(19) Europäisches Patentamt
European Patent Office
Office européen des brevets

(11) EP 0 304 013 B1

(12) EUROPEAN PATENT SPECIFICATION

(45) Date of publication and mention of the grant of the patent:
12.06.1996 Bulletin 1996/24

(21) Application number: 88113283.1

(22) Date of filing: 16.08.1988

(51) Int Cl.6: C12N 15/00, C12P 21/02, C12N 1/20, C07K 14/00, G01N 33/68, G01N 33/577, C12N 5/00, C12Q 1/68, C07K 16/00, C12P 21/00 // (C12P21/00, C12R1:91)

(54) **A novel senile amyloid precursor protein and an antibody specific for the same**

Seniles amyloides Vorläufer-Protein und dafür spezifischer Antikörper

Protéine précurseur de l'amyloide sénile et un anticorps spécifique

(84) Designated Contracting States:
CH DE FR GB IT LI SE

(30) Priority:
15.08.1987 JP 203298/87
21.08.1987 JP 207995/87
18.11.1987 JP 291404/87
11.12.1987 JP 313228/87
05.02.1988 JP 25260/88
10.02.1988 JP 29366/88
19.02.1988 JP 37905/88
25.05.1988 JP 125660/88

(43) Date of publication of application:
22.02.1989 Bulletin 1989/08

(73) Proprietor: Asahi Kasei Kogyo Kabushiki Kaisha
Osaka-shi Osaka 530 (JP)

(72) Inventors:
- Kitaguchi, Nobuya
  Fuji-shi Shizuoka-ken (JP)
- Takahashi, Yasuyuki
  Fuji-shi Shizuoka-ken (JP)
- Tokushima, Yasuo
  Fuji-shi Shizuoka-ken (JP)
- Itoh, Hirataka
  Fuji-shi Shizuoka-ken (JP)

(74) Representative: Boeters, Hans Dietrich, Dr. et al
Patentanwälte Boeters & Bauer,
Bereiteranger 15
81541 München (DE)

(56) References cited:
WO-A-88/03951

- NATURE, vol. 325, 19 February 1987, London (GB); J. KANG et al., pp. 733-736
- SCIENCE, vol. 235, 20 February 1987, Washington, DC (US); R.E. TANZI et al., pp. 880-884
- SCIENCE, vol. 235, 20 February 1987, Washington, DC (US; D. GOLDGABER et al., pp. 877-880
- NATURE, vol. 331, 11 February 1988, London (GB); N. KITAGUCHI et al., pp. 530-533

EP 0 304 013 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Background Of The Invention

Field Of The Invention

The present invention relates to a novel protein and a DNA coding for the protein. More particularly, the present invention relates to a novel senile plaque amyloid precursor protein which can be utilized for diagnosis of dysbolism in the central nervous system, such as senile dementia, and a DNA coding for the protein. The present invention also relates to a complementary DNA to the above-mentioned DNA and RNA's corresponding to the above-mentioned DNA's. Further, the present invention relates to an antibody specific for the above-mentioned protein. Furthermore, the present invention relates to a human chromosomal DNA fragment coding for the above-mentioned protein. The above-mentioned protein, antibody, DNA's, RNA's and human chromosomal DNA fragment are useful for diagnosis of dysbolism in the central nervous system. The present invention further relates to a method for detecting at least a portion of a senile plaque amyloid precursor protein, a DNA coding for the protein or an RNA corresponding to the DNA.

In the present specification, amino acids and peptides are represented using abbreviations, as indicated below, approved by the IUPAC-IUB Commission on Biochemical Nomenclature (CBN). With respect to amino acids and the like having isomers, those represented by the following abbreviations are of the L-configuration unless otherwise specified. Further, unless otherwise specified, the left ends and right ends of the amino acid sequences of peptides are the N-terminus and C-terminus, respectively.

Gln: glutamine residue
Asp: aspartic acid residue
Pro: proline residue
Tyr: tyrosine residue
Val: valine residue
Lys: lysine residue
Glu: glutamic acid residue
Ala: alanine residue
Asn: asparagine residue
Leu: leucine residue
Phe: phenylalanine residue
Gly: glycine residue
His: histidine residue
Ser: serine residue
Thr: threonine residue
Ile: isoleucine residue
Trp: tryptophan residue
Arg: arginine residue
Met: methionine residue
Cys: cysteine residue

Polydeoxyribonucleotides and oligodeoxyribonucleotides are represented by sequences of deoxyribonucleotide residues which are abbreviated as follows:

A: 2′-deoxyadenylic acid residue
C: 2′-deoxycytidylic acid residue
G: 2′-deoxyguanylic acid residue
T: thymidylic acid residue

Unless otherwise specified, the left and right ends of the sequences of deoxyribonucleotides are the 5′-end and 3′-end, respectively.

Discussion of Related Art

In recent years, the increase of patients suffering from diseases in the central nervous system, including senile dementia, has become a social problem due to the increase in population of the advanced ages. There are many of the above type diseases, the cause of which has not been elucidated and effective diagnostic and therapeutic methods have not yet been developed. Senile dementia of Alzheimer's type (hereinafter referred to as "SDAT") is one of such diseases.

Recently, an autopsy of the brains of dead SDAT patients was conducted and it was found that a substance called senile plaque was deposited in the brain of the SDAT patient in a relatively large amount as compared to the brain of a normal person. Since then, the relationship between the senile plaque and SDAT has been given attention. Nowadays, it is known that when a specimen of the brain of an SDAT patient is stained with Congo red and observed through an optical microscope, senile plaque assumes a red color, and when the stained specimen is observed through a polarization microscope it is caused to assume an apple green color due to double refraction. Further, by the observation of the specimen of the brain of an SDAT patient through an electron microscope, it was found that senile plaque has a fibrous structure. From the above findings, it is suggested that senile plaque is comprised of one of the proteins which are called amyloid.

Glenner et al and Masters et al reported that the protein which constitutes the senile plaque amyloid of an SDAT patient is the same as the constituent protein of cerebrovascular amyloid of the patient and the protein is deposited in the brain of a Down's syndrome patient (G. G. Glenner et al, Biochem. Biophys. Res. Commun., 122, 1131-1135 (1984) and C. L. Masters et al, Proc. Natl. Acad. Sci., USA, 82, 4245-4249 (1985)).

Kang et al succeeded in the cloning of a gene coding for a senile plaque amyloid precursor protein (hereinafter referred to as "SPAP") from a brain cDNA library and they reported the base sequence of the gene together with the amino acid sequence of the precursor protein presumed from the base sequence of the gene (J. Kang et al, Nature, 325, 733-736 (1987)). The SPAP having the amino acid sequence reported by Kang et al is hereinafter often referred to as "AP".

On the other hand, Tanzi et al reported that the gene coding for SPAP is expressed in various fetal organs, including brain and spleen, and that mRNA coding for SPAP is expressed in a normal human brain in an amount as much as that of the mRNA expressed in the brain of an SDAT patient (R. E. Tanzi el al, Science, 235, 880-884 (1987)). Therefore, the relationship between the gene coding for SPAP and SDAT is unclear.

It was reported that an antibody against a senile plaque precursor protein was prepared using as an antigen a synthetic peptide comprising an amino acid sequence which corresponds to a portion of the amino acid sequence of an amyloid deposited in a brain (C. W. Wong et al, Proc. Natl. Acad. Sci., USA, 82, 8729-8732 (1985); C. L. Masters et al, EMBO. J., 4 (11), 2757-2763 (1985)). By immuno-histochemical examination, it has been found that the antibody reacts with a senile plaque amyloid and a cerebrovascular amyloid. However, it has not yet been confirmed whether the amyloid protein in blood and cerebrospinal fluid (hereinafter referred to as "CSF") can be detected. Further, according to the report of Patridge et al, the substance which was detected in blood and CSF using an antiserum against the above-mentioned synthetic peptide was found to be immunoglobulin G (W. M. Partridge et al, Biochem. Biophys. Res. Commun., 145 (1), 241-248 (1987)). This report suggests that the utility of the antibody against the above-mentioned synthetic peptide is unclear.

Delavar et al reported that the duplication of the SPAP gene is observed in the genes of an SDAT patient and a Down's sydrome patient (J. M. Delabar et al, Science, 235, 1390-1392 (1987)). In this report, Delabar et al used the following method. That is, the DNA's of SDAT patients and Down's syndrome patients are subjected to Southern blotting analysis. In practicing the hybridization, two probes, namely a labeled pro-$\alpha_2$-collagen gene probe and a labeled SPAP gene probe are used. By comparison of the radioactive intensity of a band which is detected by the SPAP gene probe with that of a band which is detected by the pro-$\alpha_2$-collagen gene probe, the copy number of the SPAP gene in the chromosome is obtained. However, this method is disadvantageous because it is necessary to coincide the reactivity of the SPAP gene probe to the SPAP gene with the reactivity of the pro-$\alpha_2$-collagen gene probe to the pro-$\alpha_2$-collagen gene, and because there are many factors which adversely affect the radioactive intensity.

On the other hand, it is reported that the duplication of the SPAP gene is not observed in many patients suffering from familial and sporadic Alzheimer's diseases (St. George-Hyslop et al, Science, 238, 664-666 (1987); R. E. Tanzi et al, Science, 238, 666-669 (1987); and M. B. Podlsny et al, Science, 238, 669-671 (1987)).

Further, it is reported that the results of the analysis of polymorphism of restriction fragment length showed that the SPAP gene is not identical with the gene locus directly relating to the familial Alzheimer's disease (FAD) (C. Van Broeckhoven et al, Nature, 329, 153-155 (1987) and R. E. Tanzi et al, Nature, 329, 156-157 (1987)). However, it is possible that the product produced by expression of the gene locus relating to FAD directly or indirectly affects the production and metabolism of the SPAP, causing the amyloid to be deposited.

WO-A-88/03 951 is prior art according to Article 54 (3) and (4) EPC as far as three of its altogether four claimed priorities are concerned. Thus, WO-A-88/03 951 describes an amino acid sequence starting from amino acid 671 (methionine) of a senile plaque amyloid precursor protein (which will be defined later in the description of the invention) and a corresponding DNA sequence starting with nucleotide 2011 of a DNA coding for said protein.

Nevertheless and as mentioned above, the mechanism of SDAT has not yet been elucidated. Under such circumstances, it is earnestly desired in the art to elucidate the mechanism of SDAT and develop a method for detecting a biochemical change in the brain accompanying SDAT or a change in DNA which causes SDAT, in order to diagnose SDAT.

Summary Of The Invention

With a view toward developing a method for diagnosis of SDAT, the present inventors have made extensive and intensive studies. As a result, the following novel findings have been made.

Illustratively stated, in view of the fact that the metabolism of various substances in the brain of an SDAT patient is inhibited, that is, the activities of choline acetyl transferase, acetylcholine esterase, etc. and the amount of somatostatin are decreased in brains of SDAT patients as compared to those of an age-matched human controls, the present inventors have made studies on the metabolism of the SPAP in the brain of an SDAT patient. In reference to the metabolism of the SPAP, the inventors have made studies on the expression of the SPAP gene. As a result, the present inventors have found an mRNA coding for a novel senile plaque amyloid precursor protein having a protease inhibiting activity.

Particularly, the present inventors synthesized an oligodeoxyribonucleotide probe having a base sequence which corresponds to a portion of an mRNA coding for the known SPAP, namely AP, and conducted the screening of an SPAP-producing cell line from various central nervous system cell lines using the synthesized probe. As a result, it was found that a glioblastoma ATCC HTB-14 produced the SPAP. Then, poly(A)RNA's were obtained from the cell line, and a cDNA library was prepared from the poly(A)RNA's and subjected to colony hybridization using the above-mentioned synthetic probe. As a result, a cDNA clone which codes for a novel protein having such a structure that a novel peptide consisting of 76 amino acids is inserted in an intermediate portion of the known SPAP has unexpectedly been obtained together with a cDNA clone which codes for the known SPAP. The novel amyloid precursor protein encoded by the cDNA is hereinafter often referred to as "NAP".

The base sequence of the cDNA coding for NAP is shown in Fig. 1(A). In Fig. 1(A), the cDNA coding for the known SPAP, namely AP, comprises a base sequence consisting of a sequence of the 1st to 865th bases and, ligated thereto, a sequence of from the 1091st to the 2310th bases. That is, the cDNA coding for the known SPAP has the same base sequence as that obtained by deleting the 866th to 1090th bases from the cDNA coding for the novel SPAP, namely NAP. In the case of the cDNA coding for the known SPAP, namely AP, the 865th base "G" and the 1091st and 1092nd bases "TT" shown in Fig. 1(A) constitute a codon for a valine. The novel peptide found by the present inventors has an amino acid sequence corresponding to the amino acids of from the 289th amino acid Glu to the 364th amino acid Leu shown in Fig. 1(A) (the novel peptide is hereinafter often referred to as "INS76"). As shown in Fig. 1(A), the base sequence coding for the INS76 consists of the 865th to 1092nd bases. However, the novel base sequence found by the present inventors consists of the 866th to 1090th bases shown in Fig. 1(A). Even when the novel base sequence found by the present inventors is inserted in the base sequence coding for the known SPAP, the resultant base sequence maintains its reading frame and codes for a protein having an amino acid sequence which is 75 amino acids larger than that of the known SPAP, namely AP.

Further, the present inventors found that the INS76 has a homology to bovine pancreatic trypsin inhibitor (hereinafter often referred to as "BPTI") and human inter-$\alpha$-trypsin inhibitor (hereinafter often referred to as "h-I$\alpha$TI") in amino acid sequence as shown in Fig. 8 (homologous portions are indicated by boxes). For example, the amino acid sequence of INS76 is homologous to that of BPTI in that they have commonly 26 amino acids including 6 cysteins. Then, the present inventors have examined trypsin inhibiting activity of the INS76 and have found that the INS76 has an activity of inhibiting trypsin, one of the proteases.

Furthermore, the present inventors have succeeded in obtaining a human chromosomal DNA fragment coding for the above-mentioned novel protein comprising an amino acid sequence of the INS76 and having a protease inhibiting activity. Illustratively stated, the screening of a chromosomal gene library prepared from a human lymphocyte DNA is conducted using as a probe a DNA fragment obtained by cleavage of the cDNA coding for the NAP mentioned above or a synthetic oligonucleotide to obtain various clones containing a domain relating to expression of a base sequence coding for INS76 in the chromosomal gene. Then, the present inventors analysed the thus obtained clones with respect to its restriction sites to prepare a restriction map, and determined the position at which the probe hybridizes the clones by Southern hybridization method and also determined the base sequences of the clones. As a result, it has been found that the same base sequence as that of from the 663rd to 1224th bases shown in Fig. 1(A), which base sequence is a portion of the base sequence coding for the NAP, is constituted of four exons in the human chromosomal SNAP gene. The four exons correspond to the base sequences of the 663rd to 865th bases, of the 866th to 1033rd bases, of the 1034th to 1090th bases and of the 1091st to 1224th bases of Fig. 1(A), respectively. These four exons are designated exon H, exon I, exon J and exon K, respectively.

It has further been found that the protein NAP containing INS76 is encoded by the mRNA comprising a base sequence corresponding to exons H, I, J and K (hereinafter referred to as "H-I-J-K type mRNA"). On the other hand, the known SPAP, namely AP, is encoded by the mRNA comprising a base sequence corresponding to exons H and K (hereinafter referred to as "H-K type mRNA"). Then, it has been found that different mRNA's are transcribed from one gene (this phenomenon is called alternative splicing). In view of the occurrence of alternative splicing, the present inventors have made a further study with respect to possibilities of extistences of an H-I-K type mRNA and an H-J-K type mRNA and found that the H-I-K type mRNA is really present in the cell of a cell line derived from a human brain and in a human brain. Then, the present inventors have succeeded in obtaining a cDNA corresponding to the H-I-K type mRNA. The cDNA of the the H-I-K type mRNA has the base sequence as shown in Fig. 1(B). The cDNA coding for the H-I-K type SPAP has such a structure that a base sequence corresponding to the 168 bases of from the 866th to the 1033rd bases shown in Fig. 1(B) is inserted in the base sequence of the cDNA corresponding to the H-K type mRNA. With respect to the amino acid sequence, it has been found that the protein encoded by the H-I-K type mRNA has such a structure that the 289th amino acid Val of the AP is replaced by an amino acid sequence corresponding to the amino acid sequence of 56 amino acids of from the 289th amino acid Glu to the 345th amino acid Ile shown in Fig. 1(B). The polypeptide having an amino acid sequence of the 289th to 345th amino acids shown in Fig. 1(B) is hereinafter often referred to as "INS57". Further, the protein having the full amino acid sequence represented in Fig. 1(B) is hereinafter often referred to as "NAP3". The present inventors have also found that the INS57 has a homology to BPTI and h-I$\alpha$TI in amino acid sequence as shown in Fig. 9 (homologous portions are indicated by boxes). For example, the amino acid sequence of the INS57 is homologous to that of BPTI in that they have commonly 27 amino acid including 6 cysteins. Then, the present inventors have examined a trypsin inhibiting activity of the INS57 and have found that the INS57 has a trypsin inhibiting activity.

As mentioned above, the present inventors have successfully found two types of novel SPAP's, namely, NAP and NAP3, other than the known SPAP, namely AP.

The present inventors have also found that the amounts of the H-I-J-K type and/or H-I-K type mRNA's expressed in human brain from the SPAP gene increase according to the advance in age. Further, it has also been found that the amounts of the H-I-J-K type and/or H-I-K type mRNA's in the brain of an SDAT patient are likely to be higher than those in the normal human brain. In view of the above-mentioned facts, the present inventors have made extensive and intensive studies, and found that the diagnosis of the SDAT can be conducted using a DNA or mRNA having at least a portion of a base sequence coding for the above-mentioned protein NAP or NAP3.

Based on the above-mentioned novel findings, the present invention has been completed.

It is, therefore, an object of the present invention to provide a protein which is a raw material for producing an antibody which is useful for the diagnosis of abnormal brain metabolism.

It is another object of the present invention to provide a DNA coding for the above-mentioned protein, which is useful for producing the protein by recombinant DNA technique.

It is still another object of present invention to provide a antibody specific for the above-mentioned protein, which is useful for the diagnosis of abnormal brain metabolism.

It is a further object of the present invention to provide a DNA probe or an RNA probe useful for determining the amount of an mRNA coding for a substance causing the abnormal metabolism in a brain cell or a cell reflecting brain metabolism for the diagnosis of the above-mentioned brain abnormality.

It is still a further object of the present invention to provide a DNA probe or an RNA probe useful for detecting a specific base sequence in a chromosomal DNA, which relates to the production of a substance causing the abnormal brain matabolism.

It is a further object of the present invention to provide a chromosomal DNA fragment coding for SPAP, which can be used for producing a pathological model animal for SDAT such as a transgenic animal for use in research on the abnormal production and metabolism of SPAP.

The foregoing and other objects, features and advantages of the present invention will be apparent to those skilled in the art from the following detailed description and appended claims taken in connection with the accompanying drawings.

## Brief Description Of The Drawings

In the drawings:

Figs. 1(A)-a through c in total show the base sequence of a cDNA coding for NAP, which has a BamHI-cleaved end at its 5′-end and a HindIII-cleaved end at its 3′-end, and also show an amino acid sequence of NAP;

Figs. 1(B)-a through c in total show the base sequence of a cDNA coding for NAP3, which has a BamHI-cleaved end at its 5′-end and a HindIII-cleaved end at its 3′-end, and also show an amino acid sequence of NAP3;

Fig. 2 shows the restriction maps of (A) a human chromosomal DNA fragment of about 37 Kb comprising exons I, J and K of the SPAP gene; (B) human chromosomal DNA fragments respectively contained in phage clones $\lambda$24 ($\lambda$32), $\lambda$31 and $\lambda$35 and a subclone (pAP246B) of the phage clone $\lambda$24; and (C) a DNA insert of a subclone pGAP-K3 of the phage clone $\lambda$35;

Fig. 3 shows restriction maps of (A) a human chromosomal DNA fragment of about 6.7 Kb comprising exon H, which is inserted in 57 clone; and (B) a human chromosomal DNA fragment of about 1 Kb comprising exon H, which is a portion of the DNA fragment (A);

Fig. 4 shows the base sequence of exon H and portions of introns adjacent to the exon H;

Fig. 5 shows the base sequence of exon I and portions of introns adjacent to the exon I;

Fig. 6 shows the base sequence of exon J and portions of introns adjacent to the exon J;

Fig. 7 shows the base sequence of exon K and portions of introns adjacent to the exon K;

Fig. 8 shows the amino acid sequence of protein INS76 of the present invention which is a portion of protein NAP, and the amino acid sequences respectively of a portion of bovine pancreatic trypsin inhibitor (BPTI) and a portion of human inter-α-trypsin inhibitor (h-IαTI);

Fig. 9 shows the amino acid sequence of the protein INS57 of the present invention which is a portion of protein NAP3, and the amino acid sequences respectively of a portion of bovine pancreatic trypsin inhibitor (BPTI) and a portion of human inter-α-trypsin inhibitor (h-IαTI);

Fig. 10(A) shows a flow chart illustrating the construction of a plasmid pGBP1 containing a cDNA coding for a full amino acid sequence of protein AP (known SPAP);

Fig. 10(B) shows a flow chart illustrating the construction of a plasmid pGBP2 containing a cDNA coding for a full amino acid sequence of the protein NAP of the present invention;

Figs. 11(A), (B) and (C) show flow charts illustrating the constructions of plasmid pSVMTAP for the expression of AP, plasmid pSVMTNAP for the expression of a DNA of the NAP of the present invention, and plasmid pSVMTNAP3 for the expression of a DNA of the NAP3 of the present invention, respectively;

Fig. 12(A) shows a flow chart illustrating the construction of plasmid pSPGT2TX in which the DNA coding for a protease inhibitory (PI) region of the NAP of the present invention, which region has an amino acid sequence corresponding to the amino acid sequence of the 289th to 361st amino acids in Fig. 1(A), is subcloned;

Fig. 12(B) shows a flow chart illustrating the construction of plasmid pPItrp314 comprising a trp promoter and, downstream thereof, a cDNA coding for the PI region of the NAP of the present invention;

For. 12(C) shows a flow chart illustrating the construction of plasmid pPIXa3142 comprising a trp promoter and, downstream thereof, a cDNA coding for the PI region and factor Xa-recognizing amino acid sequence;

Fig. 12(D) shows a flow chart of constructing a plasmid pIXTtrp726 for the expression of a DNA coding for a fused protein comprising the PI region, factor Xa-recognizing sequence and TNF;

Figs. 12(E), (F) and (G) show flow charts of constructing plasmids pPItrp75-1, pPItrp75-2 and pPItrp75-16 which contain respectively 1 unit, 2 units and 16 units of a DNA region comprising a ribosome-binding site and a base sequence coding for the PI region;

Fig. 12(H) shows a flow chart of constructing plasmids pAPKB1865 and pAPKB1877 which are precursor plasmids for the expression of a DNA coding for a secretable type AP and a DNA coding for a secretable type NAP of the present invention, respectively;

Fig. 12(I) shows a flow chart of constructing plasmids pSVMT592 and pSVMT667 which are plasmids for the expression of a DNA coding for a secretable AP and a DNA coding for a secretrable NAP of the present invention, respectively;

Fig. 12(J) shows a flow chart of constructing a plasmid pSVMT648 for the expression of a DNA coding for a secretable NAP3 of the present invention;

Fig. 12(K) shows a flow chart of constructing a plasmid pSVMT-PIXT for the expression of a DNA coding for a fused protein comprising a secretable PI region of the present invention, a factor Xa-recognizing sequence and TNF;

Fig. 12(L) shows a flow chart of constructing a plasmid pSVMT-APPI for the expression of a DNA coding for a secretable PI region of the present invention;

Fig. 12(M) shows an amino acid sequence of the protein produced by the expression from the plasmid pSVMT-APPI, and a base sequence coding for the protein;

Fig. 12(N) shows a flow chart of constructing a plasmid pSVMDPI for the expression of a DNA coding for a fused protein comprising a signal peptide and a secretable PI region of the present invention;

Fig. 13 shows a flow chart of constructing a plasmid pSGP3TX for obtaining a DNA probe and RNA probe of the present invention;

Fig. 14 shows the results of autoradiography obtained by Southern hybridization of normal human DNA's digested with BamHI, EcoRI and HindIII, respectively, with a probe (a) of the present invention;

Fig. 15 shows the results of autoradiography obtained by Southern hybridization of normal human DNA's digested with BamHI, EcoRI and HindIII, respectively, with a synthetic oligonucleotide IE-K5; and

Fig. 16 is a graph showing the relationships between the activities of various serine proteases and the concentrations of the protein of the present invention.

Disclosure Of The Invention

According to the present invention, there is provided a protein comprising an amino acid sequence represented by the following formula (I):

Val-Cys-Ser-Glu-Gln-Ala-Glu-Thr-Gly-Pro-Cys-
Arg-Ala-Met-Ile-Ser-Arg-Trp-Tyr-Phe-Asp-Val-
Thr-Glu-Gly-Lys-Cys-Ala-Pro-Phe-Phe-Tyr-Gly-
Gly-Cys-Gly-Gly-Asn-Arg-Asn-Asn-Phe-Asp-Thr-
Glu-Glu-Tyr-Cys-Met-Ala-Val-Cys-Gly-Ser-Ala

... (I).

The protein of the present invention comprises an amino acid sequence of formula (I), which amino acid sequence corresponds to the amino acid sequence of the 290th to 344th amino acids shown in Fig. 1(B). The protein of the present invention may consist essentially of the amino acid sequence represented by formula (I). Alternatively, the protein may be in the form of a protein comprising the amino acid sequence of formula (I) and, attached thereto at its N-terminus and/or C-terminus, at least one amino acid or amino acid sequence of other peptides. Examples of proteins comprising the amino acid sequence of formula (I) and at least one amino acid or amino acid sequence of other peptides include the following proteins (1) and (2).

(1) A protein which comprises an amino acid sequence represented by the following formula (II):

Glu-(I)-Ile (II)

wherein (I) is a peptide having the same amino acid sequence as defined above.

(2) A protein which comprises an amino acid sequence represented by the following formula (IV):

Glu-(I)-Met-Ser-Gln-Ser-Leu-Leu-Lys-Thr-Thr-
Gln-Glu-Pro-Leu-Ala-Arg-Asp-Pro-Val-Lys-Leu

... (IV)

wherein (I) is a peptide having the same amino acid sequence as defined above.

The proteins of the present invention also include a senile plaque amyloid precursor protein comprising the amino acid sequence of formula (I). As mentioned before, there are three types of senile plaque amyloid precursor proteins, namely, AP, NAP and NAP3. The AP which does not contain in its amino acid sequence the amino acid sequence of formula (I) is already known and is not included in the proteins of the present invention. The proteins of the present invention include the NAP and NAP3, both of which comprise in their respective amino acid sequences an amino acid sequence of formula (I). The NAP3 and NAP of the present invention have amino acid sequences represented by the following formulae (III) and (V), respectively:

$X_1$-(I)-$X_2$ (III)

wherein (I) is a peptide having the same amino acid sequence as defined above, $X_1$ is a peptide having an amino acid sequence of the 1st to 289th amino acids shown in Fig. 1(B), and $X_2$ is a peptide having an amino acid sequence of the 345th to 751st amino acids shown in Fig. 1(B), and

$X_3$-(I)-$X_4$ (V)

wherein (I) is a peptide having the same amino acid sequence as defined above, $X_3$ is a peptide having an amino acid sequence of the 1st to 289th amino acids shown in Fig. 1(A), and $X_4$ is a peptide having an amino acid sequence of the 345th to 770th amino acids shown in Fig. 1(A).

The protein of the present invention is not restricted to the above-mentioned proteins respectively comprising the amino acid sequences of formulae (I) to (V). The protein of the present invention also includes a protein comprising at least the amino acid sequence of formula (I) as long as the protein has a protease inhibiting activity.

The protein of the present invention may or may not contain at least one sugar residue.

In another aspect of the present invention, there is provided a DNA comprising at least one base sequence selected from the group consisting of a base sequence coding for a protein of the present invention mentioned above and a base sequence complementary thereto.

In a further aspect of the present invention, there is provided a DNA comprising at least one base sequence selected from the group consisting of a base sequence represented by the following formula (VI):

```
1         10         20         30         40
AGGTGTGCTC TGAACAAGCC GAGACGGGGC CGTGCCGAGC

        50         60         70         80
AATGATCTCC CGCTGGTACT TTGATGTGAC TGAAGGGAAG

        90        100        110        120
TGTGCCCCAT TCTTTTACGG CGGATGTGGC GGCAACCGGA

       130        140        150        160
ACAACTTTGA CACAGAAGAG TACTGCATGG CCGTGTGTGG

     168
CAGCGCCA                           ... (VI),
```

and a base sequence complementary thereto.

The DNA of the present invention comprises a base sequence of the above-mentioned formula (VI). In the base sequence of formula (VI), the base sequence of the 3rd to 167th bases codes for the amino acid sequence represented by formula (I) as mentioned above. The DNA of the present invention may comprise the base sequence represented by formula (VI) and, attached thereto at its 5'-end and/or 3'-end, at least one base or base sequence other than that of formula (VI). Examples of DNA's comprising a base sequence of formula (VI) and at least one base or base sequence other than that of formula (VI) include the following DNA's (1) to (5).

(1) A DNA which comprises at least one base sequence selected from the group consisting of a base sequence represented by the formula (VII):

$$G\text{-}(VI)\text{-}TT \qquad (VII)$$

wherein (VI) is a DNA having the same base sequence as defined above,
and a base sequence complementary thereto.

(2) A DNA which comprises at least one base sequence selected from the group consisting of a base sequence represented by the formula (VIII):

$$Y_1\text{-}(VI)\text{-}Y_2 \qquad (VIII)$$

wherein (VI) is a DNA having the same base sequence as defined above, $Y_1$ is a DNA having a base sequence of the 1st to 865th bases shown in Fig. 1(B), and $Y_2$ is a DNA having a base sequence of the 1034th to 2253rd base shown in Fig. 1(B),
and a base sequence complementary thereto.

(3) A DNA which comprises at least one base sequence selected from the group consisting of a base sequence represented by the formula (IX):

$$(VI)\text{-}Y_3 \qquad (IX)$$

wherein (VI) is a DNA having the same base sequence as defined above, and $Y_3$ is a DNA having a base sequence represented by the following formula (X): TG

TCCCAAAGTT TACTCAAGAC TACCCAGGAA

CCTCTTGCCC GAGATCCTGT TAAAC ... (X),

and a base sequence complementary thereto.

(4) A DNA which comprises at least one base sequence selected from the group consisting of a base sequence represented by the formula (XI):

$$G\text{-(VI)-}Y_3\text{-TT} \qquad \text{(XI)}$$

wherein (VI) is a DNA having the same base sequence as defined above, and $Y_3$ has the same meaning as defined above,

and a base sequence complementary thereto.

(5) A DNA which comprises at least one base sequence selected from the group consisting of a base sequence represented by the formula (XII):

$$Y_4\text{-(VI)-}Y_5 \qquad \text{(XII)}$$

wherein (VI) is a DNA having the same base sequence as defined above, $Y_4$ is a DNA having a base sequence of the 1st to 865th bases shown in Fig. 1(A), and $Y_5$ is a DNA having a base sequence of the 1034th to 2310th bases shown in Fig. 1(A),

and a base sequence complementary thereto.

The above-mentioned DNA of the present invention may be used for producing various proteins as mentioned above by recombinant DNA technique.

The DNA of the present invention may be produced by a conventional method from a human cell line such as a glioblastoma (for example, a cell line ATCC HTB-14, ATCC HTB-16 or ATCC HTB-17), a neuroblastoma (for example, a cell line ATCC HTB-10 or HTB-11) or a neuroglioma (for example, a cell line ATCC HTB-148), or a tissue such as a brain tissue. Illustratively stated, poly(A)RNA's are extracted from the above-mentioned cell line or tissue, and using the poly(A)RNA's, a cDNA library is prepared. On the other hand, a base sequence of about 12 to 100 continuously sequenced bases is selected from the base sequence indicated in Fig. 1(A) or (B), and an oligodeoxyribonucleotide complementary to the selected base sequence is synthesized using a customary DNA synthesizer. In this connection, it is preferred to select a base sequence so that at least a portion of the base sequence coding for the amino acid sequence of formula (II) or (IV) may be included in the selected base sequence. Using the thus synthesized oligodeoxyribonucleotide as a probe, the above-obtained cDNA library is screened and the intended DNA of the present invention is cloned according to a conventional method. Thus, there is obtained a DNA of the present invention.

The DNA of the present invention may also be organo-chemically synthesized by a conventional method. For example, the DNA of the present invention may be synthesized by a solid phase method using a commercially available DNA synthesizer.

Further, the DNA of the present invention having a base sequence coding for protein NAP3 having an amino acid sequence of formula (III) as mentioned above may be produced by deletion of the connecting portion between exon I and exon K from the DNA having a base sequence as shown in Fig. 1(A) using an appropriate deleter, for example, a DNA having a base sequence of the formula:

**5'-CTGTTGTAGGAATGGCGCTGCCAC-3'.**

Using the above-mentioned DNA, the protein of the present invention may be produced by a recombinant DNA technique using a conventional host-vector system. Hereinbelow, the method for producing the protein of the present invention will be explained.

First, an explanation is given with respect to the case where a higher eukaryote such as a mammalian cell or an insect cell, for example a silkworm cell is used as a host cell. The DNA of the present invention is ligated downstream of a promoter and, if desired, to an initiation codon ATG. Further, if desired, a termination signal such as a polyadenylation signal may be ligated to the C-terminus of the DNA of the present invention. The resultant DNA is inserted in an appropriate replicable expression vector to obtain a replicable recombinant DNA. The replicable recombinant DNA is transferred into a mammalian cell to obtain a transformant. The transformant is cultured so that the protein of the present invention which is encoded by the above-mentioned DNA is produced by expression of the DNA in the cell or in the culture medium. For example, when the DNA having a base sequence of formula (VIII) is used, the protein NAP3 of the present invention can be produced. On the other hand, when the DNA having a base sequence of formula (XII) is used, the protein NAP of the present invention can be produced.

As mentioned before, the protein having a amino acid sequence of formula (II) is designated INS57 and encoded by exon I of the human chromosonal DNA coding for NAP3. Further, the protein having an amino acid sequence of formula (IV) is designated INS76 and encoded by exons I and J of the human chromosomal DNA coding for NAP. The proteins INS57 and INS76 of the present invention may be produced by recombinant DNA technique using a human chromosomal DNA fragment comprising exon I or exons I and J.

In the base sequence coding for the NAP3 or NAP, there is a base sequence coding for a transmembrane domain which has an affinity to the cell membrane of a host cell. Particularly, in the case of the base sequence shown in Fig. 1(A), the base sequence of the 2098th to 2169th bases codes for the transmembrane domain, and in the case of the base sequence shown in Fig. 1(B), the base sequence of the 2041st to 2112nd bases codes for the transmembrane domain. When the DNA portion coding for the transmembrane domain is deleted from the DNA of the present invention and the DNA not containing the base sequence of the transmembrane domain is used for the production of the protein of the present invention by recombinant DNA technique, the protein produced in the host cell is secreted from the cell into the culture medium. Such a type of protein of the present invention is hereinafter often referred to as "a secretory protein".

Further, when a DNA coding for a signal peptide is ligated upstream of the DNA of the present invention and the termination signal so that the resultant DNA may constitute a reading frame so as to enable a fused peptide comprising said signal peptide and the protein of the present invention to be produced by DNA expression, and the resultant DNA is expressed in a host cell, the protein produced in a host cell can be secreted from the cell into the culture medium. The secretion of the protein into the culture medium is advantageous in that it is not necessary to disrupt the host cell for obtaining the protein and, therefore, isolation of the protein can easily be conducted. Further, if a serum-free medium or a medium containing an extremely small amount of a serum is used, isolation and purification of the protein of the present invention can be conducted more easily because the amount of protein impurities present in the culture medium can be reduced.

Examples of promoters which may be used for the production of the protein of the present invention include an SV40 early promoter, an SV40 late promoter, a viral promoter, such as mouse mammary tumor virus (MMTV), a promoter of a gene coding for a constituent protein of a cell, such as actin and tublin, and an inducible promoter, such as a metallothionein promoter and a heat shock protein promoter.

Examples of vectors which may be used for the production of the protein of the present invention include viral vectors, such as SV40 virus and bovine papilloma virus. However, if desired, the DNA coding for the protein of the present invention may also be transferred into a host cell without inserting it in a vector.

In practicing the transformation of a host cell with the DNA coding for the protein of the present invention or a recombinant DNA containing the DNA, an appropriate marker may generally be ligated to the DNA or recombinant DNA, and transferred into a host cell. The marker may also be transferred into a host cell simultaneoulsy with the transfection of the DNA or the recombinant DNA into a host cell without ligating the marker to the DNA or the recombinant DNA (co-transfection). By the use of a marker, a transformant can easily and effectively be selected from host cells remaining untransformed by the criterion of the phenotypical trait imparted by the marker. Examples of markers include a gene for neomycin resistance and a gene coding for dihydrofolate reductase (hereinafter referred to as "DHFR"). For example, when the gene for neomycin resistance is used as a marker, a transformant can be selected from host cells remaining untransformed by the criterion of the resistance to an antibiotic G418.

When the DHFR gene is used as a marker and ligated to the vicinity of the DNA coding for the protein of the present invention, it is advantageous that replication and expression of the DNA coding for the protein of the present invention in a host cell can be increased in the presence of methotrexate which is an inhibitor of DHFR, leading to a high yield production of the protein of the present invention.

Examples of host cells include a COS-1 cell derived from a simian kidney, a CHO cell derived from a Chinese hamster, a mouse C127 cell, an NIH3T3 cell, a rat 3T3 cell and the like. The COS-1 cell may generally be used for the transient expression of the DNA. On the other hand, the CHO cell may generally be used for the constitutive expression of DNA.

Next, an explanation is given with respect to the case where a microorganism is used as a host cell. Examples of microorganisms which may be used for the production of the present protein include Escherichia coli, Bacillus subtilis and, Saccharomyces cerevisiae.

The DNA coding for the protein of the present invention is ligated downstream of a promoter, and an initiation codon ATG, if any, as in the case where a higher eukaryote is used as a host cell. If desired, a termination signal may be ligated to the C-terminus of the DNA coding for the present protein. Further, if desired, a DNA coding a signal peptide may be ligated between the promoter and the DNA coding for the above-mentioned protein, constituting a reading frame so that a fused protein of the signal peptide and the above-mentioned protein is produced by expression of the DNA's. The fused peptide can be secreted out from the host cell into the culture medium, or transferred into the periplasm of the host cell.

As the promoter, various promoters may be used as long as the promoter can work in a host cell employed. For example, when E. coli is used as a host cell, it is preferred to use a trp promoter, a tac promoter, a lac promoter and a PL promoter from the standpoint of efficiency of expression.

Generally, a low molecular peptide is unstable in a host cell because the peptide is easily degraded by a protease present in a host cell. For preventing the degradation of the peptide, it is preferred to use an ATP-dependent protease-deficient strain ($lon^-$) such as E. coli Y1089 or to culture a transformant at a relatively low temperature, for example, at 25°C.

The protein of the present invention may be produced in the form of a fused protein with another protein, in which the present protein is bonded to another protein directly or indirectly through an intervening peptide. In order to produce a fused protein, a DNA coding for the other protein and, if desired, a DNA coding for an intervening peptide are ligated to the DNA coding for the above-mentioned protein. Examples of intervening peptides include an activated blood coagulation factor X(factor Xa)-recognizing sequence.

Examples of proteins which may be fused with the protein of the present invention include a tumor necrosis factor (TNF), β-galactosidase, γ-interferon (γ-IFN) and the like.

A protein comprised of the protein of the invention fused with another protein is advantageous in that the present protein can easily and efficiently be purified utilizing the characteristic properties of the other protein bonded to the present protein. For example, when the fused protein is the present protein fused with a protein having a lymphokine activity, such as TNF, and is purified by high performance liquid chromatography etc., the fraction containing the fused protein can be identified by the criterion of the lymphokine activity. Of course, it is possible that the fraction containing the present protein can be identified by the criterion of the protease inhibiting activity of the protein of the present invention. However, in most cases, it is not easy to distinguish the protease inhibiting activity of the present protein from those of the substances inherent in a host cell. Therefore, it is advantageous to produce a fused protein comprised of the present protein and another protein having a characteristic property which is not possessed by substances derived from the host cell. Moreover, it is also possible to effectively purify a fused protein by affinity chromatography using a column packed with a supporting material having, bonded thereto, an antibody specific for the protein fused with the present protein.

Where a fused protein has such a structure that the present protein is bonded to another protein through an intervening peptide having a factor Xa-recognizing amino acid sequence, since the intervening peptide can be cleaved by treatment with factor Xa, the present protein can advantageously be separated from the fused protein by treatment with factor Xa.

The protein of the present invention may also be organo-chemically synthesized by a conventional method, for example a method described in Seikagaku Jikken Koza (Lectures on Experiments of Biochemistry), Vol. 1, Tanpakushitsu-no-kagaku (Protein Chemistry) IV, edited by Japanese Society of Biochemistry, published by Tokyo Kagaku Dojin, Japan. Further, the present protein may also be organo-chemically synthesized by a solid phase method using a commercially available peptide synthesizer.

Further, the proteins of the present invention having amino acid sequences (III) and (V), namely protein NAP3 and NAP, may also be produced by culturing a glioblastoma cell line ATCC HTB-14, ATCC HTB-16 or ATCC HTB-17, a neuroblastoma cell line ATCC HTB-10 or ATCC HTB-11, or a neuroglioma cell line ATCC HTB-148.

The thus produced protein of the present invention may be purified by a conventional technique, for example, high performance liquid chromatography, ion exchange chromatography, gel filtration, affinity chromatography, polyacrylamide gel electrophoresis, isoelectric focusing, salting-out, etc. Those techniques may be used alone or in combination.

As mentioned above, the protein of the present invention has a protease inhibiting activity. This protease inhibiting activity is due to the affinity of the present protein to a protease. Therefore, the present protein may advantageously be purified by affinity chromatography using a column packed with a protease-bonded supporting material, such as an agarose or polyacrylamide to which trypsin or chymotrypsin is bonded. This affinity chromatography is useful for purifying the protein of the present invention having a relatively short amino acid sequence. However, where the present protein has a relatively long amino acid sequence, there would be a danger that the protein of the present invention is degraded by the protease bonded to a supporting material packed in the column.

Thus, the protein of the present invention can be obtained in substantially pure form.

The protein of the present invention may be used as an antigen for producing an antibody specific for the present protein. The antibody specific for the present protein is useful for detecting a human brain senile plaque amyloid precursor protein (SPAP). The detection of SPAP is useful for the diagnosis of senile dimentia of Alzheimer's type (SDAT).

Accordingly, in a further aspect of the present invention, an antibody specific for a human brain senile plaque amyloid precursor protein selected from the group consisting of amino acid sequences of formula (III) and of formula (V) as mentioned above is provided.

The antibody of the present invention may be produced by a customary immunization method using the above-mentioned protein as an antigen.

Generally, an antibody specific for a certain protein can be produced using as an antigen a peptide having an amino acid sequence of at least 5 continuously sequenced amino acids in the amino acid sequence of the protein. Therefore, a portion of the protein of the present invention may also be used as an antigen for producing the antibody. Particularly, a peptide comprising a sequence of at least five continuously sequenced amino acids in an amino acid sequence represented by the formula:

**Glu-Val-Val-Arg-(II)-Pro-Thr-Thr-Ala**

wherein (II) is a peptide having the amino acid sequence of formula (II), may be used as an antigen. Further, a peptide comprising a sequence of at least five continuously sequenced amino acids in an amino acid sequence represented by the formula:

**Glu-Val-Val-Arg-(IV)-Pro-Thr-Thr-Ala**

wherein (IV) is a peptide having the amino acid sequence of formula (IV), may also be used as an antigen. The above-mentioned peptides are also included in the present invention. Such peptides may be produced by recombinant DNA technique as in the case of the production of the protein of the present invention as mentioned above. The peptides may also be organo-chemically produced by a customary liquid phase or solid phase synthetic method. In the case of the solid phase method, the peptides may be produced using a commercially available peptide synthesizer.

The peptide or protein of the present invention as such may be used as an antigen. However, when the peptide of the present invention is comprised of up to about 20 amino acids, the titer of the antibody produced using the peptide as an antigen is not so high. Therefore, where a peptide comprised of about up to 20 amino acids is used as an antigen, for increasing the titer of an antibody to be produced, it is preferred that the peptide be bonded to a high molecular substance and the resultant peptide compound be used as an antigen. The peptide to be bonded to a high molecular weight substance is generally comprised of 5 amino acids or more, preferably 10 amino acids or more from the standpoint of antigenicity.

Examples of high molecular weight substances include albumins of various animals, a keyhole limpet hemocyanin (hereinafter often referred to as "KLH") and the like. The bonding of the peptide to the high molecular weight substance may be conducted by a customary method such as an active ester method using a carbodiimide compound, and an MBS method. The MBS method is explained below. A cystein is bonded to the N-terminus or C-terminus of the peptide of the present invention by an amide bond. Then, the thiol group of the cystein is reacted with m-maleiimidobenzoic acid N-hydroxy succinimide ester (hereinafter referred to as "MBS"). Then, the resultant is bonded to the amino group of a high molecular substance. Thus, there is obtained a peptide compound which may be used for producing the antibody of the present invention.

The antibody of the present invention includes an antiserum, a polyclonal antibody and a monoclonal antibody.

Hereinbelow, an explanation is given with respect to the method for producing the antibody using the peptide or protein of the present invention or the above-mentioned peptide compound.

An antiserum may be produced by immunizing an animal with the peptide, protein or peptide compound according to a customary method. Examples of animals which may be used for the immunization include rabbit, mouse, goat, guinea pig, donkey, rat, sheep, horse, hamster, cattle, chicken, dog, cat and the like. The dose of the peptide, protein and peptide compound varies according to the type of an animal and the type of the antigen employed. For example, when a rabbit and a peptide compound are used as the animal to be immunized and as the antigen, respectively, about 100 to 5000 μg/dose of the peptide compound may generally be injected into a rabbit together with a Freund's complete adjuvant. The injection of the peptide compound into the rabbit is repeated about 4 to 8 times at intervals of 1 to 3 weeks. When the peptide moiety of the peptide compound is comprised of 20 amino acids or more, for increasing the titer of an antibody to be produced, it is preferred that the second to the final injections be conducted using as an antigen the peptide not bonded to a high molecular substance.

After completion of the immunization, the blood of the animal is collected and subjected to centrifugation to separate the blood into a supernatant and a precipitate. The supernatant is collected. The thus obtained supernatant is an antiserum of the present invention.

A polyclonal antibody of the present invention can be obtained by subjecting the antiserum as obtained above to purification by a customary method. For example, the polyclonal antibody of the present invention may be obtained as follows. To the antiserum is added a salt which is customarily used for salting-out, such as ammonium sulfate, to thereby precipitate a protein in the antiserum. The precipitate is collected by centrifugation and dissolved in a neutral buffer such as a phosphate buffer. The resultant solution is dialyzed to remove the salt used for salting-out from the solution. The solution is then subjected to purification by a customary technique, such as ion exchange chromatography and affinity column chromatography using an affinity column containing an anti-immunoglobulin antibody, a peptide compound used as an antigen for the immunization, protein A or the like. Thus, there is obtained a fraction containing the purified polyclonal antibody of the present invention.

A monoclonal antibody of the present invention may be produced using the peptide or protein of the present invention, or the above-mentioned peptide compound by a customary method. For example, a cell line capable of producing the antibody of the present invention is prepared by a well-known cell fusion technique (Nature, 256 (6), 495-497 (1975)) and the cell line is cultured to thereby produce a monoclonal antibody in the culture medium. The cell line capable of producing the antibody of the present invention may also be prepared by a method in which a normal cell producing the antibody is treated with a virus such as an Epstein-Barr virus to mutate the cell to a cell capable of being cultured for a long time.

The cell fusion technique will be explained below in detail. First, an immunizable animal such as mouse is immunized with the peptide or protein of the present invention, or the above-mentioned peptide compound. After completion of the immunization, a spleen is excised from the animal. From the spleen, cells capable of producing an antibody (B lymphocytes) are obtained. The thus obtained cells are fused with cells of a cell line which can infinitely reproduce (the cell line is hereinafter simply referred to as "parent cell line"). The cell fusion is conducted in the presence of a fusion promoting agent. It is preferred that the parent cell line have a characteristic that it cannot multiply in a culture medium in the absence or presence of a certain substance. Examples of such cell lines include a cell line deficient in hypoxanthine-guanine phosphoribosyl transferase (hereinafter referred to as "HGPRT") and a cell line deficient in thymidine kinase (hereinafter referred to as "TK"). The cell line deficient in HGPRT or TK cannot multiply in a HAT medium which contains hypoxanthine, aminopterin and thymidine. However, when the cell line is fused with the above-mentioned B lymphocyte, the HGPRT activity or TK activity of the cell line is revived so that the resultant fused cell can multiply even in the HAT medium. Utilizing the above-mentioned characteristic of the parent cell line, the fused cell is selected from the parent cell line remaining unfused using the HAT medium. Further, the fused cell is also separated from the B lymphocyte remaining unfused by culturing for a long time because the B lymphocyte per se cannot multiply and dies within a short period of time. Thus, there are obtained fused cells. From the thus obtained cells, a fused cell capable of producing the intended antibody is selected. By culturing the selected fused cell, the monoclonal antibody is produced in the culture medium of the fused cell.

As the animal used for immunization to obtain B lymphocytes capable of producing the antibody, there may generally be employed a BALB/c mouse and the like.

The immunization may generally be conducted as follows. The peptide or protein of the present invention, or the peptide compound mentioned above is injected as an antigen subcutaneously or intraperitoneally to a BALB/c mouse at a dose of about 0.1 to 100 μg together with a Freund's complete adjuvant. The injection of the antigen is repeated about 2 to 6 times at intervals of 1 to 2 weeks. Thereafter, a solution of the present peptide or the peptide compound in physiological saline is injected intraperitoneally or intravenously into the mouse to complete the immunization. 3 to 4 days later, a spleen is excised from the mouse and reduced to spleen cells including B lymphocytes.

The thus obtained spleen cells are fused with cells of the parent cell line. Examples of parent cell lines include HGPRT-deficient strains derived from a myeloma cell of a BALB/c mouse, such as P3-X63-Ag8 strain and P3-X63-Ag8-U1 (hereinafter referred to simply as "P3U1") strain; P3/NSI/1-Ag4-1 strain; P3X63-Ag8.653 strain; and SP2/O-Ag-14 strain.

As the fusion promoting agent, there may generally be used a polyethylene glycol (PEG), a liposome which is an artificial lipid vesicle, a virus such as Sendai virus (HVJ) and the like. The cell fusion may also be conducted by an electro-fusion technique in which an electric pressure is applied to the cells without using a fusion promoting agent.

When an HGPRT-deficient strain such as the P3-X63-Ag8 strain or P3U1 strain is used as the parent cell line, cells of the parent cell line which are fused with an antibody-producing cells, can be easily and effectively selected from the cells remaining unfused by culturing in a HAT medium as mentioned before. From the thus obtained fused cells, a fused cell capable of producing an antibody which has excellent reactivity with the peptide or protein of the present invention is selected by a customary immunological assay method.

Thus, there is obtained a fused cell (hybridoma) capable of producing a monoclonal antibody of the present invention.

Using the hybridoma, the monoclonal antibody of the present invention may be produced as follows. The hybridoma is injected into the abdominal cavity of a mouse stimulated with pristine and the like. After predetermined period of time, the abdominal dropsy is collected. Thus, there is obtained a solution containing the monoclonal antibody of the present invention.

The solution containing the monoclonal antibody may also be obtained by culturing the hybridoma in a nutrient medium.

From the thus obtained solution in the form of the abdominal dropsy or the culture medium, the antibody of the present invention may be isolated and purified by a customary purification method. For example, the solution containing the antibody is subjected to salting-out using a salt such as ammonium sulfate to precipitate a protein. The precipitate is collected by centrifugation and dissolved in a neutral buffer such as a phosphate buffer. The resultant solution is dialyzed to remove the salt used for the salting-out from the solution. Then, the solution is subjected to purification by a customary technique such as an ion exchange chromatography or an affinity chromatography using a column containing an anti-immunoglobulin antibody, an antigen used for the immunization, protein A or the like.

Thus, there is obtained a monoclonal antibody of the present invention in substantially pure form.

The above-mentioned cell line capable of producing the antibody of the present invention is also included in the scope of the present invention. Therefore, according to the present invention, there is provided a cell line which is capable of producing a monoclonal antibody specific for at least a portion of human brain senile plaque amyloid precursor protein, which portion has a protease inhibiting activity. Examples of cell lines include a mouse hybrid cell line ADI1-7-5-2, a mouse hybrid cell line ADI1-4-6-1 and a mouse hybrid cell line ADI1-05-44-2. The mouse hybrid cell lines ADI1-7-5-2, ADI-4-6-1 and ADI1-05-44-2 are deposited at the Fermentation Research Institute, Japan under the accession numbers FERM P-9757, FERM P-9861 and FERM P-9856, respectively.

In a further aspect of the present invention, there is provided a human chromosomal DNA fragment comprising as an exon a base sequence represented by the following formula (VI):

```
1        10         20         30         40
AGGTGTGCTC TGAACAAGCC GAGACGGGGC CGTGCCGAGC

         50         60         70         80
AATGATCTCC CGCTGGTACT TTGATGTGAC TGAAGGGAAG

         90        100        110        120
TGTGCCCCAT TCTTTTACGG CGGATGTGGC GGCAACCGGA

        130        140        150        160
ACAACTTTGA CACAGAAGAG TACTGCATGG CCGTGTGTGG

      168
 CAGCGCCA                             ... (VI).
```

The base sequence of the formula (VI) is designated exon I as mentioned before.

The human chromosomal DNA fragment may further comprise at least a portion of a base sequence of the base numbers 1 to 85 indicated in Fig. 5 and at least a portion of a base sequence of the base numbers 254 to 457 indicated in Fig. 5, attached to the base sequence of the formula (VI) at its 5'-end and 3'end, respectively.

Further, the human chromosomal DNA fragment may further comprises a base sequence as shown in Fig. 6, and which has a molecular length of about 6.6 kb and a restriction endonuclease cleavage map shown in (B) of Fig. 2 as a BamHI-BamHI insert in plasmid pAP246B. In the base sequence shown in Fig. 6, there is a base sequence designated exon J as mentioned before (in Fig. 6, the base sequence of exon J is underlined, and the other portions are intron portions).

Furthermore, the human chromosomal DNA fragment may further comprises base sequences respectively shown in Figs. 6 and 7 and which has a molecular length of about 37 kb and a restriction endonuclease cleavage map as shown in (A) of Fig. 2. In the base sequence shown in Fig. 7, there is a base sequence as designated exon K as mentioned before (in Fig. 7, the base sequence of exon K is underlined, and the other portions are intron portions).

According to the present invention, there is also provided a human chromosomal DNA comprising the base sequence of exon J, that is a human chromosomal DNA fragment comprising as an exon a base sequence represented by the following formula (X):

```
TG TCCCAAAGTT TACTCAAGAC TACCCAGGAA

CCTCTTGCCC GAGATCCTGT TAAAC        ... (X).
```

The above-mentioned human chromosomal DNA fragment may further comprise at least a portion of base sequence of the 1st to 195th bases shown in Fig. 6 and at least a pqrtion of a base sequence of the 253rd to 395th bases shown in Fig. 6, attached to said base sequence of formula (X) at its 5'-end and 3'-end, respectively.

According to the present invention, there is further provided a human chromosomal DNA fragment comprising a base sequence as shown in Fig. 4, and a human chromosomal DNA fragment comprising a base sequence as shown in Fig. 7. The human chromosomal DNA fragment comprising the base sequence of Fig. 4 contains the base sequence of exon H as mentioned before (in Fig. 4, the base sequence of exon H is underlined, and the other portions are intron portions). This human chromosomal DNA fragment includes a human chromosomal DNA fragment which has a molecular length of about 6.7 kb and a restriction endonuclease cleavage map as shown in (A) of Fig. 3. On the other hand, the human chromosomal DNA fragment comprising the base sequence of Fig. 7 contains the base sequence of exon K as mentioned before (in Fig. 7, the base sequence of exon K is underlined, and the other portions are intron portions).

The above-mentioned human chromosomal DNA fragment may be produced by a customary recombinant DNA technique as follows. A chromosomal DNA is obtained from a human cell or human tissue by a customary method and digested with an appropriate restriction enzyme to obtain DNA fragments. The DNA fragments are respectively inserted in replicable vectors to obtain a gene library. The gene library is subjected to screening by a customary method using a probe having a base sequence coding for at least a portion of the cDNA coding for the SPAP or at least a portion of a intron as mentioned above. It is preferred to use a probe having a base sequence containing at least a portion of the base sequence of exons H, I, J or K. It is also possible that a probe having a base sequence of at least a potion of an exon other than exons H, I, J and K mentioned above is used to obtain a chromosomal gene coding for SPAP, and using the obtained chromosomal gene, the intended human chromosomal DNA fragment is obtained by a customary technique of gene walking.

The above-mentioned human chromosomal DNA fragment relates to expression of a portion of a human brain senile plaque amyloid precursor protein, which portion has a protease inhibiting activity.

Generally, it is known that there is polymorphism in the human chromosomal gene, such as deletion, insertion or point mutation. It is considered that some of such polymorphism relates to a hireditary disease and an inherited character which is likely to cause a body to suffer from a specific disease. The human chromosomal DNA fragment of the present invention includes such polymorphic mutant DNA fragments of the above-mentioned human chromosomal DNA fragment. The polymorphic mutant DNA fragments may also be obtained from human chromosomal genes by cloning in the same manner as mentioned above.

The human chromosomal DNA fragment of the present invention may be used for producing the protein of the present invention by recombinant DNA technique. The human chromosomal DNA fragment may also be used for the detection of alternative splicing of the SPAP gene in vitro. Further, the human chromosomal DNA fragment can be used for producing a transgenic animal which is useful as a model animal for studying the SDAT. The transgenic animal may be produced by a method in which the DNA fragment is inserted in an appropriate expression vector and the resultant recombinant is transferred into a fertilized ovum of an animal, and the resultant ovum is grown.

In a further aspect of the present invention, there is provided an RNA corresponding to the above-mentioned DNA of the present invention. Further, according to the present invention, there is provided an RNA corresponding to the human chromosomal DNA fragment of the present invention. The RNA of the present invention comprises the same base sequence as that of the DNA or human chromosomal DNA fragment of the present invention except that deoxyadenylic acid (A), deoxycytidylic acid (C), deoxyguanylic acid (G) and thymidylic acid (T) in the base sequence of the DNA corresponds to adenylic acid, guanylic acid, cytidylic acid and uridylic acid, respectively. The RNA of the present invention may be produced by a customary recombinant DNA technique using RNA polymerase. That is, a certain RNA polymerase-secific promoter is ligated upstream of the above-mentioned DNA of the present invention, and the resultant recombinant DNA is treated with RNA polymerase specific for the above-mentioned promoter, to obtain the RNA of the present invention. The RNA of the present invention may also be organo-chemically synthesized by a customary method.

The RNA of the present invention may advantageously be used as a probe for the detection of mRNA coding for NAP or NAP3 produced in a brain cell or other cells for the diagnosis of the SDAT. The RNA of the present invention may also be used in combination with an appropriate translation system for producing the protein of the present invention by recombinant DNA technique.

According to the present invention, there is further provided a method for detecting a human brain senile protein amyloid precursor protein (SPAP) selected from the group consisting of amino acid sequences of formula (III) and of formula (V) as mentioned above, which comprises subjecting a sample to immunoassay using an antibody of the present invention as mentioned before.

The sample may generally be obtained from a human body. Examples of samples obtained from a human body include a cerebrospinal fluid (CSF), plasma, serum, a brain tissue section, a brain tissue homogenate, a brain tissue extract and the like.

According to the present invention, the immunoassay may be conducted by a customary method. For example, when the CSF, plasma, serum, brain tissue homogenate or brain tissue extract is used as a sample, the immunoassay may be conducted by a solid phase enzyme-linked immunosorbent assay (hereinafter often referred to as "solid phase ELISA") or an immunoblotting method. Examples of immunoblotting methods include dot blotting method, Western blotting method and the like. With respect to the brain tissue section, the immunoassay may be conducted by a customary immuno-histochemical method.

The solid phase ELISA may be conducted as follows. A sample such as CSF, plasma, serum and brain tissue extract is put in a well of, for example, a polystyrene-made microplate and, then, a buffer such as a carbonate-bicarbonate buffer or a phosphate buffer is put in the well. The mixture is stationarily incubated for a predetermined period of time so that proteins in the sample are adsorbed on the inner surface of the well. The incubation is generally conducted in a carbonate-bicarbonate buffer at 4 °C overnight. The incubation may also be conducted at room temperature for 2 hours, or at 37 °C for 1 hour. Then, the plate is washed with a phosphate buffer. The washing may also be conducted using a buffer containing a surfactant. After the washing of the plate, the antibody of the present invention is put in the well as a primary antibody, and the plate is incubated to advance an antigen-antibody reaction. Then, the plate is washed with a buffer, and a solution containing a predetermined concentration of an enzyme-labeled anti-immunoglobulin (Ig) antibody is added in the well as a secondary antibody to react with the antibody of the present invention which has been reacted with an antigen adsorbed on the plate. Then, the plate is washed, and a substrate such as o-phenylenediamine is added to the plate to determine the enzyme activity of the reaction mixture. It is also possible that instead of the enzyme-labeled anti-Ig antibody, a biotin-labeled antibody is used for reacting with the antibody of the present invention and, then, an enzyme-labeled avidin is reacted with the biotin-labeled antibody (ABC method). Further, the solid ELISA may also be conducted by a customary peroxidase-antiperoxidase (PAP) method.

The enzyme activity determined indirectly represents the amount of the portion of a human brain senile plaque amyloid precursor protein, having a protease inhibiting activity present in the sample employed.

In practicing the solid phase ELISA, a fluorescent substance-labeled or a radioisotope-labeled anti-mouse Ig antibody may also be used instead of the above-mentioned enzyme-labeled anti-Ig antibody.

Further, the solid phase ELISA may also be conducted by the so-called sandwich immunoassay method in which two types of monoclonal antibodies of the present invention which are capable of recognizing different epitopes, are used. Particularly, one monoclonal antibody is adsorbed on a microplate as a primary antibody. Then, a sample is applied to the plate to react an antigen in the sample with the primary monoclonal antibody adsorbed on the plate. Then, the other monoclonal antibody of the present invention is reacted with the antigen which has been reacted with the primary antibody. Then, a secondary antibody as mentioned above is reacted with the antigen-monoclonal antibody complex. This method is advantageous from the standpoint of sensitivity.

The dot blotting may be conducted as follows. A small amount (generally 0.2 to 10 μl) of a sample such as CSF, plasma, serum, brain tissue extract and brain tissue homogenate is dotted on a nitrocellulose filter or a nylon filter. The filter is subjected to blocking with a bovine serum albumin (hereinafter referred to as "BSA"), a gelatin, a skim milk or the like in a buffer such as a Tris-HCl buffer. Then, the antibody of the present invention is reacted with the antigen on the filter. After completion of the reaction, the resultant filter is washed with a buffer. Then, an enzyme-labeled anti-Ig antibody is applied to the filter as a secondary antibody to react the secondary antibody with the antibody of the present invention. The filter is washed. Then, to the filter is added a substrate, and the enzyme activity is determined based on the thickness of color assumed by the enzyme-substrate reaction. Examples of substrates include 4-chloronaphthol, diaminobenzidine and the like. As the secondary antibody, a labeled protein A may also be used instead of the above-mentioned anti-Ig antibody.

The Western blotting may be conducted as follows. A sample such as CSF, plasma, serum, brain tissue extract, brain tissue homogenate as such or after being boiled with sodium dodecyl sulfate (hereinafter referred to as "SDS"), is subjected to polyacrylamide gel electrophoresis (hereinafter referred to as "PAGE"). The resultant gel is equilibrated with a transfer buffer and, then, the protein in the gel is electrotransferred into a nitrocellulose filter or a nylon filter. The thus obtained filter is treated in the same manner as in the case of the above-mentioned dot blotting to thereby determine the amount of the protein comprising at least a portion of a human brain SPAP in the sample.

The immuno-histochemical assay of a brain tissue section sample may be conducted by a customary method as follows. A brain of a dead patient is frozen, or fixed with formalin and embedded in paraffin. From the thus obtained brain, a tissue section is prepared. The tissue section is treated with trypsin. The treatment of trypsin may generally be conducted using 0.1 % aqueous trypsin solution at 37 °C for 10 min. However, the trypsin treatment is not always required. The resultant tissue section is subjected to assay using the antibody of the present invention by the above-mentioned ABC method or a peroxidase-antiperoxidase (PAP) method. In the PAP method, diaminobenzidine is generally used as a substrate of peroxidase.

In practicing the above-mentioned immunoassay, the antibody of the present invention may be labeled with a dye such as rhodamine and fluorescein isothiocyanate (FITC); an enzyme such as peroxidase; or a radioactive isotope, so that the assay can be conducted without using a secondary antibody.

The human brain SPAP is occasionally glycosylated, that is, contains a sugar chain, and it is possible that the sugar chain will adversely affect reactivity to the antibody of the present invention to thereby decrease the sensitivity of the detection. Therefore, if desired, a sample may be treated with a glycohydrolase to remove most of sugar chain from the protein, so that the sensitivity of the detection is increased. Examples of glycohydrolases include heparinase, heparinase II, heparinase III, γ-N-acetyl-D-glucosamidase, endoglycosidase, exoglycosidase, hyaluronidase and the like. In this connection, it is preferred that the antibody of the present invention be prepared using an antigen containing no sugar chain or, even if any, a minimized amount of sugar chain.

The antiserum or the fraction of the polyclonal antibody obtained by the above-mentioned purification may generally be diluted to an appropriate degree and the dilution is used for immunoassay. In the case of the solid phase ELISA, the antiserum may generally be diluted about 100 to about 4000 times, preferably about 200 to about 1000 times. In the case of the Western blotting, the antiserum may generally be diluted about 100 to about 2000 times, preferably about 200 to about 1000 times. In the case of the solid phase ELISA, the antiserum or the fraction containing the polyclonal antibody may generally be diluted about 200 to about 4000 times, preferably 100 to about 500 times. When the polyclonal antibody is used for immunoassay, a polyclonal antibody solution containing the polyclonal antibody at such a concentration as is equal to the IgG concentration of the antiserum of the present invention may generally be prepared and diluted as in the case of the antiserum of the present invention. On the other hand, the fraction of the monoclonal antibody obtained by the above-mentioned purification may generally be diluted about up to 50 times, preferably about 1 to 10 times.

Further, according to the present invention, there is provided a method for detecting a DNA coding for a polypeptide having a protease inhibiting activity which is a portion of a human brain senile plaque amyloid precursor protein, which comprises subjecting a sample to assay by hybridization using a probe selected from the group consisting of (i) a DNA comprising at least a portion of a base sequence of formulae (VI), (VIII) or (XII) as mentioned above, (ii) a complementary DNA to the DNA and (iii) RNA's corresponding thereto.

The detection of a DNA coding for a polypeptide which is a portion of a human brain senile plaque amyloid precursor protein (SPAP)having a protease inhibitory activity, provides useful information for the diagnosis of SDAT.

As the probe, the above-mentioned DNA of the present invention may be used. Further, there may also be used a portion of the DNA of the present invention as a probe. In order to conduct an effective hybridization of the DNA to be detected with a probe with high specificity, it is preferred that a probe has a base sequence of at least twelve continuously sequenced bases. Therefore, the probe to be used in the method of the present invention should preferably have a base sequence of at least twelve continuously sequenced bases in the base sequence of the DNA of the present invention. Particularly, it is preferred that a portion of the DNA of the present invention to be used as a probe has a sequence of at least twelve continuously sequenced bases, more preferably seventeen continuously sequence bases in a base sequence of the formula:

G GTGGTTCGAG-(VI)-TT CCTACAACA

wherein (VI) is a DNA having the same base sequence as defined for formula (VI) mentioned before, or a sequence of at least twelve continuously sequenced bases, more preferably seventeen continuously sequenced bases in a base sequence of the formula:

G GTGGTTCGAG-(IX)-TT CCTACAACA

wherein (IX) is a DNA having the same base sequence as defined in formula (IX) mentioned before.

As the probe, there may also be used a complementary DNA to the above-mentioned DNA or an RNA corresponding to the above-mentioned DNA.

A sample to be subjected to assay by hybridization may generally be a DNA obtained from a human body. For example, a DNA prepared from poly(A)RNA obtained from a human blood lymphocyte and the other human cells may be used.

The assay may be conducted by a customary hybridization method such as Southern blotting and dot blotting.

The assay by the Southern blotting method may be conducted as follows. The chromosomal DNA is extracted from a human blood lymphocyte or other human cell by a customary method as described later in Example. The chromosomal DNA is digested with an appropriate restriction enzyme to obtain DNA fragments. The DNA fragments are subjected to gel electrophoresis. The resultant DNA fragments electrophoresed on the gel are transferred onto a membrane filter such as a nylon filter electrically or by capillarity. On the other hand, the above-mentioned DNA or RNA to be used as a probe is labeled with a radioactive isotope such as $^{32}P$ or $^{35}S$, or a fluorescent substance by a customary method. The labeled probe is hybridized with the above-obtained membrane filter. The DNA fragment on the filter hybridized with the probe can be detected as a band by the radioactivity or fluorescence. Based on the base sequence of the probe employed, the position of the band detected, the intensity of the band, etc., the abnormality in SPAP gene is determined. That is, the base sequence of the probe hybridized, and the position and intensity of the band obtained with respect to the sample are compared to those obtained with respect to the normal sample to determine the abnormality.

The assay by the dot blotting may be conducted as follows. The chromosomal DNA obtained in the same manner as mentioned above is subjected to gene amplification according to a customary method, for example, the method of Nakamura [Yusuke Nakamura, "Saibo Kogaku" (Cell Technology), 5 (8), 755-760(1986),JAPAN]. The chromosomal DNA may also be used without being subjected to gene amplification. The chromosomal DNA is then digested with an appropriate restriction enzyme to obtain DNA fragments. The DNA fragments are adsorbed on a membrane filter such as a nitrocellulose filter or a nylon filter. The above-mentioned digestion of the chromosomal DNA may not necessarily be conducted. Therefore, the chromosomal DNA as such may also be adsorbed on a membrane filter. Then, the hybridization is conducted using a labeled probe as mentioned above. The abnormality in the chromosomal DNA of a sample is evaluated from the intensity of a dot in the same manner as mentioned above with respect to the Southern blotting.

Further, the abnormal duplication of the SPAP gene in a chromosomal DNA, which repetition of the SPAP causes various diseases to occur in a central nervous system, may be detected by the Southern blotting or dot blotting in substantially the same manner as mentioned above except that the intensity of the band or dot on the membrane filter is compared to that of the band or dot obtained by hybridization of the DNA fragments or chromosomal DNA with a probe coding for a protein generally existing in nature, such as actin, tublin or τ protein.

The above-mentioned DNA probe may be produced by recombinant DNA technique as mentioned before. The DNA probe may also be organo-chemically synthesized using a customary DNA synthesizer.

The DNA of the present invention and a complementary DNA thereto may be complementarily annealed to each other to obtain a double-stranded DNA. When the molecular length of the DNA is long, the double-stranded DNA may be prepared by organo-chemical synthesis in combination with enzyme reactions such as ligation and DNA polymerization.

The resultant double stranded DNA may be multiplied by recombinant DNA technique using a customary host-vector system. That is, the DNA is inserted in a replicable vector to obtain a recombinant DNA. The replicable recombinant DNA is transferred into a host cell to form a transformant. By culturing the transformant, the DNA is multiplied with the replication of the recombinant DNA. Examples of vectors include plasmids pBR322, pUC18, pUC19, pUC118, pUC119 and the like. Examples of hosts include Escherichia coli, Bacillus subtilis, Saccharomyces cerevisiae and an animal cell.

After culturing the transformant, the multiplied DNA may be isolated from the transformant as follows. The recombinant DNA is isolated from the transformant by a customary plasmid isolation technique. That is, the transformant is lysed by means of an alkali and/or a lysozyme. From the lysed cell, a protein is removed by a customary method to obtain a plasmid fraction. The plasmid fraction is then subjected to purification by a customary technique such as ultracentrifugation using cesium chloride, or high performance liquid chromatography.

The RNA probe may be produced as follows. The DNA of the present invention is inserted in a vector carrying a promoter specific for SP6RNA polymerase or T7RNA polymerase to obtain a recombinant DNA. Using the SP6RNA polymerase or T7RNA polymerase, the DNA of the present invention in the recombinant DNA is transcribed to the corresponding RNA having a base sequence complementary to that of the DNA. The thus obtained RNA may be labeled with a radioisotope or a fluorescent substance. The RNA may also be organo-chemically synthesized according to a customary method.

The above-mentioned DNA probe or RNA probe may also be used for detecting an mRNA coding for a polypeptide having a protease inhibiting activity.

Accordingly, in a further aspect to the present invention, there is provided a method for detecting an RNA coding for a polypeptide having a protease inhibiting activity, which polypeptide is a portion of a human brain senile plaque amyloid precursor protein, which comprises subjecting a sample to assay by hybridization using a probe selected from the group consisting of (i) a complementary DNA to a DNA comprising at least a portion of a base sequence of formula (VI), (VIII) or (XII) as mentioned above and (ii) RNA's corresponding thereto.

A sample to be assayed may generally be obtained from a human body. Examples of samples include a brain tissue and tissues of the other organ, a blood lymphocyte and the like. The sample should be treated so as not to degrade the mRNA present in the sample because the mRNA is relatively unstable and degradable as compared to a DNA. For example, a fresh tissue or cell containing the mRNA is rapidly frozen in a liquid nitrogen. A fresh sample as such may also be used as long as the assay is readily conducted.

The mRNA may be isolated from the tissue or cell by a customary method as described in Example 1. Using the thus isolated mRNA as a sample, the assay is conducted by a customary hybridization technique such as Northern blotting and dot blotting. The Northern blotting and the dot blotting may be conducted in substantially the same manner as in the Southern blotting and the dot blotting which is conducted for detecting a DNA as mentioned above, except that a probe is hybridized with an mRNA instead of a DNA.

By changing the base sequence of the DNA probe or RNA probe, each of mRNA's coding for AP (the known SPAP), NAP and NAP3 (the novel SPAP's of the present invention) may be detected. Comparison between the amounts of the thus detected mRNA's coding for AP, NAP and NAP3, respectively, is useful for the diagnosis of SDAT or the abnormal metabolism in the central nervous system.

Generally, it is known that an abnormal base sequence in the introns of a chromosomal gene causes a certain disease. For example, it is known that β thalassemia, an anemia, is caused by an abnormal base sequence in the introns of a globin gene. Illustratively stated, one base in the base sequence of an intron of a globin gene is changed and, as a result, an intron termination signal and an exon initiation signal are newly formed in the intron. By the formation of the new intron termination signal and new exon initiation signal, an abnormal mRNA is transcribed from the globin gene. The production of the abnormal mRNA causes the thalassemia.

Further, it is also known that there is an enhancer sequence in an intron, which controls the transcription of a chromosomal gene into an mRNA. If the enhancer sequence in an intron is changed by the insertion or deletion of a base, or by point mutation, the gene expression may be adversely affected, causing a disease.

Moreover, it is known that there is a base sequence in a respective intron of a gene, which is necessary for appropriately removing RNA portions having base sequences of introns from an immature mRNA comprising base sequences of exons and introns, to thereby form a mature mRNA consisting of the base sequences of exons (this process is called "splicing"). Examples of such base sequences include a pyrimidine-enriched base sequence present in the 3′-end region of an intron; and a consensus sequence between various genes, which is a branch point of splicing and present in the 3′-end region of 10 to 50 bases in a respective intron and which is represented, for example, by the following formula:

**YXYTRAY,**

or

CTTGXXYYY

wherein Y is C or T, R is A or G, and X is A, G, C or T.

Furthermore, it is known that there are universal sequences in introns at the border regions with exons, that is, a base sequence GT at the 5'-end of the intron and a base sequence AG at the 3'-end of the intron. These sequences also participate in splicing of the immature mRNA. Further, it is known that a certain base sequence in the base sequence of an exon also participates in splicing of the immature mRNA.

The above-mentioned base sequences are important to the proper expression of a gene. However, if such base sequences are changed by the insertion or deletion of a base, or by the point mutation, the proper expression of a gene is no longer conducted, causing a disease.

Further, it is also known that various types of mRNA's are produced from one gene by alternative splicing. In the case of such a gene, it is possible that if the base sequence of the gene is changed as mentioned above, the relative amounts of mRNA's produced from the gene are changed, causing a disease.

Accordingly, with respect to the senile dimentia of Alzheimer's disease type (SDAT) also, it is possible that the SDAT is caused by the change in base sequence of exons and/or introns of the human chromosomal gene coding for SPAP. Such a change in base sequence of SPAP gene may be detected by hybridization using as a probe at least a portion of the human chromosomal DNA of the present invention.

Therefore, in still a further aspect of the present invention, there is provided a method for detecting a human chromosomal DNA fragment relating to expression of a polypeptide having a protease inhibiting activity, the polypeptide being a portion of a human brain senile plaque amyloid precursor protein, which comprises subjecting a sample to assay by hybridization using as a probe at least a portion of a human chromosomal DNA fragment as mentioned before.

A sample to be used for the assay is the same as used in the method for detecting the DNA of the present invention.

The assay may be conducted by a customary hybridization technique such as Southern blotting and dot blotting as explained before.

As the probe, the above-mentioned human chromosomal DNA fragment of the present invention may be used. Further, there may also be used a portion of the human chromosomal DNA fragment of the present invention as a probe. In order to conduct an effective hybridization of the human chromosomal DNA fragment to be detected with a probe with high specificity, it is preferred that a probe has a base sequence of at least twelve continuously sequenced bases. Therefore, the probe to be used in the method of the present invention should preferably have a base sequence of at least twelve continuously sequenced bases, more preferably seventeen continuously sequenced bases in the base sequence of the human chromosomal DNA fragment of the present invention.

The protein of the present invention is useful for preparing an antibody specific for a human brain senile plaque amyloid precursor protein (SPAP). The thus prepared antibody is advantageously used for detecting the SPAP in CSF, plasma, serum, etc. obtained from a human body. The SPAP relates to SDAT. Therefore, the antibody is useful for the diagnosis of SDAT.

The protein of the present invention having a protease inhibiting activity has an affinity to serine proteases such as trypsin, chymotrypsin, plasmin, kallikrein, factor Xa and elastase. Therefore, the protein of the present invention can be used as a ligand for purifying the serine proteases. The protein of the present invention is more advantageously used as a ligand for purifying trypsin and chymotrypsin.

Further, the present protein may also be used for treating a pancreatitis, a peptic ulcer, an atherosclerosis, an inflammation of a connective tissue, an injury or necrosis of a vascular system or the like, and for repressing the fibrinogenolysis of an injured portion.

The antibody of the present invention can specifically react with at least a portion of a human brain SPAP present in CSF, plasma, serum, etc. As mentioned above, the human brain SPAP relates to SDAT. Therefore, the present antibody is useful for the diagnosis of SDAT.

The DNA and RNA of the present invention can advantageously be used as a probe for detecting the DNA or mRNA coding for NAP or NAP3 (novel SPAP) in a tissue of a brain etc. Therefore, the present DNA or RNA is useful for the diagnosis of SDAT.

The DNA and RNA of the present invention can also be used for producing the protein of the present invention by recombinant DNA technique.

The human DNA fragment of the present invention can advantageously be used as a probe for detecting the abnormality in the base sequence of the SPAP gene. The abnormality in the base sequence of the SPAP gene may cause SDAT. Therefore, the human chromosomal DNA fragment of the present invention is also useful for the diagnosis of SDAT. The present human chromosomal DNA fragment can also be used for producing the protein of the present invention by recombinant DNA technique.

The present invention will now be described in more detail with reference to the Examples and Referential Examples, which should not be construed as being limiting the present invention.

In the Examples and Referential Examples, the literature mentioned as "the manual of Maniatis et al." means the following book:

T. Maniatis et al., Molecular Cloning A Laboratory Manual, Cold Spring Harbor Laboratory, Cold Spring Harbor, N. Y., 1982.

The following abbreviations and terms are used in the Examples and Referential Examples.

Ligation buffer: a solution containing 0.05 M Tris (pH 7.4), 0.01 M $MgCl_2$, 0.01 M dithiothreitol, 1 mM spermidine, and 1 mg/ml bovine serum albumin.

6 x SSC: a solution containing 0.9 M sodium chloride and 90 mM sodium citrate.

50 x Denhardt: a solution prepared by dissolving in 500 ml of water 5 g of Ficoll (manufactured and sold by Pharmacia Fine Chemicals AB, Sweden), 5 g of polyvinyl pyrrolidone, and 5 g of bovine serum albumin (manufactured and sold by Calbiochem Co., Ltd., U.S.A.; crystalline grade).

PBS(-): Dulbecco's phosphate buffered saline ($Ca^{2+}$ and $Mg^{2+}$ are not contained).

Luria-Bertani medium: a solution prepared by dissolving 10 g of bacto trypton, 5 g of yeast extract and 5 g of sodium chloride in 1 ℓ of water.

PBS: Dulbecco's phosphate buffered saline.
TBS: 0.05M Tris-HCl (pH 7.5) containing 0.9 w/w% NaCl.
FCS: fetal calf serum.

Example 1

[Cloning of cDNA's coding for novel senile plaque amyloid precursor proteins (NAP and NAP3)]

Step 1

[Culturing of Glioblastoma ATCC HTB-14 cell line and preparation of a poly(A)RNA]

Glioblastoma ATCC HTB-14 cell line is cultured in Eagle's Minimum Essential Medium based on Eerle's balanced salts containing non-essential amino acids and sodium pyruvate (hereinafter referred to as "EMEM") and containing 15 % FCS under the culture conditions in accordance with the protocol of ATCC . About $5x10^8$ cells of the resultant cultured cells are collected, and RNA is isolated from the collected cells according to the method of Chirgwin et al [J. M. Chirgwin; A.E. Przygyla; R.J. MacDonald and W.J. Rutter, Biochemistry 18, 5294 (1979)]. The RNA obtained is purified by oligo(dT) cellulose column chromatography, thereby obtaining 205 μg of a poly(A)RNA fraction (as calculated from the optical density at 260 nm).

Step 2

(Cloning of the C-terminal region of the cDNA coding for a senile plaque amyloid precursor protein)

5 μg of vector pUC19 (manufactured and sold by Takara Shuzo Co., Ltd., Japan) is digested with BamHI and HindIII and treated with bacterial alkaline phosphatase (hereinafter referred to as "BAP"), followed by phenol extraction at least three times to remove the BAP. The resultant product is subjected to 1 % low melting point agarose gel electrophoresis and a band portion corresponding to the vector is cut off from the resultant gel. From the cut-off gel, the vector is extracted (the resultant vector is designated "vector a").

Separately, using 10 μg of poly(A)RNA isolated from the glioblastoma ATCC HTB-14 in the above Step 1, oligo $(dT)_{15}$ as a primer and a cDNA synthesis kit (manufactured and sold by Boehringer Mannheim Yamanouchi K.K., Japan), single-stranded cDNA complementary to the poly(A)RNA of the glioblastoma ATCC HTB-14 is synthesized and then, using the obtained single-stranded cDNA, double-stranded cDNA is synthesized in accordance with the protocol accompanying the kit . Then, the double-stranded cDNA is purified by phenol extraction and ethanol precipitation. The thus obtained double-stranded cDNA is digested with BamHI and HindIII and subjected to 1 % low melting point agarose gel electrophoresis. A band portion corresponding to the cDNA fragments each having a molecular length of about 1 to 2 kilo base pairs (hereinafter referred to as "kb") is cut off from the resultant gel. Then, the cDNA fragments are extracted from the gel, and purified by phenol extraction and ethanol precipitation.

A half amount of the above-obtained BamHI-HindIII cDNA fragments and about 210 ng of the above-obtained vector a are ligated to each other at 15 °C for 3 hours in a ligation buffer containing T4 ligase and adenosine triphosphate (hereinafter referred to as "ATP"). Using the resultant ligation reaction mixture, Escherichia coli MC1061 (manufactured and sold by Pharmasia Fine Chemicals AB, Sweden)is transformed and then cultured in accordance with the method described in the manual of Maniatis et al, thereby obtaining about 35,000 colonies of the transformed Escherichia coli having resistance to ampicillin.

The colonies are transferred onto a Whatmann®-541 filter, and hybridization is performed at 55 °C for 2 hours using as a probe a synthetic deoxyoligonucleotide AM-4 (5′-ACCATGAGTCCAATGATTGCAC-3′) labeled with radioactive $^{32}P$ at its 5′-terminus. After completion of the hyblidization, the resultant filter is washed with 6 x SSC solution at room temperature for 10 min 2 times and then further washed with 6 x SSC solution at 55 °C for 5 min. The resultant filter is subjected to autoradiography. As a result, it is found that there are about 200 positive colonies. The respective positive colonies are separately cultured on an agar medium (Luria-Bertani medium containing ampicillin) to isolate individual colonies and each of the cultured colonies is transferred onto a filter. Then, the transferred colony is again subjected to hybridization with the AM-4 probe, followed by the same washing operation as mentioned above. Then, the filter is subjected to autoradiography to identify each of the positive clones. 24 Positive clones are selected from the above-obtained positive clones. From the 24 clones, plasmids are extracted by a rapid, small-scale plasmid isolation method in accordance with the description on pages 368 to 369 of the manual of Maniatis et al. With respect to the plasmids obtained, restriction mapping analysis is conducted. As a result, it is found that plasmids obtained from 18 clones of the 24 clones have such a structure as can give DNA fragments of about 1.4 kb and about 2.7 kb when digested with BamHI and HindIII and can give DNA fragments of about 0.5 kb and about 3.6 kb when digested by EcoRI.

Step 3

(Cloning the N-terminal region of the cDNA coding for a senile plaque amyloid precursor protein)

5 µg of vector pUC19 is digested by BamHI and treated with BAP and then subjected to phenol extraction several times. The resultant digest is subjected to 1 % low melting point agarose gel electrophoresis and a band portion corresponding to the vector is cut off from the resultant gel. From the cut-off gel, the vector fragment is extracted (the vector is designated "vector b").

Separately, using 10 µg of poly(A)RNA isolated from glioblastoma ATCC HTB-14, 4 µg of AM-1(5′-CTTCATATCCTGAGTCATGTCG-3′) as a primer, and a cDNA synthesis kit (manufactured and sold by Boehringer Mannheim Yamanouchi K.K., Japan), single-stranded cDNA complementary to the poly(A)RNA of glioblastoma ATCC HTB-14 is synthesized and then, using the single-stranded cDNA, double-stranded cDNA is synthesized in accordance with the protocol accompanying the kit. Then, the double-stranded cDNA is purified by phenol extraction and ethanol precipitation. The thus obtained double-stranded cDNA is digested with BamHI and subjected to 1 % low meting point agarose gel electrophoresis and a band portion corresponding to the cDNA having a length of about 0.7 to 2 kb is cut off from the resultant gel. From the resultant gel, the cDNA fragments are obtaind. Then, the cDNA fragments are purified by phenol extraction and ethanol precipitation.

The above-obtained BamHI-fragments of the cDNA and 200 ng of the above-obtained vector b are ligated to each other at 15 °C for 6 hours in a ligation buffer containing T4 ligase and ATP. The resultant reaction mixture is heated at 65 °C for 10 min and then readily used for transforming Escherichia coli MC1061 in accordance with the manual of Maniatis et al. The resistant transformants are cultured, thereby obtaining about 9,000 colonies having resistance to ampicillin.

The colonies are transferred onto a Whatmann®541 filter. Using as a probe a synthetic deoxyoligonucleotide AM-3 (5′-CATTCATGTGCATGTTCAGTCTG-3′) labeled at its 5′-terminus with radioactive $^{32}P$ by means of $\gamma$-($^{32}P$)ATP and T4 kinase, hybridization is performed at 55 °C for 2 hours. After completion of the hybridization, the resultant filter is washed with 6 x SSC solution at room temperature for 10 min 2 times and then further washed with 6 x SSC solution at 55 °C for 5 min. The resultant filter is subjected to autoradiography. As a result, it is found that about 20 positive colonies are obtained. The respective positive colonies are cultured on an agar medium (Luria-Bertani medium containing ampicillin) to isolate individual colonies and the respective colony is transferred onto a filter. Then, the filter is again subjected to hydridization with the AM-3 probe, followed by the same washing operation as mentioned above. The filter is subjected to autoradiography to identify positive clones. 12 positive clones are selected at random from the positive clones, and from the 12 clones, plasmids are extracted by a rapid, small-scale plasmid isolation method in accordance with the manual of Maniatis et al. The plasmids obtained are analyzed by digestion with BamHI. As a result, it is found that a plasmid isolated from one clone consists of an insert of about 1.4 Kb and a vector of about 2.7 Kb (the clone is designated "BB-1"); plasmids isolated from the other 10 clones consist of an insert of about 1.6 Kb and a vector of about 2.7 Kb (the clones are designated "BB-2"); and a plasmid isolated from the remaining one clone consists of an insert of 1.5 to 1.6 Kb and a vector of about 2.7 Kb (the clone is designated "BB-3"), and it is further found that the insert of the plasmid from BB-3 is a little shorter than that of the plasmid isolated from clone BB-2. Then, the plasmids are digested with BamHI and XhoI. As a result, it is found that plasmids isolated from clone BB-1 consist of three fragments of about 2.7 Kb, about 1.0 Kb and about 0.4 Kb; plasmids isolated from clone BB-2 consist of three fragments of about 2.7 Kb, about 1.2 Kb and about 0.4 Kb; and plasmids isolated from clone BB-3 consist of three fragments of about 2.7 kb, about 1.1 Kb and about 0.4 Kb.

Further, plasmids isolated from clones BB-2 and BB-3 are analyzed by digestion with XhoII. As a result, plasmids isolated from clone BB-2 are found to be able to give three fragments of 900 bp, 500 bp and 200 bp and fragments derived from the vector, and plasmids isolated from clone BB-3 are found to be able to give two fragments of 1,400 bp and 200 bp and fragments derived from the vector.

The plasmids isolated from clones BB-1, BB-2 and BB-3 are designated plasmids pBB-1, pBB-2 and pBB-3, respectively. Clone BB-3 was deposited with the Fermentation Research Institute (hereinafter often referred to as "FRI") of the Agency of Industrial Science and Technology located in Ibaraki, Japan, under the name of E. coli K-12 Strain MC1061 (pBB-3) on February 10, 1988 and has been assigned accession No. FERM BP-1989.

Step 4

(Construction of the whole cDNA's for SPAP)

The plasmid as obtained in Step 2 is digested with BamHI and treated with BAP and then subjected to phenol extraction. The resultant product is subjected to 1 % low melting point agarose gel electrophoresis and a band portion corresponding to the vector is cut off from the resultant gel. From the gel, vector pUC19 containing the C-terminal region of the cDNA (this vector is designated "vector c").

Separately, each of plasmids pBB-1, pBB-2 and pBB-3 as obtained in Step 3 which respectively contain the N-terminal regions of the cDNA's coding for SPAP which regions have different molecular lengths is digested with BamHI. The mixture of digested plasmids are subjected to 1 % low melting point agarose gel electrophoresis and a band portion corresponding to each of the digested plasmids of pBB-1, pBB-2 and pBB-3, having molecular lengths of about 1.4 Kb, about 1.6 kb and about 1.5 to 1.6 Kb, respectively, is cut off from the resultant gel, followed by purification by phenol extraction and ethanol precipitation, thereby obtaining cDNA fragments desired respectively from pBB-1, pBB-2 and pBB-3.

The above-obtained vector c are separately ligated to each of the above-obtained cDNA fragments in a ligation buffer containing ATP and T4 ligase at 15°C for 3 hours and then at 4°C for 18 hours. The resultant reaction mixture is heated at 65°C for 10 min. Then, using each of the heated reaction mixture, transformation of competent cells of Escherichia coli HB101 (manufactured and sold by Takara Shuzo Co., Ltd., Japan) is conducted in accordance with the protocol accompanying the competent cells of HB101, and then the transformed cells are cultured, thereby obtaining about 1,000 clones having resistance to ampicillin. 12 Clones are selected from each of the BB-1-derived clones, BB-2-derived clones and BB-3-derived clones, and plasmid DNA's are obtained from the selected clones by a rapid, small scale plasmid isolation method. The plasmid DNA's obtained are analyzed by digestion with XhoI and HindIII. In accordance with the results of the analysis, three types of clones derived respectively from pBB-1, pBB-2 and pBB-3, in which clones the respective cDNA of the N-terminal region and the respective cDNA of the C-terminal region are inserted in the right direction, are selected.

The plasmid having the cDNA derived from plasmid pBB-1 comprises a cDNA insert having the same size as that of the portion of from the BamHI site to HindIII site of the 5′-terminal region of the cDNA coding the known SPAP (AP). This plasmid is designated pGBP1. The plasmid having a novel cDNA derived from clone BB-2 contains a cDNA insert having a molecular length of about 0.2 Kb larger than that of the cDNA insert of plasmid pGBP1. This plasmid is designated pGBP2. The clone that contains plasmid pGBP2 is designated E. coli K12 strain HB101 (pGBP2). Further, the novel plasmid that comprises the cDNA derived from BB-3 contains a cDNA insert having a molecular length of about 0.2 kb larger than that of the cDNA insert of plasmid pGBP1 but a little smaller than that of the cDNA insert of plasmid pGBP2. This plasmid is designated pGBP3.

E. coli K12 strain HB101 (pGBP2) was deposited with the Fermentation Research Institute of the Agency of Industrial Science and Technology located in Ibaraki, Japan, on August 18, 1987. E. coli K12 strain HB101 (pGBP2) was also deposited with the American Type Culture Collection (ATCC) on August 18, 1987 and has been assigned accession No. 67502.

The constructions of plasmids pGBP1 and pGBP2 are diagrammatically shown in Fig. 10(A) and Fig. 10(B), respectively.

Step 5

(Determination of base sequence)

With respect to pGBP1, pGBP2 and pGBP3, a portion of the inserted cDNA is digested with restriction enzymes, and the resultant fragments are inserted into M13 vectors (manufactured and sold by Takara Shuzo Co., Ltd., Japan) by a technique of subcloning. The subcloning is conducted by two methods, namely, a method in which only the restriction sites of the cDNA are utilized for an insertion site and a method in which restriction sites are utilized in combination with the deletion method of Henikoff et al [S. Henikoff et al, Gene, Vol. 28, pages 351 to 359 (1984)]. The operation is performed in accordance with the protocol accompanying M13 Sequencing Kit manufactured and sold by Takara Shuzo Co., Ltd., Japan. Subsequently, the entire base sequences of the inserts are determined in accordance with the method of Sanger et al. [F. Sanger, Science, Vol. 214, pages 1205-1210 (1981)]. With respect to plasmid pGBP2, the results of the base sequence determination of the cDNA insert are shown in Fig. 1(A). With respect to plasmid pGBP3, the results of the base sequence determination of the cDNA insert are shown in Fig. 1(B). The difference between the base sequence of the cDNA insert of plasmid pGBP1 and that of plasmid pGBP2 resides in that the former lacks the base sequence of from the 866th base A to the 1090th base C in the base sequence of the latter [see Fig. 1(A)]. The base sequence of the cDNA insert of plasmid pGBP1 coincides with that of the cDNA coding for the known SPAP, namely, AP.

Fig. 8 shows the homology of a portion of the novel protein encoded by plasmid pGBP2, which portion has an amino acid sequence corresponding to amino acid sequence of the 289th to 364th amino acids, to each of BPTI (bovine pancreatic trypsin inhibitor) and h-IαTI (human-inter-α-trypsin inhibitor), both of which are known trypsin inhibitors.

Further, Fig. 9 shows the homology of a portion of the novel protein encoded by plasmid pGBP3, which portion has an amino acid sequence corresponding to the amino acid sequence of the 289th to 345th amino acids, to each of BPTI and h-IαTI.

Example 2

[Expression of cDNA's coding for novel senile plaque amyloid precursor proteins (NAP and NAP3)]

Using the following DNA fragments A to D, plasmid pSVMT103 having a restriction map shown in Fig. 11 is constructed.

(A) A BamHI-HindIII fragment of about 0.5 kb derived from an oligo(dG)-tailed pL1 linker containing SV40 replication origin (manufactured and sold by Pharmasia Fine Chemicals AB, Sweden) (DNA fragment A);

(B) A BamHI-HindIII fragment of about 3.0 kb derived from an oligo(dT)-tailed pcDV1 plasmid primer (manufactured and sold by Pharmasia Fine Chemicals AB, Sweden) containing pBR322 replication origin, ampicilline resistant gene, and SV40 poly(A) signal (DNA fragment B);

(C) A BamHI-BglII DNA fragment of about 0.3 kb containing a mouse metallothionein promoter (D.M. Durnam et al, Proc. Natl. Acad. Sci., USA, 77, 6511-6515 (1980)) (DNA fragment C); and

(D) A BglII-BamHI DNA fragment containing human interferon cDNA derived from plasmid pGI101 (ATCC 39690) (DNA fragment D).

On the other hand, plasmid pGBP2 prepared in Step 4 of Example 1 is cleaved by HindIII and PvuI and then partially digested with BamHI. The resultant is subjected to 1 % low melting point agarose gel electrophoresis, to thereby isolate fragment of about 3.0 kb.

Plasmid pSVMT103 is cleaved by BglII and HindIII and then subjected to 1 % low melting point agarose gel electrophoresis, to thereby isolate vector fragments of about 3.5 kb (this vector fragment is designated "vector d").

The above-obtained fragment of about 3.0 kb and vector d are ligated to each other by means of T4 ligase, and using the resultant mixture, E. coli MC1061 is transformed, to thereby obtain transformants having resistance to ampicillin. 4 Clones of transformants are selected from the transformants, and plasmid DNA is isolated from the selected clones by a rapid, small-scale plasmid isolation method. The plasmid DNA obtained is digested with BamHI and EcoRI and subjected to agarose gel electrophoresis. As a result, it is found that the plasmid DNA isolated from 3 clones of the 4 clones has such a structure as can give fragments of about 0.5 kb, about 0.9 kb, about 1.8 kb and about 2.9 kb by digestion with BamHI and EcoRI. The plasmid is designated "plasmid pSVMTNAP". The above procedure for the construction of plasmid pSVMTNAP is diagrammatically shown in Fig. 11(B).

Substantially the same procedure as described above is repeated except that plasmid pGBP1 obtained in Step 4 of Example 1 is employed in place of plasmid pGBP2, to obtain a DNA fragment of about 2.8kb from plasmid pGBP1 and then ligate the obtained DNA fragment to vector d, thereby obtaining a plasmid which has the replication origin of SV40 and mouse metallothionein promoter and, downstream thereof, the DNA coding for the known SPAP and, further downstream thereof, SV40 early polyadenylation sequence. This plasmid is designated "plasmid pSVMTAP". The procedure for the construction of plasmid pSVMTAP is diagrammatically shown in Fig. 11(A).

Substantially the same procedure as described above is further repeated except that plasmid pGBP3 obtained in Step 4 of Example 1 is employed in place of plasmid pGBP2, to obtain a DNA fragment of about 3.0 kb and then ligate the obtained DNA fragment to vector d, thereby obtaining a plasmid which has the replication origin of SV40 and mouse metallothionein promoter and, downstream thereof, the DNA cording NAP3 and, further downstream thereof, SV40 early polyadenylation sequence. This plasmid is designated "plasmid pSVMTNAP3". The procedure for the construction of plasmid pSVMTNAP3 is diagrammatically shown in Fig. 11(C).

With 10 μg of each of the above-obtained pSVMTNAP, pSVMTAP and pSVMTNAP3, 1 x $10^6$ cells of simian cell line COS-1 [Y. Gluzman, Cell, 23 175 (1981)](manufactured and sold by Dainippon Pharmaceutical Co., Ltd., Japan; catalog No.09-1650) are transfected in sucrose-containing PBS (272 mM sucrose, 7 mM sodium phosphate, 1 mM $MgCl_2$, pH7.4). In practicing the transfection, electric pulses are applied to cells 2 times at intervals of 30 sec under such conditions as a voltage of 400 V, a capacitor of 3 μF and a time constant of 1.0 to 1.3 msec, using Gene Pulser® manufactured and sold by Bio-Rad Laboratories, USA.

The resultant cells transfected respectively with pSVMTNAP, pSVMTAP and pSVMTNAP3 are each separately cultured in Dulbecco's modified minimum essential medium containing 10 % FCS at 37 °C for 24 hours in an atmosphere of an air having a $CO_2$ concentration of 5 % . Then, each culture medium is replaced with a fresh one and culturing is further performed for 43 hours. Thereafter, the cells are scraped off by means of a scraper and sufficiently washed with PBS(-) to remove FCS contained in the medium from the surface of the cells. The thus obtained cells are subjected to extraction in a extraction buffer (0.1 M triethanolamine, 0.3 M NaCl, 0.01 M $CaCl_2$, pH7.8) using Dounce's homogenizer.

The resultant respective cell extracts are separately subjected to measurement of trypsin inhibiting activity by a combination of the method of Kassel et al [B. Kassel et al, Methods in Enzymology, 19, 844-852 (1970)] with the method of Kanaoka et al [Y. Kanaoka et al., Chemical & Pharmaceutical Bulletin (Japanese Society of pharmacy) 25(11), 3126-3128 (1977)]. That is, the measurement is conducted as follows. 0.1 ml of a porcine trypsin solution (obtained by dissolving 1 mg of a trypsin powder (manufactured and sold by Sigma Chemical Company, USA) in 5 ml of 2 mM hydrochloric acid), 0.2 ml of 0.01 M Tris-HCl buffer (pH8.0) and cell extract of such an amount as contain 100 μg of protein (if desired, the cell extract is diluted with 0.01 M Tris-HCl buffer) are put in a cuvette for spectrofluorophotometry and allowed to stand for 5 min. Then, to the resultant mixture is added a solution containing 1 mM N-α-benzoyl-DL-arginine-7-amido-4-methylcoumarin (BAMCA)(manufactured and sold by Sigma Chemical Company, USA) and 0.01 M Tris-HCl buffer (pH8.0) (prepared by dissolving BAMCA in 1 ml of dimethyl sulfoxide and then adding the resultant solution to 100 ml of 0.01 M Tris-HCl buffer) so that the total volume becomes 3.0 ml, followed by quick stirring. Then, the resultant mixture is irradiated with a light of 380 nm for exciting the mixture and the change rate in fluorescence at 440 nm is measured. The change rate in fluorescence represents the hydrolysis ratio of BAMCA. That is, the measurement of the change rate in fluorescence gives the trypsin activity remaining in the mixture. From the remaining trypsin activity, the trypsin inhibiting activity of each of plasmids pSVMTNAP and pSVMTNAP3 can be evaluated. Two series of the above-mentioned procedures are conducted, namely, Experiment A and Experiment B. In Experiment A, transfections of plasmid pSVMTNAP and pSVMTAP are conducted twice (Experiment A-1 and Experiment A-2, respectively). The above-mentioned measurement of the change rate in fluorescence is conducted 8 times with respect to the cell extract of Experiment A-1 and 5 times with respect to the cell extract of Experiment A-2. On the other hand, in Experiment B, transfections of plasmid pSVMTNAP3 and pSVMTAP are also conducted twice (Experiment B-1 and Expreiment B-2, respectively). The measurement of the change rate is conducted 5 times with respect to the cell extract of Experiment B-1 and 4 times with respect to the cell extract of Experiment B-2.

On the other hand, a cell extract is obtained from COS-1 cells which are not transfected with any plasmid but treated with electric pulses, and this control cell extract is subjected to measurement of the change rate in fluorescence in the same manner as mentioned above, to determine the trypsin activity with respect to the control cell extract.

The trypsin activity obtained with respect to each of the cell extracts derived from the cells containing plasmids pSVMTAP, pSVMTNAP and pSVMTNAP3, respectively, is compared with that obtained with respect to the the control cell extract derived from the cell containing no plasmid, and expressed in terms of the ratio (%) relative to the trypsin activity obtained with respect to the control cell extract. The averages and standard deviations of the obtained trypsin activities are shown in Tables 1(a) and (b).

Incidentally, it is noted that the trypsin activity obtained with respect to the control cell extract is 45 to 58 % relative to the trypsin activity of the system consisting of a porcine trypsin solution and BAMCA. This is due to the trypsin inhibiting activity of cell-line COS-1 itself.

| Table 1(a) | | | |
|---|---|---|---|
| Plasmid | None | pSVMTAP | pSVMTNAP |
| Experiment A-1 | 100.0±15.0 | 94.0±12.4 | 29.0±15.9** |
| Experiment A-2 | 100.0±10.4 | 101.0±5.5 | 36.8±23.5* |
| Table 1(b) | | | |
| Plasmid | None | pSVMTAP | pSVMTNAP3 |
| Experiment B-1 | 100.0±13.2 | 102.0±8.0 | 31.4±14.5** |
| Experiment B-2 | 100.0±11.0 | 98.5±3.6 | 34.3±22.9* |

Note:
1) The values are average ± standard deviation
2) **: level of significance is more than 99 % as examined by t-test, and
*: level of significance is more than 95 % as examined by t-test.

As apparent from Table 1, with respect to each of the extracts derived from pSVMTNAP-transfected cells and pSVMTNAP3-transfected cells, the trypsin activity is significantly lowered as compared to those obtained with respect to the extracts from pSVMTAP-transfected cells and cells containing no plasmid.

Example 3

(Expression of a cDNA coding for a fused protein comprised of a peptide having a protease inhibiting activity, which is encoded by a portion of NAP cDNA, and a human tumor necrosis factor (TNF) in Escherichia coli)

Step 1

(Subcloning of a portion of NAP cDNA coding for a peptide having a protease inhibiting activity)
A plasmid for the preparation of an RNA probe is prepared in accordance with the flow chart shown in Fig. 12(A). In Fig. 12 (A), the black portion indicates a portion of a base sequence corresponding to the sequence of the 913rd to 1137th bases in the DNA sequence shown in Fig. 1(A).

3 µg of plasmid pGBP2 is digested with AccI and XhoI and subjected to 1 % low melting point agarose gel electrophoresis, followed by extraction and purification of the resultant AccI-XhoI fragment of 900 bp from the gel. Then, the fragment is digested with XhoII at 37 °C for 1.5 hours. The conditions of the resultant reaction mixture is adjusted for TaqI digestion and, the fragment in the mixture is digested with TaqI at 65 °C for 1 hour. The resultant reaction mixture is extracted with phenol and subjected to buffer solution exchange by rapid gel filtration in accordance with the method described in the manual of Maniatis et al. Then, the resultant mixture is subjected to 1 % low melting point agarose gel electrophoresis, followed by extraction and purification of the resultant TaqI-XhoII fragment of about 230 bp from the gel.

Separately, 2 µg of plasmid pSP6/T7-19 having SP6 promoter and T7 promoter (manufactured and sold by Bethesda Research Laboratories, Inc., U.S.A.) is digested with AccI and BamHI and subjected to 1 % low melting point agarose gel electrophoresis, followed by extraction and purification of the resultant vector fragment from the gel.

The above-obtained TaqI-XhoII fragment and the vector fragment are reacted with each other using T4 ligase in a ligation buffer in the presence of ATP at 15 °C for 2 hours so that the TaqI-XhoII fragment and the vector fragment are ligated. Using the resultant reaction mixture, Escherichia coli MC1061 is transformed and cultured in accordance with the description of the manual of Maniatis et al., thereby obtaining colonies of ampicillin resistant transformants. From these colonies, 12 colonies are arbitrarily selected and subjected to rapid, small-scale isolation of plasmid DNA. The resultant plasmid isolated from each of the colonies is digested with ScaI and subjected to agarose gel electrophoresis. From the results of the electrophoresis, 3 clones out of 12 clones are found to be the desired clones which have a plasmid comprising a fragment of about 1.5 kb and a fragment of about 1.7 kb. The thus obtained plasmid contained in the 3 clones is designated "plasmid pSPGP2TX".

This plasmid comprises a base sequence of a portion of the NAP cDNA, coding for a peptite having a protease inhibiting activity (hereinafter this peptide is often referred to simply as "PI"), which base sequence is located downstream of the T7 promoter, and has such a structure that a sense RNA can be synthesized using T7RNA polymerase in vitro and that an anti-sense RNA can be synthesized using SP6RNA polymerase in vitro.

Step 2

(Construction of an expression plasmid having a trp promoter)

Plasmid pHNtrp535 which is capable of expressing human tumor necrosis factor (hereinafter referred to as "TNF") under the control of a trp promoter is constructed as follows.

In accordance with the method of Shirai et al (T. Shirai et al, Nature, 313, 803-806 (1985)), plasmid pHNtac4 is prepared. The plasmid is cleaved by EcoRI and HincII and subjected to electrophoresis to thereby obtain a DNA fragment of about 220 bp having a portion of a tac promoter.

Separately, four DNA fragments, TRP-1, TRP-2, TRP-3 and TRP-4, respectively having the following base sequences are organo-chemically synthesized:

**TRP-1** 5'-GACAATTAATCATCGAACTAGTTAACT-3'

**TRP-2** 5'-AGTACGCAAGTTCACGTAAAAAGGGTATCG-3'

**TRP-3** 5'-GATCCGATACCCTTTTTACGTGAACTTG-3'

**TRP-4** 5'-CGTACTAGTTAACTAGTTCGATGATTAATTGTC-3'.

The synthesized DNA fragments and the above-obtained DNA fragment are ligated to each other by means of T4 DNA ligase. The ligation product is cleaved by EcoRI and BamHI and subjected to electrophoresis to thereby obtain a trp promoter fragment of about 270 bp.

On the other hand, plasmid pHNtac4 is cleaved by XhoII and HindIII to obtain a DNA fragment of about 600 bp containing human TNF gene. The thus obtained DNA fragment is ligated to the above-obtained trp promoter fragment and ECORI-HindIII-cleaved plasmid pBR327 (X. Soberon et al, Gene, 9, 287-296 (1980)) by means of T4 ligase. With the ligation product, cells of E. coli MC1061 are transformed. From the thus obtained transformants, plasmids are isolated and subjected to restriction analysis to confirm that in the plasmid, the human TNF gene is inserted downstream of the trp promoter. The thus obtained plasmid is designated "plasmid pHNtrp535".

Step 3

(Construction of a plasmid for expression of PI)

Plasmid pPItrp314 comprising a trp promoter and, ligated to downstream thereof, an initiation codon ATG and a DNA coding for PI located, is prepared in accordance with the flow chart shown in Fig. 12(B). The preparation of the plasmid pPItrp314 is as follows.

Plasmid pUC18(manufactured and sold by Takara Shuzo Co., Ltd., Japan) is cleaved by HindIII and BamHI to obtain a HindIII-BamHI fragment of about 2.7 kb.

On the other hand, plasmid pHNtrp535 is cleaved by HindIII and EcoRI to obtain a HindIII-EcoRI fragment of about 250 bp containing a trp promoter.

Further, human transforming growth factor β (hereinafter referred to as "TGF-β") cDNA is isolated from cDNA's prepared from poly(A)RNA of epidermal carcinoma cell line KB (ATCC CCL17) in accordance with the method of Derynck et al (R. Derynck et al, Nature, 316, 701-705, (1985)). To the portion of cDNA coding for mature TGF-β are ligated a fragment having a BamHI-cleaved end and an initiation codon ATG at its 5′-end, and a fragment having a HindIII-cleaved end at its 3′-end, to thereby obtain a DNA fragment of about 0.7 kb.

The above-obtained fragments are ligated to each other to obtain expression plasmid pTGFβtrp. Plasmid pTGFβtrp is cleaved by NcoI, and both ends of the cleaved plasmid are made blunt by means of DNA polymerase I Klenow fragment. The resultant plasmid is cleaved by EcoRI and subjected to electrophoresis to thereby obtain a vector fragment containing a trp promoter.

On the other hand, plasmid pSPGP2TX prepared in Step 1 is cleaved by restriction enzyme TaqI, and both ends of the cleaved plasmid are made blunt by means of DNA polymeraseI Klenow fragment (manufactured and sold by Takara Shuzo Co., Ltd., Japan). The resultant cleaved plasmid is digested with EcoRI. The resultant fragment is subjected to 1 % low melting point agarose gel electrophoresis, and a fragment of about 240 bp containing a portion of cDNA coding for PI is obtained from the gel. The above-obtained fragments are ligated to each other using T4 ligase to prepare a plasmid. The plasmid is transferred into Escherichia coli JM105 to obtain a transformant. The thus obtained plasmid is designated "plasmid pPItrp314".

Using a M13 sequencing kit (manufactured and sold by Takara Shuzo Co., Ltd., Japan), a base sequence of a portion of plasmid pPItrp314 is determined. As a result, it is confirmed that the DNA coding for PI is ligated to an initiation codon ATG and a codon for Arg, constituting a reading frame, and that the codon ATG is located downstream from the trp promoter.

Step 4

(Construction of a plasmid capable of expressing a DNA coding for a fused protein of PI and human TNF)

A synthetic linker (Xa link AS) having a base sequence shown in Fig. 12(C) is organo-chemically prepared. The linker has an AvaI site at one end and an SstI site at the other end and codes for an amino acid sequence which is recognized and cleaved by factor Xa (K. Nagai and C. Thogersen, Nature, 309, 810-812, (1984).

Plasmid pPItrp314 obtained in Step 1 is cleaved by restriction enzymes AvaI and SstI and subjected to 1 % low melting point agarose gel electrophoresis, followed by recovery of the resultant DNA fragment from the gel. The DNA fragment is ligated with the above-obtained synthetic linker to prepare plasmid pPIXa3142. The procedure for the construction of the plasmid is shown in Fig. 12 (C).

Separately, plasmid pHNtrp535 is cleaved by SstI and PstI and subjected to 1 % low melting point agarose gel electrophoresis, to obtain a fragment of about 3 kb. Plasmid pHNtrp535 is separately cleaved by PstI and HindIII and subjected to electrophoresis to obtain a fragment of about 0.9 kb.

The thus prepared fragments are ligated with a fragment of about 0.5 kb prepared by cleaving the above-mentioned pPIXa3142 by HindIII and SstI. The resultant plasmid is transferred into Escherichia coli MC1061. This plasmid comprises a trp promoter and, located downstream thereof, an initiation codon ATG, a codon for Arg and a DNA coding for a fused protein comprised of a peptide of 71 amino acids containing PI, a peptide having an amino acid sequence recognized by factor Xa and a human TNF. With this plasmid construction, the DNA coding for a fused protein is capable of being expressed in Escherichia coli. This plasmid is designated "plasmid pIXTtrp726".

Step 5

(Production of a fused protein by gene expression in Escherichia coli)

Plasmid pIXTtrp726 obtained in Step 4 is transferred into cells of Escherichia coli Y1089 (manufactured and sold by Funakoshi Phermaceutical Co., Ltd., Japan) to obtain transformants. The transformants are cultured using L medium containing 50 µg/ml of ampicillin and 5 µg/ml of tetracycline at 37 °C overnight. 1 ml of the resultant culture is added to 50 ml of M9 culture medium containing 50 µg/ml of ampicillin, 5 µg/ml of tetracycline, 1 % of Casamino acid and 0.2 % of glucose and cultured at 37 °C for 24 hours. 2 ml of the resultant culture is subjected to centrifugation to collect the transformants. The transformants are suspended in 20 mM Tris-hydrochloric acid (pH7.5). The suspension is subjected to ultrasonication using Ohtake sonicator (manufactured and sold by Otake Seisakusho, Japan) to lyse the transformants, and subjected to centrifugation to obtain a supernatant. The activity of TNF contained in the supernatant is assayed by the criterion of the cytotoxicity against a mouse fibroblast L cell according to a modification of the method of Ruff et al (M.R. Ruff and G.E. Gifford, J. Immunol, 126, 1279, (1981)).

Separately, substantially the same procedure as mentioned above is repeated except that plasmid pBR327 is used as a control instead of plasmid pIXTtrp726, to thereby determine the TNF activity.

As a result, it is found that the supernatant derived from the transformant containing pIXTtrp726 exhibits 338 units of cytotoxicity against L cell per ml of the supernatant, whereas the supernatant derived from the transformant containing pBR327 as a control scarcely exhibits cytotoxicity against L cell.

An aliquot of the above-obtained transformants containing pIXTtrp726 is boiled in SDS-sample buffer and subjected to SDS-polyacrylamide gel electrophoresis using 15 % polyacrylamide gel in accordance with the method of Laemmli (U.K. Laemmli, Nature, 227, 680-685, (1970)). After the gel electrophoresis, the resultant is transferred from the gel to a nitrocellulose filter using Trans Brot® cell (manufactured and sold by Bio-Rad Laboratories, U.S.A.) in accordance with the protocol provided for the product. The filter is subjected to immunodetection of TNF using anti-human TNF rabbit antiserum in accordance with Immunodetection Application Guide of Bethesda Research Laboratories Inc., U.S.A. As a result, it is found that color development appears at a band portion of 28 kilodalton which is assumed to correspond to a fused protein comprising PI, a factor Xa-recognizing sequence and human TNF. Thus, the production of a fused protein by gene expression in Escherichia coli is confirmed.

Step 6

(Purification of a fused protein)

The transformant Escherichia coli Y1089 containing pIXTtrp726 obtained in Step 5 is cultured in 2 ℓ of the same culture medium as used in Step 5 at 37 °C for 24 hours. The resultant culture is subjected to centrifugation to collect the transformants. The transformants are suspended in 200 ml of a solution containing 20 mM Tris-hydrochlolic acid (pH7.5) and 1 mM phenylmethanesulfonyl fluoride (PMSF). The transformants are lysed by means of Manton-Gaulin press (manufactured and sold by Gaulin Corp., USA) and subjected to centrifugation to collect a supernatant.

The thus obtained supernatant is applied to a column (1 cm in diameter, 10 cm in length) packed with Sepharose 4B (manufactured and sold by Pharmacia Fine Chemicals AB, Sweden) to which an anti-human TNF monoclonal antibody is immobilized, at a flow rate of 2 ml/hour to cause a fused protein in the supernatant to be adsorbed on the column. The column is then washed with 40 ml of PBS(-) 4 times. Then, the fused protein is eluted out with 20 ml of PBS(-) containing 1M potassium thiocyanate (KSCN). The resultant eluate is desalted and concentrated using Centricon® 10 (manufactured and sold by Amicon Corp., USA) to thereby obtain 1 ml of a solution of the fused protein in 20 mM Tris-hydrochloric acid (pH7.5).

The trypsin inhibiting activity and cytotoxicity against L cell of the thus obtained purified fused protein are measured. The fused protein exhibits a trypsin inhibiting activity comparable to that of 106 ng/ml of BPTI and has 328 units/ml of cytotoxicity against L cell.

Example 4

(Construction of a plasmid capable of highly expressing the DNA coding for PI, and expression of the DNA)

Step 1

(Construction of a plasmid capable of highly expressing the DNA coding for PI)

Plasmid pIXTtrp726 obtained in Step 4 of Example 3 is cleaved by restriction enzyme StuI, and ligated to XbaI linker DNA (5′-CTCTAGAG-3′) using T4 ligase. The resultant DNA is cleaved by XbaI and EcoRI and subjected to 1 % low melting point agarose gel electrophoresis, followed by recovery and purification of the resultant fragment of about 0.5 kb from the gel. The fragment is inserted in plasmid pUC19 which is cleaved by XbaI and EcoRI. The resultant plasmid is designated "plasmid pPItrp75-1". The plasmid is transferred into Escherichia coli MC1061. As shown Fig. 12(E), this plasmid which is capable of expressing the DNA coding for PI and which comprises a trp promoter and, located downstream thereof, an initiation codon an Arg codon, a base sequence which codes for a PI-containing an amino acid sequence of 71 amino acids, an Arg codon, a Leu codon and a stop codon.

Next, a plasmid containing 16 units of the DNA coding for PI which is capable of highly expressing PI is prepared in accordance with the flow chart shown in Fig. 12(F). Illustratively stated, the above-mentioned plasmid pPItrp75-1 is cleaved by restriction enzymes SpeI and PstI and subjected to 1 % low melting point agarose gel electrophoresis, followed by recovery and purification to obtain a SpeI-PstI DNA fragment of about 270 bp containing a ribosome binding site and DNA coding for PI. The thus obtained fragment is inserted in a XbaI-PstI DNA fragment prepared by cleaving plasmid pPItrp75-1 by XbaI and PstI. In this insertion reaction, the SpeI cleaved end of the SpeI-PstI DNA fragment and the XbaI cleaved end of the XbaI-PstI DNA fragment are ligated to each other so that the sensitivity of the junction site to enzymes SpeI and XbaI disappears. Using the reaction mixture containing the thus prepared plasmid, Escherichia coli HB101 is transformed. The plasmid isolated from the thus obtained transformant is designated "plasmid pPItrp75-2" [Fig. 12(F)].

Thereafter, the DNA fragment which contains the ribosome binding site and DNA coding for PI is doubled repeatedly in accordance with the flow chart shown in Fig. 12(G) to thereby obtain plasmid pPItrp 75-16 which comprises 16 DNA units containing a ribosome binding site and DNA coding for PI.

Step 2

(Expression of the DNA coding for PI in Escherichia coli containing plasmid pPItrp75-16)

Plasmid pPItrp75-16 obtained in Step 1 is transferred into cells of Escherichia coli JM109 (manufactured and sold by Funakoshi Pharmaceutical Co., Ltd., Japan). Then, the resultant transformant is cultured, lysed and subjected to centrifugation to collect a supernatant in accordance with the procedure as described in Step 5 of Example 3. To 2 ml of the supernatant is added 8 ml of acetone. The resultant mixture is allowed to stand at -20 °C for 1 hour and then subjected to centrifugation. The resultant precipitate is collected. The precipitate is suspended in 1 ml of 20 mM Tris-hydrochloric acid (pH7.5) and the suspension is subjected to centrifugation to collect a supernatant, thereby removing insoluble impurities.

The trypsin inhibiting activity of the thus obtained supernatant is measured in the same manner as in Example 3. BPTI (manufactured and sold by Sigma Chemical Co., USA) is used as a standard sample for the measurement of trypsin inhibiting activity and a cell extract obtained from a transformant containing plasmid pBR327 is used as a control. As a result, it is found that the cell extract derived from the transformant containing pBR327 exhibits trypsin inhibiting activity comparable to 68 ng/ml of BPTI, whereas the supernatant obtained from the transformant containing plasmid pPItrp75-16 exhibits trypsin inhibiting activity comparable to 235 ng/ml of BPTI.

Example 5

(Expression of secretable PI, NAP and NAP3 in animal cell)

Step 1

(Construction of plasmid capable of expressing the DNA's respectively coding for secretable PI, NAP and NAP3)

Plasmid pSVMTAP and plasmid pSVMTNAP obtained in Example 2 are each separately cleaved by KpnI and BglII and subjected to 1 % low melting point agarose gel electrophoresis to obtain a fragment of 1.7 kb derived from plasmid pSVMTAP and a fragment of 1.9 kb derived from plasmid pSVMTNAP, both of which contain neither a base sequence coding for a membrane domain nor a base sequence coding for a cytoplasmic domain. Each of the fragment of 1.7 kb and the fragment of 1.9 kb is separately ligated to a fragment prepared by cleaving plasmid pUC18 by KpnI and BamHI to obtain a plasmid containing AP DNA and a plasmid containing NAP DNA. Each of the obtained plasmids is transferred into cells of Escherichia coli JM105 (manufactured and sold by Phamacia Fine Chemicals AB, Sweden). The plasmid containing AP DNA and the plasmid containing NAP DNA are respectively designated "plasmid pAPKB1865" and "plasmid pAPKB1877" [see Fig. 12(H)]. As shown in Fig. 12(H), each of the plasmids has a Leu codon and a stop codon derived from plasmid pUC18 which are located downstream of the BglII-BamHI junction at which KpnI-BamHI fragment is inserted.

The above-obtained plasmids pAPKB1865 and pAPKB1877 are separately cleaved by KpnI and HindIII and subjected to 1 % low melting point agarose gel electrophoresis to obtain a fragment of 1.7 kb and a fragment of 1.9 kb which are derived from plasmids pAPKB1865 and pAPKB1877, respectively. Separately, pSVMTNAP is cleaved by KpnI and HindIII and subjected to 1 % low melting point agarose gel electrophoresis to obtain a fragment of about 3 kb. To the thus obtained fragment as a vector is ligated each of the above-obtained fragment of 1.7 kb and the fragment of 1.9 kb. Each of the resultant plasmids is separately transferred into cells of Escherichia coli. The resultant plasmid derived from AP DNA and the plasmid derived form NAP DNA are respectively designated "plasmid pSVMT592" and "plasmid pSVMT667" [see Fig. 12(I)].

Plasmid pBB3 obtained in Step 3 of Example 1 which codes for the N-terminal amino acid sequence of NAP3 is cleaved by KpnI and BamHI and subjected to 1 % low melting point agarose gel electrophoresis to obtain a fragment of about 1.4 kb. Separately, plasmid pSVMT667 is cleaved by KpnI and HindIII and subjected to 1 % low melting point agarose gel electrophoresis to obtain a fragment of about 3 kb. Plasmid pSVMT667 is also cleaved by BamHI and HindIII and subjected to 1% low melting point agarose gel electrophoresis to obtain a fragment of 0.4 kb. The thus obtained three fragments are ligated to each other to obtain a plasmid. The plasmid is transferred into Escherichia coli MC1061 (manufactured and sold by Pharmacia Fine Chemicals AB, Sweden; ATCC 53338). The plasmid is designated "plasmid pSVMT648" [see Fig. 12(J)].

Plasmid pSVMT592, pSVMT648 and pSVMT667 are respectively capable of expressing the DNA's coding for secretable proteins of AP, NAP3 and NAP, in which proteins a transmembrane domain and a cytoplasmic domain are not contained.

Step 2

(Production of secretable AP, NAP and NAP3 in COS-1 cell)

With 20 μg of plasmid pSVMT667, 4 x $10^6$ of COS-1 cells are transfected in a solution of 272 mM sucrose, 7 mM sodium phosphate (pH7.4) and 1 mM $MgCl_2$ by applying an electrical pulse twice at a condenser capacity of 3 μF at a voltage of 400V using Gene Pulser® (manufactured and sold by Bio-Rad Laboratories, U.S.A.). The transfected cells are cultured in a Dulbecco's modified minimum essential medium containing 10 % FCS at 37 °C in an air containing 5 % of $CO_2$ for 24 hours. The cultured cells are then washed with PBS(-) to remove serum (FCS). The washed cells are transferred to a culture medium containing no serum and cultured for 48 hours. The resultant culture medium (hereinafter referred to as "supernatant 1") is collected and a fresh medium is added to the cells. The cells are further cultured for 48 hours. Then, the culture medium (hereinafter referred to as "supernatant 2") is recovered. The trypsin inhibiting activities in supernatant 1 and supernatant 2 are measured as follows.

50 μl of a supernatant is serially diluted with a PBS containing 1 % bovine serum albumin (BSA, manufactured and sold by Sigma Chemical Company, USA) on a plate having 96 wells so that 50 μl of samples diluted from 2 to 1024 times are prepared. 50 μl of a trypsin solution having a concentration of 1.25 unit/ml (manufactured and sold by Sigma Chemical Company, USA) is added to each of 50 μl of the diluted samples. The resultant mixtures are allowed to stand at room temperature for 10 minutes and then to the mixtures is added 100 μl of a solution prepared by dissolving 1.8 mg/ml Nα-Benzoyl-DL-Arginine-p-Nitroamilide Hydrochloride (hereinafter referred to as "BAPNH") (manufactured and sold by Sigma Chemical Company, USA). The resultant mixtures are allowed to stand at 37 °C overnight. Then, the absorbance at 405 nm of the resultant is measured using Multiskan® (manufactured and sold by Titertech Co., USA). An absorbance versus dilution rate is plotted on a graph. From the graph, the dilution rate (Ic50) which give a half of the net value of absorbance obtained from the background absorbance value and the maximum absorbance value is determined. At the same time, an absorbance versus dilution rate is plotted on a graph with respect to a BPTI solution having a BPTI concentration of 400 ng/ml. From the graph, Ic50 is determined with respect to BPTI. The trypsin inhibiting activity of the supernatant is calculated in accordance with the following formula:

$$\text{Trypsin inhibiting activity (ng/ml)} = \frac{\text{Ic50(supernatant}}{\text{Ic50(BPTI)}} \times 400$$

When trypsin inhibiting activity of the original sample is low, the supernatant is concentrated using Centricon® (manufactured and sold by Amicon Co., USA).

The trypsin inhibiting activities of supernatants 1 and 2 are shown in Table 2.

Substantially the same procedure as mentioned above is repeated except that plasmids pSVMT648 and pSVMT592 are separately used instead of plasmid pSVMT667, to determine the trypsin inhibiting activities of plasmids pSVMT648 and pSVMT592. The results are also shown in Table 2.

Table 2

| | pSVMT667 | pSVMT648 | pSVMT592 |
|---|---|---|---|
| Supernatant 1 | 150ng/ml | 40ng/ml | 3ng/ml |
| Supernatant 2 | 110ng/ml | 20ng/ml | 4ng/ml |

Example 6

(Production and secretion of a fused protein comprised of PI and human TNF by an animal cell)

Step 1

(Construction of plasmid for expression of DNA coding for a fused protein of PI and human TNF)

In order to express the DNA coding for a fused protein obtained in Step 4 of Example 3 in an animal cell to produce the fused protein and secrete the fused protein out of the cell, plasmid pSVMT-tPA is used. The plasmid pSVMT-tPA is constructed using the following DNA fragments:

(1) a BglII-HindIII DNA fragment of about 3.7 kb derived from plasmid pSVMT-103 and having an mMT promoter, SV40 replication origin, gene for ampicillin resistance, replication origin of plasmid pBR322 and SV40 transcription termination sequence; and
(2) a DNA fragment having such a structure that a BamHI-cleaved site and a HindIII-cleaved site are respectively attached to the ends of a DNA fragment of about 2.2 kb isolated from a human tPA cDNA in accordance with Pennica et al method (D. Pennica et al, Nature, 301, 214-221 (1983)).

Plasmid pSVMT-tPA is cleaved by BglII and HindIII and subjected to electrophoresis to obtain a fragment of about 3.7 kb.

On the other hand, pIXTtrp726 obtained in Step 4 of Example 3 is cleaved by restriction enzymes BamHI and HindIII and subjected to 1 % low melting point agarose gel electrophoresis to obtain a fragment of about 0.8 kb.

The thus obtained two fragments are ligated in accordance with the flow chart shown in Fig. 12(K) to prepare a plasmid. The plasmid is transferred into Escherichia coli MC1061. The plasmid is designated "plasmid pSVMT-PIXT". This plasmid comprises an mMT promoter, and ligated to downstream thereof, DNA coding for a signal sequence of tissue plasminogen activator (t-PA), and a DNA coding for a fused protein comprised of PI, a factor Xa-recognizing sequence and human TNF.

Step 2

(Production and secretion of a fused protein by COS-1 cell)

Plasmid pSVMT-PIXT obtained in Step 1 and plasmid pSVMT-tPA as a control are each separately transfected in COS-1 cells in accordance with the method in Step 2 of Example 5. The transfected COS-1 cells are cultured in a culture medium. The culture medium is collected and a fresh one is added to the cells 3 times every 2 days. The collected media are respectively designated "supernatants 1, 2 and 3" in the obtaining order. The trypsin inhibiting activities and TNF activities respectively in terms of BPTI and cytotoxicity against L cell of each of the supernatants are respectively measured in accordance with the same methods as in Step 2 of Example 5 and as in Step 5 of Example 3. The results are shown in Table 3.

Table 3

| | | Trypsin inhibiting activity | TNF activity |
|---|---|---|---|
| pSVMT-PIXT | Supernatant 1 | 184 ng/ml | 172units/ml |
| | Supernatant 2 | 316 ng/ml | 287units/ml |
| | Supernatant 3 | 242 ng/ml | 206units/ml |
| pSVMT-tPA | Supernatant | <37 ng/ml | <20units/ml |

Separately, each of the supernatants is concentrated using Centricon®. An aliquot of each of the concentrated supernatants is subjected to SDS-polyacrylamide gel electrophoresis. After the electrophoresis, proteins in the gel are stained with Coomassie Brilliant Blue. As a result, it is found that color development appears at a band portion corresponding to 28 to 31 kilodalton on the lanes of the supernatants derived from pSVMT-PIXT, whereas no band appears at such a position on a lane of the supernatant derived from the control plasmid. Further, immunodetection of TNF is conducted using anti-human TNF rabbit antiserum in accordance with the same method as in Step 4 of Example 3. As a result, an immunoreactive band is detected at the band portion of about 28 to 31 Kilodalton.

From the above results, it is confirmed that a fused protein is produced and secreted by COS-1 cells and that the fused protein exhibits trypsin inhibiting activity and TNF activity.

Example 7

(1) (Expression of a DNA coding for secretable PI in animal cell and production of the same on a large scale)

Step 1

(Construction of plasmid capable of expressing for secretory PI)

Plasmid pPItrp75-1 is cleaved by BamHI and HindIII and subjected to 1 % low melting point agarose electrophoresis to obtain a fragment of about 240 bp. The thus obtained fragment is ligated to the vector fragment obtained in Step 1 of Example 6 by digesting pSVMT-tPA by BglII and HindIII to prepare a plasmid. The plasmid is transferred into Escherichia coli MC1061. The plasmid is designated pSVMT-APPI [see Fig. 12(L)].

This plasmid comprises an mMT promoter, and ligated to downstrem thereof, a DNA coding for a protein of 111 amino acids which comprises a signal peptide of t-PA and a PI-containing amino acid sequence [see Fig. 12(M)].

Step 2

(Expression of secretable PI in COS-1 cell)

Plasmid pSVMT-APPI is transferred in COS-1 cells in the same manner as in Step 2 of Example 5 to obtain transfectants. The transfectants are cultured in a culture medium. The culture medium is collected and a fresh medium is added to the cells 4 times every 2 days so that four supernatant media are obtained. The trypsin inhibiting activities of the supernatant media are measured in the same manner as in Step 2 of Example 5. As a result, it is confirmed that the four supernatant media exhibit trypsin inhibiting activities respectively corresponding to 844, 844, 880 and 511 ng/ml of BPTI.

Another aliquot of each of the supernatant media is fractionated by means of Centricon 10 and the presence or absence of a trypsin inhibiting activity is determined with respect to each fraction. As a result, it is found that a trypsin inhibiting activity is present in the fraction having a molecular weight of 10 kilodalton or more. The fraction is subjected to SDS-polyacrylamide gel electrophoresis, followed by staining with a Coomassie Brilliant Blue. The results are compared with the results of the SDS-polyacrylamide gel electrophoresis and Coomassie Brilliant Blue staining as obtained in step 2 of Example 6 with respect to the supernatant of the culture of COS-1 cells transfected with plasmid pSVMT-tPA as a control. As a result, there is detected at a position corresponding to the molecular weight of 15 to 19 kilodalton a band which is not detected in the case of the supernatant of the culture of COS-1 cells transfected with plasmid pSVMT-tPA and which is thus considered to be ascribed to the secreted PI (hereinafter referred to as "sPI"). Since the sPI has an estimated molecular weight of about 8 kilodalton after secretion, the above results suggest that the sPI is glycosylated in COS-1 cells. Then, the fraction is subjected to SDS-polyacrylamide gel electrophoresis, followed by periodic acid-Schiff staining for detecting a sugar chain. As a result, it is found that the above-mentioned band portion is strongly stained, and the glycosylation of sPI is confirmed.

Step 3

(Mass production of the sPI)

COS-1 cells are transfected with plasmid pSVMT-APPI by the method described in step 2 of Example 5. The transfection procedure is effected forty times in total and the medium is renewed three times at intervals of two days. Thus, 1.2 ℓ of a supernatant of the medium is obtained.

To the obtained supernatant is added acetone to a final concentration of 40 %. The resultant mixture is allowed to stand at -20 °C for 1 hr and then subjected to centrifugation at 5000 rpm at -10 °C for 20 min. After the centrifugation, the supernatant is collected.

To the collected supernatant is added acetone to a final concentration of 80 % and the resultant mixture is allowed to stand at -20 °C for 1 hr. The mixture is then subjected to centrifugation and the formed precipitate is collected.

The precipitate is suspended in 50 ml of 20 mM Tris-HCl (pH 7.5). The resultant suspension is subjected to centrifugation at 5000 rpm at 4 °C for 10 min and the formed supernatant is collected.

To 50 ml of the thus obtained supernatant are added 150 units of TPCK-treated insoluble trypsin attached to beaded agarose (produced and sold by Sigma Chemical Company, U.S.A.). The resultant mixture is shaken at 37 °C for 10 min to cause the inhibitor to be adsorbed on the beaded agarose. The thus obtained suspension is charged into Select S Column (trade name of a column produced and sold by 5′prime- 3′prime Co., Ltd., USA) and the beaded agarose is collected. The separation of the beaded agarose from the buffer is effected by centrifugation at 1000 rpm for 1 min. The collected beaded agarose is subjected to elution three times with 6 ml of an eluent consisting of 0.3 M NaCl, 10 mM $CaCl_2$ and 10 mM HCl (pH 2). The eluate obtained in each elution is neutralized with 5 N NaOH. The eluates obtained by three separate elutions are mixed and the mixture (18 ml) is concentrated while desalting, by means of Centricon 10 to obtain 1 ml of a PBS(-) buffer solution. The trypsin inhibiting activity of the sPI solution obtained in each purification step is assayed. The results are shown in Table 4 given below.

Step 4

(Analysis of the purified sPI)

An aliquot of the purified sPI solution obtained in step 3 of Example 7 is subjected to SDS-polyacrylamide gel electrophoresis, followed by staining with a Coomassie Brilliant Blue. As a result, there is obtained a broad single band corresponding to the molecular weight of 15 to 19 kilodalton. Separately, another aliquot of the purified PI solution obtained in step 3 of Example 7 is subjected to SDS-polyacrylamide gel electrophoresis, followed by staining according to the method of Zacharius et al [see Zacharius, R.M., et al, Anal. Biochem., 30, 148-152 (1969)]. As a result, it is found that the purified sPI obtained in step 3 of Example 7 is glycosylated.

In addition, the amino acid sequence of the purified sPI is determined up to the nineteenth amino acid as from the N-terminus by means of Gaseous Phase Protein Sequencer Model 470A (produced and sold by Applied Biosystems, USA), according to the protocol provided for the apparatus. As a result, it is found that the sPI contains peptides 1 and 2 respectively having the amino acid sequences shown in Table 5 in an amount ratio of 1:2.

Table 4

| Solution containing sPI | Vol. (ml) | Inhibitor concentration in terms of BPTI (μg/ml) | Inhibiting activity in terms of BPTI (μg) | Yield (%) |
|---|---|---|---|---|
| Supernatant obtained immediately after the incubation | 1200 | 0.56 | 670 | 100 |
| Supernatant collected in step (3) | 50 | 11 | 550 | 82 |
| Flow through | 50 | 0.5 | 25 | 4 |
| Fraction obtained by the first elution | 6 | 30.8 | 185 | |
| Fraction obtained by the second elution | 6 | 28.2 | 169 | 62 |
| Fraction obtained by the third elution | 6 | 10.5 | 63 | |
| Ultimate concentrate | 1 | 352 | 352 | 52 |

Table 5

| | |
|---|---|
| Peptide 1 | 1Gly-Ala-Arg-Ser-Met-Arg-Glu-Val- X -Ser- X - X -Ala- X -(Thr)-(Gly)- X - X - X19 |
| Peptide 2 | 1Ser-Met-Arg-Glu-Val- X -Ser-Glu-Gln -Ala-Glu-Thr-Gly-Pro- X - X -Ala -Met-Ile19 |

In the above amino acid sequences, the amino acids in the parenthesis are estimated amino acids and X represents undetermined amino acids. Further, the underline indicates the PI regions.

From the above results, it is apparent that after the secretable PI is expressed in COS-1 cells, the t-PA signal sequence thereof is cleaved at two sites whereas the regions having trypsin inhibiting activity are maintained.

(2) (Expression of a DNA coding for secretable PI in animal cell and production of the same on a large scale)

Step 1

(Construction of plasmid pSV2MDPI)

In accordance with the flow chart shown in Fig. 12 (N), plasmid pSV2MDPI containing a gene coding for dihydrofolate reductase (DHFR) and a DNA coding for secretory PI is constructed. Illustratively stated, plasmid pSVMT-APPI prepared in Step 1 of Example 7-(1) is digested with BamHI to obtain a fragment containing a DNA coding for PI. On the other hand, plasmid pSV2DHFR (ATCC 37146) is digested with BamHI and treated with alkaline phosphatase. The resultant plasmid is ligated to the above-obtained fragment to obtain a recombinant. With the recombinant, cells of E. coli JM105 are transformed. Thus, a recombinant plasmid containing the DHFR gene and the secretable PI DNA which are ligated in a proper direction is obtained. The plasmid is designated pDMPI. This plasmid is completely digested with BglII and, then, partially digested with BamHI to obtain a DNA fragment containing an SV40 poly(A)signal and a DNA coding for PI. This fragment is ligated to a vector fragment obtained by digesting plasmid pSVDHFR with BglII and treating the digest with alkaline phosphatase, to thereby obtain a recombinant in which the BglII-BamHI fragment is inserted in proper direction. The recombinant is designated "plasmid pSV2MDPI".

Step 2

(Transformation of CHO cell with plasmid pSV2MDPI and expression in the transformed cell)

About 4 μg of plasmid pSV2-neo(ATCC 37150) and about 20 μg of plasmid pSV2MDPI prepared in Step 1 of Example 7-(2) are mixed with each other, followed by precipitation with ethanol. The precipitate is air-dried and dissolved in 450 μl of TE solution (pH7.9, 1 mM Tris-HCl buffer, 0.1 mM EDTA). To the solution is added 500 μl of a 2 x HBS (50 mM HEPES, 280 mM NaCl, 1.5 mM $Na_2HPO_4$, pH 7.12). To the mixture is dropwise added 50 μl of 2.5 M $CaCl_2$, and the resultant mixture is kept on ice for 10 min. On the other hand, cells of CHO-KI strain (ATCC CCLD 61) are inoculated onto a plate for tissue culture which has a diameter of 6 cm and contained Ham's F-12 medium (manufactured by Flow Laboratories, Inc., U.S.A., catalog No. 10-421-20) containing 10 % (v/v) FCS and 1 % (v/v) penicillin-streptomycin (manufactured by Flow Laboratories, Inc., U.S.A., catalog No. 16-700-49) so that the amount of cells become about 5 x $10^5$ per plate. The inoculated cells were incubated at 37 °C overnight in an air containing 5 % $CO_2$.

Then, the medium is replaced with a fresh medium of the same kind, followed by incubation for 3 hours. Over the resultant CHO-KI cells, the above-mentioned plasmid DNA solution to which the $CaCl_2$ solution has been dropwise added is layered and the incubation is conducted at 37 °C for about 8 hours. Then, the plate is washed twice with 5 ml of PBS(-) (manufactured by Flow Laboratories, Inc., U.S.A., catalog No. 28-103-05) and further washed with 5 ml of the above-mentioned medium, and a fresh medium of the same type is added to the plate, followed by culturing for about 16 hours.

The resultant cells adhering to the plate are peeled off using a trypsin solution and transplanted onto 4 plates for tissue culture plate having a diameter of 10 cm, followed by incubation. 24 hours later, the medium is replaced with a selective medium. The selective medium has such a composition that to the above-mentioned medium is added 400 µg/ml of genetisin G-418 (manufactured by GIBCO, U.S.A., catalog No. 860-1811). The plates are cultured for about 2 weeks while replacing the medium with a fresh medium on every three or four days, to thereby perform the cloning of G418 resistant cells. One of the cell clones obtained by the above-mentioned procedure is separately grown on plates for tissue culture having a diameter of 10 cm until confluent growth is attained. The cells are washed with PBS (-) three times and a fresh medium containing no serum is added to the cells, followed by culturing for 2 days. After the culturing, the supernatant of the medium is collected and subjected to measurement of trypsin inhibiting activity in the same manner as in Step 2 of Example 5. As a result, it is found that the supernatant of the medium has a trypsin inhibiting activity corresponding to 87 ng/ml of BPTI.

The supernatant is subjected to purification in the same manner as in Step 3 of Example 7-(1) to obtain a fraction containing sPI. The fraction is subjected to SDS-polyacrylamide electrophoresis and PAS staining. As a result, it is found that the fraction gives a single band corresponding to the molecular weight of about 15 to 19 kilodaltons, and the PI in the fraction is glycosylated.

Example 8

[Preparation of a peptide having an amino acid sequence Arg-Ala-Met-Ile-Ser-Arg-Trp-Tyr-Phe-Asp-Val-Thr-Glu-Gly-Lys-Cys (corresponding to the amino acid seference of from the 301st to 316th amino acids in Fig.1(A))]

A peptide having the above-mentioned amino acid sequence is prepared by means of a peptide synthesizer (Model 430A produced and sold by Applied Biosystems, USA). The average yield is 97.9 %. The obtained peptide is purified by removing the protecting groups from the peptide with hydrogen fluoride, followed by ion exchange and then subjecting the peptide to reversed phase high-performance liquid chromatography. The chromatography is effected using ERC-ODS-1161 column and, as an eluent, a gradient of 0.1 % aqueous solution of trifluoroacetic acid (liquid A) and acetonitrile containing 0.1 % trifluoroacetic acid (liquid B). In practicing the elution, the volume ratio of liquid B to liquid A is increased from 0 % to 100 % at a flow rate of 2 ml/min for a period of 50 min. An eluate is repeatedly collected at the main peak, thereby obtaining about 20 mg of a purified peptide. The peptide is subjected to the analysis of amino acid composition, and the amino acid sequence of the peptide is analyzed by a gaseous phase peptide sequencer (Model 470A produced and sold by Applied Biosystems, USA). As a result, it is confirmed that the obtained peptide is the desired peptide.

Example 9

[Preparation of a peptide having an amino acid sequence Trp-Tyr-Phe-Asp-Val-Thr-Glu-Gly-Lys-Cys (corresponding to the amino acid sequence of the 307th to 316th amino acids in Fig.1(A)]

A peptide having the above-mentioned amino acid sequence is prepared, purified and analyzed in substantially the same manner as in Example 8. The average yield is 98.0 % and the total amount of the purified peptide obtained is about 16 mg.

Example 10

[Preparation of a peptide having the amino acid sequence Val-Thr-Glu-Gly-Lys-Cys (corresponding to the amino acid sequence of the 311st to the 316th amino acids in Fig.1(A)]

A peptide having the above-mentioned amino acid sequence is prepared, purified and analyzed in substantially the same manner as in Example 8. The average yield is 98.8 % and the total amount of the purified peptide obtained is about 10 mg.

Example 11

[Preparation of a peptide having an amino acid sequence Ser-Val-Glu-Glu-Val-Val-Arg-Glu-Val-Cys (corresponding to the amino acid sequence of the 282nd to 291st amino acids in Fig.1(A)]

A peptide having the above-mentioned amino acid sequence is prepared, purified and analyzed in substantially the same manner as in Example 8. The total amount of the purified peptide obtained is about 8 mg.

Example 12

(1) [Preparation of a peptide having an amino acid sequence Gln-Ala-Glu-Thr-Cys (corresponding to the amino acid sequence formed by adding Cys to the C-terminus of the amino acid sequence of the 294th to 297th amino acids in Fig.1(A))].

A peptide having the above-mentioned amino acid sequence is prepared and purified in substantially the same manner as in Example 8. The total amount of the purified peptide obtained is about 15 mg.

(2) [Preparation of a peptide having an amino acid sequence Gly-Ser-Ala-Met-Ser-Gln-Ser-Leu-Leu-Lys-Thr-Thr-Gln-Glu-Pro-Leu-Ala-Arg (corresponding to the amino acid sequence of the 342nd to 359th amino acids in Fig. 1 (A))].

Example 13

[Bonding to a carrier (KLH)]

KLH and MBS are reacted according to the method described in "Zoku-Seikagaku Jikken Koza 5, Men-eki Seikagaku Kenkyuho (Lectures on Experimental Biochemistry, Second series, Vol. 5, Immunological and biochemical Assay)", edited by the Japanese Society of Biochemistry, published by Tokyo Kagaku Dojin, Japan (1986), pp. 84-87, to bond the m-maleimide benzoic acid-N-hydroxysuccinimide (MBS) to the amino group of KLH by amide linkage, thereby obtaining MBS-acyl-KLH. The obtained substance is purified by gel filtration and reacted with each of the peptides obtained in Examples 8 to 11 and Example 12-(1) to obtain 5 types of KLH-bonded peptides. Hereinafter, the KLH-bonded peptides obtained from the peptides obtained in Examples 8, 9, 10, 11 and 12-(1) are respectively referred to as KLH-1, KLH-2, KLH-3, KLH-4 and KLH-5. The bonding efficiencies of the peptides [peptide/KLH-bonded peptide x 100 (%, w/w)] are 28.6 % for KLH-1, 20.5 % for KLH-2, 10.8 % for KLH-3, 21.4 % for KLH-4 and 11.4 % for KLH-5.

Example 14

[Preparation of antiserum]

To KLH-1 obtained in Example 13 is added PBS(-) to a final protein concentration of 1.0 mg/ml. To 500 µl of the thus obtained solution is added an equivolume of Freund's complete adjuvant and mixed well. The resultant mixture is injected to a rabbit (No. 1) subcutaneously four times at intervals of two weeks to immunize the rabbit. One weeks after the final injection, about 20 cc of blood is collected from the rabbit. From the collected blood, about 10 cc of serum is obtained.

The titer of the antiserum is assayed by a solid phase enzyme-linked immunosorbent assay (ELISA) using the peptide obtained in Example 8 as an antigen and o-phenylenediamine as a color developing reagent. The details of the assay are as follows. The peptide obtained in Example 8 is added to a 96-well microplate in an amount of 1 µg per well and allowed to stand for 1 hr at room temperature in sodium carbonate-bicarbonate buffer (pH 9.6) to cause the peptide to be adsorbed on the wells. The wells are washed three times with PBS(-) and treated with PBS(-) containing 5 % of fetal calf serum at room temperature for 1 hr. The above-obtained serum of the rabbit is diluted to various degrees as indicated in Table 6 to obtain various dilutions. The dilutions are each separately added to the wells of the microplate and allowed to stand at room temperature for 1 hr. The wells of the microplate is washed three times with PBS(-) and treated with peroxidase-labeled anti-rabbit IgG antiserum (goat) at room temperature for 1 hr. After the treatment, the wells of the microplate are washed with PBS(-) and each separately treated with a solution of o-phenylenediamine in a buffer containing citric acid at room temperature for 30 min to effect a reaction. The reaction is terminated by the addition of hydrochloric acid. The absorbance of the solution in each well is measured at 482 mm. Substantially the same procedure as mentioned above is repeated using another rabbit (No. 2). The results are shown in Table 6 given below.

To evaluate nonspecific reaction, antigen-free wells are also treated with antiserum in the same manner as described above.

Table 6

| | Absorbance at 492 mm | | | |
|---|---|---|---|---|
| Degree of Dilution | Antigen-free well | | Antigen 1 μg/well | |
| | Rabbit No. 1 | Rabbit No. 2 | Rabbit No. 1 | Rabbit No. 2 |
| X 200 | 0.035 | 0.029 | 2 | 1.693 |
| X 400 | 0.012 | 0.025 | 2 | 1.464 |
| X 800 | 0.018 | 0.016 | 1.761 | 0.943 |
| X 1600 | 0.013 | 0.000 | 1.401 | 0.633 |
| X 3200 | 0.007 | 0.015 | 0.857 | 0.297 |
| X 6400 | 0.048 | 0.045 | 0.547 | 0.179 |
| X 12800 | 0.006 | 0.034 | 0.365 | <u>0.116</u> |
| X 25600 | 0.032 | 0.040 | 0.184 | 0.083 |
| X 51200 | 0.040 | 0.039 | 0.127 | 0.052 |
| X 102400 | 0.033 | 0.039 | <u>0.116</u> | 0.067 |
| X 204800 | 0.051 | 0.056 | 0.175 | 0.055 |

As is apparent from the above, there are significant differences between the results obtained from the antigen-free wells and those obtained from the wells containing 1 μg antigen/well within the range of dilution up to X 102400 for rabit No. 1 and within the range of dilution up to X 12800 for rabit No. 2.

Example 15

(Purification of an antigen from HTB-14 cells)

ATCC HTB14 cells are cultured by the same method in Step 1 of Example 1. Then, about $1x10^9$ cells are collected from the culture medium by means of a scraper. The collected cells are lysed in PBS(-) (pH7.4) containing 1 % Triton X-100, 0.5 % deoxycholic acid and 10 mM EDTA in the presence of 2 mM phenylmethane sulfonyl fluoride (PMSF) as a protease inhibitor, and the residue of the cells are removed by centrifugation to obtain a cell solution. The cell solution is purified by gel filtration and DEAE column chromatography. Through the purification, the fraction which reacts with the antiserum obtained in Example 14 is concentrated step by step, to obtain a protein. The thus obtained protein reacted strongly with the antiserum obtained in Example 14.

The protein is subjected to partial sequencing of the amino acid sequence. The partial amino acid sequence thus obtained is identical with a portion of the amino acid sequence of INS 76.

Example 16

[Preparation of a monoclonal antibody]

KLH-1 obtained in Example 13 is added to PBS(-) to obtain a solution having a protein concentration of 1 mg/ml. To 750 μl of the solution is added an equivolume of a Freund's complete adjuvant and mixed well to obtain a perfect water-in-oil type emulsion. The emulsion is subcutaneously injected to 10 six-week old female BALB/c mice in an amount of 130 μl per mouse four times at intervals of one week. One week after the final injection, about 50 μl of blood is collected from one of the eyegrounds of each mouse. From the blood of each mouse, 20 to 30 μl of serum is obtained. The antibody titer of the serum of each mouse is roughly determined by a solid phase ELISA in substantially the same manner as in Example 14 except that peroxidase-bonded anti-mouse IgG (H+L) antibody (goat) is used instead of peroxidase-bonded anti-rabit IgG antiserum (goat), and a mouse having the highest antibody titer is selected. To the selected mouse is intraperitoneally injected a solution of 12 μg of the peptide obtained in Example 8 in 120 μl of saline as a booster. 3 days after the boost, the spleen is excised from the mouse. Then, the spleen cells are loosened with forceps in Hank's solution and separated from the spleen membrane. At the same time, the entire blood of the mouse is collected from the heart. From the collected blood, 350 μl of a mouse antiserum is obtained.

$5x10^8$ cells of the thus obtained spleen cells are sufficiently mixed with $3x10^8$ cells of mouse myeloma cell line P3U1 in RPMI1640 medium. To the resultant mixture is added 0.3 ml of a 50 % polyethylene glycol solution and stirred slowly to obtain a solution. The solution is allowed to stand in an atmosphere containing 5 % $CO_2$ at 37°C for 7 min to effect cell fusion. After the cell fusion, the cells are collected from the solution, washed and added to RPMI 1640 medium containing 20 % FCS to obtain a cell suspension. The suspension is allowed to stand in an atmosphere containing 5 % $CO_2$ at 37°C for 1 hr. Then, the cell suspension is put into the wells of 35 plates of 96-well microplate in an amount of 100 µl per well. One day later, RPMI1640 medium containing 13.61 mg/ℓ of hypoxanthine, 0.18 mg/ℓ aminopterin, 3.88 mg/ℓ thymidine and 20 % of fetal calf serum (hereinafter referred to as "HAT medium") is added to the wells of the plates in an amount of 100 µl per well. About 2 weeks after the cell fusion, colonies have grown in 778 wells. 100µl of the supernatant of each of the 778 wells are subjected to determination of antibody titer in substantially the same manner as described in Example 14 except that the peptide obtained in Example 8 is used as the antigen in an amount of 50 ng per well instead of 1 µg per well. From the results of the determination, 30 colonies of which the culture supernatants show high antibody titer are selected. The 30 colonies corresponding to the 30 serums are further grown. From the colonies, 5 clones of cell line which produces the desired monoclonal antibody are obtained through cloning by limiting dilution-culture method and determination of antibody titer by the above-described method.

One of the 5 clones is designated "cell line ADI1-7-5-2". Cell line ADI1-7-5-2 has been deposited with the Fermentation Research Institute under the accession number FERM BP-1994 (date of deposit: December 11, 1987).

Example 17

[Preparation of a monoclonal antibody]

KLH-1 obtained in Example 13 is added to PBS(-) to obtain a solution having a protein concentration of 2 mg/ml. To 750 µl of the solution is added an equivolume of a Freund's complete adjuvant and mixed well to obtain a perfect water-in-oil type emulsion. The emulsion is subcutaneously injected to 8 six-week old female BALB/c mice in an amount of 150 µl per mouse six times at intervals of one week. Ten days after the final injection, about 50 µl of blood is collected from one of the eyegrounds of each mouse. From the blood of each mouse, 20 to 30 µl of serum is obtained. The antibody titer of the serum of each mouse is roughly determined by a solid phase ELISA in substantially the same manner as in Example 16 and a mouse having the highest antibody titer is selected. As a booster, a solution of 70 µg of the peptide obtained in Example 8 in 120 µl of saline is intraperitoneally injected to the selected mouse. 3 days after the boost, the spleen is excised from the mouse. Then, the splenocyte is loosened with forceps in Hank's solution and separated from the spleen membrane. At the same time, the entire blood of the mouse is collected from the heart. From the collected blood, 250 µl of a mouse antiserum is obtained.

$2.8x10^8$ cells of the thus obtained spleen cells are sufficiently mixed with $3x10^8$ cells of mouse myeloma cell line P3U1 in RPMI1640 medium. To the resultant mixture is added 0.3 ml of a 50 % polyethylene glycol solution and stirred slowly to obtain a solution. The solution is allowed to stand in an air containing 5 % $CO_2$ at 37°C for 7 min to effect cell fusion. After the cell fusion, the cells are collected from the solution, washed and added to RPMI 1640 medium containing 20 % FCS to obtain a cell suspension. The suspension is allowed to stand in an air containing 5 % $CO_2$ at 37°C for 1 hr. Then, the cell suspension is put into the wells of 35 plates of 96-well microplate in an amount of 100 µl per well. One day later, HAT medium is added to the wells of the plates in an amount of 100 µl per well. About 2 weeks after the cell fusion, colonies have grown in 550 wells. 100µl of the supernatant of each of the 550 wells are subjected to determination of antibody titer in substantially the same manner as described in Example 16. From the results of the determination, 25 colonies of which the culture supernatants show high antibody titer are selected. The 25 colonies are further grown. From the colonies, 6 clones of cell line which produce the desired monoclonal antibody are obtained through cloning by limiting dilution-culture method and determination of antibody titer by the above-described method.

The monoclonal antibody produced by each of the 6 clones in the respective supernatant is subjected to the following assay to determine its antigenic specificity and affinity to antigens.

The peptide obtained in Example 8, aprotinin (BPTI) (manufactured and sold by Sigma Chemical Company, USA) and Urinary Trypsin Inhibitor (produced and sold by Japan Chemical Research Co., Ltd.) (hereinafter referred to as "UTI") are plated to a microplate adapted for ELISA in four different equimolar amounts, i.e., 20 ng, 100 ng, 1 µg and 10 µg for the peptide; 62 ng, 310 ng, 3.1 µg, and 31 µg for aprotinin; and 670 ng, 3.35 µg, 33.5 µg and 335 µg for UTI. At the same time, control wells having no antigen plated thereto are provided.

The antibody titer of the supernatant of each of the culture media respectively containing the 6 clones is assayed by a solid phase ELISA in substantially the same manner as described in Example 14 except that the above-provided antigens and microplate are used instead of the antigen and microplate used in Example 14 and that peroxidase-bonded anti-mouse IgG(H+L) antibody (goat) is used instead of peroxidase-bonded anti-rabbit IgG antiserum (goat). As a result, it is found that all of the supernatants react only with the peptide obtained in Example 8 and that the reactivity with the peptide varies according to the type of supernatant. The clone corresponding to the supernatant which reacts most strongly with the peptide is designated "cell line ADI1-4-6-1". Cell line ADI1-4-6-1 has been deposited with FRI under the accession number FERM BP-1995 (date of deposit: February 9, 1988).

Example 18

[Preparation of a monoclonal antibody]

Substantially the same procedure as in Example 16 is repeated except that KLH-2 obtained in Example 13 is used as the antigen instead of KLH-1 to obtain 3 clones of cell line which produce the desired monoclonal antibody.

Example 19

[Preparation of monoclonal antibody]

Substantially the same procedure as in Example 16 is repeated except that KLH-3 obtained in Example 13 is used as the antigen instead of KLH-1 to obtain 1 clone of cell line which produces the desired monoclonal antibody.

Example 20

[Preparation of a monoclonal antibody]

Substantially the same procedure as in Example 16 is repeated except that KLH-4 obtained in Example 13 is used as the antigen instead of KLH-1 to obtain 3 clones of cell line which produce the desired monoclonal antibody.

Example 21

[Preparation of a monoclonal antibody]

Substantially the same procedure as in Example 16 is repeated except that KLH-5 obtained in Example 13 is used as the antigen instead of KLH-1 to obtain 1 clones of cell line which produce the desired monoclonal antibody.

Example 22

(Preparation of a monoclonal antibody)

Substantially the same procedure as in Example 16 is repeated except that the protein obtained in Example 15 is used as the antigen instead of KLH-1 to obtain 8 clones of a cell line which produce the desired monoclonal antibody.

One of the clones has been designated "cell line ADI1-05-44-2". The ADI1-05-44-2 is deposited with the Fermentation Research Institute under the Budapest Treaty on February 5, 1988 under the accession No. FERM BP-1988.

Example 23

(Preparation of a monoclonal antibody)

Freund's complete adjuvant (FCA) is added, in equal amounts, to a PBS(-) solution (150 μg/500 μℓ) of the purified secreted PI (hereinafter simply referred to as "sPI") obtained in Example 7, and well emulsified. The resultant mixture is injected into the abdomen of each of five 6-week old BALB/C female mice in an amount of 200 μℓ intraperitoneally. Further, two weeks later, a mixture of PI (250 μg/500 μℓ) and Freund's incomplete adjuvant (FIA) is injected into each of the mice in an amount of 200 μℓ intraperitoneally. Moreover, two further weeks later, sPI (50 μg/40 μℓ in PBS) is intravenously injected as a booster into one of the mice. Two days after the injection of the booster, the spleen of the mouse is excised, thereby obtaining $1x10^8$ spleen cells. These spleen cells are fused with mouse myeloma cell line SP2/0-Ag-14 and screened in substantially the same manner as in Example 16, thereby obtaining two clones of hybridomas which are capable of producing a monoclonal antibody. 50 ng of sPI is used as an antigen in the solid phase ELISA for the screening at this step. The titers are measured by the solid phase ELISA in the same manner as in Example 14, with respect to the serum (i.e., antisera to sPI) obtained at the time of excising the spleen from the mouse and the results are shown in Table 7.

Table 7

| Amount of Antigen / Antiserum Dilution | Antiserum not present | 500X | 1000X | 2000X | 4000X | 8000X | 16000X | 32000X |
|---|---|---|---|---|---|---|---|---|
| 0 | -0.078 | -0.078 | -0.076 | -0.078 | -0.079 | -0.079 | -0.079 | -0.076 |
| 100 ng | 0.194 | > 2 | > 2 | 1.240 | 0.685 | 0.400 | 0.275 | 0.135 |
| 100 ng | 0.060 | > 2 | > 2 | 1.372 | 0.796 | 0.405 | 0.231 | 0.147 |
| 50 ng | 0.127 | > 2 | > 2 | 1.222 | 0.702 | 0.364 | 0.233 | 0.183 |
| 50 ng | 0.083 | > 2 | > 2 | 1.188 | 0.638 | 0.361 | 0.228 | 0.126 |
| 25 ng | 0.108 | > 2 | 1.487 | 0.746 | 0.369 | 0.189 | 0.128 | 0.071 |
| 25 ng | 0.067 | > 2 | 1.554 | 0.707 | 0.409 | 0.159 | 0.089 | 0.043 |
| 12.5 ng | 0 | 1.498 | 0.721 | 0.372 | 0.144 | 0.069 | 0.029 | 0.091 |

Example 24

(Characterization of monoclonal antibodies)

The immunogloblin class of each of the monoclonal antibodies obtained in Examples 16, 17 and 22 is determined using the isotyping kit including a class-specific anti-mouse antiserum (manufactured and sold by Amersham Co., Ltd., U.S.A.). The results are shown in Table 8 below. All of the monoclonal antibodies are IqM, and the L chains of the monoclonal antibodies are classified as κ.

Table 8

| Class | Monoclonal Antibody | | |
|---|---|---|---|
| | Example 16 | Example 17 | Example 22 |
| λ | - | - | - |
| κ | + | + | + |
| IgG3 | - | - | - |
| IgG2b | - | - | - |
| IgG2a | - | - | - |
| IgG1 | - | - | - |
| IgM | + | + | + |
| IgA | - | - | - |

Example 25

(1) (Assay of serum, CSF and cerebral tissue extract)

With respect to the sera, CSF's and cerebral tissue extracts from SDAT patients, dot blotting is performed using the monoclonal antibody obtained in Example 17. As the sera and CSF's, use is made of samples from three 46 to 85-year-old, mild to serious SDAT patients and from two non-demented patients. As the cerebral tissue extract, use is made of a sample of a frontal cortex from one SDAT and from non-demented patients. The sera are used as they are, and the CSF's are concentrated 8-fold by centrifugation in vacuum prior to use thereof. The cerebral tissue extract is obtained as a supernatant by homogenizing the cerebral tissue, by means of Dounce's homogenizer, in the extraction buffer mentioned in Example 2 to which phenylmethanesulfonyl fluoride (PMSF) has been added in a final concentration of 1 mM, followed by centrifugation.

1 μl of each of the thus obtained samples of serum, CSF and cerebral tissue extract is dotted onto a nitrocellulose filter, air dried for 15 min, and subjected to blocking with a 3 % BSA/TBS solution at 4°C overnight. The filter is rinsed with TBS, and shaked at room temperature for 1 hr in a first antibody solution obtained by diluting two-fold the supernatant of the medium containing a monoclonal antibody obtained in Example 17 with a 1 % BSA/TBS solution. Then, the filter is washed, and color development on the filter is performed using, as a second antibody, horseradish peroxidase-labeled anti-mouse IgG (H+L) antibody (goat) and, as a coloring agent, 4-chloronaphthol. As a first antibody control, a solution obtained by diluting 1000-fold non-immunized mouse serum is used. As a result, deeper color development than that of the controls is observed with respect to all of the cerebral tissue extracts, sera and CSF's.

(2) (Assay of glycohydrolase-treated serum, CSF and cerebral tissue extract)

Dot blotting is performed in substantially the same manner as in Example 25-(1), except that the antiserum obtained in Example 14 is used as a first antibody, that biotinylated goat anti-rabbit IgG (H+L) antibody is used as a second antibody, and that samples obtained by treating with heparinase II each of the serum, CSF and cerebral tissue extract obtained in Example 25-(1) are employed. The treatment with heparinase II is performed using 0.7 U/ml heparinase II (Manufactured and sold by Sigma Chemical Company, U.S.A.), in 10 mM Tris buffer (pH7.0) in the presence of 1.5 mM $CaC\ell_2$, for 24 hr. Thereafter, desalting and concentration are performed using Centricon® 10, thereby obtaining samples to be tested. As a result, deeper color development than that of the heparinase II non-treated ones is observed with respect to all of the cerebral tissue extract, serum and CSF. That is, the difference in color intensity between SDAT patients and the control case has become greater by the glycohydrolase treatment.

Example 26

(1) (Assay of serum, CSF and cerebral tissue extract)

Assay of each of the serum, CSF and cerebral tissue extract from SDAT patients by the solid-phase ELISA method is performed using the mouse antiserum obtained in Example 23.

As the sera and CSF's, use is made of samples from three 46 to 85-year-old, mild to serious SDAT patients and from two non-demented patients. As the cerebral tissue extract, use is made of frontal cortex samples from one SDAT patient and one control brain. The sera are used as they are, and the CSF's is concentrated 8-fold by centrifugation in vacuum prior to use thereof. The cerebral tissue extract is obtained as a supernatant by homogenizing the cerebral tissue, by means of Dounce's homogenizer, in the extraction buffer mentioned in Example 2 to which PMSF has been added in a final concentration of 1 mM, followed by centrifugation.

100 μl of each of the thus obtained serum and CSF and 400 μg of each of the cerebral tissue extracts after protein assay are separately put into the wells of 96-well microplates. 11 μl of 0.5 M $NaHCO_3$ buffer is added to each well so as to cause it to exhibit a pH value of 9.6, and the plate is incubated at 4°C overnight to effect adsorption.

Subsequently, the wells are subjected to blocking in a 3 % BSA/PBS solution at room temperature for 3 hr. Then, the wells are rinsed with 0.1 % BSA/PBS, and 100 μl of a solution obtained by diluting 1000-fold the antiserum prepared in Example 23 with 1 % BSA/PBS is put in each of the wells. The plates are incubated at room temperature for 1 hr. The wells are washed, and color development on the plates is performed using, as a second antibody, biotinylated goat anti-mouse IgG (H+L) antibody and horseradish peroxidase streptavidin complex, and further using, as a coloring agent, o-phenylenediamine. As a first antibody control, a solution obtained by diluting 1000-fold non-immunized mouse serum is used. As a result, deeper color development than that of the control is observed with respect to all of the cerebral tissue extract, serum and CSF samples from patients.

(2) (Assay of glycohydrolase-treated serum, CSF and cerebral tissue extract)

Assay by the solid phase ELISA method is performed in substantially the same manner as in Example 26-(1), except that the antiserum obtained in Example 14 is used as a first antibody, that biotinylated goat anti-rabbit IgG (H+L) antibody is used as a second antibody, and that samples obtained by treating with heparinase II each of the serum, CSF and cerebral tissue extract obtained in Example 26-(1) are employed. The treatment with heparinase II is performed using 0.7 U/ml heparinase II (manufactured and sold by Sigma Chemical Company, U.S.A.), in the presence of $1.5 \times 10^{-3}$ M $CaC\ell_2$, in 10 mM Tris buffer (pH7.0) for 24 hr. Thereafter, desalting and concentration are performed using Centricon 10, thereby obtaining samples to be tested. As a result, deeper color development than that of the heparinase II non-treated ones is observed with respect to all of the cerebral tissue extracts, sera and CSF's. Further, the difference in coloring intensity between SDAT patients and the control case has become greater.

Example 27

(Assay of serum, CSF and cerebral tissue extract)

Assay of the sera, CSF's and cerebral tissue extracts from SDAT patients by the solid-phase ELISA method is performed using the monoclonal antibody obtained in Example 22.

As the sera and CSF's, use is made of samples from three 46 to 85-year-old, mild to serious SDAT patients and from two non-demented conrols. As the cerebral tissue extracts, use is made of frontal cortex samples from one SDAT patient and one control brain. The sera are used as they are, and the CSF's are concentrated 8-fold by centrifugation in vacuum prior to use thereof. The cerebral tissue extract is obtained as a supernatant by homogenizing the cerebral tissue, by means of Dounce's homogenizer, in the extraction buffer mentioned in Example 2 to which PMSF has been added in a final concentration of 1 mM, followed by centrifugation.

100 μl of each of the thus obtained sera and CSF's and 400 μg of each of the cerebral tissue extracts after protein assay are separately put into the wells of 96-well microplates. 11 μl of 0.5 M $NaHCO_3$ buffer is added to each well so as to cause it to exhibit a pH value of 9.6, and the plate is incubated at 4°C overnight to effect adsorption.

Subsequently, the wells are subjected to blocking with a 3 % BSA/PBS solution at room temperature for 3 hr. Then, the wells are rinsed with 0.1 % BSA/PBS, and 100 μl of the culture supernatant of monoclonal antibody-producing strain prepared in Example 22 is put in each of the wells. The plates are incubated at room temperature for 1 hr. The wells are washed, and color development on the plates is performed using, as a second antibody, biotinylated goat anti-mouse IgG (H+L) antibody horseradish peroxidase streptavidin complex, and further using, as a coloring agent, o-phenylenediamine. As a first antibody control, a solution obtained by diluting 1000-fold non-immunized mouse serum is used. As a result, deeper color development than that of the control is observed with respect to all of the cerebral tissue extract, serum and CSF samples from patients.

Example 28

(Synthesis of an oligodeoxynucleotide)

Oligodeoxynuclotide (AM-5) having a base sequence complementary to the base sequence 5′ - CAATGATCTCCCGCTGGTAC - 3′ [corresponding to the base sequence of from the 905th to 924th bases in Fig. 1(A)] is synthesized using a DNA synthesizer (Model 380A manufactured and sold by Applied Biosystems Co., Ltd., U.S.A.). According to the protocol of Applied Biosystems Co., Ltd., purification is performed by high performance liquid chromatography. Thus, about 70 μg of the desired product is recovered.

Example 29

(Synthesis of an RNA probe)

Plasmid pSPGP2TX obtained in Step 1 of Example 3 is digested with HindIII, and purified by phenol extraction and ethanol precipitation. Using, as a template, the HindIII-digested pSPGP2TX, and also using SP6 RNA polymerase (included in the SP6 System RPN1506 kit manufactured and sold by Amersham Co., Ltd., U.S.A.), labeling reaction is conducted at 37°C for 1 hr using 1 μCi of [α-$^{32}$P]UTP (uridine triphosphate), 400 μM of each of ATP, GTP and CTP according to the protocol of Amersham Co., Ltd. Thus, there is obtained a radioactive RNA probe complementary to TaqI-XhoII fragment of pGBP2 (corresponding to the base sequence of from the 861st to 1082nd bases in Fig. 1).

Example 30

(Synthesis of a cDNA probe)

15 μg of plasmid pSPGP2TX obtained in Example 3 Step 1 is digested with AvaI and PstI. Subsequently, electrophoresis on 1.5 % agarose gel is performed, and a gel portion containing a fragment of about 220 bp is cut off. From this gel portion, AvaI-PstI fragment is isolated using Geneclean® sold by Funakoshi Pharmaceutical Co., Ltd., Japan in accordance with the protocol of the company. 200 ng of this fragment is labelled with 50 μCi of deoxycytidine 5′-[α-$^{32}$P]-triphosphate (dCTP) using the nick translation kit manufactured and sold by Takara Shuzo Co., Ltd., Japan in accordance with the protocol appended to the kit. The thus obtained reaction mixture is treated with phenol /chloroform, and the labeled cDNA probe is purified using Nick-Column® manu factured and sold by Pharmacia Fine chemicals AB, Sweden. Thus, there is obtained a cDNA probe having a radioactivity of about 2x10$^7$ cpm.

Example 31

(Synthesis of a cDNA probe)

The AvaI-PstI fragment obtained in Example 30 is labelled with [α-$^{32}$P]dCTP using Multiprime® DNA labelling system manufactured and sold by Amersham Co., Ltd., U.S.A., in accordance with the protocol appended to the system, and purified. Thus, there is obtained a cDNA probe having a specific radioactivity of about 1x10$^9$ cpm/μg.

Example 32

(Northern blot analysis)

Poly(A)RNA's are obtained in accordance with the method of Chirguin et al described in Example 1 Step 1 from glioblastoma strain ATCC HTB-14, glioblastoma strain ATCC HTB-16, glioblastoma strain ATCC HTB-17, human fetus brain (10-week-old) and SDAT patient cerebrum. Further, poly(A)RNA separated from the hippocampus region of the brain of 70-year-old human is purchased from Clontech Co., Ltd., U.S.A.

5 μg of each of these poly(A)RNA's is denatured with glyoxal, and subjected to electrophoresis according to the method described on page 200 of the laboratory manual of Maniatis et al. Subsequently, the RNA's are electrically transferred to nylon filters (Zeta-Probe® manufactured and sold by Bio-Rad Laboratories, U.S.A.).

The operations after transfer to filter up to autoradiography are performed according to the protocol of Bio-Rad Laboratories. With respect to the filters, hybridization is performed using, as a probe, synthetic oligonucleotide AM-5 obtained in Example 28 which has its 5′-end labelled with radioactive $^{32}$P using T4 kinase (hereinafter referred to as "synthetic probe $^{32}$P-AM-5"). That is, the above-mentioned filters are prehybridized at 55°C for 2 hr in a solution containing 0.75 M NaCℓ, 25 mM sodium phosphate (pH7.0), 1 % glycine, 75 mM sodium citrate, 0.1 % SDS, 0.01 % (w/v) Ficoll, 0.01 % (w/v) bovine serum albumin, 0.01 % (w/v) polyvinyl pyrrolidone and 150 μg/ml salmon sperm DNA. Then, the filters are hybridized with the synthetic probe $^{32}$P-AM-5 (5x10$^5$ cpm/ml) at 55°C for 2 hr. Thereafter, the filters are washed with 6xSSC at room temperature for 10 min, washed twice at 55°C for 10 min, and subjected to autoradiography.

The poly(A)RNA's from glioblastoma strains ATCC HTB-14 and HTB-17 exhibit a strong band at about 3.4 kb (kilobase). The poly(A)RNA from human fetus brain exhibits a relatively weak band at the same position, and that from 70-year-old human brain (Clontech Co., Ltd.) exhibits a stronger band at the same position. The poly(A)RNA from SDAT patient brain exhibits a band at the same position, which is stronger than that from 70-year-old human brain.

Substantially the same results as obtained with synthetic probe AM-5 are obtained with both of the RNA probe prepared in Example 29 and the cDNA probes prepared in Examples 30 and 31. Incidentally, when these RNA and cDNA probes are used, washing of the filters is performed using, as a washing buffer, 0.1 x SSC/0.1 % SDS, in place of 6xSSC.

Example 33

(Synthesis of an RNA probe)

A plasmid for synthesizing an RNA probe is constructed according to the flow chart shown in Fig. 13 in which the portion corresponding to the base sequence of from the 866th to 1033rd bases in the base sequence of Fig. 1(B) is blacked. That is, 3 μg of pGBP3 is digested with AccI and BalI, and subjected to electrophoresis on 1 % low melting point agarose gel. A fragment of about 1.4 kbp is isolated. Subsequently, the resultant AccI-BalI fragment is digested with Taq I at 65°C for 1 hr. The resultant reaction mixture is subjected to phenol extraction, and then to buffer exchange by the rapid gel filtration method according to the method described in the laboratory manual of Maniatis et al. Thereafter, electrophoresis on 1 % low melting point agarose gel is performed, and TaqI-TaqI fragment of about 220 bp is isolated and purified.

On the other hand, 2 μg of plasmid pSP6/T7-19 (product of Bethesda Research Laboratories Inc., U.S.A.) containing SP6 promoter and T7 promoter is digested with AccI, and subjected to electrophoresis on 1 % low melting point agarose gel. From the gel, a desired vector is isolated. This vector is hereinafter referred to as "vector f".

The thus obtained TaqI fragment and vector f are ligated at 15 °C for 2 hr using T4 ligase in the presence of ATP in a ligase buffer. Using the resultant reaction mixture, Escherichia coli MC1061 is transformed and cultured in accordance with the laboratory manual of Maniatis et al, thereby obtaining ampicillin resistant colonies. From these colonies, 12 colonies are arbitrarily selected, and plasmids are isolated according to the conventional method of rapid, small-scale plasmid isolation (mini-prep method). The isolated plasmids are digested with ScaI and EcoRI, and subjected to electrophoresis on 5 % polyacrylamide gel. As a result, it is found that five transformant clones each give a plasmid containing a fragment of about 110 bp in addition to the fragment attributed to the vector, and four transformant clones each give a plasmid containing a fragment of about 180 bp in addition to the fragment attributed to the vector. Of these clones, clones giving a plasmid containing a fragment of about 110 bp are the desired clones. The thus obtained plasmid contained in the clones are hereinafter referred to as "plasmid pSPGP3TX". Plasmid pSPGP3TX is digested with HindIII, and purified by phenol extraction and ethanol precipitation. Using, as a template, the HindIII-digested plasmid pSPGP3TX, and also using SP6 RNA polymerase (included in the SP6 System RPN1506 kit manufactured and sold by Amersham Co., Ltd., U.S.A.), labeling reaction is conducted at 37 °C for 1 hr using 1 μCi of [α-$^{32}$P]UTP (uridine triphosphate), 400 μM of each of ATP, GTP and CTP according to the protocol of Amersham Co., Ltd. Thus, there is obtained a radioactive RNA probe complementary to TaqI fragment of pGBP3, which includes a base sequence corresponding to the base sequence of the 862nd to 1079th bases in Fig. 1(B).

Example 34

(Synthesis of cDNA probe)

In substantially the same manner as in Example 33, AccI-BalI fragment is prepared from plasmid pGBP3 and TaqI fragment is separated from AccI-BalI fragment. 200 ng of this fragment is labelled with 50 μCi of deoxycytidine 5′-[α-$^{32}$P]triphosphate(dCTP) using the nick translation kit manufactured and sold by Takara Shuzo Co., Ltd., Japan, in accordance with the protocol appended to the kit. The thus obtained reaction mixture is treated with phenol/chloroform, and the labeled cDNA is purified using Nick-column® manufactured and sold by Pharmacia Fine Chemicals AB, Sweden. Thus, there is obtained a cDNA probe having a radioactivity of about 2 x $10^7$ cpm.

Example 35

(Synthesis of oligodeoxynucleotide)

Oligodeoxynucleotide AM-12 having the following base sequence:

**5'-CAGAGCACACCTCTCGAACCACCT-3'**

which can specifically recognize with the junction between exon H and exon I of each of H-I-K type and H-I-J-K type mRNA's is synthesized in substantially the same manner as in Example 28, thereby obtaining 40 μg of a purified product of oligodeoxynucleotide AM-12.

Example 36

(Synthesis of oligodeoxynucleotide)

Oligodeoxynucleotide AM-13 having the following base sequence:

**5'-CTGTTGTAGGAATGGCGCTGCCAC-3'**

which can specifically recognize with the junction between exon I and exon K of H-I-K type mRNA is synthesized in substantially the same manner as in Example 28, thereby obtaining 50 µg of a purified product of oligodeoxyribonucleotide AM-13.

Reference Example 1

(Synthesis of oligodeoxynucleotide)
Oligodeoxynucleotide AM-11 having the following base sequence:

5'-CTGTTGTAGGAACTCGAACCACCT-3'

which can specifically recognize with the junction between exon H and exon K of H-K type mRNA is synthesized in substantially the same manner as in Example 28, thereby obtaining 34 µg of a purified product of oligodeoxynucleotide AM-11.

Example 37

(Synthesis of oligodeoxynucleotide)
Oligodeoxynucleotide AM-14 having the following base sequence:

5'-CTGTTGTAGGAAGTTTAACAGGAT-3'

which can specifically recognize with the junction between exon J and exon K of each of H-J-K type and H-I-J-K type mRNA's is synthesized in substantially the same manner as in Example 28, thereby obtaining 43 µg of a purified product of oligodeoxynucleotide AM-14.

Example 38

(Synthesis of oligodeoxynucleotide)
Oligodeoxynucleotide AM-HI2 having the following base sequence:

5'-AAACTTTGGGACACTCGAACCACCTC-3'

which can specifically recognize with the junction between exon H and exon J of H-J-K type mRNA is synthesized in substantially the same manner as in Example 28, thereby obtaining 38 µg of a purified product of oligodeoxynucleotide AM-HI2.

Example 39

(Synthesis of oligodeoxynucleotide)
Oligodeoxynucleotide AM-I1I2 having the following base sequence:

5'-AAACTTTGGGACATGGCGCTGCCACA-3'

which can specifically recognize with the junction between exon I and exon J of H-I-J-K type mRNA is synthesized in substantially the same manner as in Example 28, thereby obtaining 35 µg of a purified product of oligodeoxynucleotide AM-I1I2.

Example 40

(Northern blot)
2.5 µg of each of the poly(A)RNA's from glioblastoma strain ATCC HTB-14, human fetus brain (10-week-old) and hippocampus region of 70-year-old human (product of Clontech Co., Ltd., U.S.A.) is electrophoresed and transferred to a filter in substantially the same manner as in Example 32. This is repeated to prepare a total of four filters with respect to each of the above-mentioned poly(A)RNA's. The oligodeoxynucleotides AM-11, AM-12, AM-13 and AM-14 synthesized in Example 35, Example 36, Example 37 and Reference Example 1, respectively, are labelled with radioactive $^{32}P$-γ-ATP. Using, as a probe, the thus labelled DNA's, hybridization on the above-mentioned filters is performed in substantially the same manner as described in Example 32.

As a result of autoradiography, it is found that all of the poly(A)RNA's exhibit a band at a position corresponding to the molecular length of 3.2 to 3.4 kb. In connection with the intensity of the band, the following is observed. That is, with respect to the poly(A)RNA isolated from glioblastoma strain ATCC HTB-14, the amount of mRNA (H-K type mRNA) hybridizing with AM-11 is small, and there are observed strong hybridizations with AM-12, AM-13 and AM-14 to a substantially equal degree. With respect to the poly(A)RNA isolated from fetus brain, the mRNA is strongly hybridized with AM-11, and the hybridizations with AM-12, AM-13 and AM-14 are significantly less than that with AM-11. With respect to the poly(A)RNA isolated from brain hippocampus region of 70-year-old human, the hybridizations with AM-12 and AM-13 are strong.

Example 41

(Northern blot analysis)

A poly(A)RNA's are obtained from 1 g to 5 g of each of frontal lobes of two patients of SDAT (89 years old woman and 87 years old man) and four control humans (from 59 years old human to 90 years old human) in accordance with the method of Chirgwin et al as described in Step 1 of Example 1.

Northern blot analysis is conducted with respect to 2.4 μg of each of these poly(A)RNA's according to the method as described in Example 32.

Illustratively stated, first hybridization is conducted using the labeled cDNA probe (for the detection for NAP or NAP3 mRNA) obtained in Example 30 on a filter. The resultant filter is subjected to autoradiography to measure the intensity of the band at which the cDNA probe is hybridized. Then, the filter is washed with 0.1 x SSC solution at 65 °C for 6 hours. The filter is subjected to autoradiography to confirm that the probe is completely removed from the filter. Using the filter thus obtained, second hybridization is conducted using as a control probe a human β-actin probe (manufactured and sold by Wako Pure Chemical Industries, Ltd., Japan), and then, subjected to autoradiography to measure the intensity of the band at which the control probe is hybridized. With respect to each of SDAT patients and control humans, the intensity of the band at which the cDNA probe obtained in Example 30 is hybridized is compared with that of the band at which the β-actin probe is hybridized, so that the relative amount of the expressed mRNA coding for NAP or NAP3 to the expressed mRNA coding for β-actin is obtained. As a result, it is found that the relative amounts of mRNA coding for NAP or NAP3 in the case of the SDAT patients are larger than those in the case of control humans.

Example 42

(Cloning of exons I and J of human chromosomal SPAP gene)

Step 1

(Analysis of a human chromosomal gene by the method of Southern blot)

Using Ficol-Paque (manufactured and sold by Pharmacia Fine Chemicals AB, Sweden), peripheral blood lymphocytes are separated from 10 ml of the blood of healthy human according to the appended protocol. Then, according to the method of Shimizu et al [Kenji Shimizu et al, "DNA transfection and selection", View of Oncogene Research (an extra edition of Chemistry Today vol. 2, Japan), 246-249 (1985)], a chromosomal DNA is extracted from the lymphocytes and purified, thereby obtaining 110 μg of chromosomal DNA.

10 μg each of the chromosomal DNA thus obtained is separately digested individually with restriction enzymes BamHI, EcoRI and HindIII. The resultant DNA digests are extracted with phenol and subjected to ethanol precipitation, thereby collecting DNA fragments. Then, the thus obtained DNA fragments are subjected to agarose gel electrophoresis and transferred onto a zeta probe membrane filter. The $^{32}$P-labeled cDNA probe (hereinafter referred to as "probe a") prepared in Example 30 is used as a hybridization probe. The probe is added to a buffer comprising 25 mM sodium phosphate (pH 6.5), 5 x SSC solution, 2 x Denhardt's, 100 μg/ml of denatured sermon sperm DNA and 50 % of formamide so that the resultant mixture exhibits a radioactivity of 1 x $10^6$ cpm/ml. Then, the resultant mixture is applied to the filter and incubated at 42 °C for 20 hours to conduct hybridization. The filter is washed with 0.1 x SSC solution containing 0.1 % SDS at 55 °C and, then, subjected to autoradiography. The results are shown in Fig. 14. As shown in Fig. 14, with respect to the DNA fragments respectively obtained by the digestions with BamHI, EcoRI and HindIII, each of the fragments of 6.6 kb, 7.4 kb and 8.2 kb is individually hybridized with the probe. Further, it is found that only single gene coding for a protein having a protease inhibiting activity which is hybridized with NAP cDNA, is present in the human genome.

Step 2

(Cloning of a portion of a human chromosomal SPAP gene, coding for a protein having a protease inhibiting activity)

Human chromosomal DNA clones which contain a portion of SPAP gene coding for a protein having a protease inhibiting activity is obtained by screening from about $10^6$ plaques of human genomic DNA library (manufactured and sold by Clontech Laboratories Inc., U.S.A.) which comprises bacteriophage λEMBL-3 and, ligated thereto, chromosomal DNA fragments obtained from human lymphocytes.

25 agar plates (150 mm in diameter) each having 4 x $10^4$ plaques of the above-mentioned library are prepared. Then, according to the method of Benton et al [W.D. Benton & R.W. Davis, Science 196, 180-182(1977)], transfer of the plaques to a nitrocellulose filter and screening by hybridization are conducted.

$^{32}P$-labeled probe a as prepared in Example 30 is added to the same buffer as used in Step 1 so that the final concentration of the probe becomes 1 x $10^5$ cpm/ml. The filters are put in the buffer and incubated at 42 °C 16 hours to conduct hybridization. The resultant filters are washed with 0.1 x SSC solution containing 0.1 % SDS at 55 °C and, then, subjected to autoradiography, to thereby obtain two positive spots. The phages corresponding to the positive spots are extracted from the agar plate. Using the extracted phages, the plaque hybridization is conducted in the same manner as mentioned above to obtain two purified positive phage clones. Thus obtained phage clones are designated "λ24" and "λ25", respectively.

Step 3

(Analysis of λ24 and λ25)

With λ24 and λ25 obtained in Step 2, cells of Escherichia coli LE392 are infected. Then, according to the method of Sclhavy et al [Sclhavy, T.J., Berman, M.L., Enquist, L.W., Experiments with Gene Fusion, 140-143, (1984)], the phage DNA is recovered from the infected cell culture. The thus obtained phage DNA is digested individually with restriction enzymes BamHI, EcoRI and HindIII and subjected to analysis by agarose gel electrophoresis. Both of the λ24 and λ25 are found to contain a human chromosomal DNA of about 20 kb and, further, the digestion patterns between the digestion with the restriction enzymes are found to coincide with each other. These results indicate that these phage clones are identical [Fig. 2(B)]. In Fig. 2, "E-" is an abbreviation of exon.

Step 4

(Subcloning of λ24 DNA fragment and determination of a restriction map)

The BamHI-digested λ24 phage DNA which is obtained in step 2 is subjected to agarose gel electrophoresis and transferred to a zeta probe membrane filter in accordance with the method as described in the protocol of Bio-Rad, USA (BIO-RAD news, May 1985, p.1 to 2). Using $^{32}P$-labeled probe a as prepared in Example 30, Southern blot analysis of the filter is conducted. As a result, it is found that about 6.6 kb of DNA fragment is hybridized with the probe. The DNA fragment corresponding to the hybridized DNA fragment on the filter is extracted from the gel and purified. The thus obtained DNA fragment is ligated to vector pUC18 which has been digested with BamHI and treated with BAP in ligation buffer using T4 ligase in the presence of ATP. Using the reaction mixture, the cells of Escherichia coli JM105 are transformed to obtain ampicillin-resistant colonies of the transformants in accordance with the method as described in the Laboratory manual of Maniatis et al, p.252 to 253. 6 Colonies are selected from the thus obtained ampicillin resistant colonies, and plasmid DNA's are extracted from the colonies according to a rapid, small-scale plasmid isolation method. The obtained plasmid DNA's are digested with BamHI and subjected to agarose gel electrophoresis. As a result, it is confirmed that all of the plasmids contain 6.6 kb of the desired DNA fragment. One of the plasmids is designated "plasmid pAP246B" and the clone containing the plasmid is designated E. coli K-12 strain JM105(pAP246B).

E. coli K-12 strain JM105(pAP246B) is deposited at Fermentation Research Institute under the accession number FERM BP-1987 on February 1, 1988.

Plasmid pAP246B is digested with each of restriction enzymes EcoRI, AvaI, HindIII, ScaI and BglII and subjected to agarose gel electrophoresis. In Fig. 2(B), a restriction map of a chromosomal DNA fragment inserted in plasmid pAP246B is shown. In order to identify the position of an exon present in the fragment, plasmid pAP246B is digested with restriction enzyme ScaI alone and with various combinations of restriction enzyme ScaI with other restriction enzymes as indicated in Fig.2(B), and analyzed by Southern blot hybridization using $^{32}P$-labeled probe a as obtained in Example 30. As a result, it is found that about 2 kb of BamHI-ScaI fragment and about 1.5 kb of ScaI fragment are individually hybridized with the probe, and exons are present in these fragments.

Step 5

(Determination of the base sequence of exon I)

About 2 kb of BamHI-ScaI fragment is cut off from plasmid pAP246B and purified. After subcloning of the fragment into vector M13, the base sequence of the fragment is determined by sequencing in accordance with the method of Sanger et al [Sanger, F., Science, 214, 1205-1210, (1981)] and in accordance with the protocol appended to M13 sequencing kit manufactured and sold by Takara Shuzo Co., Ltd., Japan. The sequencing strategy, namely, directions and lengths of the sequencing is indicated by arrows in Fig.2(C).

Thus, there is determined the base sequence of the fragment with respect to 226 bases, which sequence corresponds to the base sequence of from the 1st to 226th bases in Fig. 5.

Further, for the purpose of the determination of a base sequence of the 3′-region of the above-mentioned fragment, 1.5 kb of ScaI-EcoRI fragment is cut off from plasmid pAP246B. This fragment is subcloned into vector M13. Then, the base sequence of the fragment is determined in the same manner as mentioned above. As a result, the fragment comprises a base sequence corresponding to the base sequence of the 227th to 457th bases in Fig. 5.

The above-obtained base sequences of the fragments (457 bases in total) are compared with the base sequence of NAP cDNA. As a result, it is found that the base sequence of 168 bases of from the 86th to 253rd bases in Fig. 5 is identified as an exon. This exon is designated "exon I".

Step 6

(Determination of the base sequence of exon J)

On the basis of the results of Step 3, 1.5 kb ScaI fragment of plasmid pAP246B is isolated. On the other hand, vector pUC18 is digested with SmaI and treated with BAP to obtain a vector fragment. Then, both the fragments are ligated to each other by means of T4 ligase and transfected into cells of Escherichia coli JM105 in the same manner as in Step 4. The thus obtained transformed cells are transferred onto Whatmann 541 filter, and then subjected to screening in the same manner as in Step 2 of Example 1 using $^{32}P$-labeled NAP cDNA fragment (probe a) prepared in Example 30. Prasmids are extracted from the resultant 12 positive colonies by a rapid, small-scale plasmid isolation method. The prasmids are digested with EcoRI and HindIII and analyzed. As a result, it is confirmed that the desired fragment is inserted in the plasmids. One of the plasmids is designated "plasmid pAP-SS1.5".

Further, in order to identify the position of an exon present in plasmid pAP-SS1.5, plasmid pAP-SS1.5 is digested with restriction enzymes EcoRI, BglII and AvaI. The digests are subjected to Southern blot analysis using the above-mentioned probe a. As a result, it is found that an exon is present in a BglII-EcoRI fragment of about 0.6 kb.

This fragment is isolated and subcloned in vector M13 in the same manner as described in Step 4 and the base sequence of the fragment is determined. As a result, the fragment comprises a base sequence corresponding to the base sequence of 395 bases in Fig.6.

The above-mentioned base sequence is compared with the base sequence of NAP cDNA. As a result, it is found that a base sequence of 57 bases of from the 196th to 252nd bases in Fig. 6 is identified as an exon. This exon is designated "exon J".

Example 43

(Cloning of exon K)

Step 1

(Preparation of a probe)

A λ24DNA obtained in Step 3 of Example 42 is digested with restriction enzymes EcoRI and SalI and subjected to agarose gel electrophoresis. Then, a DNA fragment of about 7 kb is extracted from the gel and purified. The thus obtained DNA fragment is inserted in vector pUC18 which has been digested with EcoRI and SalI, to obtain plasmid pGAP-ES247. This plasmid pGAP-ES247 is digested completely with HindIII and, then, partially with PstI and subjected to analysis by agarose gel electrophoresis. As a result, it is found that PstI site is present in the DNA fragment at a position of around the 400th base counted from the 3′-end thereof.

8 μg of plasmid pGAP-ES247DNA is digested with restriction enzyme PstI and subjected to agarose gel electrophoresis. A fragment of about 400 bp is extracted from the gel. This DNA fragment is labeled with $^{32}P$ in the same manner as in Example 30. The thus obtained $^{32}P$-labeled DNA fragment is designated "probe b" and used in the following screening.

Step 2

(Screening and purification of clones)

Using $^{32}P$-labeled probe b prepared in Step 1 above, plaque hybridization is conducted in the same manner as in Step 2 of Example 42. As a result, 6 positive spots which are hybridized with probe b are obtained from $6 \times 10^5$ plaques. Then, the second plaque hybridization is conducted with respect to the obtained plaques to individually purify 6 phage clones. The thus obtained 6 phage clones are designated λ31, λ32, λ33, λ34, λ35 and λ36, respectively.

Step 3

(Analysis of each clone)

According to the method as described in Step 3 of Example 42, each phage clone prepared in Step 2 and λ24 DNA prepared in Step 1 of Example 42 are respectively digested with various restriction enzymes, and the digestion patterns of each clone and λ24 DNA are compared by agarose gel electrophoresis. As a result, it is found that the digestion patterns of λ32, λ33, λ34 DNA completely coincide with that of λ24 DNA and these phages are the same clone. Further, it is found that the digestion patterns of λ31 DNA coincide with that of λ36 DNA and these are the same clones, but a DNA fragment coincident with a digest of λ24 DNA is not detected. Furthermore, it is found that most of DNA fragments obtained from λ35 DNA coincide with those of λ24 DNA except for a novel EcoRI-HindIII fragment of about 3 kb.

These λ31, λ32, λ35 and λ36 DNA's are digested with restriction enzymes EcoRI and SalI and subjected to analysis by hybridization by the same method as described in Step 4 of Example 42. Illustratively stated, the DNA digests-transferred filter is hybridized with $^{32}P$-labeled synthetic oligonucleotide INS-3′ (5′-GGGGTACTGGCTGCTGTTGTAGGAA-3′) as a probe in a buffer containing 1 x $10^5$ cpm/ml of the probe at 55°C for 2 hours. The resultant filter is washed with 6 x SSC solution containing 0.1 % SDS at 50°C, and subjected to autoradiography. As a result, a fragment of about 15 kb, a fragment of about 15 kb and a fragment of about 3 kb are individually hybridized with the probe with respect to phages λ31, λ36 and λ35, respectively. On the other hand, with respect to phage λ32, no fragments are hybridized.

Step 4

(Determination of a base sequence of exon K and introns adjacent thereto)

A EcoRI-SalI DNA fragment of about 3.0 kb obtained from λ35 DNA is subjected to subcloning in vector pUC18 which has been digested with EcoRI and SalI. The thus obtained subcloned plasmid is designated "plasmid pGAP-K3". Escherichia coli JM105 is transformed with plasmid pGAP-K3. The resultant transformant is designated E. coli K-12 strain JM105 (pGAP-K3).

E. Coli K-12 strain JM105 (pGAP-K3) is deposited at Fermentation Research Institute under the accession number FERM BP-1996 on April 8, 1988.

The plasmid pGAP-K3 is digested with various restriction enzymes and subjected to agarose gel electrophoresis. Then, using $^{32}P$-labeled INS-3′ prepared in Step 3 as a probe, hybridization of the digests is conducted. Thus, a restriction map of a chromosomal DNA containing an exon K is obtained and a position of an exon in the chromosomal DNA is determined [Fig.2(C)].

According to the sequencing strategy as indicated by arrows in Fig. 2(C), plasmid pGAP-K3 is digested with various restriction enzymes, and the resultant fragments are extracted and subjected to subcloning into M13 vector. Then, the base sequence of each of the subcloned digests is determined in the same manner as in Step 5 of Example 1. Thus, there is obtained a base sequence of 953 bases as shown in Fig. 7. By the comparison of the above-obtained base sequence with that of NAP cDNA, it is found that the underlined base sequence of from the 623rd to 756th bases in Fig.7 is of an exon. The exon is designated "exon K".

Example 44

(Cloning of exon H)

Step 1

(First screening using a synthetic probe)

According to the method as described in Step 2 of Example 42, screening is conducted by plaque hybridization using a synthetic probe mentioned below. Illustratively stated, a synthetic oligonucleotide INS-5′ (5-CTCGAACCAC-CTCTTCCACAGACTC-3′) labeled with $^{32}P$ at its 5′-end is added to a buffer containing 5 x SSC, 1 % glycine, 25 mM phosphate, 5 x Denhardt and 0.1 % SDS, at an oligonucleotide concentration of 4 x $10^5$ cpm/ml. Then, the plaque-transferred nitrocellulose filter as obtained in Step 2 of Example 42 is put in the buffer and incubated at 55 °C for 4 hours to conduct hybridization. The resultant filter is washed with 6 x SSC solution containing 0.1 % of SDS at 55°C and subjected to autoradiography, thereby obtaining 8 positive spots.

Step 2

(Selection and purification of clone)

Plasmid pGBP2DNA obtained in Step 4 of Example 1 is digested with restriction enzymes AccI and TaqI, and the resultant digests are subjected to agarose gel electrophoresis. Then, a fragment of about 640 bp is extracted from the gel and purified. The resultant fragment is labeled with $^{32}P$ in the same manner as described in Example 30. The thus obtained $^{32}P$-labeled fragment is designated "probe c".

Phages corresponding to the above-obtained 8 positive spots are extracted from an agar and, subjected to plaque hybridization with probe c. As a result, 2 phage clones are hybridized with probe c. Then, the plaque hybridization is subjected again to purify the phage clones. The thus obtained purified phage clones are designated λ51 and λ57, respectively.

Step 3

(Restriction analysis of λ51 and λ57 DNA's)

According to the method described in Step 3 of Example 42, each DNA of λ51 and λ57 obtained in Step 2 above is isolated. The thus obtained DNA's are digested with restriction enzymes EcoRI and SalI and subjected to agarose gel electrophorisis. As a result, it is found that three fragments of about 1.2 kb, about 2.0 kb and about 5.7 kb are common to λ51 and λ57 DNA's. These DNA fragments are analyzed by Southern blot hybridization using probe c prepared in Step 2 above. 5.4 kb fragment of λ51 DNA and 6.7 kb fragment of λ57 DNA are hybridized with probe c. These fragments are extracted from the agarose gel and purified. Then, the resultant fragments are individually ligated to vector pUC19 digested with EcoRI and SalI using T4 ligase to thereby obtain plasmid pGAP-ES51 containing λ51-derived DNA and plasmid pGAP-ES57 containing λ57-derived DNA.

Each of plasmids pGAP-ES51 and pGAP-ES57 is digested with various restriction enzymes and subjected to agarose gel electreophoresis. As a result, it is found that plasmid pGAP-ES57DNA has a restriction map shown in Fig. 3(A). Further, it is found that plasmid pGAP-ES51 is 1300 bp shorter than plasmid pGAP-ES57, but plasmids pGAP-ES51 and pGAP-ES57 have DNA fragments derived from a common chromosomal gene. In order to identify the position of an exon in the DNA insert of the plasmids, the plasmids are digested with various restriction enzymes and subjected to agarose gel electrophoresis. Then, the DNA digests are analyzed by Southern blot hybridization using the synthetic probe INS-5′ obtained in Step 1 above. As a result, it is found that an exon exists at the position indicated in the restriction map of Fig. 3(B).

Step 4

(Determination of base sequence of exon H and introns adjacent thereto)

According to the sequencing strategy shown in Fig. 3(B), the sequencing of plasmid pGAP-ES57 is conducted as follows. The plasmid is digested with restriction enzymes RsaI, PstI and TaqI. Then, the obtained fragments are purified and subjected to subcloning. The base sequence of each of the fragments is determined in the same manner as in Step 5 of Example 1. Thus, a base sequence of 895 bases shown in Fig. 4 is obtained. The thus obtained base sequence is compared with that of NAP cDNA. As a result, it is found that a base sequence corresponding to the base sequence of from the 490th to 692nd bases in Fig. 4 is of an exon. This exon is designated "exon H".

Example 45

(Southern blot analysis of chromosomal DNA using synthetic probe specific for the splice junction of exon I)

Human chromosomal DNA prepared in Step 1 of Example 42 is digested with BamHI, EcoRI and HindIII and the resultant digests are analyzed by Southern blot analysis using synthetic oligonucleotide int-I5 (5′- ATCTCCTCTGATT-AGAGGTGTGCTC-3′) labeled with $^{32}P$ at its 5′-end which is specific for the splice junction of exon I. In practicing the Southern blot analysis, the hybridization is conducted in a buffer containing 25mM sodium phosphate (pH6.5), 5 x SSC, 1 % glycin, 5 X Denhardt's and 0.1 % SDS, and 4 x $10^5$ cpm/ml of $^{36}P$-labeled int-I5 at 55°C for 4 hours. The resultant filter is washed with 6 x SSC containing 0.1 % SDS at 55°C and then, subjected to autoradiography. As a result, the same hybridization pattern as obtained in Step 1 of Example 42 is obtained. That is, 6.6 kb fragment, 7.4 kb fragment and 8.2 kb fragment are hybridized with the probe in the case of the digestions with BamHI, EcoRI and HindIII, respectively. The results are shown in Fig. 14.

Example 46

(Southern blot analysis of a chromosomal DNA using a synthetic probe specific for the splice junction of exon K)

The filter used in Example 45 is washed with 0.1 x SSC solution containing 0.5 % SDS at 65°C for 1 hour to remove synthetic probe int-I5 hybridized with the filter. The filter is subjected to autoradiography to confirm that int-I5 is completely removed. Then, using $^{32}P$-labeled synthetic oligonucleotide probe IE-K5 (5′-TCTACTTTATAGTTCCTACAACAGCA-3') specific for the splice junction of exon K, hybridization is conducted in the same manner as in Example 45 and subjected to autoradiography. As a result, it is found that about 3.8 kb fragment, about 15 kb fragment and about 7.2 kb fragment are individually hybridized with the probe in the case of the digestions with BamHI, EcoRI and HindIII, respectively.

Example 47

The protease inhibiting activities of the secreted PI obtained in Example 7 against various proteases are determined.

As protease, those listed in Table 8 below are used.

Table 8

| Enzyme | Origin | Manufacturer |
|---|---|---|
| trypsin | porcine pancreas | Sigma Chemical Company, U.S.A. |
| chymotrypsin | bovine pancreas | Funakoshi Pharmaceutical Co., Ltd.,Japan |
| factor Xa | bovine plasma | Sigma Chemical Company, U.S.A. |
| kallikrein | human urine | Funakoshi Pharmaceutical Co., Ltd.,Japan |
| kallikrein | human plasma | Funakoshi Pharmaceutical Co., Ltd.,Japan |
| plasmin | human plasma | Sigma Chemical Company, U.S.A. |

Further, the following substrates are used.

Table 9

| Enzyme | Substrate |
|---|---|
| trypsin | Benzoyl-Arg-AMC |
| chymotrypsin | Ala-Ala-Phe-AMC |
| factor Xa | Boc-Ile-Glu-Gly-Arg-AMC |
| kallikrein | Boc-Phe-Arg-AMC |
| plasmin | Boc-Val-Leu-Lys-AMC |

Note: AMC 7-amido-4-methylcoumarin

In determining the protease inhibiting activities, the following buffer and substrate solution are used.

Buffer: a 50 mM Tris-HCℓ buffer (pH8.0) containing 100 mM NaCℓ and 10 mM $CaCℓ_2$.

Substrate solution: the same buffer as mentioned above except that 0.2 M substrate and 1 % (w/w) dimethylsulfoxide are contained.

The procedure for the determination of the protease inhibiting activities of the sPI of the present invention is as follows.

The sPI is dissolved in the above-mentioned buffer at various concentrations. To each of the resultant solutions is added an enzyme solution containing the above-mentioned protease and allowed to stand for 5 min. The concentrations of enzymes are set so that the hydrolysis rates of the substrate by the enzymes are almost same. To the resultant mixture is added an equal amount of the substrate solution. With the passage of time, the resultant mixture is continuously subjected to determination of the intensity of fluorescence at 450 nm which is obtained under the light excitation with 365 nm. Based on the obtained intensities of the fluorescence, the reaction rate of each enzyme-substrate reaction is calculated. Then the relative activity of each enzyme is calculated by the following formula:

$$\frac{A}{B} \times 100\ (\%)$$

wherein A is the reaction rate of an enzyme in the presence of the secretable sPI, and B is the reaction rate of the enzyme in the absence of the secretable sPI.

The results are shown in Fig. 16.

Further, the PI concentrations which inhibit 50 % of activities of the enzymes ($IC_{50}$) are shown in the following Table 10.

Table 10

| Enzyme | $Ic_{50}(x10^{-5}M)$ |
|---|---|
| trypsin | 7.0 |
| chymotrypsin | 1.8 |
| factor Xa | >77 |
| kallikrein (plasma) | 77 |
| kallikrein (urine) | >77 |
| plasmin | 38 |

From Table 10, it is apparent that sPI has high inhibiting activities against trypsin and chymotrypsin.

## Claims

**1.** A protein comprising an amino acid sequence represented by the following formula (I):

Val-Cys-Ser-Glu-Gln-Ala-Glu-Thr-Gly-Pro-Cys-
Arg-Ala-Met-Ile-Ser-Arg-Trp-Tyr-Phe-Asp-Val-
Thr-Glu-Gly-Lys-Cys-Ala-Pro-Phe-Phe-Tyr-Gly-
Gly-Cys-Gly-Gly-Asn-Arg-Asn-Asn-Phe-Asp-Thr-
Glu-Glu-Tyr-Cys-Met-Ala-Val-Cys-Gly-Ser-Ala ... (I).

**2.** A protein comprising an amino acid sequence represented by the following formula (II):

Glu-(I)-Ile (II)

wherein (I) is a peptide having the same amino acid sequence as defined by formula (I) in claim 1.

**3.** A protein comprising an amino acid sequence represented by the following formula (III):

$X_1$-(I)-$X_2$ (III)

wherein (I) is a peptide having the same amino acid sequence as defined by formula (I) in claim 1, $X_1$ is a peptide having an amino acid sequence corresponding to the amino acid sequence of the 1st to 289th amino acids shown in Fig. 1 (B), and $X_2$ is a peptide having an amino acid sequence corresponding to the amino acid sequence of the 345th to 751st amino acids shown in Fig. 1 (B).

**4.** A protein comprising an amino acid sequence represented by the following formula (IV):

Glu-(I)-Met-Ser-Gln-Ser-Leu-Leu-Lys-Thr-Thr-
Gln-Glu-Pro-Leu-Ala-Arg-Asp-Pro-Val-Lys-Leu
... (IV)

wherein (I) is a peptide having the same amino acid sequence as defined by formula (I) in claim 1.

**5.** A protein comprising an amino acid sequence represented by the following formula (V):

$X_3$-(I)-$X_4$ (V)

wherein (I) is a peptide having the same amino acid sequence as defined by formula (I) in claim 1, $X_3$ is a peptide having an amino acid sequence corresponding to the amino acid sequence of the 1st to 289th amino acids shown in Fig. 1 (A), and $X_4$ is a peptide having an amino acid sequence corresponding to the amino acid sequence of the 345th to 770th amino acids shown in Fig. 1 (A).

**6.** The protein according to any one of claims 1 to 5, which is a human brain senile plaque amyloid precursor protein.

**7.** The protein according to any one of claims 1 to 5, which has a protease inhibiting activity.

**8.** A DNA comprising at least one base sequence selected from the group consisting of a base sequence represented by the following formula (VI):

```
1        10         20         30         40
AGGTGTGCTC TGAACAAGCC GAGACGGGGC CGTGCCGAGC

         50         60         70         80
AATGATCTCC CGCTGGTACT TTGATGTGAC TGAAGGGAAG

         90        100        110        120
TGTGCCCCAT TCTTTTACGG CGGATGTGGC GGCAACCGGA

        130        140        150        160
ACAACTTTGA CACAGAAGAG TACTGCATGG CCGTGTGTGG

      168
CAGCGCCA                           ... (VI),
```

and a base sequence complementary thereto.

**9.** A DNA comprising at least one base sequence selected from the group consisting of a base sequence represented by formula (VII):

$$G\text{-}(VI)\text{-}TT \quad (VII)$$

wherein (VI) is a DNA having the same base sequence as defined by formula (VI) in claim 8, and a base sequence complementary thereto.

**10.** A DNA comprising at least one base sequence selected from the group consisting of a base sequence represented by the following formula (VIII):

$$Y_1\text{-}(VI)\text{-}Y_2 \quad (VIII)$$

wherein (VI) is a DNA having the same base sequence as defined by formula (VI) in claim 8, $Y_1$ is a DNA having a base sequence corresponding to the sequence of the 1st to 865th bases shown in Fig. 1 (B), and $Y_2$ is a DNA having a base sequence corresponding to the base sequence of the 1034th to 2253rd bases shown in Fig. 1 (B), and a base sequence complementary thereto.

**11.** A DNA comprising at least one base sequence selected from the group consisting of a base sequence represented by the following formula (IX):

$$(VI)\text{-}Y_3 \quad (IX)$$

wherein (VI) is a DNA having the same base sequence as defined by formula (VI) in claim 8, and $Y_3$ is a DNA having a base sequence represented by the following formula (X):

```
TG TCCCAAAGTT TACTCAAGAC TACCCAGGAA CCTCTTGCCC

GAGATCCTGT TAAAC  ... (X),
```

and a base sequence complementary thereto.

**12.** A DNA comprising at least one base sequence selected from the group consisting of a base sequence represented by the following formula (XI):

$$G\text{-(VI)-}Y_3\text{-TT} \qquad (XI)$$

wherein (VI) is a DNA having the same base sequence as difined by formula (VI) in claim 8, and $Y_3$ has the same meaning as defined by formula (X) in claim 11,
and a base sequence complementary thereto.

**13.** A DNA comprising at least one base sequence selected from the group consisting of a base sequence represented by the following formula (XII):

$$Y_4\text{-(VI)-}Y_5 \qquad (XII):$$

wherein (VI) is a DNA having the same base sequence as defined by formula (VI) in claim 8, and $Y_4$ is a DNA having a base sequence corresponding to the base sequence of the 1st to 865th bases shown in Fig. 1 (A), and $Y_5$ is a DNA having a base sequence corresponding to the base sequence of the 1034th to 2310th bases shown in Fig. 1 (A),
and a base sequence complementary thereto.

**14.** An RNA corresponding to a DNA of claims 8 to 13.

**15.** A human chromosomal DNA fragment comprising as an exon a base sequence represented by the following formula (VI):

```
1        10         20         30         40
AGGTGTGCTC TGAACAAGCC GAGACGGGGC CGTGCCGAGC

         50         60         70         80
AATGATCTCC CGCTGGTACT TTGATGTGAC TGAAGGGAAG

         90        100        110        120
TGTGCCCCAT TCTTTTACGG CGGATGTGGC GGCAACCGGA

        130        140        150        160
ACAACTTTGA CACAGAAGAG TACTGCATGG CCGTGTGTGG

     168
CAGCGCCA                              ... (VI).
```

**16.** The human chromosomal DNA fragment according to claim 15, which further comprises at least a portion of a base sequence corresponding to the base sequence of the 1st to 85th bases shown in Fig. 5 and at least a portion of a base sequence corresponding to the base sequence of the 254th to 457th bases shown in Fig. 5, attached to said base sequence of formula (VI) at its 5'-end and 3'-end, respectively.

**17.** The human chromosomal DNA fragment according to claim 16, which further comprises a base sequence as shown in Fig. 6 and which has a molecular length of about 6.6 kb and a restriction endonuclease cleavage map as shown in (B) of Fig. 2 as a BamHI-BamHI insert in plasmid pAP246B, said plasmid pAP246B being carried by Escherichia coli K-12 strain JM105 (pAP246B) deposited with the Fermentation Research Institute, Japan, under accession No. FERM BP 1987.

**18.** The human chromosomal DNA fragment according to any one of claims 15 to 17, which relates to expression of a portion of a human brain senile plaque amyloid precursor protein, said portion having a protease inhibiting activity.

**19.** A human chromosomal DNA fragment comprising as an exon a base sequence represented by the following formula (X):

TG TCCCAAAGTT TACTCAAGAC TACCCAGGAA

CCTCTTGCCC GAGATCCTGT TAAAC ... (X).

**20.** The human chromosomal DNA fragment according to claim 19, which further comprises at least a protion of a base sequence corresponding to the base sequence of the 1st to 195th bases shown in Fig. 6 and at least a portion of a base sequence corresponding to the base sequence of the 253rd to 395th bases shown in Fig. 6, attached to said base sequence of formula (X) at its 5'-end and 3'-end, respectively.

**21.** An RNA corresponding to a DNA of claims 15 to 22.

**22.** A replicable recombinant DNA comprising a replicable expression vector and, ligated thereto, a promotor and a DNA coding for a protein comprising an amino acid sequence of formula (III) as defined in claim 3.

**23.** The replicable recombinant DNA according to claim 22, which further comprises a DNA coding for a signal peptide, said DNA coding for a signal peptide being ligated between said promotor and said DNA coding for said protein, constituting a reading frame so as to enable a fused peptide comprising said signal peptide and said protein to be produced by expression of said DNA's.

**24.** A transformant comprising a microorganism or cell and, contained therein, a replicable recombinant DNA comprising a replicable expression vector and, ligated thereto, a promotor and a DNA coding for a protein comprising an amino acid sequence of formula (III) as defined in claim 3.

**25.** A replicable recombinant DNA comprising a replicable expression vector and, ligated thereto, a promotor and a DNA coding for a protein comprising an amino acid sequence of formula (V) as defined in claim 5.

**26.** The replicable recombinant DNA according to claim 25, which further comprises a DNA coding for a signal peptide, said DNA coding for a signal peptide being ligated between said promotor and said DNA coding for said protein, constituting a reading frame so as to enable a fused peptide comprising said signal peptide and said protein to be produced by expression of said DNA's.

**27.** A transformant comprising a microorganism or cell and, contained therein, a replicable recombinant DNA comprising a replicable expression vector and, ligated thereto, a promotor and a DNA coding for a protein comprising an amino acid sequence of formula (V) as defined in claim 5.

**28.** Escherichia coli K-12 strain HB101 (pGBP2) deposited with the American Type Culture Collection, U.S.A., under Accession No. ATCC 67502.

**29.** Escherichia coli K-12 strain JM105 (pAP246B) deposited with the Fermentation Research Institute, Japan, under Accession No. FERM BP-1987.

**30.** An antibody specific for a human brain senile plaque amyloid precursor protein selected from the group consisting of amino acid sequences of formula (III) as defined in claim 3 and of formula (V) as defined in claim 5.

**31.** The antibody according to claim 30, which is a monoclonal antibody produced by a cell line selected from the group consisting of a mouse hybrid cell line ADI1-7-5-2, a mouse hybrid cell line ADI1-4-6-1 and a mouse hybrid cell line ADI1-05-44-2, said mouse hybrid cell line ADI1-7-5-2 being deposited with the Fermentation Research Institute, Japan, under the accession No. FERM BP 1994, said mouse hybrid cell line ADI1-4-6-1 being deposited with the Fermentation Research Institute, Japan, under the accession No. FERM BP 1995, and said mouse hybrid cell line ADI1-05-44-2 being deposited with the Fermentation Research Institute, Japan, under the accession No. FERM BP 1988.

**32.** A method for detecting a human brain senile plaque amyloid precursor protein selected from the group consisting of amino acid sequences of formula (III) as defined in claim 3 and of formula (V) as defined in claim 5, which comprises subjecting a sample to immunoassay using an antibody of claim 30 specific for said amino acid sequence.

**33.** A method for detecting a DNA of claim 8, which comprises subjecting a sample to assay by hybridization using a probe selected from the group consisting of (i) a DNA comprising at least a portion of a base sequence of formula (VI) as defined in claim 8, (ii) a complementary DNA to said DNA and (iii) RNA's corresponding thereto.

**34.** A method for detecting a DNA of claim 10, which comprises subjecting a sample to assay by hybridization using a probe selected from the group consisting of (i) a DNA comprising at least a portion of a base sequence of formula (VIII) as defined in claim 10, (ii) a complementary DNA to said DNA and (iii) RNAs corresponding thereto.

**35.** A method for detecting a DNA of claim 13, which comprises subjecting a sample to assay by hybridization using a probe selected from the group consisting of (i) a DNA comprising at least a portion of a base sequence of formula (XII) as defined in claim 13, (ii) a complementary DNA to said DNA and (iii) RNAs corresponding thereto.

**36.** A method for detecting a DNA of claim 8 which is present in cDNA coding for a human senile amyloid precursor protein, which comprises subjecting a sample to assay by hybridization using a probe selected from the group consisting of (i) a DNA comprising at least twelve continuously sequenced bases in a base sequence of formula

G GTGGTTCGAG - (VI) - TT CCTACAACA

wherein (VI) is a DNA having the same base sequence as defined by formula (VI) in claim 8, (ii) a complementary DNA to said DNA and (iii) RNAs corresponding thereto.

**37.** A method for detecting a DNA of claim 11 which is present in cDNA coding for a human brain senile amyloid precursor protein, which comprises subjecting a sample to assay by hybridization using a probe selected from the group consisting of (i) a DNA comprising at least twelve continuously sequenced bases in a base sequence of formula

G GTGGTTCGAG - (IX) - TT CCTACAACA

wherein (IX) is a DNA having the same base sequence as defined by formula (IX) in claim 11, (ii) a complementary DNA to said DNA and (iii) RNAs corresponding thereto.

**38.** A method for detecting an RNA corresponding to the DNA of claim 8, which comprises subjecting a sample to assay by hybridization using a probe selected from the group consisting of (i) a complementary DNA to a DNA comprising at least a portion of a base sequence of formula (VI) as defined in claim 8, and (ii) an RNA corresponding thereto.

**39.** A method for detecting an RNA corresponding to the DNA of claim 10, which comprises subjecting a sample to assay by hybridization using a probe selected from the group consisting of (i) a complementary DNA to a DNA comprising at least a portion of a base sequence of formula (VIII) as defined in claim 10, and (ii) an RNA corresponding thereto.

**40.** A method for detecting an RNA corresponding to the DNA of claim 13, which comprises subjecting a sample to assay by hybridization using a probe selected from the group consisting of (i) a complementary DNA to a DNA comprising at least a portion of a base sequence of formula (XII) as defined in claim 13, and (ii) an RNA corresponding thereto.

**41.** A method for detecting an RNA corresponding to the DNA of claim 8, which comprises subjecting a sample to assay by hybridization using a probe selected from the group consisting of (i) a complementary DNA to at least twelve continuously sequenced bases of the formula

G GTGGTTCGAG - (VI) - TT CCTACAACA

wherein (VI) is a DNA having the same base sequence as defined by formula (VI) in claim 8, and (ii) an RNA corresponding to said complementary DNA (i).

**42.** A method for detecting an RNA corresponding to the DNA of claim 11, which comprises subjecting a sample to assay by hybridization using a probe selected from the group consisting of (i) a complementary DNA to at least twelve continuously sequenced bases of the formula

G GTGGTTCGAG - (IX) - TT CCTACAACA

wherein (IX) is a DNA having the same base sequence as defined by formula (IX) in claim 11, and (ii) an RNA corresponding to said complementary DNA (i).

**43.** A method for detecting a human chromosomal DNA fragment relating to expression of a polypeptide having a protease inhibiting activity, said polypeptide being a portion of a human brain senile plaque amyloid precursor protein, which comprises subjecting a sample to assay by hybridization using as a probe at least a portion of any one of human chromosomal DNA fragments of claims 15 to 17, and claims 19 to 20.

**44.** A probe for use in detecting a DNA or RNA coding for a polypeptide related to human brain senile plaque amyloid precursor protein, which comprises a base sequence selected from the group consisting of (i) a DNA comprising at least twelve continuously sequenced bases in a base sequence of the formula

G GTGGTTCGAG - (VI) - TT CCTACAACA

wherein (VI) is a DNA having the same base sequence as defined by formula (VI) in claim 8, (ii) a complementary DNA to said DNA and (iii) RNAs corresponding thereto.

**45.** A probe for use in detecting a DNA or RNA coding for a polypeptide related to human brain senile plaque amyloid precursor protein, which comprises a base sequence selected from the group consisting of (i) a DNA comprising at least twelve continuously sequenced bases in a base sequence of the formula

G GTGGTTCGAG - (IX) - TT CCTACAACA

wherein (IX) is a DNA having the same base sequence as defined by formula (IX) in claim 11, (ii) a complementary DNA to said DNA and (iii) RNAs corresponding thereto.

**Patentansprüche**

**1.** Protein, aufweisend eine durch die folgende Formel (I) wiedergegebene Aminosäuresequenz:

Val-Cys-Ser-Glu-Gln-Ala-Glu-Thr-Gly-Pro-Cys-
Arg-Ala-Met-Ile-Ser-Arg-Trp-Tyr-Phe-Asp-Val-
Thr-Glu-Gly-Lys-Cys-Ala-Pro-Phe-Phe-Tyr-Gly-
Gly-Cys-Gly-Gly-Asn-Arg-Asn-Asn-Phe-Asp-Thr-
Glu-Glu-Tyr-Cys-Met-Ala-Val-Cys-Gly-Ser-Ala ... (I).

**2.** Protein, aufweisend eine durch die folgende Formel (II) wiedergegebene Aminosäuresequenz:

Glu-(I)-Ile (II)

worin (I) ein Peptid mit derselben Aminosäuresequenz ist wie in Formel (I) in Anspruch 1 definiert.

**3.** Protein, aufweisend eine durch die folgende Formel (III) wiedergegebene Aminosäuresequenz:

$X_1$-(I)-$X_2$ (III)

worin (I) ein Peptid mit derselben Aminosäuresequenz ist wie in Formel (I) in Anspruch 1 definiert, $X_1$ ein Peptid mit einer Aminosäuresequenz entsprechend der Aminosäuresequenz der 1. bis 289. der in Fig. 1 (B) dargestellten Aminosäuren und $X_2$ ein Peptid mit einer Aminosäuresequenz entsprechend der Aminosäuresequenz der 345. bis 751. der in Fig. 1 (B) dargestellten Aminosäuren.

**4.** Protein, aufweisend eine durch die folgende Formel (IV) wiedergegebene Aminosäuresequenz:

Glu-(I)-Met-Ser-Gln-Ser-Leu-Leu-Lys-Thr-Thr-
Gln-Glu-Pro-Leu-Ala-Arg-Asp-Pro-Val-Lys-Leu

... (IV)

worin (I) ein Peptid mit derselben Aminosäuresequenz ist wie in Formel (I) in Anspruch 1 definiert.

**5.** Protein, aufweisend eine durch die folgende Formel (V) wiedergegebene Aminosäuresequenz:

$$X_3\text{-(I)-}X_4 \qquad \text{(V)}$$

worin (I) ein Peptid mit derselben Aminosäuresequenz ist wie in Formel (I) in Anspruch 1 definiert, $X_3$ ein Peptid mit einer Aminosäuresequenz entsprechend der Aminosäuresequenz der 1. bis 289. der in Fig. 1 (A) dargestellten Aminosäuren und $X_4$ ein Peptid mit einer Aminosäuresequenz entsprechend der Aminosäuresequenz der 345. bis 770. der in Fig. 1 (A) dargestellten Aminosäuren.

**6.** Protein gemäß einem der Ansprüche 1 bis 5, welches ein Senil-Plaque-Amyloid-Precursor-Protein des menschlichen Gehirns ist.

**7.** Protein gemäß einem der Ansprüche 1 bis 5, das eine Protease inhibierende Aktivität besitzt.

**8.** DNA, aufweisend wenigstens eine Basensequenz, die aus der Gruppe ausgewählt ist, die aus einer Basensequenz besteht, die durch die folgende Formel (VI) wiedergegeben wird:

```
1         10         20         30         40
AGGTGTGCTC TGAACAAGCC GAGACGGGGC CGTGCCGAGC

        50         60         70         80
AATGATCTCC CGCTGGTACT TTGATGTGAC TGAAGGGAAG

       , 90        100        110        120
TGTGCCCCAT TCTTTTACGG CGGATGTGGC GGCAACCGGA

       130        140        150        160
ACAACTTTGA CACAGAAGAG TACTGCATGG CCGTGTGTGG

      168
CAGCGCCA                          ... (VI),
```

und einer dazu komplementären Basensequenz.

**9.** DNA, aufweisend wenigstens eine Basensequenz, die aus einer Gruppe ausgewählt ist, die aus einer Basensequenz besteht, die durch die folgende Formel (VII) wiedergegeben wird:

G-(VI)-TT (VII)

worin (VI) eine DNA mit derselben Basensequenz ist wie durch Formel (VI) in Anspruch 8 definiert, und eine dazu komplementäre Basensequenz.

**10.** DNA, aufweisend wenigstens eine Basensequenz, die aus der Gruppe ausgewählt ist, die aus der Basensequenz besteht, die durch die folgende Formel (VIII) wiedergegeben wird:

$$Y_1\text{-(VI)-}Y_2 \qquad \text{(VIII)}$$

worin (VI) eine DNA mit derselben Basensequenz ist wie durch Formel (VI) in Anspruch 8 definiert, $Y_1$ eine DNA

mit einer Basensequenz entsprechend der Sequenz der 1. bis 865. der in Fig. 1 (B) dargestellten Basen und $Y_2$ eine DNA mit einer Basensequenz entsprechend der Basensequenz der 1034. bis 2253. der in Fig. 1 (B) dargestellten Basen, und eine dazu komplementäre Basensequenz.

**11.** DNA, aufweisend wenigstens eine Basensequenz, die aus einer Gruppe ausgewählt ist, die aus einer Basensequenz besteht, die durch die folgende Formel (IX) wiedergegeben wird:

$$(VI)\text{-}Y_3 \qquad (IX)$$

worin (VI) eine DNA mit derselben Basensequenz ist wie durch Formel (VI) in Anspruch 8 definiert und $Y_3$ eine DNA mit einer durch die folgende Formel (X) wiedergegebene Basensequenz:

```
TG TCCCAAAGTT TACTCAAGAC TACCCAGGAA CCTCTTGCCC

GAGATCCTGT TAAAC                                  ... (X)
```

und eine dazu komplementäre Basensequenz.

**12.** DNA, aufweisend wenigstens eine Basensequenz, die aus der Gruppe ausgewählt ist, die aus einer Basensequenz besteht, die durch die nachfolgende Formel (XI) wiedergegeben wird:

$$G\text{-}(VI)\text{-}Y_3\text{-}TT \qquad (XI)$$

worin (VI) eine DNA mit derselbens Basensequenz ist wie durch Formel (VI) in Anspruch 8 definiert und $Y_3$ dieselbe Bedeutung hat wie durch Formel (X) in Anspruch 11 definiert, und eine dazu komplementäre Basensequenz.

**13.** DNA, aufweisend wenigstens eine Basensequenz, die aus der Gruppe ausgewählt ist, die aus einer Basensequenz besteht, die durch die folgende Formel (XII) wiedergegeben wird:

$$Y_4\text{-}(VI)\text{-}Y_5 \qquad (XII)$$

worin (VI) eine DNA mit derselben Basensequenz ist wie durch Formel (VI) in Anspruch 8 definiert, $Y_4$ eine DNA mit einer Basensequenz entsprechend der Basensequenz der 1. bis 865. der in Fig. 1 (A) dargestellten Basen und $Y_5$ eine DNA mit einer Basensequenz entsprechend der Basensequenz der 1043. bis 2310. der in Fig. 1 (A) dargestellten Basen, und eine dazu komplementäre Basensequenz.

**14.** RNA entsprechend einer DNA nach den Ansprüche 8 bis 13.

**15.** Menschliches, chromosomales DNA-Fragment, als ein Exon eine Basensequenz aufweisend, die durch die folgende Formel (VI) wiedergegeben wird:

```
1         10         20         30         40
AGGTGTGCTC TGAACAAGCC GAGACGGGGC CGTGCCGAGC

        50         60         70         80
AATGATCTCC CGCTGGTACT TTGATGTGAC TGAAGGGAAG

      , 90        100        110        120
TGTGCCCCAT TCTTTTACGG CGGATGTGGC GGCAACCGGA

       130        140        150        160
ACAACTTTGA CACAGAAGAG TACTGCATGG CCGTGTGTGG

      168
CAGCGCCA                              ... (VI),
```

**16.** Menschliches, chromosomales DNA-Fragment gemäß Anspruch 15, das ferner wenigstens einen Teil einer Basensequenz aufweist, die der Basensequenz der 1. bis 85. der in Fig. 5 dargestellten Basen entspricht, und wenigstens einen Teil einer Basensequenz, die der Basensequenz der 254. bis 457. der in Fig. 5 dargestellten Basen entspricht, wobei diese jeweils an ihrem 5'-Ende und 3'-Ende mit der Basensequenz von Formel (VI) verbunden sind.

**17.** Menschliches, chromosomales DNA-Fragment gemäß Anspruch 16, das ferner eine Basensequenz wie in Fig 6 dargestellt aufweist und das eine molekulare Länge von ungefähr 6,6 kB besitzt und eine wie in (B) von Fig. 2 dargestellte Restriktions-Endonuclease-Spaltungskarte als ein BamHI-BamHI-Einschub in Plasmid pAP246B, wobei das Plasmid pAP246B von Escherichia coli K-12 Stamm JM105 (pAP246B) getragen wird, der beim Fermentation Research Institute, Japan, unter der Zugangsnummer FERM BP 1987 hinterlegt ist.

**18.** Menschliches, chromosomales DNA-Fragment gemäß einem der Ansprüche 15 bis 17, das mit der Expression eines Teils eines menschlichen Senil-Plaque-Amyloid-Precursor-Proteins des menschlichen Gehirns verbunden ist, wobei dieser Teil eine Protease inhibierende Aktivität aufweist.

**19.** Menschliches, chromosomales DNA-Fragment, das als ein Exon eine Basensequenz aufweist, die durch die folgend Formel (X) wiedergegeben wird:

```
TG TCCCAAAGTT TACTCAAGAC TACCCAGGAA CCTCTTGCCC

GAGATCCTGT TAAAC                              ...(X).
```

**20.** Menschliches, chromosomales DNA-Fragment gemäß Anspruch 19, das weiterhin wenigstens einen Teil einer Basensequenz aufweist, die der Basensequenz der 1. bis 195. der in Fig. 6 dargestellten Basen entspricht und wenigstens einen Teil einer Basensequenz, die der Basensequenz der 253. bis 395. der in Fig. 6 dargestellten Basen entspricht, die jeweils über ihr 5'-Ende und ihr 3'-Ende mit der Basensequenz der Formel (X) verbunden sind.

**21.** RNA entsprechend einer DNA nach den Ansprüche 15 bis 22.

**22.** Replizierbare, rekombinante DNA, einen replizierbaren Expressionsvektor aufweisend und, daran ligiert, einen Promoter und eine DNA-Codierung für ein Protein, die eine Aminosäuresequenz der Formel (III) wie in Anspruch 3 definiert aufweist.

**23.** Replizierbare, rekombinante DNA gemäß Anspruch 22, die weiterhin eine DNA-Codierung für ein Signalpeptid aufweist, wobei die DNA-Codierung für ein Signalpeptid zwischen den Promoter und der DNA-Codierung für das Protein ligiert ist, wodurch ein Leserahmen gebildet wird, um so einem fusionierten Peptid, das das Signalpeptid und das Protein aufweist, zu ermöglichen durch Expression der DNA's hergestellt zu werden.

**24.** Transformanten, einen Mikroorganismus oder eine Zelle aufweisend und, darin enthalten, eine replizierbare, rekombinante DNA, die einen replizierbaren Expressionsvektor aufweist und, daran ligiert, einen Promoter und eine DNA-Codierung für ein Protein, die eine Aminosäuresequenz der Formel (III) wie in Anspruch 3 definiert aufweist.

**25.** Replizierbare, rekombinante DNA, aufweisend einen replizierbaren Expressionsvektor und, daran ligiert, einen Promoter und eine DNA-Codierung für ein Protein, die eine Aminosäuresequenz der Formel (V) wie in Anspruch 5 definiert aufweist.

**26.** Replizierbare, rekombinante DNA gemäß Anspruch 25, die weiterhin eine DNA-Codierung für ein Signalpeptid aufweist, wobei die DNA-Codierung für ein Signalpeptid zwischen den Promoter und der DNA-Codierung für das Protein ligiert ist, wodurch ein Leserahmen gebildet wird, um so einem fusionierten Peptid, das das Signalpeptid und das Protein aufweist, zu ermöglichen durch Expression der DNA's hergestellt zu werden.

**27.** Transformanten, einen Mikroorganismus oder eine Zelle aufweisend und, darin enthalten, eine replizierbare, rekombinante DNA, die eine replizierbaren Expressionsvektor aufweist und, daran ligiert, einen Promoter und eine DNA-Codierung für ein Protein, die eine Aminosäuresequenz der Formel (V) wie in Anspruch 5 definiert aufweist.

**28.** Escherichia coli K-12 Stamm HB101 (pGBP2), hinterlegt bei der American Type Culture Collection, USA, unter der Zugangsnummer ATCC 67502.

**29.** Escherichia coli K-12 Stamm JM105 (pAP246B), hinterlegt bei dem Fermentation Research Institute, Japan, unter der Zugangsnummer FERM BP-1987.

**30.** Antikörper, der für ein Senil-Plaque-Amyloid-Precursor-Protein des menschlichen Gehirns spezifisch ist, ausgewält aus der Gruppe bestehend aus den Aminosäuresequenzen der Formel (III) wie in Anspruch 3 definiert und der Formel (V) wie in Anspruch 5 definiert.

**31.** Antikörper gemäß Anspruch 30, der ein von einer Zellinie hergestellter monoklonaler Antikörper ist, die aus der aus einer Maus-Hybrid-Zellinie ADI1-7-5-2, einer Maus-Hybrid-Zellinie ADI1-4-6-1 und einer Maus-Hybrid-Zellinie ADI1-05-44-2 bestehenden Gruppe ausgewählt ist, wobei die Maus-Hybrid-Zellinie ADI1-7-5-2 bei dem Fermentation Resaerch Institute, Japan, unter der Zugangsnummer FERM BP 1994 hinterlegt ist, die Maus-Hybrid-Zellinie ADI1-4-6-1 bei dem Fermentation Research Institute, Japan, unter der Zugangsnummer FERM BP 1995 und die Maus-Hybrid-Zellinie ADI1-05-44-2 bei dem Fermentation Resaerch Institute, Japan, unter der Zugangsnummer FERM BP 1988.

**32.** Verfahren zum Nachweis eines Senil-Plaque-Amyloid-Precursor-Proteins des menschlichen Gehirns, ausgewählt aus der Gruppe bestehend aus den Aminosäuresequenzen der Formel (III) wie in Anspruch 3 definiert und der Formel (V) wie in Anspruch 5 definiert, das ein Unterwerfen einer Probe unter ein Immunoassay unter Verwendung eines Antikörpers nach Anspruch 30, der spezifisch für die Aminosäuresequenz ist, umfaßt.

**33.** Verfahren zum Nachweis einer DNA nach Anspruch 8, das ein Unterwerfen einer Probe unter ein Assay durch Hybridisierung umfaßt, wobei eine Sonde verwendet wird, die aus der Gruppe ausgewählt ist, die aus (i) einer DNA, aufweisend wenigstens einen Teil einer Basensequenz der Formel (VI) wie in Anspruch 8 definiert, (ii) eine zu dieser DNA komplementären DNA und (iii) damit korrespondierenden RNA's besteht.

**34.** Verfahren zum Nachweis einer DNA nach Anscpruch 10, das ein Unterwerfen einer Probe unter ein Assay durch Hybridisierung umfaßt, wobei eine Sonde verwendet wird, die aus der Gruppe ausgewählt ist, die aus (i) einer DNA, aufweisend wenigstens einen Teil einer Basensequenz der Formel (VIII) wie in Anspruch 10 definiert, (ii) eine zu dieser DNA komplementären DNA und (iii) damit korrespondierenden RNA's besteht.

**35.** Verfahren zum Nachweis einer DNA nach Anspruch 13, das ein Unterwerfen einer Probe unter ein Assay durch Hybridisierung umfaßt, wobei eine Sonde verwendet wird, die aus der Gruppe ausgewählt ist, die aus (i) einer DNA, aufweisend wenigstens einen Teil einer Basensequenz der Formel (XII) wie in Anspruch 13 definiert, (ii) eine zu dieser DNA komplementären DNA und (iii) damit korrespondierenden RNA's besteht.

**36.** Verfahren zum Nachweis einer DNA nach Anspruch 8, die in einer cDNA-Codierung für ein menschliches Senil-Plaque-Precursor-Protein vorliegt, das ein Unterwerfen einer Probe unter ein Assay durch Hybridisierung umfaßt, wobei eine Sonde verwendet wird, die aus der Gruppe ausgewählt ist, die aus (i) einer DNA, aufweisend wenigstens zwölf kontinuierlich sequenzierte Basen in einer Basensequenz der Formel

G GTGGTTCGAG-(VI)-TT CCTACAACA

worin (VI) eine DNA mit derselben Basensequenz ist wie durch Formel (VI) in Anspruch 8 definiert, (ii) eine zu dieser DNA komplementären DNA und (iii) damit korrespondierenden RNA's besteht.

**37.** Verfahren zum Nachweis einer DNA nach Anspruch 11, die in einer cDNA-Codierung für ein menschliches Senil-Plaque-Precursor-Protein vorliegt, das ein Unterwerfen einer Probe unter ein Assay durch Hybridisierung umfaßt, wobei eine Sonde verwendet wird, die aus der Gruppe ausgewählt ist, die aus (i) einer DNA, aufweisend wenigstens zwölf kontinuierlich sequenzierte Basen in einer Basensequenz der Formel

G GTGGTTCGAG-(IX)-TT CCTACAACA

worin (IX) eine DNA mit derselben Basensequenz ist wie durch Formel (IX) in Anspruch 11 definiert, (ii) eine zu dieser DNA komplementären DNA und (iii) damit korrespondierenden RNA's besteht.

**38.** Verfahren zum Nachweis einer RNA, die der DNA nach Anspruch 8 entspricht, das ein Unterwerfen einer Probe unter ein Assay durch Hybridisierung umfaßt, wobei eine Sonde verwendet wird, die aus der Gruppe ausgewählt ist, die besteht aus (i) einer DNA, die komplementär zu einer DNA ist, die wenigstens einen Teil einer Basensequenz von Formel (VI) wie in Anspruch 8 definiert aufweist, und (ii) einer damit korrespondierenden RNA.

**39.** Verfahren zum Nachweis einer RNA, die der DNA nach Anspruch 10 entspricht, das ein Unterwerfen einer Probe unter ein Assay durch Hybridisierung umfaßt, wobei eine Sonde verwendet wird, die aus der Gruppe ausgewählt ist, die besteht aus (i) einer DNA, die komplementär zu einer DNA ist, die wenigstens einen Teil einer Basensequenz von Formel (VIII) wie in Anspruch 10 definiert aufweist, und (ii) einer damit korrespondierenden RNA.

**40.** Verfahren zum Nachweis einer RNA, die der DNA nach Anspruch 13 entspricht, das ein Unterwerfen einer Probe unter ein Assay durch Hybridisierung umfaßt, wobei eine Sonde verwendet wird, die aus der Gruppe ausgewählt ist, die besteht aus (i) einer DNA, die komplementär zu einer DNA ist, die wenigstens einen Teil einer Basensequenz von Formel (XII) wie in Anspruch 13 definiert aufweist, und (ii) einer damit korrespondierenden RNA.

**41.** Verfahren zum Nachweis einer RNA, die der DNA nach Anspruch 8 entspricht, das ein Unterwerfen einer Probe unter ein Assay durch Hybridisierung umfaßt, wobei eine Sonde verwendet wird, die aus der Gruppe ausgewählt ist, die besteht aus (i) einer DNA, die komplementär zu wenigstens zwölf kontinuierlich sequenzierten Basen der Formel

G GTGGTTCGAG-(VI)-TT CCTACAACA

ist, worin (VI) eine DNA mit derselben Basensequenz ist wie durch Formel (VI) in Anspruch 8 definiert und (ii) einer zu der komplementären DNA (i) korrespondierenden RNA.

**42.** Verfahren zum Nachweis einer RNA, die der DNA nach Anspruch 11 entspricht, das ein Unterwerfen einer Probe unter ein Assay durch Hybridisierung umfaßt, wobei eine Sonde verwendet wird, die aus der Gruppe ausgewählt ist, die besteht aus (i) einer DNA, die komplementär zu wenigstens zwölf kontinuierlich sequenzierten Basen der Formel

G GTGGTTCGAG-(IX)-TT CCTACAACA

ist, worin (IX) eine DNA mit derselben Basensequenz ist wie durch Formel (IX) in Anspruch 11 definiert und (ii) einer zu der komplementären DNA (i) korrespondierenden RNA.

**43.** Verfahren zum Nachweis eines menschlichen, chromosomalen DNA-Fragments, das mit der Expression eines Polypeptids verbunden ist, das eine Protease inhibierende Wirkung besitzt, wobei das Polypeptid ein Teil eines Senil-Plaque-Amyloid-Precursor-Proteins des menschlichen Gehirns ist, das ein Unterwerfen einer Probe unter ein Assay durch Hybridisierung umfaßt, wobei wenigstens ein Teil von igendeinem der menschlichen, chromosomalen DNA-Fragmente nach den Ansprüchen 15 bis 17 und 19 bis 20 als Sonde verwendet wird.

**44.** Sonde zur Verwendung beim Nachweis einer DNA oder RNA, die ein Polypeptid codieren, das mit einem Senil-Plapque-Amyloid-Precursor-Protein des menschlichen Gehirns verbunden ist, die eine Basensequenz aufweist, die aus der Gruppe ausgewählt ist, die besteht aus (i) einer DNA, die wenigstens zwölf kontinuierlich sequenzierte Basen in einer Basensequenz der Formel

G GTGGTTCGAG-(VI)-TT CCTACAACA

aufweist, worin (VI) eine DNA mit derselben Basensequenz ist wie durch Formel (VI) in Anspruch 8 definiert, (ii) einer DNA, die zu dieser DNA komplementär ist und (iii) damit korrespondierenden RNA's.

**45.** Sonde zur Verwendung eines Nachweises einer DNA oder RNA, die ein Polypeptid codiert, das mit einem Senil-Plapque-Amyloid-Precursor-Protein des menschlichen Gehirns verbunden ist, die eine Basensequenz aufweist, die aus der Gruppe ausgewählt ist, die besteht aus (i) einer DNA, die wenigstens zwölf kontinuierlich sequenzierte Basen in einer Basensequenz der Formel

G GTGGTTCGAG-(IX)-TT CCTACAACA

aufweist, worin (IX) eine DNA mit derselben Basensequenz ist wie durch Formel (IX) in Anspruch 11 definiert, (ii) einer DNA, die zu dieser DNA komplementär ist und (iii) damit korrespondierende RNA's.

**Revendications**

1. Protéine comprenant une séquence d'acides aminés représentée par la formule (I) qui suit :

   Val-Cys-Ser-Glu-Gln-Ala-Glu-Thr-Gly-Pro-Cys-
   Arg-Ala-Met-Ile-Ser-Arg-Trp-Tyr-Phe-Asp-Val-
   Thr-Glu-Gly-Lys-Cys-Ala-Pro-Phe-Phe-Tyr-Gly-
   Gly-Cys-Gly-Gly-Asn-Arg-Asn-Asn-Phe-Asp-Thr-
   Glu-Glu-Tyr-Cys-Met-Ala-Val-Cys-Gly-Ser-Ala ...(I).

2. Protéine comprenant une séquence d'acides aminés représentée par la formule (II) :

   Glu-(I)-Ile (II)

   où (I) est un peptide ayant la même séquence d'acides aminés que celle définie par la formule (I) de la revendication 1.

3. Protéine comprenant une séquence d'acides aminés représentée par la formule (III) qui suit :

   $X_1$-(I)-$X_2$ (III)

   où (I) est un peptide ayant la même séquence d'acides aminés que celle définie par la formule (I) de la revendication 1, $X_1$ est un peptide ayant une séquence d'acides aminés correspondant à la séquence d'acides aminés des premier à 289ème acides aminés montrés à la figure 1 (B), et $X_2$ est un peptide ayant une séquence d'acides aminés correspondant à la séquence d'acides aminés des 345ème à 751ème acides aminés montrés à la figure 1 (B).

4. Protéine comprenant une séquence d'acides aminés représentée par la formule (IV) :

   Glu-(I)-Met-Ser-Gln-Ser-Leu-Leu-Lys-Thr-Thr-
   Gln-Glu-Pro-Leu-Ala-Arg-Asp-Pro-Val-Lys-Leu
   ...(IV)

   où (I) est un peptide ayant la même séquence d'acides aminés que celle définie par la formule (I) de la revendication 1.

5. Protéine comprenant une séquence d'acides aminés représentée par la formule (V) qui suit :

   $X_3$-(I)-$X_4$ (V)

   où (I) est un peptide ayant la même séquence d'acides aminés que celle définie par la formule (I) de la revendication 1, $X_3$ est un peptide ayant une séquence d'acides aminés correspondant à la séquence d'acides aminés des premier à 289ème acides aminés montrés à la figure 1 (A), et $X_4$ est un peptide ayant une séquence d'acides aminés correspondant à la séquence d'acides aminés de 345ème à 770ème acides aminés montrés à la figure 1 (A).

6. Protéine selon l'une quelconque des revendications 1 à 5, qui est une protéine précurseur de l'amyloïde en plaque sénile du cerveau humain.

7. Protéine selon l'une quelconque des revendications 1 à 5, qui a une activité d'inhibition de la protéase.

8. ADN comprenant au moins une séquence de bases sélectionnée dans le groupe consistant en une séquence de bases représentée par la formule (VI) qui suit :

```
1         10         20         30         40
AGGTGTGCTC TGAACAAGCC GAGACGGGGC CGTGCCGAGC
          50         60         70         80
AATGATCTCC CGCTGGTACT TTGATGTGAC TGAAGGGAAG
          90        100        110        120
TGTGCCCCAT TCTTTTACGG CGGATGTGGC GGCAACCGGA
         130        140        150        160
ACAACTTTGA CACAGAAGAG TACTGCATGG CCGTGTGTGG
    168
CAGCGCCA                          ...(VI),
```

et la séquence des bases qui lui est complémentaire.

9. ADN comprenant au moins une séquence de bases sélectionnée dans le groupe consistant en une séquence de bases représentée par la formule (VII) :

$$G\text{-(VI)-}TT \qquad \text{(VII)}$$

où (VI) est un ADN ayant la même séquence des bases que celle définie par la formule (VI) de la revendication 8, et la séquence des bases qui lui est complémentaire.

10. ADN comprenant au moins une séquence de bases sélectionnée dans le groupe consistant en une séquence de bases représentée par la formule (VIII) qui suit :

$$Y_1\text{-(VI)-}Y_2 \qquad \text{(VIII)}$$

où (VI) est un ADN ayant la même séquence de bases que celle définie par la formule (VI) de la revendication 8, $Y_1$ est un ADN ayant une séquence de bases correspondant à la séquence des première à 865ème bases montrées à la figure 1(B), et $Y_2$ est un ADN ayant une séquence de bases correspondant à la séquence de bases des 1034ème à 2253ème bases montrées à la figure 1(B),
et une séquence de bases qui lui est complémentaire.

11. ADN comprenant au moins une séquence de bases sélectionnée dans le groupe consistant en une séquence de bases représentée par la formule (IX) qui suit :

$$\text{(VI)-Y3} \qquad \text{(IX)}$$

où (VI) est un ADN ayant la même séquence de bases que celle définie par la formule (VI) de la revendication 8, et $Y_3$ est un ADN ayant une séquence de bases représentée par la formule (X) qui suit :

```
TG  TCCCAAAGTT  TACTCAAGAC   TACCCAGGAA   CCTCTTGCCC

GAGATCCTGT  TAAAC      ... (X),
```

et une séquence de bases qui lui est complémentaire.

12. ADN comprenant au moins une séquence de bases sélectionnée dans le groupe consistant en une séquence de bases représentée par la formule (XI) qui suit :

$$G\text{-(VI)-}Y_3\text{-}TT \qquad \text{(XI)}$$

où (VI) est un ADN ayant la même séquence de bases que celle définie par la formule (VI) de la revendication 8,

et $Y_3$ a la même signification que celle définie par la formule (X) de la revendication 11, et une séquence de bases qui lui est complémentaire.

**13.** ADN comprenant au moins une séquence de bases sélectionnée dans le groupe consistant en une séquence de bases représentée par la formule (XII) :

$$Y_4\text{-(VI)-}Y_5 \qquad \text{(XII) :}$$

où (VI) est un ADN ayant la même séquence de bases que celle définie par la formule (VI) de la revendication 8, et $Y_4$ est un ADN ayant une séquence de bases correspondant à la séquence de bases des première à 865ème bases que l'on peut voir à la figure 1(A), et $Y_5$ est un ADN ayant une séquence de bases correspondant à la séquence de bases des 1034ème à 2310èmes bases montrées à la figure 1(A),
et une séquence de bases qui lui est complémentaire.

**14.** ARN correspondant à un ADN des revendications 8 à 13.

**15.** Fragment d'ADN chromosomique humain comprenant comme exon une séquence de bases représentée par la formule (VI) qui suit :

```
1         10         20         30         40
AGGTGTGCTC TGAACAAGCC GAGACGGGGC CGTGCCGAGC
        50         60         70         80
AATGATCTCC CGCTGGTACT TTGATGTGAC TGAAGGGAAG
        90        100        110        120
TGTGCCCCAT TCTTTTACGG CGGATGTGGC GGCAACCGGA
       130        140        150        160
ACAACTTTGA CACAGAAGAG TACTGCATGG CCGTGTGTGG
      168
CAGCGCCA                            ...(VI).
```

**16.** Fragment d'ADN chromosomique humain selon la revendication 15, qui comprend de plus au moins une portion d'une séquence de bases correspondant à la séquence de bases des première à 85ème bases montrées à la figure 5 et au moins une portion d'une séquence de bases correspondant à la séquence de bases des 254ème à 457ème bases montrées à la figure 5, attachées à ladite séquence de bases de formule (VI) à ses extrémités 5' et 3', respectivement.

**17.** Fragment d'ADN chromosomique humain selon la revendication 16 qui comprend de plus une séquence de bases telle que montrée à la figure 6 et qui a une longueur moléculaire d'environ 6,6 kb et une carte de scission d'endonucléases de restriction telle que montrée en (B) sur la figure 2 sous la forme d'un insert BamHI-BamHI dans le plasmide pAP246B, ledit plasmide pAP246B étant porté par Escherichia coli K-12 souche JM105 (pAP246B) déposée au Fermentation Research Institute, Japon, sous le N° d'accession FERM BP 1987.

**18.** Fragment d'ADN chromosomique humain selon l'une quelconque des revendications 15 à 17, qui se rapporte à l'expression d'une portion d'une protéine précurseur de l'amyloïde en plaque du cerveau humain, ladite portion ayant une activité d'inhibition de protéase.

**19.** Fragment d'ADN chromosomique humain comprenant, comme exon, une séquence de bases représentée par la formule (X) qui suit :

```
TG  TCCCAAAGTT  TACTCAAGAC   TACCCAGGAA   CCTCTTGCCC

GAGATCCTGT  TAAAC      ...  (X).
```

**20.** Fragment d'ADN chromosomique humain selon la revendication 19, qui comprend de plus au moins une portion d'une séquence de bases correspondant à la séquence de bases des première à 295ème bases montrées à la figure 6 et au moins une portion de la séquence des bases correspondant à la séquence des bases des 253ème à 395ème bases montrées à la figure 6, attachées à ladite séquence des bases des formules (X) à ses extrémités 5' et 3', respectivement.

**21.** ARN correspondant à un ADN des revendications 15 à 22.

**22.** ADN recombinant réplicable comprenant un vecteur d'expression réplicable et, ligaturés à celui-ci, un promoteur et un ADN codant pour une protéine comprenant une séquence d'acides aminés de la formule (III) telle que définie à la revendication 3.

**23.** ADN recombinant réplicable selon la revendication 22, qui comprend de plus un ADN codant pour un peptide signal, ledit ADN codant pour un peptide signal étant ligaturé entre ledit promoteur et ledit ADN codant pour ladite protéine, constituant un cadre de lecture afin de permettre à un peptide fusionné comprenant ledit peptide signal et ladite protéine d'être produit par l'expression desdits ADN.

**24.** Transformant comprenant un micro-organisme ou cellule et, contenu dans celui-ci, un ADN recombinant réplicable comprenant un vecteur d'expression réplicable et, ligaturés à celui-ci, un promoteur et un ADN codant pour une protéine comprenant une séquence d'acides aminés de formule (III) telle que définie à la revendication 3.

**25.** ADN recombinant réplicable comprenant un vecteur d'expression réplicable et, ligaturés à celui-ci, un promoteur et un ADN codant pour une protéine comprenant une séquence d'acides aminés de la formule (V) telle que définie à la revendication 5.

**26.** ADN recombinant réplicable selon la revendication 25, qui comprend de plus un ADN codant pour un peptide signal, ledit ADN codant pour un peptide signal étant ligaturé entre ledit promoteur et ledit ADN codant pour ladite protéine, constituant un cadre de lecture afin de permettre à un peptide fusionné comprenant ledit peptide et ladite protéine d'être produit par l'expression desdits ADN.

**27.** Transformant comprenant un micro-organisme ou une cellule et, contenu dans celui-ci, un ADN recombinant réplicable comprenant un vecteur d'expression réplicable et, ligaturé à celui-ci, un promoteur et un ADN codant pour une protéine comprenant une séquence d'acides aminés de formule (V) telle que définie à la revendication 5.

**28.** Escherichia coli K-12 souche HB101 (pGBP2) déposée au American Type Culture Collection, U.S.A., sous le N° d'Accession ATCC 67502.

**29.** Escherichia coli K-12 souche JM105 (pAP246B) déposée au Fermentation Research Institute, Japon, sous le N° d'Accession FERM BP-1987.

**30.** Anticorps spécifique d'une protéine précurseur de l'amyloïde en plaque sénile du cerveau humain sélectionné dans le groupe consistant en séquences d'acides aminés de la formule (III) telle que définie à la revendication 3 et de la formule (V) telle que définie à la revendication (V).

**31.** Anticorps selon la revendication 30, qui est un anticorps monoclonal produit par une lignée de cellules sélectionnée dans le groupe consistant en une lignée de cellules hybrides de souris ADI1-7-5-2, une lignée de cellules hybrides de souris ADI1-4-6-1 et une lignée de cellules hybrides de souris ADI1-05-44-2, ladite lignée de cellules hybrides de souris ADI1-7-5-2 étant déposée au Fermentation Research Institute, Japon, sous le N° d'Accession FERM BP 1994, ladite lignée de cellules hybrides de souris ADI1-4-6-1 étant déposée au Fermentation Research Institute, Japon, sous le N° d'Accession FERM BP 1995, et ladite lignée de cellules hybrides de souris ADI1-05-44-2 étant déposée au Fermentation Research Institute, Japon, sous le N° d'Accession FERM BP 1988.

**32.** Méthode de détection d'une protéine précurseur de l'amyloïde en plaque sénile du cerveau humain sélectionnée dans le groupe consistant en séquences d'acides aminés de la formule (III) telle que définie à la revendication 3 et de la formule (V) telle que définie à la revendication 5, qui consiste à soumettre un échantillon à une immunodétermination en utilisant un anticorps de la revendication 30 spécifique pour ladite séquence d'acides aminés.

**33.** Méthode pour détecter un ADN de la revendication 8, qui consiste à soumettre un échantillon à une détermination par hybridation en utilisant une sonde sélectionnée dans le groupe consistant en (i) un ADN comprenant au moins une portion d'une séquence de bases de la formule (VI) telle que définie à la revendication 8, (ii) un ADN complémentaire dudit ADN et (iii) les ARN qui leur correspondent.

**34.** Méthode pour la détection d'un ADN de la revendication 10, qui consiste à soumettre un échantillon à une détermination par hybridation en utilisant une sonde sélectionnée dans le groupe consistant en (i) un ADN comprenant au moins une portion d'une séquence de bases de la formule (VIII) telle que définie à la revendication 10, (ii) un ADN complémentaire dudit ADN et (iii) les ARN qui leur correspondent.

**35.** Méthode de détection d'un ADN de la revendication 13 qui consiste à soumettre un échantillon à une détermination par hybridation en utilisant une sonde sélectionnée dans le groupe consistant en (i) un ADN comprenant au moins une portion d'une séquence de bases de la formule (XII) telle que définie à la revendication 13, (ii) un ADN complémentaire dudit ADN et (iii) les ARN qui leur correspondent.

**36.** Méthode de détection d'un ADN de la revendication 8 qui est présent dans l'ADNc codant pour une protéine précurseur de l'amyloïde sénile humain, qui consiste à soumettre un échantillon à une détermination par hybridation en utilisant une sonde sélectionnée dans le groupe consistant en (i) un ADN comprenant au moins douze bases continuellement mises en séquence en une séquence de bases de la formule

G GTGGTTCGAG-(VI)- TT CCTACAACA

où (VI) est un ADN ayant la même séquence de bases que celle définie par la formule (VI) de la revendication 8, (ii) un ADN complémentaire dudit ADN et (iii) les ARN qui leur correspondent.

**37.** Méthode de détection d'un ADN de la revendication 11 qui est présent dans l'ADNc codant pour une protéine précurseur de l'amyloïde sénile du cerveau humain qui consiste à soumettre un échantillon à une détermination par hybridation en utilisant une sonde sélectionnée dans le groupe consistant en (i) un ADN comprenant au moins douze bases continuellement mises en séquence dans une séquence de bases de formule

G GTGGTTCGAG-(IX)-TT CCTACAACA

où (IX) est un ADN ayant la même séquence des bases que celle définie par la formule (IX) de la revendication 11, (ii) un ADN complémentaire dudit ADN et (iii) les ARN qui leur correspondent.

**38.** Méthode de détection d'un ARN correspondant à l'ADN de la revendication 8, qui consiste à soumettre un échantillon à une détermination par hybridation en utilisant une sonde sélectionnée dans le groupe consistant en (i) un ADN complémentaire d'un ADN comprenant au moins une portion d'une séquence de bases de la formule (VI) telle que définie à la revendication 8, et (ii) un ARN qui lui correspond.

**39.** Méthode de détection d'un ARN correspondant à l'ADN de la revendication 10, qui consiste à soumettre un échantillon à une détermination par hybridation en utilisant une sonde sélectionnée dans le groupe consistant en (i) un ADN complémentaire d'un ADN comprenant au moins une portion d'une séquence de bases de la formule (VIII) telle que définie à la revendication 10, et (ii) un ARN correspondant.

**40.** Méthode de détection d'un ARN correspondant à l'ADN de la revendication 13, qui consiste à soumettre un échantillon à une détermination par hybridation en utilisant une sonde sélectionnée dans le groupe consistant en (i) un ADN complémentaire d'un ADN comprenant au moins une portion d'une séquence de bases de la formule (XII) telle que définie à la revendication 13, et (ii) un ARN correspondant.

**41.** Méthode de détection d'un ARN correspondant à l'ADN de la revendication 8, qui consiste à soumettre un échantillon à une détermination par hybridation en utilisant une sonde sélectionnée dans le groupe consistant en (i) un ADN complémentaire d'au moins douze bases mises en séquence continue de la formule

G GTGGTTCGAG-(VI)-TT CCTACAACA

où (VI) est un ADN ayant la même séquence des bases que celle définie par la formule (VI) de la revendication 8, et (ii) un ARN correspondant audit ADN complémentaire (i).

**42.** Méthode de détection d'un ARN correspondant à l'ADN de la revendication 11 qui consiste à soumettre un échantillon à une détermination par hybridation en utilisant une sonde sélectionnée dans le groupe consistant en (i) un ADN complémentaire d'au moins douze bases mises en séquence continue de la formule

G GTGGTTCGAG-(IX)-TT CCTACAACA

où (IX) est un ADN ayant la même séquence de bases que celle définie par la formule (IX) de la revendication 11, et (ii) un ARN correspondant audit ADN complémentaire (i).

**43.** Méthode de détection d'un fragment d'ADN chromosomique humain se rapportant à l'expression d'un polypeptide ayant une activité d'inhibition de protéase, ledit polypeptide étant une portion d'une protéine précurseur de l'amyloïde en plaque sénile du cerveau humain, qui consiste à soumettre un échantillon à une détermination par hybridation en utilisant comme sonde au moins une portion de l'un des fragments d'ADN chromosomique humain des revendications 15 à 17 et des revendications 19 à 20.

**44.** Sonde à utiliser pour détecter un ADN ou un ARN codant pour un polypeptide en rapport avec la protéine précurseur de l'amyloïde en plaque sénile du cerveau humain qui comprend une séquence de bases sélectionnée dans le groupe consistant en (i) un ADN comprenant au moins douze bases continuellement mises en séquence en une séquence des bases de la formule

G GTGGTTCGAG-(VI)-TT CCTACAACA

où (VI) est un ADN ayant la même séquence de bases que celle définie par la formule (VI) de la revendication 8, (ii) un ADN complémentaire dudit ADN et (iii) des ARN qui leurs correspondent.

**45.** Sonde à utiliser pour détecter un ADN ou un ARN codant pour un polypeptide en rapport avec la protéine précurseur de l'amyloïde en plaque sénile de cerveau humain qui comprend une séquence de bases sélectionnée dans le groupe consistant en (i) un ADN comprenant au moins douze bases mises continuellement en séquence en une séquence des bases de la formule

G GTGGTTCGAG-(IX)-TT CCTACAACA

où (IX) est un ADN ayant la même séquence des bases que celle définie par la formule (IX) de la revendication 11, (ii) un ADN complémentaire dudit ADN et (iii) les ARN correspondants.

# FIG. 1 (A)-a

```
* -47                                                        -1 *
G ATCCCACTCG CACAGCAGCG  CACTCGGTGC CCCGCGCAGG GTCGCG

*    1                                                                        60*
ATG.CTG.CCC.GGT.TTG.GCA.CTG.CTC.CTG.CTG.GCC.GCC.TGG.ACG.GCT.CGG.GCG.CTG.GAG.GTA
Met-Leu-Pro-Gly-Leu-Ala-Leu-Leu-Leu-Leu-Ala-Ala-Trp-Thr-Ala-Arg-Ala-Leu-Glu-Val
 1                                                                            20
*   61                                                                       120*
CCC.ACT.GAT.GGT.AAT.GCT.GGC.CTG.CTG.GCT.GAA.CCC.CAG.ATT.GCC.ATG.TTC.TGT.GGC.AGA
Pro-Thr-Asp-Gly-Asn-Ala-Gly-Leu-Leu-Ala-Glu-Pro-Gln-Ile-Ala-Met-Phe-Cys-Gly-Arg

*  121                                                                       180*
CTG.AAC.ATG.CAC.ATG.AAT.GTC.CAG.AAT.GGG.AAG.TGG.GAT.TCA.GAT.CCA.TCA.GGG.ACC.AAA
Leu-Asn-Met-His-Met-Asn-Val-Gln-Asn-Gly-Lys-Trp-Asp-Ser-Asp-Pro-Ser-Gly-Thr-Lys

*  181                                                                       240*
ACC.TGC.ATT.GAT.ACC.AAG.GAA.GGC.ATC.CTG.CAG.TAT.TGC.CAA.GAA.GTC.TAC.CCT.GAA.CTG
Thr-Cys-Ile-Asp-Thr-Lys-Glu-Gly-Ile-Leu-Gln-Tyr-Cys-Gln-Glu-Val-Tyr-Pro-Glu-Leu

*  241                                                                       300*
CAG.ATC.ACC.AAT.GTG.GTA.GAA.GCC.AAC.CAA.CCA.GTG.ACC.ATC.CAG.AAC.TGG.TGC.AAG.CGG
Gln-Ile-Thr-Asn-Val-Val-Glu-Ala-Asn-Gln-Pro-Val-Thr-Ile-Gln-Asn-Trp-Cys-Lys-Arg

*  301                                                                       360*
GGC.CGC.AAG.CAG.TGC.AAG.ACC.CAT.CCC.CAC.TTT.GTG.ATT.CCC.TAC.CGC.TGC.TTA.GTT.GGT
Gly-Arg-Lys-Gln-Cys-Lys-Thr-His-Pro-His-Phe-Val-Ile-Pro-Tyr-Arg-Cys-Leu-Val-Gly

*  361                                                                       420*
GAG.TTT.GTA.AGT.GAT.GCC.CTT.CTC.GTT.CCT.GAC.AAG.TGC.AAA.TTC.TTA.CAC.CAG.GAG.AGG
Glu-Phe-Val-Ser-Asp-Ala-Leu-Leu-Val-Pro-Asp-Lys-Cys-Lys-Phe-Leu-His-Gln-Glu-Arg

*  421                                                                       480*
ATG.GAT.GTT.TGC.GAA.ACT.CAT.CTT.CAC.TGG.CAC.ACC.GTC.GCC.AAA.GAG.ACA.TGC.AGT.GAG
Met-Asp-Val-Cys-Glu-Thr-His-Leu-His-Trp-His-Thr-Val-Ala-Lys-Glu-Thr-Cys-Ser-Glu

*  481                                                                       540*
AAG.AGT.ACC.AAC.TTG.CAT.GAC.TAC.GGC.ATG.TTG.CTG.CCC.TGC.GGA.ATT.GAC.AAG.TTC.CGA
Lys-Ser-Thr-Asn-Leu-His-Asp-Tyr-Gly-Met-Leu-Leu-Pro-Cys-Gly-Ile-Asp-Lys-Phe-Arg

*  541                                                                       600*
GGG.GTA.GAG.TTT.GTG.TGT.TGC.CCA.CTG.GCT.GAA.GAA.AGT.GAC.AAT.GTG.GAT.TCT.GCT.GAT
Gly-Val-Glu-Phe-Val-Cys-Cys-Pro-Leu-Ala-Glu-Glu-Ser-Asp-Asn-Val-Asp-Ser-Ala-Asp

*  601                                                                       660*
GCG.GAG.GAG.GAT.GAC.TCG.GAT.GTC.TGG.TGG.GGC.GGA.GCA.GAC.ACA.GAC.TAT.GCA.GAT.GGG
Ala-Glu-Glu-Asp-Asp-Ser-Asp-Val-Trp-Trp-Gly-Gly-Ala-Asp-Thr-Asp-Tyr-Ala-Asp-Gly

*  661                                                                       720*
AGT.GAA.GAC.AAA.GTA.GTA.GAA.GTA.GCA.GAG.GAG.GAA.GAA.GTG.GCT.GAG.GTG.GAA.GAA.GAA
Ser-Glu-Asp-Lys-Val-Val-Glu-Val-Ala-Glu-Glu-Glu-Glu-Val-Ala-Glu-Val-Glu-Glu-Glu

*  721                                                                       780*
GAA.GCC.GAT.GAT.GAC.GAG.GAC.GAT.GAG.GAT.GGT.GAT.GAG.GTA.GAG.GAA.GAG.GCT.GAG.GAA
Glu-Ala-Asp-Asp-Asp-Glu-Asp-Asp-Glu-Asp-Gly-Asp-Glu-Val-Glu-Glu-Glu-Ala-Glu-Glu

*  781                                                                       840*
CCC.TAC.GAA.GAA.GCC.ACA.GAG.AGA.ACC.ACC.AGC.ATT.GCC.ACC.ACC.ACC.ACC.ACC.ACC.ACA
Pro-Tyr-Glu-Glu-Ala-Thr-Glu-Arg-Thr-Thr-Ser-Ile-Ala-Thr-Thr-Thr-Thr-Thr-Thr-Thr
```

# FIG. 1 (A)-b

```
                                  865 ┐┌866
*  841                                 ┘└                                       900*
GAG.TCT.GTG.GAA.GAG.GTG.GTT.CGA.GAG.GTG.TGC.TCT.GAA.CAA.GCC.GAG.ACG.GGG.CCG.TGC
Glu-Ser-Val-Glu-Glu-Val-Val-Arg-Glu-Val-Cys-Ser-Glu-Gln-Ala-Glu-Thr-Gly-Pro-Cys
                                288 289

*  901                                                                          960*
CGA.CCA.ATG.ATC.TCC.CGC.TGG.TAC.TTT.GAT.GTG.ACT.GAA.GGG.AAG.TGT.GCC.CCA.TTC.TTT
Arg-Ala-Met-Ile-Ser-Arg-Trp-Tyr-Phe-Asp-Val-Thr-Glu-Gly-Lys-Cys-Ala-Pro-Phe-Phe

*  961                                                                         1020*
TAC.GGC.GGA.TGT.GGC.GGC.AAC.CGG.AAC.AAC.TTT.GAC.ACA.GAA.GAG.TAC.TGC.ATG.GCC.GTG
Tyr-Gly-Gly-Cys-Gly-Gly-Asn-Arg-Asn-Asn-Phe-Asp-Thr-Glu-Glu-Tyr-Cys-Met-Ala-Val

               1033 ┐┌1034
* 1021              ┘└                                                         1080*
TGT.GGC.AGC.GCC.ATG.TCC.CAA.AGT.TTA.CTC.AAG.ACT.ACC.CAG.GAA.CCT.CTT.GCC.CGA.GAT
Cys-Gly-Ser-Ala-Met-Ser-Gln-Ser-Leu-Leu-Lys-Thr-Thr-Gln-Glu-Pro-Leu-Ala-Arg-Asp
                345 346

* 1081  1090 ┐┌1091                                                            1140*
CCT.GTT.AAA.CTT.CCT.ACA.ACA.GCA.GCC.AGT.ACC.CCT.GAT.GCC.GTT.GAC.AAG.TAT.CTC.GAG
Pro-Val-Lys-Leu-Pro-Thr-Thr-Ala-Ala-Ser-Thr-Pro-Asp-Ala-Val-Asp-Lys-Tyr-Leu-Glu
            364 365

* 1141                                                                         1200*
ACA.CCT.GGG.GAT.GAG.AAT.GAA.CAT.GCC.CAT.TTC.CAG.AAA.GCC.AAA.GAG.AGG.CTT.GAG.GCC
Thr-Pro-Gly-Asp-Glu-Asn-Glu-His-Ala-His-Phe-Gln-Lys-Ala-Lys-Glu-Arg-Leu-Glu-Ala

* 1201                                                                         1260*
AAG.CAC.CGA.GAG.AGA.ATG.TCC.CAG.GTC.ATG.AGA.GAA.TGG.GAA.GAG.GCA.GAA.CGT.CAA.GCA
Lys-His-Arg-Glu-Arg-Met-Ser-Gln-Val-Met-Arg-Glu-Trp-Glu-Glu-Ala-Glu-Arg-Gln-Ala

* 1261                                                                         1320*
AAG.AAC.TTG.CCT.AAA.GCT.GAT.AAG.AAG.GCA.GTT.ATC.CAG.CAT.TTC.CAG.GAG.AAA.GTG.GAA
Lys-Asn-Leu-Pro-Lys-Ala-Asp-Lys-Lys-Ala-Val-Ile-Gln-His-Phe-Gln-Glu-Lys-Val-Glu

* 1321                                                                         1380*
TCT.TTG.GAA.CAG.GAA.GCA.GCC.AAC.GAG.AGA.CAG.CAG.CTG.GTG.GAG.ACA.CAC.ATG.GCC.AGA
Ser-Leu-Glu-Gln-Glu-Ala-Ala-Asn-Glu-Arg-Gln-Gln-Leu-Val-Glu-Thr-His-Met-Ala-Arg

* 1381                                                                         1440*
GTG.GAA.GCC.ATG.CTC.AAT.GAC.CGC.CGC.CGC.CTG.GCC.CTG.GAG.AAC.TAC.ATC.ACC.GCT.CTG
Val-Glu-Ala-Met-Leu-Asn-Asp-Arg-Arg-Arg-Leu-Ala-Leu-Glu-Asn-Tyr-Ile-Thr-Ala-Leu

* 1441                                                                         1500*
CAG.GCT.GTT.CCT.CCT.CGG.CCT.CGT.CAC.GTG.TTC.AAT.ATG.CTA.AAG.AAG.TAT.GTC.CGC.GCA
Gln-Ala-Val-Pro-Pro-Arg-Pro-Arg-His-Val-Phe-Asn-Met-Leu-Lys-Lys-Tyr-Val-Arg-Ala

* 1501                                                                         1560*
GAA.CAG.AAG.GAC.AGA.CAG.CAC.ACC.CTA.AAG.CAT.TTC.GAG.CAT.GTG.CGC.ATG.GTG.GAT.CCC
Glu-Gln-Lys-Asp-Arg-Gln-His-Thr-Leu-Lys-His-Phe-Glu-His-Val-Arg-Met-Val-Asp-Pro

* 1561                                                                         1620*
AAG.AAA.GCC.GCT.CAG.ATC.CGG.TCC.CAG.GTT.ATG.ACA.CAC.CTC.CGT.GTG.ATT.TAT.GAG.CGC
Lys-Lys-Ala-Ala-Gln-Ile-Arg-Ser-Gln-Val-Met-Thr-His-Leu-Arg-Val-Ile-Tyr-Glu-Arg

* 1621                                                                         1680*
ATG.AAT.CAG.TCT.CTC.TCC.CTG.CTC.TAC.AAC.GTG.CCT.GCA.GTG.GCC.GAG.GAG.ATT.CAG.GAT
Met-Asn-Gln-Ser-Leu-Ser-Leu-Leu-Tyr-Asn-Val-Pro-Ala-Val-Ala-Glu-Glu-Ile-Gln-Asp

* 1681                                                                         1740*
GAA.GTT.GAT.GAG.CTG.CTT.CAG.AAA.GAG.CAA.AAC.TAT.TCA.GAT.GAC.GTC.TTG.GCC.AAC.ATG
Glu-Val-Asp-Glu-Leu-Leu-Gln-Lys-Glu-Gln-Asn-Tyr-Ser-Asp-Asp-Val-Leu-Ala-Asn-Met

* 1741                                                                         1800*
ATT.AGT.GAA.CCA.AGG.ATC.AGT.TAC.GGA.AAC.GAT.GCT.CTC.ATG.CCA.TCT.TTG.ACC.GAA.ACG
Ile-Ser-Glu-Pro-Arg-Ile-Ser-Tyr-Gly-Asn-Asp-Ala-Leu-Met-Pro-Ser-Leu-Thr-Glu-Thr
```

# FIG. 1 (A)-c

```
* 1801                                                                          1860*
AAA.ACC.ACC.GTG.GAG.CTC.CTT.CCC.GTG.AAT.GGA.GAG.TTC.AGC.CTG.GAC.GAT.CTC.CAG.CCG
Lys-Thr-Thr-Val-Glu-Leu-Leu-Pro-Val-Asn-Gly-Glu-Phe-Ser-Leu-Asp-Asp-Leu-Gln-Pro

* 1861                                                                          1920*
TGG.CAT.TCT.TTT.GGG.GCT.GAC.TCT.GTG.CCA.GCC.AAC.ACA.GAA.AAC.GAA.GTT.GAG.CCT.GTT
Trp-His-Ser-Phe-Gly-Ala-Asp-Ser-Val-Pro-Ala-Asn-Thr-Glu-Asn-Glu-Val-Glu-Pro-Val

* 1921                                                                          1980*
GAT.GCC.CGC.CCT.GCT.GCC.GAC.CGA.GGA.CTG.ACC.ACT.CGA.CCA.GGT.TCT.GGG.TTG.ACA.AAT
Asp-Ala-Arg-Pro-Ala-Ala-Asp-Arg-Gly-Leu-Thr-Thr-Arg-Pro-Gly-Ser-Gly-Leu-Thr-Asn

* 1981                                                                          2040*
ATC.AAG.ACG.GAG.GAG.ATC.TCT.GAA.GTG.AAG.ATG.GAT.GCA.GAA.TTC.CGA.CAT.GAC.TCA.GGA
Ile-Lys-Thr-Glu-Glu-Ile-Ser-Glu-Val-Lys-Met-Asp-Ala-Glu-Phe-Arg-His-Asp-Ser-Gly

* 2041                                                                          2100*
TAT.GAA.GTT.CAT.CAT.CAA.AAA.TTG.GTG.TTC.TTT.GCA.GAA.GAT.GTG.GGT.TCA.AAC.AAA.GGT
Tyr-Glu-Val-His-His-Gln-Lys-Leu-Val-Phe-Phe-Ala-Glu-Asp-Val-Gly-Ser-Asn-Lys-Gly

* 2101                                                                          2160*
GCA.ATC.ATT.GGA.CTC.ATG.GTG.GGC.GGT.GTT.GTC.ATA.GCG.ACA.GTG.ATC.GTC.ATC.ACC.TTG
Ala-Ile-Ile-Gly-Leu-Met-Val-Gly-Gly-Val-Val-Ile-Ala-Thr-Val-Ile-Val-Ile-Thr-Leu

* 2161                                                                          2220*
GTG.ATG.CTG.AAG.AAG.AAA.CAG.TAC.ACA.TCC.ATT.CAT.CAT.GGT.GTG.GTG.GAG.GTT.GAC.GCC
Val-Met-Leu-Lys-Lys-Lys-Gln-Tyr-Thr-Ser-Ile-His-His-Gly-Val-Val-Glu-Val-Asp-Ala

* 2221                                                                          2280*
GCT.GTC.ACC.CCA.GAG.GAG.CGC.CAC.CTG.TCC.AAG.ATG.CAG.CAG.AAC.GGC.TAC.GAA.AAT.CCA
Ala-Val-Thr-Pro-Glu-Glu-Arg-His-Leu-Ser-Lys-Met-Gln-Gln-Asn-Gly-Tyr-Glu-Asn-Pro

* 2281                                 2313 *
ACC.TAC.AAG.TTC.TTT.GAG.CAG.ATG.CAG.AAC.TAG.
Thr-Tyr-Lys-Phe-Phe-Glu-Gln-Met-Gln-Asn-***-

*2314
ACCCCCGC CACAGCAGCC TCTGAAGTTG GACAGCAAAA CCATTGCTTC ACTACCCATC

GGTGTCCATT TATAGAATAA TGTGGGAAGA AACAAACCCG TTTTATGATT TACTCATTAT

CGCCTTTTGA CAGCTGTGCT GTAACACAAG TAGATGCCTG AACTTGAATT AATCCACACA

TCAGTAATGT ATTCTATCTC TCTTTACATT TTGGTCTCTA TACTACATTA TTAATGGGTT

TTGTGTACTG TAAAGAATTT AGCTGTATCA AACTAGTGCA TGAATAGATT CTCTCCTGAT

TATTTATCAC ATAGCCCCTT AGCCAGTTGT ATATTATTCT TGTGGTTTGT GACCCAATTA

AGTCCTACTT TACATATGCT TTAAGAATCG ATGGGGGATG CTTCATGTGA ACGTGGGAGT

TCAGCTGCTT CTCTTGCCTA AGTATTCCTT TCCTGATCAC TATGCATTTT AAAGTTAAAC

ATTTTTAAGT ATTTCAGATG CTTTAGAGAG ATTTTTTTTC CATGACTGCA TTTTACTGTA

CAGATTGCTG CTTCTGCTAT ATTTGTGATA TAGGAATTAA GAGGATACAC ACGTTTGTTT

                                            2959*
CTTCGTGCCT GTTTTATGTG CACACATTAG GCATTGAGAC TTCAAGCT
```

# FIG. 1 (B)-a

```
* -47                                              -1*
G ATCCCACTCG CACAGCAGCG   CACTCGGTGC CCCGCGCAGG GTCGCG

*   1                                                                             60*
ATG.CTG.CCC.GGT.TTG.GCA.CTG.CTC.CTG.CTG.GCC.GCC.TGG.ACG.GCT.CGG.GCG.CTG.GAG.GTA
Met-Leu-Pro-Gly-Leu-Ala-Leu-Leu-Leu-Leu-Ala-Ala-Trp-Thr-Ala-Arg-Ala-Leu-Glu-Val
 1                                                                               20
*  61                                                                            120*
CCC.ACT.GAT.GGT.AAT.GCT.GGC.CTG.CTG.GCT.GAA.CCC.CAG.ATT.GCC.ATG.TTC.TGT.GGC.AGA
Pro-Thr-Asp-Gly-Asn-Ala-Gly-Leu-Leu-Ala-Glu-Pro-Gln-Ile-Ala-Met-Phe-Cys-Gly-Arg

* 121                                                                            180*
CTG.AAC.ATG.CAC.ATG.AAT.GTC.CAG.AAT.GGG.AAG.TGG.GAT.TCA.GAT.CCA.TCA.GGG.ACC.AAA
Leu-Asn-Met-His-Met-Asn-Val-Gln-Asn-Gly-Lys-Trp-Asp-Ser-Asp-Pro-Ser-Gly-Thr-Lys

* 181                                                                            240*
ACC.TGC.ATT.GAT.ACC.AAG.GAA.GGC.ATC.CTG.CAG.TAT.TGC.CAA.GAA.GTC.TAC.CCT.GAA.CTG
Thr-Cys-Ile-Asp-Thr-Lys-Glu-Gly-Ile-Leu-Gln-Tyr-Cys-Gln-Glu-Val-Tyr-Pro-Glu-Leu

* 241                                                                            300*
CAG.ATC.ACC.AAT.GTG.GTA.GAA.GCC.AAC.CAA.CCA.GTG.ACC.ATC.CAG.AAC.TGG.TGC.AAG.CGG
Gln-Ile-Thr-Asn-Val-Val-Glu-Ala-Asn-Gln-Pro-Val-Thr-Ile-Gln-Asn-Trp-Cys-Lys-Arg

* 301                                                                            360*
GGC.CGC.AAG.CAG.TGC.AAG.ACC.CAT.CCC.CAC.TTT.GTG.ATT.CCC.TAC.CGC.TGC.TTA.GTT.GGT
Gly-Arg-Lys-Gln-Cys-Lys-Thr-His-Pro-His-Phe-Val-Ile-Pro-Tyr-Arg-Cys-Leu-Val-Gly

* 361                                                                            420*
GAG.TTT.GTA.AGT.GAT.GCC.CTT.CTC.GTT.CCT.GAC.AAG.TGC.AAA.TTC.TTA.CAC.CAG.GAG.AGG
Glu-Phe-Val-Ser-Asp-Ala-Leu-Leu-Val-Pro-Asp-Lys-Cys-Lys-Phe-Leu-His-Gln-Glu-Arg

* 421                                                                            480*
ATG.GAT.GTT.TGC.GAA.ACT.CAT.CTT.CAC.TGG.CAC.ACC.GTC.GCC.AAA.GAG.ACA.TGC.AGT.GAG
Met-Asp-Val-Cys-Glu-Thr-His-Leu-His-Trp-His-Thr-Val-Ala-Lys-Glu-Thr-Cys-Ser-Glu

* 481                                                                            540*
AAG.AGT.ACC.AAC.TTG.CAT.GAC.TAC.GGC.ATG.TTG.CTG.CCC.TGC.GGA.ATT.GAC.AAG.TTC.CGA
Lys-Ser-Thr-Asn-Leu-His-Asp-Tyr-Gly-Met-Leu-Leu-Pro-Cys-Gly-Ile-Asp-Lys-Phe-Arg

* 541                                                                            600*
GGG.GTA.GAG.TTT.GTG.TGT.TGC.CCA.CTG.GCT.GAA.GAA.AGT.GAC.AAT.GTG.GAT.TCT.GCT.GAT
Gly-Val-Glu-Phe-Val-Cys-Cys-Pro-Leu-Ala-Glu-Glu-Ser-Asp-Asn-Val-Asp-Ser-Ala-Asp

* 601                                                                            660*
GCG.GAG.GAG.GAT.GAC.TCG.GAT.GTC.TGG.TGG.GGC.GGA.GCA.GAC.ACA.GAC.TAT.GCA.GAT.GGG
Ala-Glu-Glu-Asp-Asp-Ser-Asp-Val-Trp-Trp-Gly-Gly-Ala-Asp-Thr-Asp-Tyr-Ala-Asp-Gly

* 661                                                                            720*
AGT.GAA.GAC.AAA.GTA.GTA.GAA.GTA.GCA.GAG.GAG.GAA.GAA.GTG.GCT.GAG.GTG.GAA.GAA.GAA
Ser-Glu-Asp-Lys-Val-Val-Glu-Val-Ala-Glu-Glu-Glu-Glu-Val-Ala-Glu-Val-Glu-Glu-Glu

* 721                                                                            780*
GAA.GCC.GAT.GAT.GAC.GAG.GAC.GAT.GAG.GAT.GGT.GAT.GAG.GTA.GAG.GAA.GAG.GCT.GAG.GAA
Glu-Ala-Asp-Asp-Asp-Glu-Asp-Asp-Glu-Asp-Gly-Asp-Glu-Val-Glu-Glu-Glu-Ala-Glu-Glu

* 781                                                                            840*
CCC.TAC.GAA.GAA.GCC.ACA.GAG.AGA.ACC.ACC.AGC.ATT.GCC.ACC.ACC.ACC.ACC.ACC.ACC.ACA
Pro-Tyr-Glu-Glu-Ala-Thr-Glu-Arg-Thr-Thr-Ser-Ile-Ala-Thr-Thr-Thr-Thr-Thr-Thr-Thr

* 841                             865 ┐┌ 866                                     900*
GAG.TCT.GTG.GAA.GAG.GTG.GTT.CGA.GAG.GTG.TGC.TCT.GAA.CAA.GCC.GAG.ACG.GGG.CCG.TGC
Glu-Ser-Val-Glu-Glu-Val-Val-Arg-Glu-Val-Cys-Ser-Glu-Gln-Ala-Glu-Thr-Gly-Pro-Cys
                                288 289
```

# FIG. 1 (B)-b

* 901 960*
CGA.GCA.ATG.ATC.TCC.CGC.TGG.TAC.TTT.GAT.GTG.ACT.GAA.GGG.AAG.TGT.GCC.CCA.TTC.TTT
Arg-Ala-Met-Ile-Ser-Arg-Trp-Tyr-Phe-Asp-Val-Thr-Glu-Gly-Lys-Cys-Ala-Pro-Phe-Phe

* 961 1020*
TAC.GGC.GGA.TGT.GGC.GGC.AAC.CGG.AAC.AAC.TTT.GAC.ACA.GAA.GAG.TAC.TGC.ATG.GCC.GTG
Tyr-Gly-Gly-Cys-Gly-Gly-Asn-Arg-Asn-Asn-Phe-Asp-Thr-Glu-Glu-Tyr-Cys-Met-Ala-Val

1033 1034
* 1021 1080*
TGT.GGC.AGC.GCC.ATT.CCT.ACA.ACA.GCA.GCC.AGT.ACC.CCT.GAT.GCC.GTT.GAC.AAG.TAT.CTC
Cys-Gly-Ser-Ala-Ile-Pro-Thr-Thr-Ala-Ala-Ser-Thr-Pro-Asp-Ala-Val-Asp-Lys-Tyr-Leu
345 346

* 1081 1140*
GAG.ACA.CCT.GGG.GAT.GAG.AAT.GAA.CAT.GCC.CAT.TTC.CAG.AAA.GCC.AAA.GAG.AGG.CTT.GAG
Glu-Thr-Pro-Gly-Asp-Glu-Asn-Glu-His-Ala-His-Phe-Gln-Lys-Ala-Lys-Glu-Arg-Leu-Glu

* 1141 1200*
GCC.AAG.CAC.CGA.GAG.AGA.ATG.TCC.CAG.GTC.ATG.AGA.GAA.TGG.GAA.GAG.GCA.GAA.CGT.CAA
Ala-Lys-His-Arg-Glu-Arg-Met-Ser-Gln-Val-Met-Arg-Glu-Trp-Glu-Glu-Ala-Glu-Arg-Gln

* 1201 1260*
GCA.AAG.AAC.TTG.CCT.AAA.GCT.GAT.AAG.AAG.GCA.GTT.ATC.CAG.CAT.TTC.CAG.GAG.AAA.GTG
Ala-Lys-Asn-Leu-Pro-Lys-Ala-Asp-Lys-Lys-Ala-Val-Ile-Gln-His-Phe-Gln-Glu-Lys-Val

* 1261 1320*
GAA.TCT.TTG.GAA.CAG.GAA.GCA.GCC.AAC.GAG.AGA.CAG.CAG.CTG.GTG.GAG.ACA.CAC.ATG.GCC
Glu-Ser-Leu-Glu-Gln-Glu-Ala-Ala-Asn-Glu-Arg-Gln-Gln-Leu-Val-Glu-Thr-His-Met-Ala

* 1321 1380*
AGA.GTG.GAA.GCC.ATG.CTC.AAT.GAC.CGC.CGC.CGC.CTG.GCC.CTG.GAG.AAC.TAC.ATC.ACC.GCT
Arg-Val-Glu-Ala-Met-Leu-Asn-Asp-Arg-Arg-Arg-Leu-Ala-Leu-Glu-Asn-Tyr-Ile-Thr-Ala

* 1381 1440*
CTG.CAG.GCT.GTT.CCT.CCT.CGG.CCT.CGT.CAC.GTG.TTC.AAT.ATG.CTA.AAG.AAG.TAT.GTC.CGC
Leu-Gln-Ala-Val-Pro-Pro-Arg-Pro-Arg-His-Val-Phe-Asn-Met-Leu-Lys-Lys-Tyr-Val-Arg

* 1441 1500*
GCA.GAA.CAG.AAG.GAC.AGA.CAG.CAC.ACC.CTA.AAG.CAT.TTC.GAG.CAT.GTG.CGC.ATG.GTG.GAT
Ala-Glu-Gln-Lys-Asp-Arg-Gln-His-Thr-Leu-Lys-His-Phe-Glu-His-Val-Arg-Met-Val-Asp

* 1501 1560*
CCC.AAG.AAA.GCC.GCT.CAG.ATC.CGG.TCC.CAG.GTT.ATG.ACA.CAC.CTC.CGT.GTG.ATT.TAT.GAG
Pro-Lys-Lys-Ala-Ala-Gln-Ile-Arg-Ser-Gln-Val-Met-Thr-His-Leu-Arg-Val-Ile-Tyr-Glu

* 1561 1620*
CGC.ATG.AAT.CAG.TCT.CTC.TCC.CTG.CTC.TAC.AAC.GTG.CCT.GCA.GTG.GCC.GAG.GAG.ATT.CAG
Arg-Met-Asn-Gln-Ser-Leu-Ser-Leu-Leu-Tyr-Asn-Val-Pro-Ala-Val-Ala-Glu-Glu-Ile-Gln

* 1621 1680*
GAT.GAA.GTT.GAT.GAG.CTG.CTT.CAG.AAA.GAG.CAA.AAC.TAT.TCA.GAT.GAC.GTC.TTG.GCC.AAC
Asp-Glu-Val-Asp-Glu-Leu-Leu-Gln-Lys-Glu-Gln-Asn-Tyr-Ser-Asp-Asp-Val-Leu-Ala-Asn

* 1681 1740*
ATG.ATT.AGT.GAA.CCA.AGG.ATC.AGT.TAC.GGA.AAC.GAT.GCT.CTC.ATG.CCA.TCT.TTG.ACC.GAA
Met-Ile-Ser-Glu-Pro-Arg-Ile-Ser-Tyr-Gly-Asn-Asp-Ala-Leu-Met-Pro-Ser-Leu-Thr-Glu

* 1741 1800*
ACG.AAA.ACC.ACC.GTG.GAG.CTC.CTT.CCC.GTG.AAT.GGA.GAG.TTC.AGC.CTG.GAC.GAT.CTC.CAG
Thr-Lys-Thr-Thr-Val-Glu-Leu-Leu-Pro-Val-Asn-Gly-Glu-Phe-Ser-Leu-Asp-Asp-Leu-Gln

* 1801 1860*
CCG.TGG.CAT.TCT.TTT.GGG.GCT.GAC.TCT.GTG.CCA.GCC.AAC.ACA.GAA.AAC.GAA.GTT.GAG.CCT
Pro-Trp-His-Ser-Phe-Gly-Ala-Asp-Ser-Val-Pro-Ala-Asn-Thr-Glu-Asn-Glu-Val-Glu-Pro

# FIG. 1 (B)-c

```
* 1861                                                                                1920*
GTT.GAT.GCC.CGC.CCT.GCT.GCC.GAC.CGA.GGA.CTG.ACC.ACT.CGA.CCA.GGT.TCT.GGG.TTG.ACA
Val-Asp-Ala-Arg-Pro-Ala-Ala-Asp-Arg-Gly-Leu-Thr-Thr-Arg-Pro-Gly-Ser-Gly-Leu-Thr

* 1921                                                                                1980*
AAT.ATC.AAG.ACG.GAG.GAG.ATC.TCT.GAA.GTG.AAG.ATG.GAT.GCA.GAA.TTC.CGA.CAT.GAC.TCA
Asn-Ile-Lys-Thr-Glu-Glu-Ile-Ser-Glu-Val-Lys-Met-Asp-Ala-Glu-Phe-Arg-His-Asp-Ser

* 1981                                                                                2040*
GGA.TAT.GAA.GTT.CAT.CAT.CAA.AAA.TTG.GTG.TTC.TTT.GCA.GAA.GAT.GTG.GGT.TCA.AAC.AAA
Gly-Tyr-Glu-Val-His-His-Gln-Lys-Leu-Val-Phe-Phe-Ala-Glu-Asp-Val-Gly-Ser-Asn-Lys

* 2041                                                                                2100*
GGT.GCA.ATC.ATT.GGA.CTC.ATG.GTG.GGC.GGT.GTT.GTC.ATA.GCG.ACA.GTG.ATC.GTC.ATC.ACC
Gly-Ala-Ile-Ile-Gly-Leu-Met-Val-Gly-Gly-Val-Val-Ile-Ala-Thr-Val-Ile-Val-Ile-Thr

* 2101                                                                                2160*
TTG.GTG.ATG.CTG.AAG.AAG.AAA.CAG.TAC.ACA.TCC.ATT.CAT.CAT.GGT.GTG.GTG.GAG.GTT.GAC
Leu-Val-Met-Leu-Lys-Lys-Lys-Gln-Tyr-Thr-Ser-Ile-His-His-Gly-Val-Val-Glu-Val-Asp

* 2161                                                                                2220*
GCC.GCT.GTC.ACC.CCA.GAG.GAG.CGC.CAC.CTG.TCC.AAG.ATG.CAG.CAG.AAC.GGC.TAC.GAA.AAT
Ala-Ala-Val-Thr-Pro-Glu-Glu-Arg-His-Leu-Ser-Lys-Met-Gln-Gln-Asn-Gly-Tyr-Glu-Asn

* 2221                                 2256*
CCA.ACC.TAC.AAG.TTC.TTT.GAG.CAG.ATG.CAG.AAC.TAG.
Pro-Thr-Tyr-Lys-Phe-Phe-Glu-Gln-Met-Gln-Asn-***-

 *2257
 ACCCCCGC CACAGCAGCC TCTGAAGTTG GACAGCAAAA CCATTGCTTC ACTACCCATC

GGTGTCCATT TATAGAATAA TGTGGGAAGA AACAAACCCG TTTTATGATT TACTCATTAT

CGCCTTTTGA CAGCTGTGCT GTAACACAAG TAGATGCCTG AACTTGAATT AATCCACACA

TCAGTAATGT ATTCTATCTC TCTTTACATT TTGGTCTCTA TACTACATTA TTAATGGGTT

TTGTGTACTG TAAAGAATTT AGCTGTATCA AACTAGTGCA TGAATAGATT CTCTCCTGAT

TATTTATCAC ATAGCCCCTT AGCCAGTTGT ATATTATTCT TGTGGTTTGT GACCCAATTA

AGTCCTACTT TACATATGCT TTAAGAATCG ATGGGGGATG CTTCATGTGA ACGTGGGAGT

TCAGCTGCTT CTCTTGCCTA AGTATTCCTT TCCTGATCAC TATGCATTTT AAAGTTAAAC

ATTTTTAAGT ATTTCAGATG CTTTAGAGAG ATTTTTTTTC CATGACTGCA TTTTACTGTA

CAGATTGCTG CTTCTGCTAT ATTTGTGATA TAGGAATTAA GAGGATACAC ACGTTTGTTT

                                                2902*
CTTCGTGCCT GTTTTATGTG CACACATTAG GCATTGAGAC TTCAAGCT
```

FIG. 2

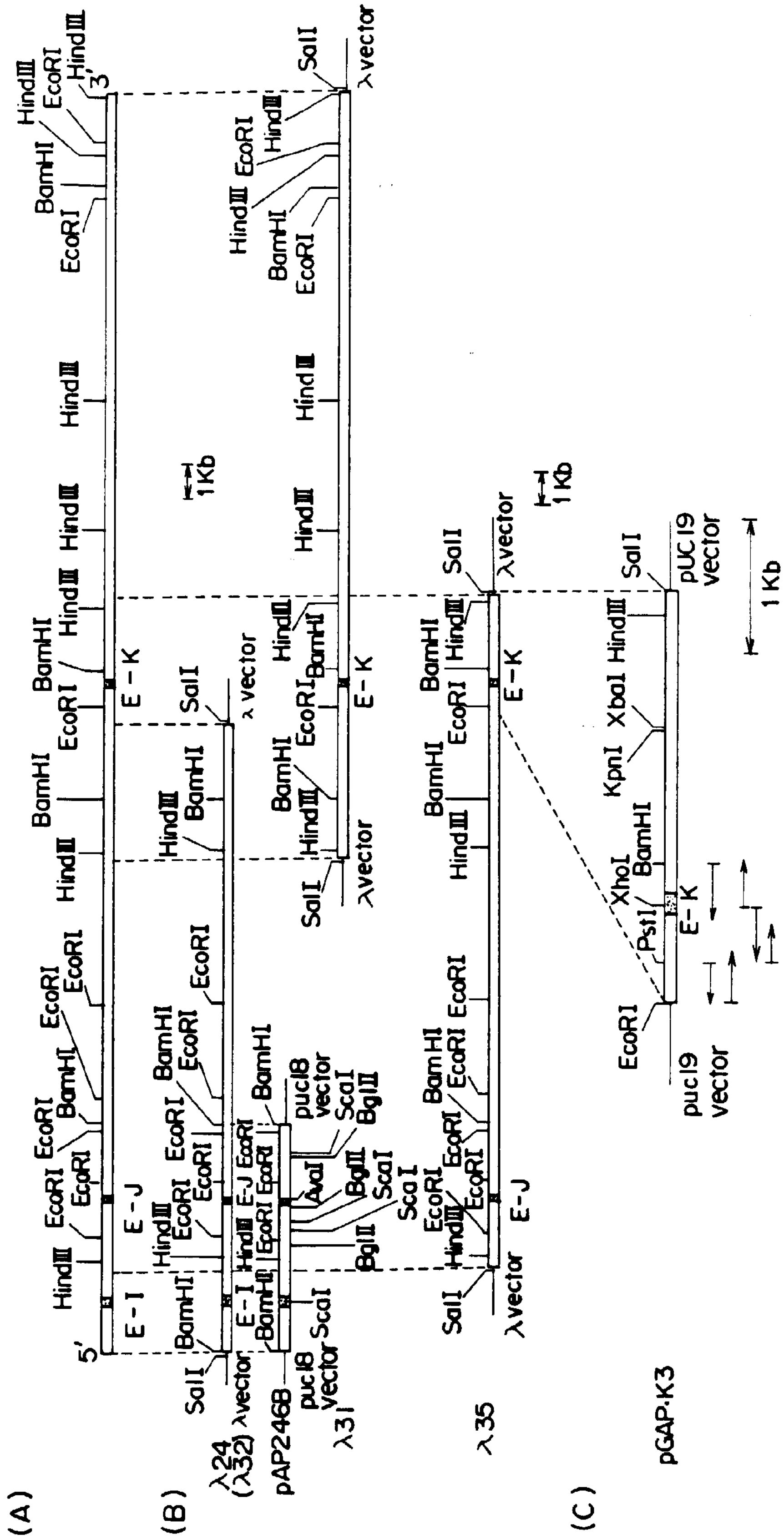
(A)
(B)
(C)
5'
3'
E-I
E-J
E-K
λ24
(λ32)
pAP246B
λ31
λ35
pGAP·K3
λ vector
puc18 vector
puc19 vector
pUC19 vector
1Kb
1 Kb
SalI
HindIII
EcoRI
BamHI
ScaI
BglII
AvaI
PstI
XhoI
KpnI
XbaI

FIG. 3

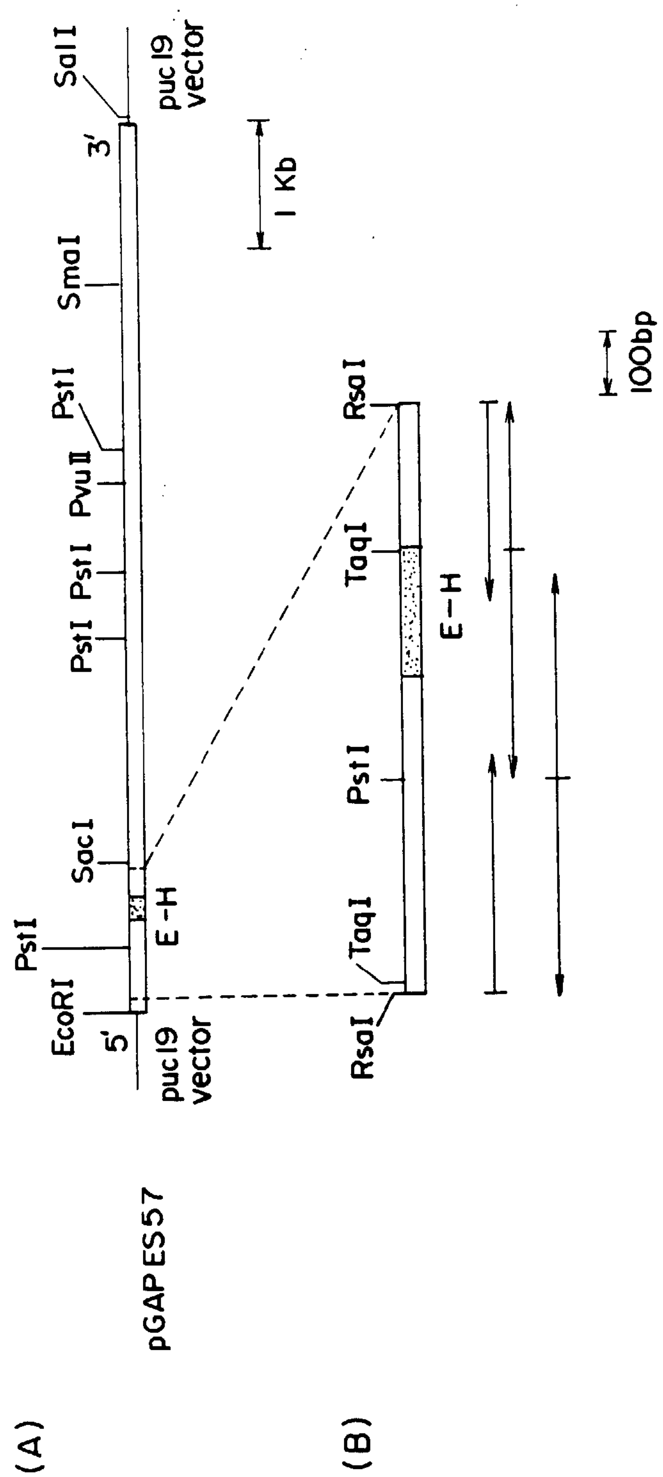
(A)
pGAPES57
SalI
puc19 vector
3'
1 Kb
SmaI
PstI
PvuII
PstI
PstI
SacI
E-H
PstI
EcoRI
5'
puc19 vector
(B)
RsaI
TaqI
E-H
PstI
TaqI
RsaI
100bp

# FIG. 4

| 10 | 20 | 30 | 40 | 50 | 60 |
|---|---|---|---|---|---|
| GTACGTTGAA | CCAATCGATA | ATATTATTAA | CAGTATTCTG | TTTTAACATG | AGAGGCGACT |
| 70 | 80 | 90 | 100 | 110 | 120 |
| TTTTGTGTAG | TATAGTCTCT | TGGTTTTCGA | CAAATGCTTA | ATGCTGTGTA | TCTATTATTA |
| 130 | 140 | 150 | 160 | 170 | 180 |
| CTTATGACAG | TATCGTGCAG | AGTAGTTTCT | AAAAATCCTC | TCTACTCACC | TATTCATCCC |
| 190 | 200 | 210 | 220 | 230 | 240 |
| TTCCCCACTC | CCCCTGAACC | ACTGGCAACC | GCTGATCTTT | TTATTGTCTC | CGTAATTTTC |
| 250 | 260 | 270 | 280 | 290 | 300 |
| CCTTTTCCAG | AATGTCCTGT | GGTTAGACTC | ATGGCATCCC | CTTTTAGCAA | CTGCAGAAAC |
| 310 | 320 | 330 | 340 | 350 | 360 |
| TAAGAACTGT | CCCCAAGGAT | AAAAATTTTT | GCAGACCCAC | TGAGCTGGGA | TTTATACTTA |
| 370 | 380 | 390 | 400 | 410 | 420 |
| GACCTGCTGA | ATTGAAGTTT | TGGGTGTGTT | TCTTCTCAAA | TTGCCAAAAT | TCCATATGGA |
| 430 | 440 | 450 | 460 | 470 | 480 |
| CGACTTTTCT | TTTCCTTCCC | TGAAATGTGT | TTAATTGACT | TTTTCTGTTA | TTTGTGTTTG |
| 490 | 500 | 510 | 520 | 530 | 540 |
| CCTTCACAGT | GAAGACAAAG | TAGTAGAAGT | AGCAGAGGAG | GAAGAAGTGG | CTGAGGTGGA |
| 550 | 560 | 570 | 580 | 590 | 600 |
| AGAAGAAGAA | GCCGATGATG | ACGAGGACGA | TGAGGATGGT | GATGAGGTAG | AGGAAGAGGC |
| 610 | 620 | 630 | 640 | 650 | 660 |
| TGAGGAACCC | TACGAAGAAG | CCACAGAGAG | AACCACCAGC | ATTGCCACCA | CCACCACCAC |
| 670 | 680 | 690 | 700 | 710 | 720 |
| CACCACAGAG | TCTGTGGAAG | AGGTGGTTCG | AGGTAATCCA | CCATTTGCTT | GGATTCCCCC |
| 730 | 740 | 750 | 760 | 770 | 780 |
| CACCCCCAAG | GAAAAGAAAG | CGTAATACCA | GAGTTGGAAA | TATCCACCCT | AGCACCACTG |
| 790 | 800 | 810 | 820 | 830 | 840 |
| CCTTCCCCAA | TCAAAAACAT | GTTTTTTTTT | CCAAAAGGCT | TCTTATGCTT | GTGAAATTTT |
| 850 | 860 | 870 | 880 | 890 | 900 |
| TTTGGTTTAA | CAAGCAAACA | ATTTCAAATA | ATGTGAAATC | TTTATTATAC | AGTAC |

# FIG. 5

| 10 | 20 | 30 | 40 | 50 | 60 |
|---|---|---|---|---|---|
| GATTAAAGAA | GTAAACGTGT | ATACATGAAC | AGAGAGACAG | TGCCTTTTCA | TGCTAAATGT |
| 70 | 80 | 90 | 100 | 110 | 120 |
| GGTTCCCCAC | ATCTCCTCTG | ATTAGAGGTG | TGCTCTGAAC | AAGCCGAGAC | GGGGCCGTGC |
| 130 | 140 | 150 | 160 | 170 | 180 |
| CGAGCAATGA | TCTCCCGCTG | GTACTTTGAT | GTGACTGAAG | GGAAGTGTGC | CCCATTCTTT |
| 190 | 200 | 210 | 220 | 230 | 240 |
| TACGGCGGAT | GTGGCGGCAA | CCGGAACAAC | TTTGACACAG | AAGAGTACTG | CATGGCCGTG |
| 250 | 260 | 270 | 280 | 290 | 300 |
| TGTGGCAGCG | CCAGTAAGTG | GACCCTTCTT | CGAGCCTGGA | CCTTTCGTCT | CTCTCGCCAC |
| 310 | 320 | 330 | 340 | 350 | 360 |
| TGACTCTGCT | TTTTGTAACA | GATTGATTTT | CCTGGTTCTT | GGGAATGGGC | CTGTTGCTAC |
| 370 | 380 | 390 | 400 | 410 | 420 |
| CACTAACCAC | ATTTCTGTCC | ACTTCTCTAA | TTGCTCAGAG | TCTCCGCAGT | ATGTTCAATC |
| 430 | 440 | 450 | 460 | | |
| ATGAGCACAC | CTCTCCGTCT | TCCCTGATAA | AGCATGT | | |

# FIG. 6

```
        10         20         30         40         50         60
AAGCTTGCAT GCCTGCAGGT CGACTCTAGA GGATCTTATG TTTATATGTT CATTTTGGTT

        70         80         90        100        110        120
TTGTTGGAGG GACCAAACCT AAGTGAGTGA TTTTGTTTGT TAGGTTGTTT TTTTGTCAGT

       130        140        150        160        170        180
GGACTCGTGC ATTTCAGCCA TCATTCCCAT GTTTCTCTTT TTGTTTTTAG TTATGTTCTC

       190        200        210        220        230        240
TTATTTTTTA CCTAGTGTCC CAAAGTTTAC TCAAGACTAC CCAGGAACCT CTTGCCCGAG

       250        260        270        280        290        300
ATCCTGTTAA ACGTACGTTG TCATTCACCT GAGGGAAGGG AAGAGGGGAG GAGGATGCTG

       310        320        330        340        350        360
CTTGGTTCAC ATAACTCCAG CATCATCACC TTCTTTGCAT GGTTTTGTGT TTCTTGAACA

       370        380        390        400
CCTGTCTTAG TAAAATGTTT CTTCCCATTA CCTTG
```

# FIG. 7

| 10 | 20 | 30 | 40 | 50 | 60 |
|---|---|---|---|---|---|
| GAATTCATTG | ACTATTGTCA | AATTTATGAA | GACAGAAAAG | TCCTTTTGTG | ATTCAAAAAT |
| 70 | 80 | 90 | 100 | 110 | 120 |
| GTGTTTATTG | ATGTTTATAT | ATCTTTTGTA | GTGTTTCCCA | TACTGTCTCA | TGAAGGTTGT |
| 130 | 140 | 150 | 160 | 170 | 180 |
| CTTCGTAATT | CAGTTATCTA | AAATACATCA | TACAATTTAC | CTTTTCCCTG | GGGTGCCGTT |
| 190 | 200 | 210 | 220 | 230 | 240 |
| ATTCTACAAC | AGGCTAATTA | CAGATATCTG | TGGTTTCTGC | AGGAGTCTTA | GTGTATGATA |
| 250 | 260 | 270 | 280 | 290 | 300 |
| TTGATGATAT | CATCATTTAC | TTTCAAAATT | GCAAAATAGT | AATTTAGGAA | AGCATGGGGA |
| 310 | 320 | 330 | 340 | 350 | 360 |
| TTTGGAAAAA | CATGTCTTCA | GCACCAACTG | TTTTGCTCTT | TGCATGCTTG | TTTCGTAAAG |
| 370 | 380 | 390 | 400 | 410 | 420 |
| AACTCCTAAT | GCTATAATTG | AAAATGGACC | ATTTTAAAGA | TTTTTCCTTC | ATTCTGTACT |
| 430 | 440 | 450 | 460 | 470 | 480 |
| TGGGAGTGGT | GAAAGACATC | CTTACTGTGC | TGCACAGTGT | CTCATGGTGT | TCTCTTAAAC |
| 490 | 500 | 510 | 520 | 530 | 540 |
| AGCATTAACG | TCTTGTATGC | GCTGCTTTAC | TAAATTCTCT | GTTCTGAGAA | ATAACTGAAA |
| 550 | 560 | 570 | 580 | 590 | 600 |
| ATACGGCTTT | CTATTAAACG | AGTGGATTAT | TCTGTTGTTG | TTGGCTTTTT | TTTCTCAAAC |
| 610 | 620 | 630 | 640 | 650 | 660 |
| CTCCTTCTCT | TCTACTTTAT | AGTTCCTACA | ACAGCAGCCA | GTACCCCTGA | TGCCGTTGAC |
| 670 | 680 | 690 | 700 | 710 | 720 |
| AAGTATCTCG | AGACACCTGG | GGATGAGAAT | GAACATGCCC | ATTTCCAGAA | AGCCAAAGAG |
| 730 | 740 | 750 | 760 | 770 | 780 |
| AGGCTTGAGG | CCAAGCACCG | AGAGAGAATG | TCCCAGGTAA | GTCTGGCTCT | TCCATCATTC |
| 790 | 800 | 810 | 820 | 830 | 840 |
| AGCCCTACGA | TATTGGGAAA | CCTGAGCTTG | CCTCTGCCTC | AGTCTCCCCA | CAGGCCTGCT |
| 850 | 860 | 870 | 880 | 890 | 900 |
| GGCTTTATCA | AGATCTTTAA | AGATGTAAAG | TTCTAATTTT | AAATGTTTAC | TGTGTGGGCA |
| 910 | 920 | 930 | 940 | 950 | 960 |
| CAGTTTGTGG | GTTTTTTGCA | TCCTCCGTGA | ACATGCTGCC | GTAGGAAGGA | TCC |

# FIG. 8

NAP 289 Glu Val Cys Ser Glu Gln Ala Glu Thr Gly Pro Cys Arg Ala Met Ile Ser
BPTI 17 Asp Phe Cys Leu Glu Pro Pro Tyr Thr Gly Pro Cys Lys Ala Arg Ile Ile
h-IαTI 80 Ala Ala Cys Asn Leu Pro Val Ile Arg Gly Pro Cys Arg Ala Phe Ile Gln

NAP 306 Arg Trp Tyr Phe Asp Val Thr Glu Gly Lys Cys Ala Pro Phe Phe Tyr Gly Gly Cys Gly
BPTI 34 Arg Tyr Phe Tyr Asn Ala Lys Ala Gly Leu Cys Gln Thr Phe Val Tyr Gly Gly Cys Arg
h-IαTI 97 Leu Trp Ala Phe Asp Ala Val Lys Gly Lys Cys Val Leu Phe Pro Tyr Gly Gly Cys Gln

NAP 326 Gly Asn Arg Asn Asn Phe Asp Thr Glu Glu Tyr Cys Met Ala Val Cys Gly Ser Ala Met
BPTI 54 Ala Lys Arg Asn Asn Phe Lys Ser Ala Glu Asp Cys Met Arg Thr Cys Gly Gly Ala Ile
h-IαTI 117 Gly Asn Gly Asn Lys Phe Tyr Ser Glu Lys Glu Cys Arg Glu Tyr Cys Gly Val Pro Gly

NAP 346 Ser Gln Ser Leu Leu Lys Thr Thr Gln Glu Pro Leu Ala Arg Asp Pro Val Lys Leu 364
BPTI 74 Gly Pro Trp Gly Lys Thr Gly Gly Arg Ala Glu Gly Glu Gly Lys Gly 89
h-IαTI 137 Asp Glu Asp Glu Glu Leu Leu 143

# FIG. 9

| | | | | | | | | | | | | | | | | | | |
|---|---|---|---|---|---|---|---|---|---|---|---|---|---|---|---|---|---|---|
| NAP3 | 289 | Glu | Val | Cys | Ser | Glu | Gln | Ala | Glu | Thr | Gly | Pro | Cys | Arg | Ala | Met | Ile | Ser |
| BPTI | 17 | Asp | Phe | Cys | Leu | Glu | Pro | Pro | Tyr | Thr | Gly | Pro | Cys | Lys | Ala | Arg | Ile | Ile |
| h-IαTI | 80 | Ala | Ala | Cys | Asn | Leu | Pro | Val | Ile | Arg | Gly | Pro | Cys | Arg | Ala | Phe | Ile | Gln |

| | | | | | | | | | | | | | | | | | | | | |
|---|---|---|---|---|---|---|---|---|---|---|---|---|---|---|---|---|---|---|---|---|
| NAP3 | 306 | Arg | Trp | Tyr | Phe | Asp | Val | Thr | Glu | Gly | Lys | Cys | Ala | Pro | Phe | Phe | Tyr | Gly | Gly | Cys | Gly |
| BPTI | 34 | Arg | Tyr | Phe | Tyr | Asn | Ala | Lys | Ala | Gly | Leu | Cys | Gln | Thr | Phe | Val | Tyr | Gly | Gly | Cys | Arg |
| h-IαTI | 97 | Leu | Trp | Ala | Phe | Asp | Ala | Val | Lys | Gly | Lys | Cys | Val | Leu | Phe | Pro | Tyr | Gly | Gly | Cys | Gln |

| | | | | | | | | | | | | | | | | | | | | | |
|---|---|---|---|---|---|---|---|---|---|---|---|---|---|---|---|---|---|---|---|---|---|
| NAP3 | 326 | Gly | Asn | Arg | Asn | Asn | Phe | Asp | Thr | Glu | Glu | Tyr | Cys | Met | Ala | Val | Cys | Gly | Ser | Ala | Ile |
| BPTI | 54 | Ala | Lys | Arg | Asn | Asn | Phe | Lys | Ser | Ala | Glu | Asp | Cys | Met | Arg | Thr | Cys | Gly | Gly | Ala | Ile |
| h-IαTI | 117 | Gly | Asn | Gly | Asn | Lys | Phe | Tyr | Ser | Glu | Lys | Glu | Cys | Arg | Glu | Tyr | Cys | Gly | Val | Pro | Gly |

FIG. 10 (A)

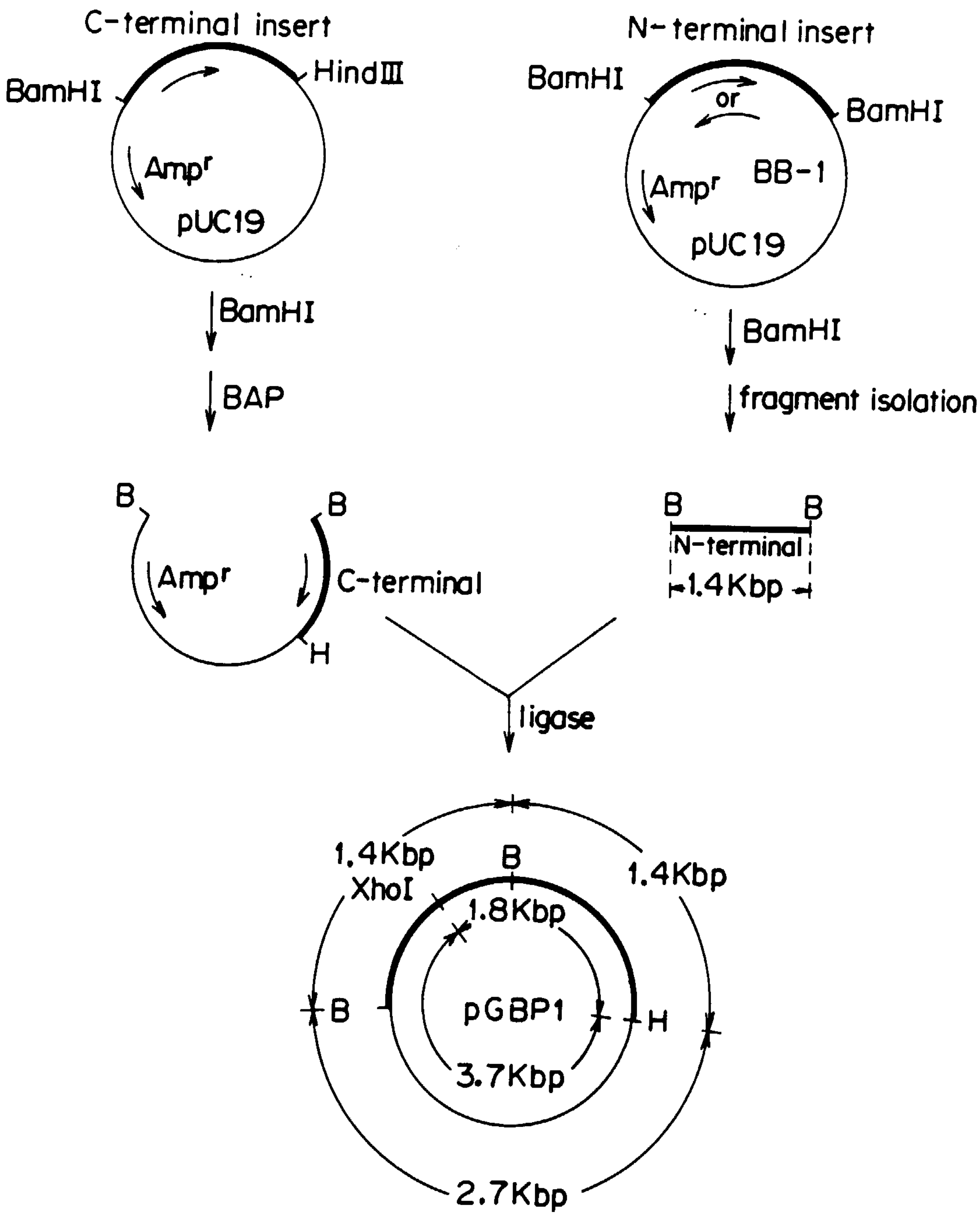
C-terminal insert
BamHI
HindIII
Amp^r
pUC19
N-terminal insert
BamHI
or
BamHI
Amp^r
BB-1
pUC19
BamHI
BAP
BamHI
fragment isolation
B
B
Amp^r
C-terminal
H
B
B
N-terminal
1.4Kbp
ligase
1.4Kbp
XhoI
B
1.4Kbp
1.8Kbp
B
pGBP1
H
3.7Kbp
2.7Kbp

# FIG. 10 (B)

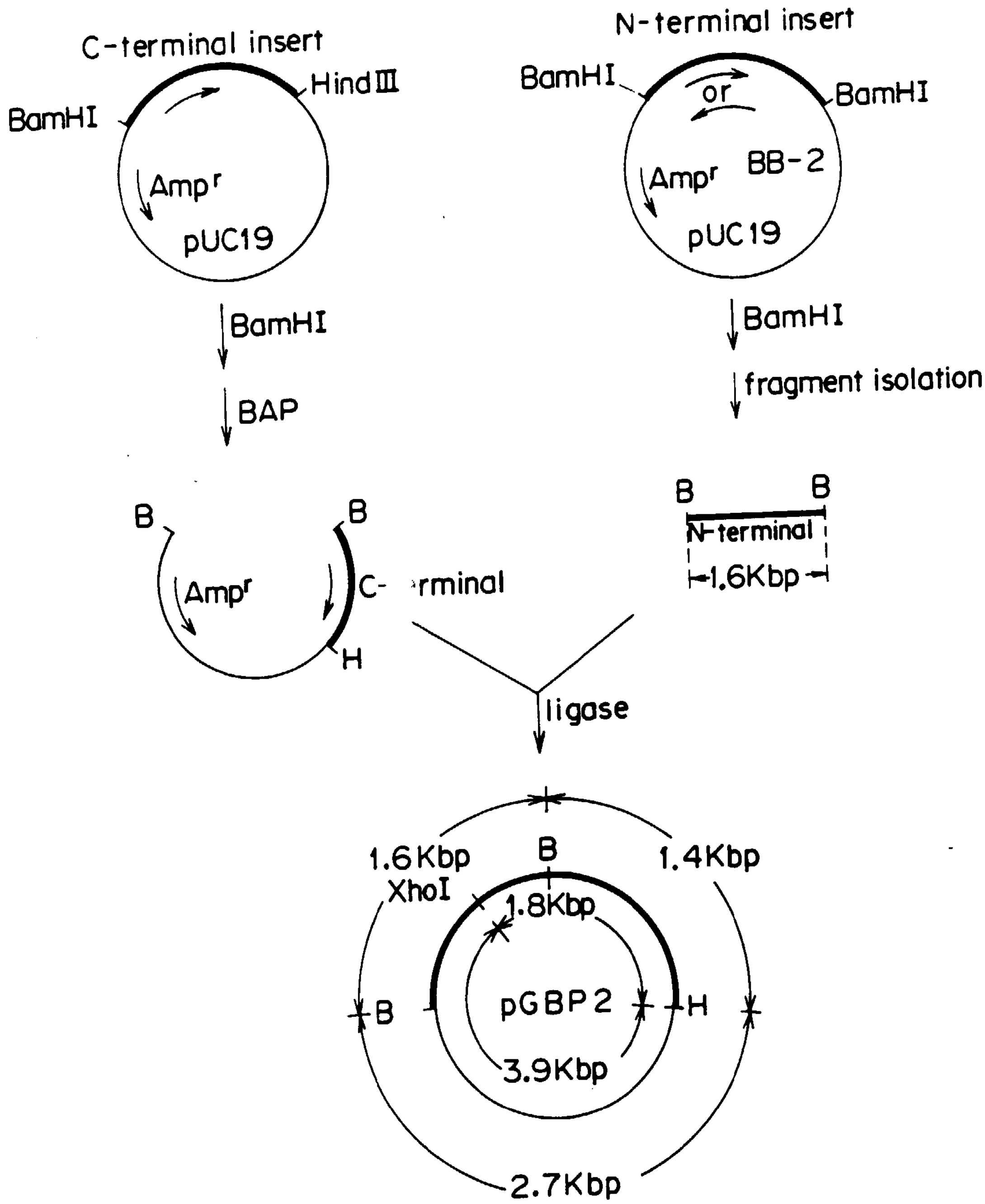
C-terminal insert
BamHI
HindIII
Amp^r
pUC19
N-terminal insert
BamHI
or
BamHI
BB-2
Amp^r
pUC19
BamHI
BAP
BamHI
fragment isolation
B
B
Amp^r
C- rminal
H
B
B
N-terminal
1.6Kbp
ligase
1.6Kbp
B
1.4Kbp
XhoI
1.8Kbp
B
pGBP2
H
3.9Kbp
2.7Kbp

FIG.11 (A)

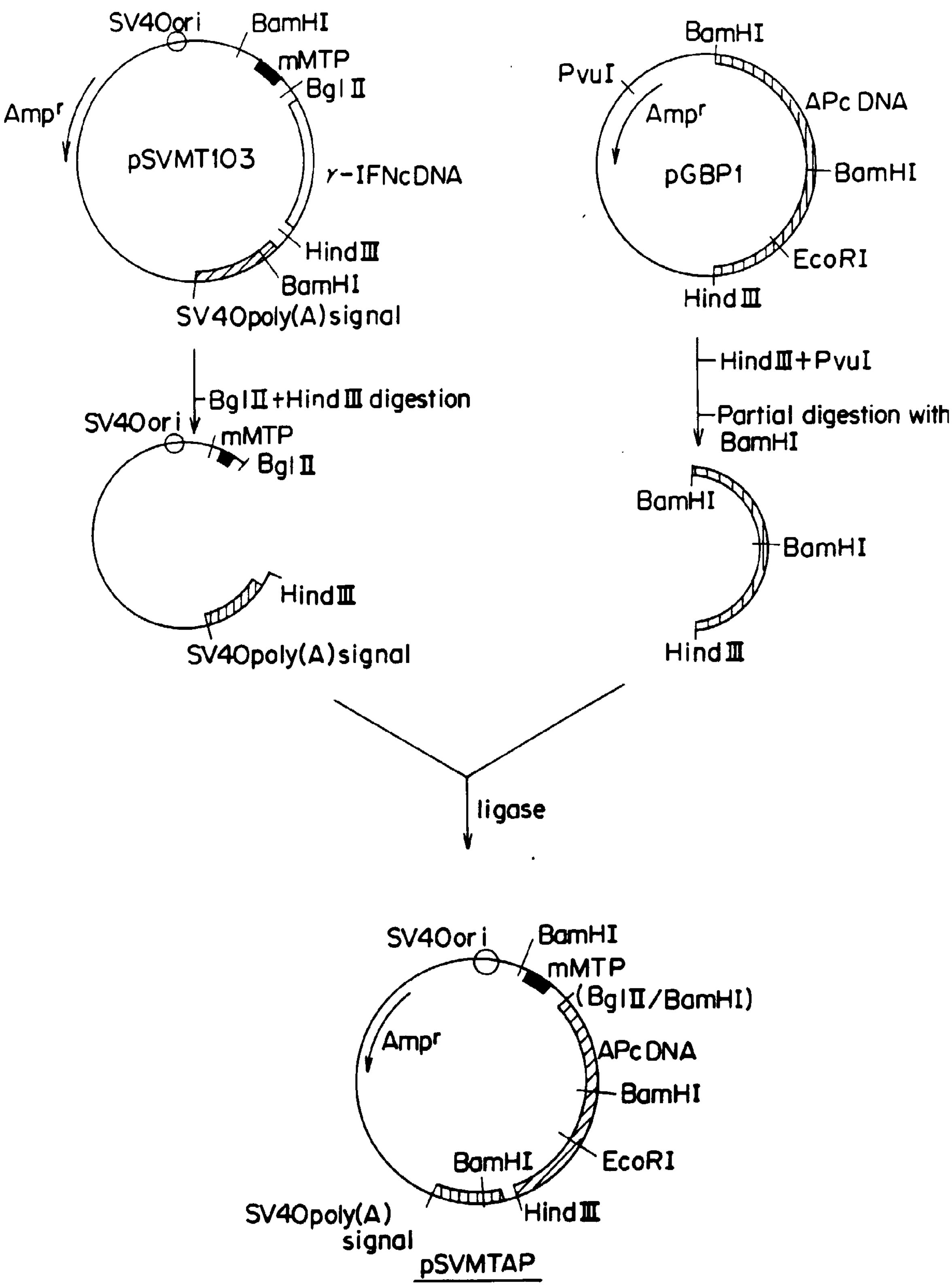
SV40ori
BamHI
mMTP
Bgl II
Amp^r
pSVMT103
γ-IFNcDNA
HindIII
BamHI
SV40poly(A)signal
BamHI
PvuI
APc DNA
Amp^r
pGBP1
BamHI
EcoRI
HindIII
BglII+HindIII digestion
HindIII+PvuI
Partial digestion with BamHI
SV40ori
mMTP
BglII
HindIII
SV40poly(A)signal
BamHI
BamHI
HindIII
ligase
SV40ori
BamHI
mMTP
(BglII/BamHI)
Amp^r
APcDNA
BamHI
BamHI
EcoRI
SV40poly(A) signal
HindIII
pSVMTAP

# FIG. 11 (B)

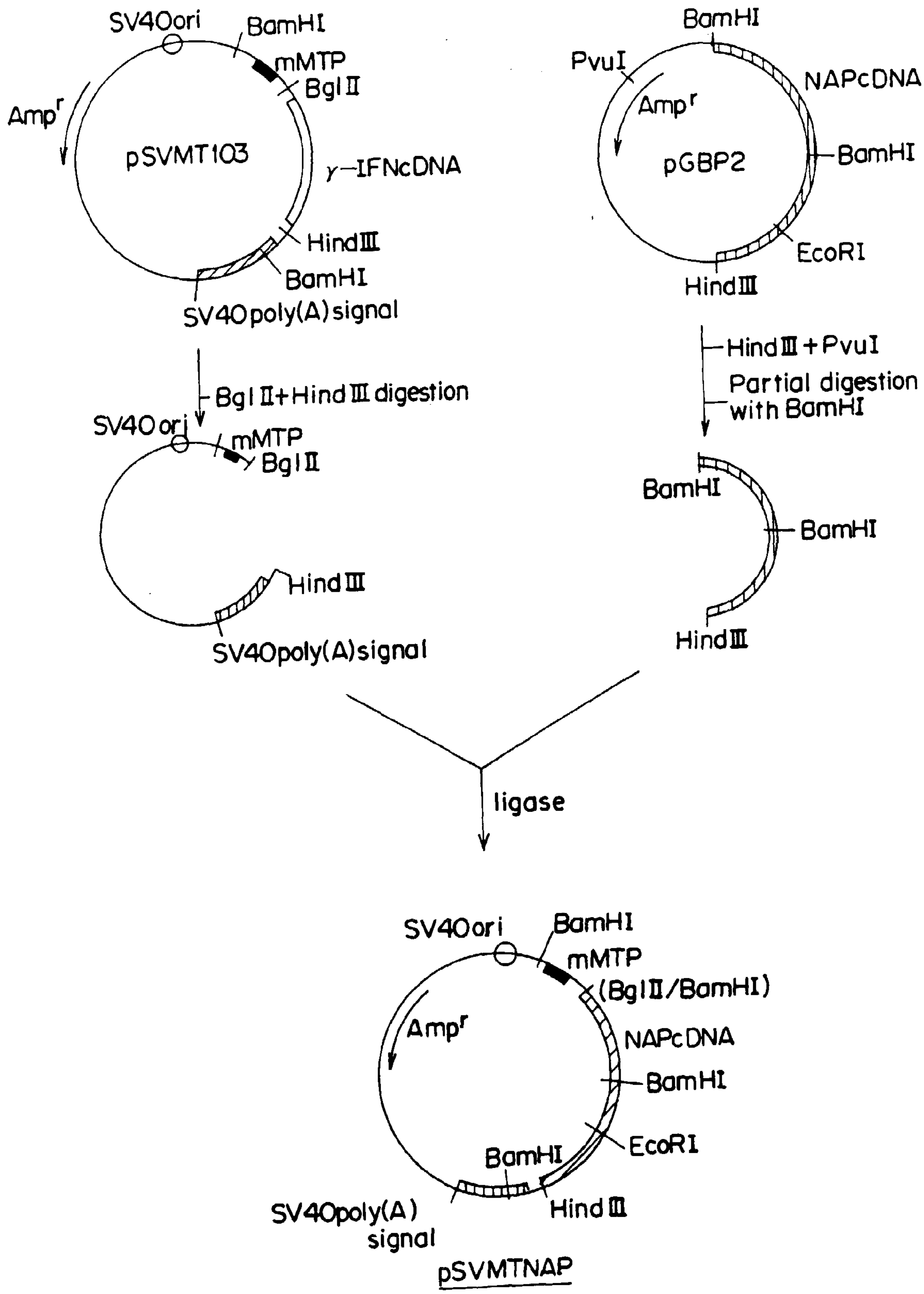
SV40ori
BamHI
mMTP
BglII
Amp^r
pSVMT103
γ-IFNcDNA
HindIII
BamHI
SV40poly(A)signal
BamHI
PvuI
NAPcDNA
Amp^r
pGBP2
BamHI
EcoRI
HindIII
BglII+HindIII digestion
HindIII+PvuI
Partial digestion with BamHI
SV40 ori
mMTP
BglII
HindIII
SV40poly(A)signal
BamHI
BamHI
HindIII
ligase
SV40ori
BamHI
mMTP
(BglII/BamHI)
Amp^r
NAPcDNA
BamHI
EcoRI
BamHI
SV40poly(A) signal
HindIII
pSVMTNAP

# FIG. 11 (C)

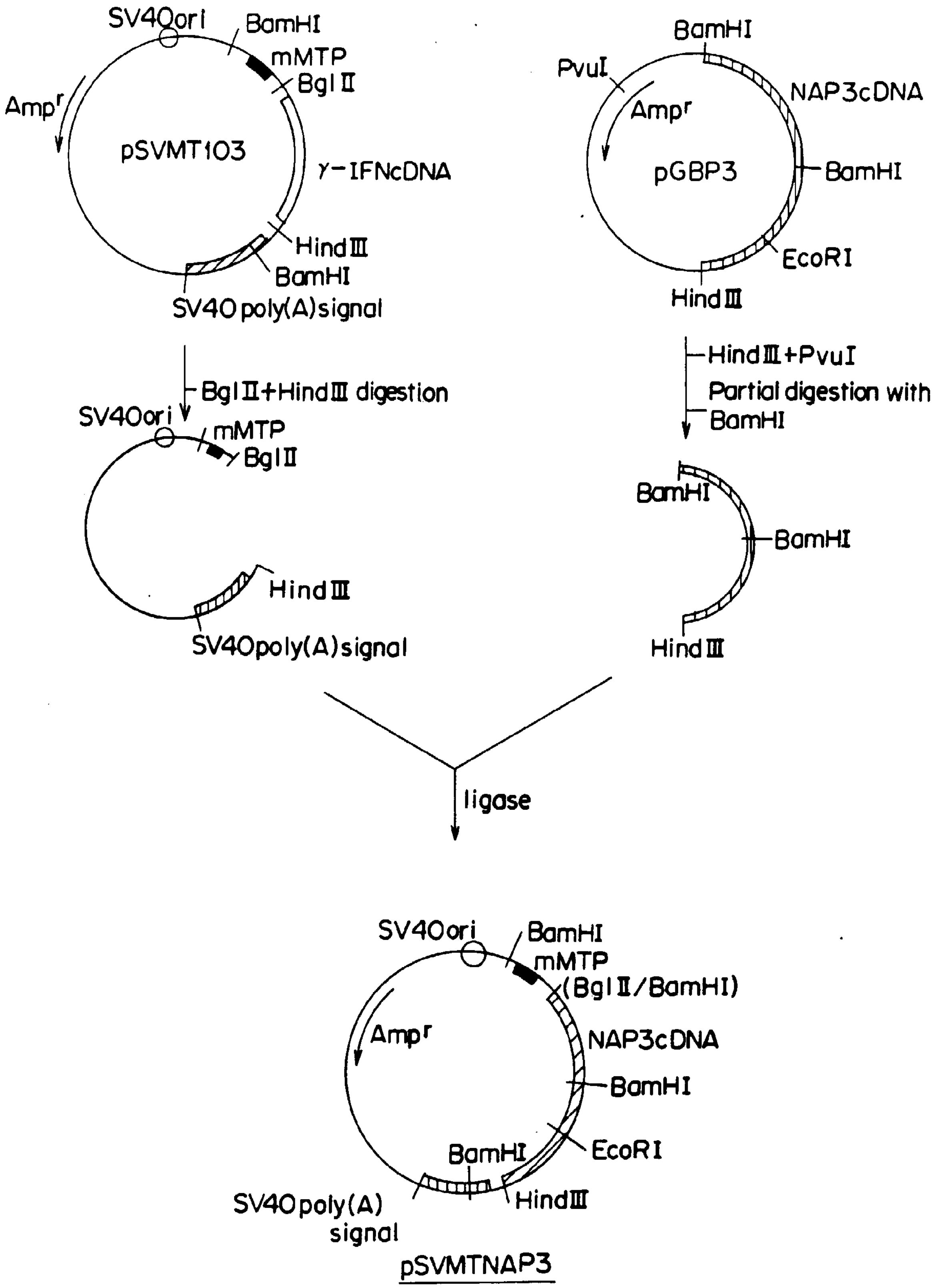
SV40ori
BamHI
mMTP
BglII
Amp^r
pSVMT103
γ-IFNcDNA
HindIII
BamHI
SV40 poly(A) signal
BamHI
PvuI
Amp^r
NAP3cDNA
pGBP3
BamHI
EcoRI
HindIII
BglII+HindIII digestion
HindIII+PvuI
Partial digestion with BamHI
SV40ori
mMTP
BglII
HindIII
SV40 poly(A) signal
BamHI
BamHI
HindIII
ligase
SV40ori
BamHI
mMTP
(BglII/BamHI)
Amp^r
NAP3cDNA
BamHI
EcoRI
BamHI
SV40 poly(A) signal
HindIII
pSVMTNAP3

FIG. 12 (A)

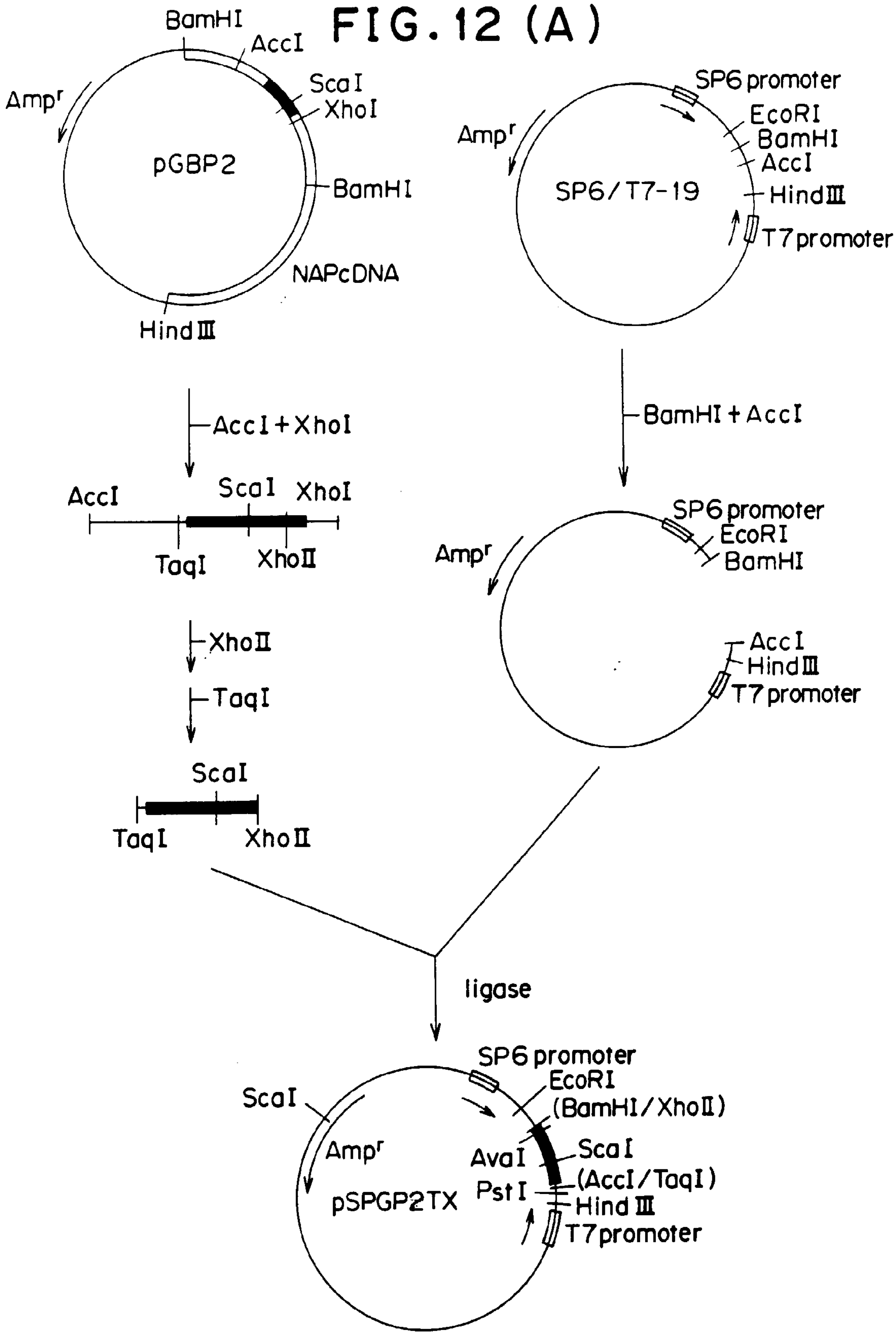
BamHI
AccI
ScaI
XhoI
Ampr
pGBP2
BamHI
NAPcDNA
HindIII
SP6 promoter
EcoRI
BamHI
AccI
Ampr
SP6/T7-19
HindIII
T7 promoter
AccI+XhoI
BamHI+AccI
AccI
ScaI
XhoI
TaqI
XhoII
SP6 promoter
EcoRI
BamHI
Ampr
XhoII
TaqI
AccI
HindIII
T7 promoter
ScaI
TaqI
XhoII
ligase
SP6 promoter
EcoRI
(BamHI/XhoII)
ScaI
Ampr
AvaI
ScaI
(AccI/TaqI)
PstI
HindIII
pSPGP2TX
T7 promoter

# FIG. 12 (B)

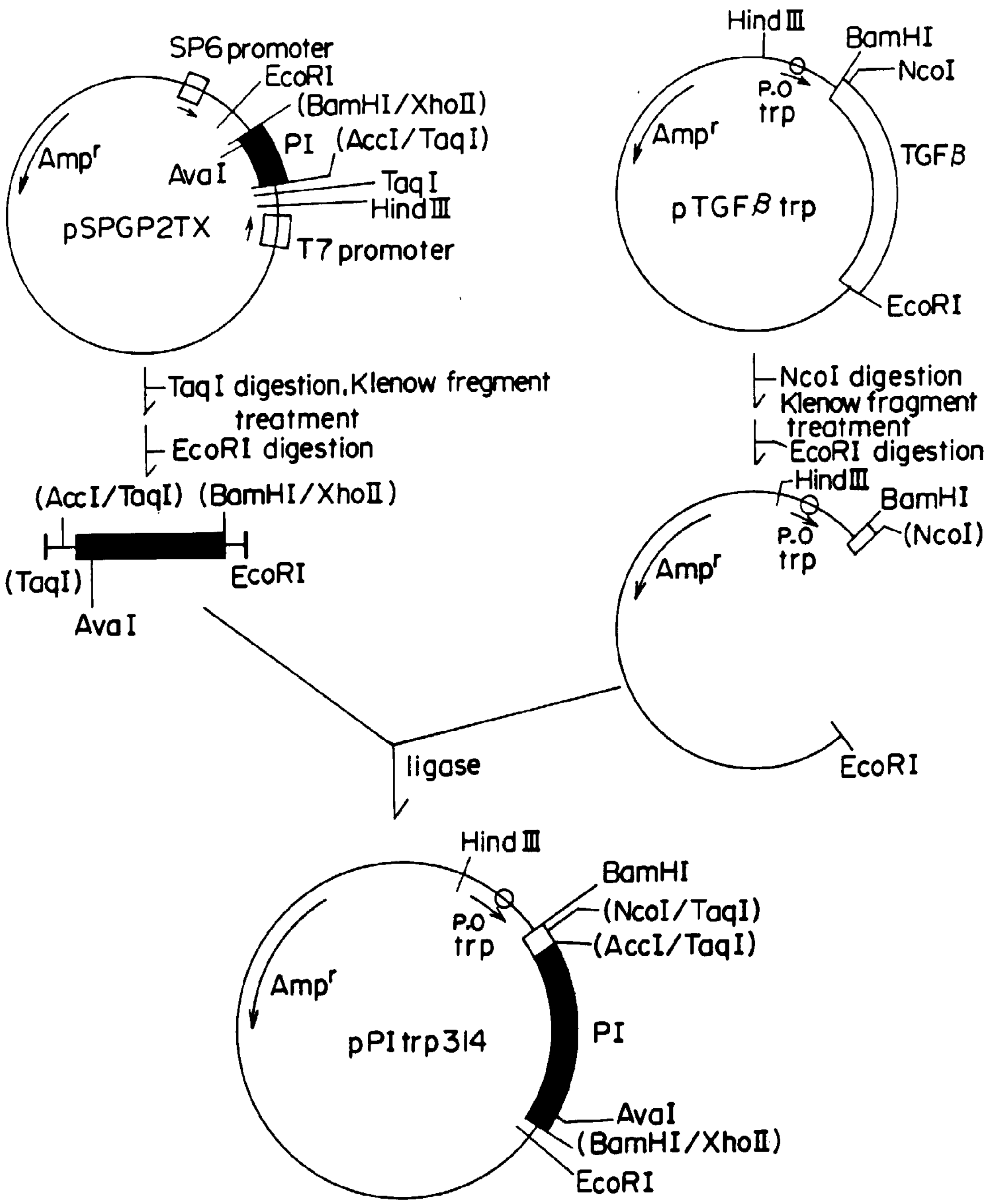
SP6 promoter
EcoRI
(BamHI/XhoII)
PI
(AccI/TaqI)
Amp^r
AvaI
TaqI
HindIII
pSPGP2TX
T7 promoter
HindIII
BamHI
NcoI
P.O
trp
Amp^r
TGFβ
pTGFβ trp
EcoRI
TaqI digestion, Klenow fregment treatment
EcoRI digestion
NcoI digestion
Klenow fragment treatment
EcoRI digestion
(AccI/TaqI) (BamHI/XhoII)
(TaqI)
EcoRI
AvaI
HindIII
BamHI
(NcoI)
P.O
trp
Amp^r
EcoRI
ligase
HindIII
BamHI
(NcoI/TaqI)
(AccI/TaqI)
P.O
trp
Amp^r
pPI trp314
PI
AvaI
(BamHI/XhoII)
EcoRI

FIG. 12 (C)

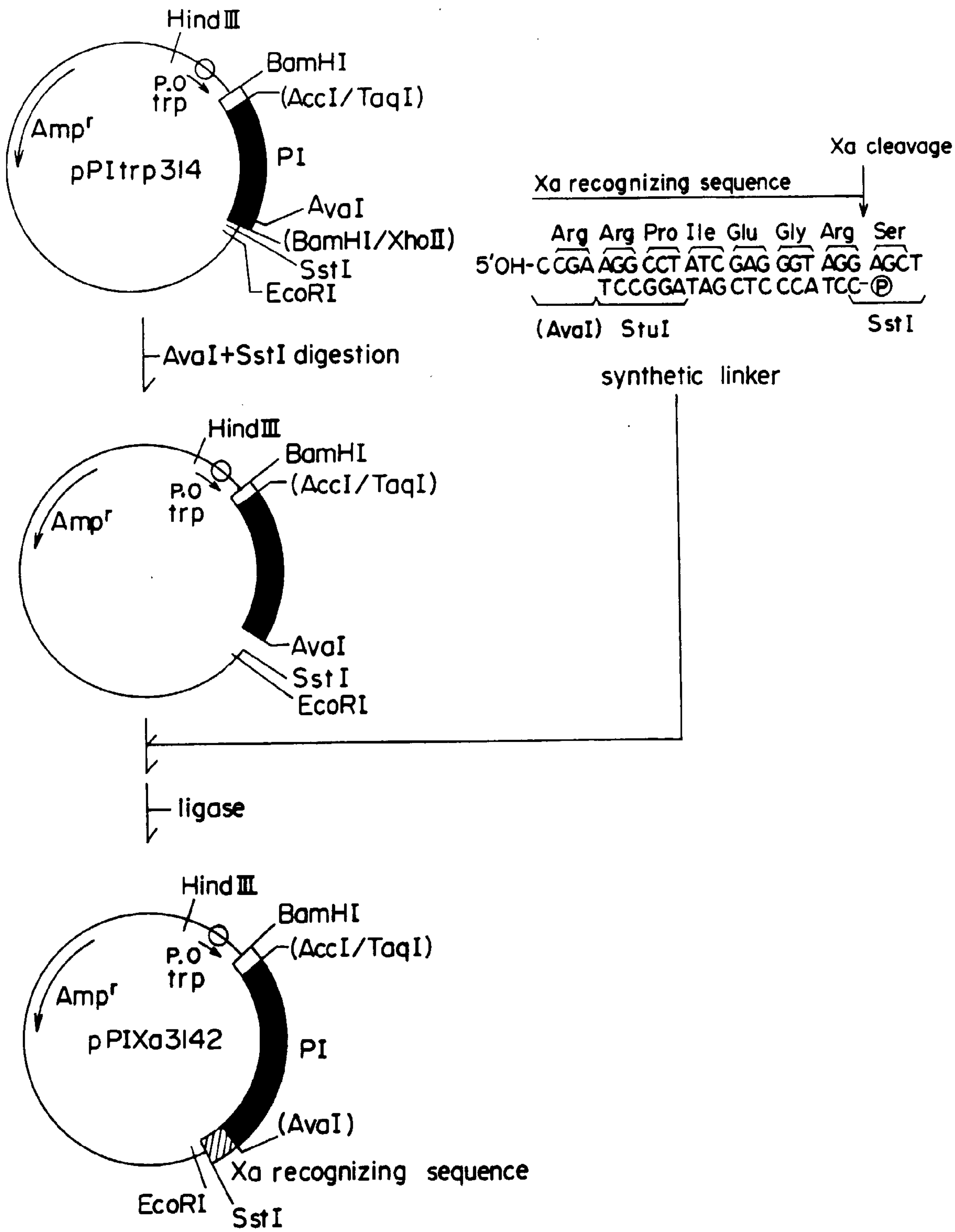
HindIII
BamHI
P.O trp
(AccI/TaqI)
Amp^r
pPItrp314
PI
AvaI
(BamHI/XhoII)
SstI
EcoRI
AvaI+SstI digestion
Xa cleavage
Xa recognizing sequence
Arg Arg Pro Ile Glu Gly Arg Ser
5'OH-CCGA AGG CCT ATC GAG GGT AGG AGCT
TCCGGATAG CTC CCA TCC-Ⓟ
(AvaI) StuI
SstI
synthetic linker
HindIII
BamHI
P.O trp
(AccI/TaqI)
Amp^r
AvaI
SstI
EcoRI
ligase
HindIII
BamHI
P.O trp
(AccI/TaqI)
Amp^r
pPIXa3142
PI
(AvaI)
Xa recognizing sequence
EcoRI
SstI

FIG. 12 (D)

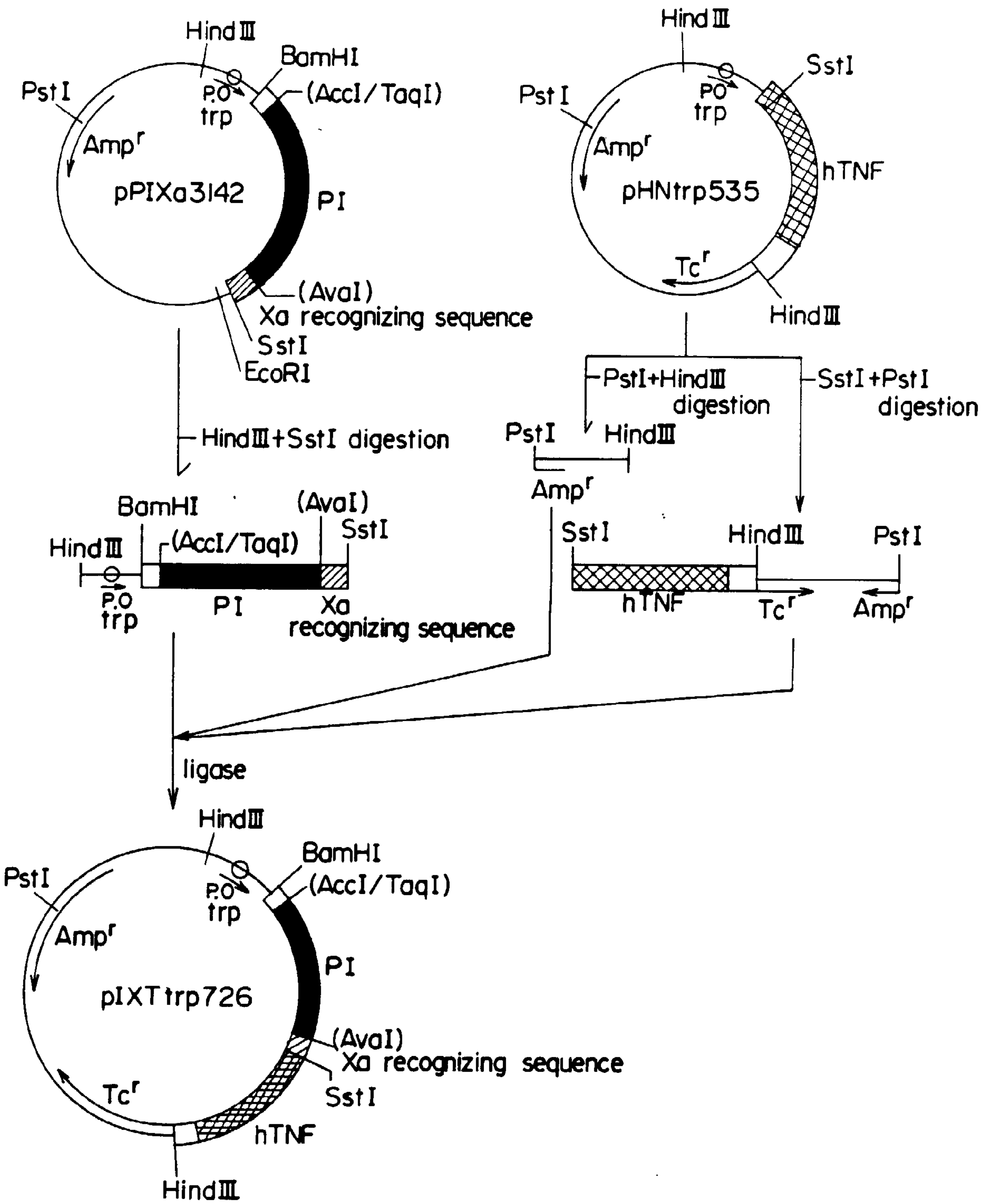
HindIII
BamHI
PstI
P.O
trp
(AccI/TaqI)
Ampr
pPIXa3142
PI
(AvaI)
Xa recognizing sequence
SstI
EcoRI
HindIII+SstI digestion
HindIII
SstI
PO
trp
PstI
Ampr
pHNtrp535
hTNF
Tcr
HindIII
PstI+HindIII digestion
SstI+PstI digestion
Xa recognizing sequence
ligase
pIXTtrp726

# FIG. 12 (E)

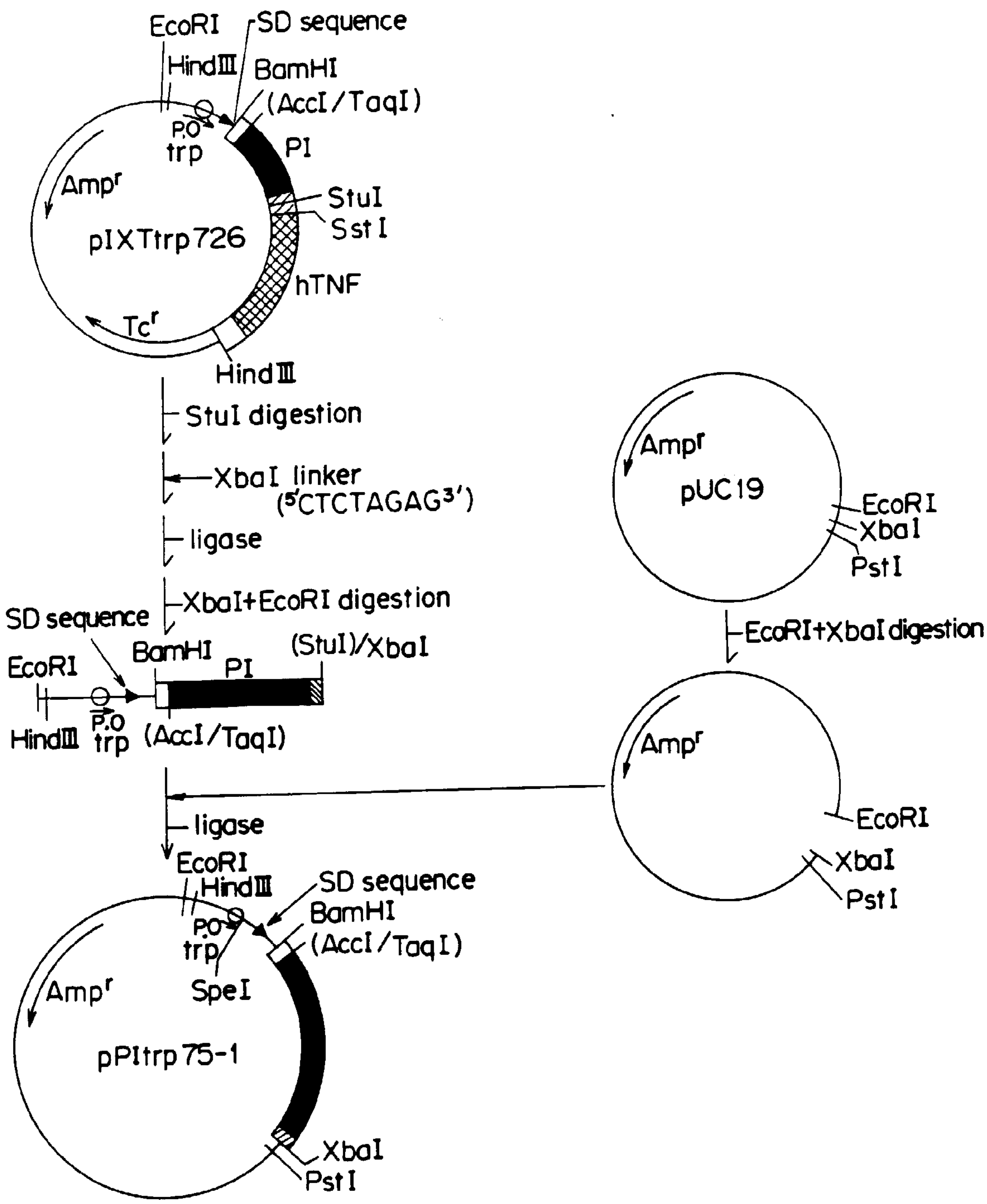
EcoRI
SD sequence
HindIII
BamHI
(AccI/TaqI)
P.O
trp
PI
Amp^r
StuI
SstI
pIXTtrp726
hTNF
Tc^r
HindIII
StuI digestion
XbaI linker
(5'CTCTAGAG3')
ligase
XbaI+EcoRI digestion
SD sequence
EcoRI
BamHI
PI
(StuI)/XbaI
HindIII
P.O
trp
(AccI/TaqI)
Amp^r
pUC19
EcoRI
XbaI
PstI
EcoRI+XbaI digestion
Amp^r
EcoRI
XbaI
PstI
ligase
EcoRI
HindIII
SD sequence
BamHI
P.O
(AccI/TaqI)
trp
Amp^r
SpeI
pPItrp75-1
XbaI
PstI

# FIG.12 (F)

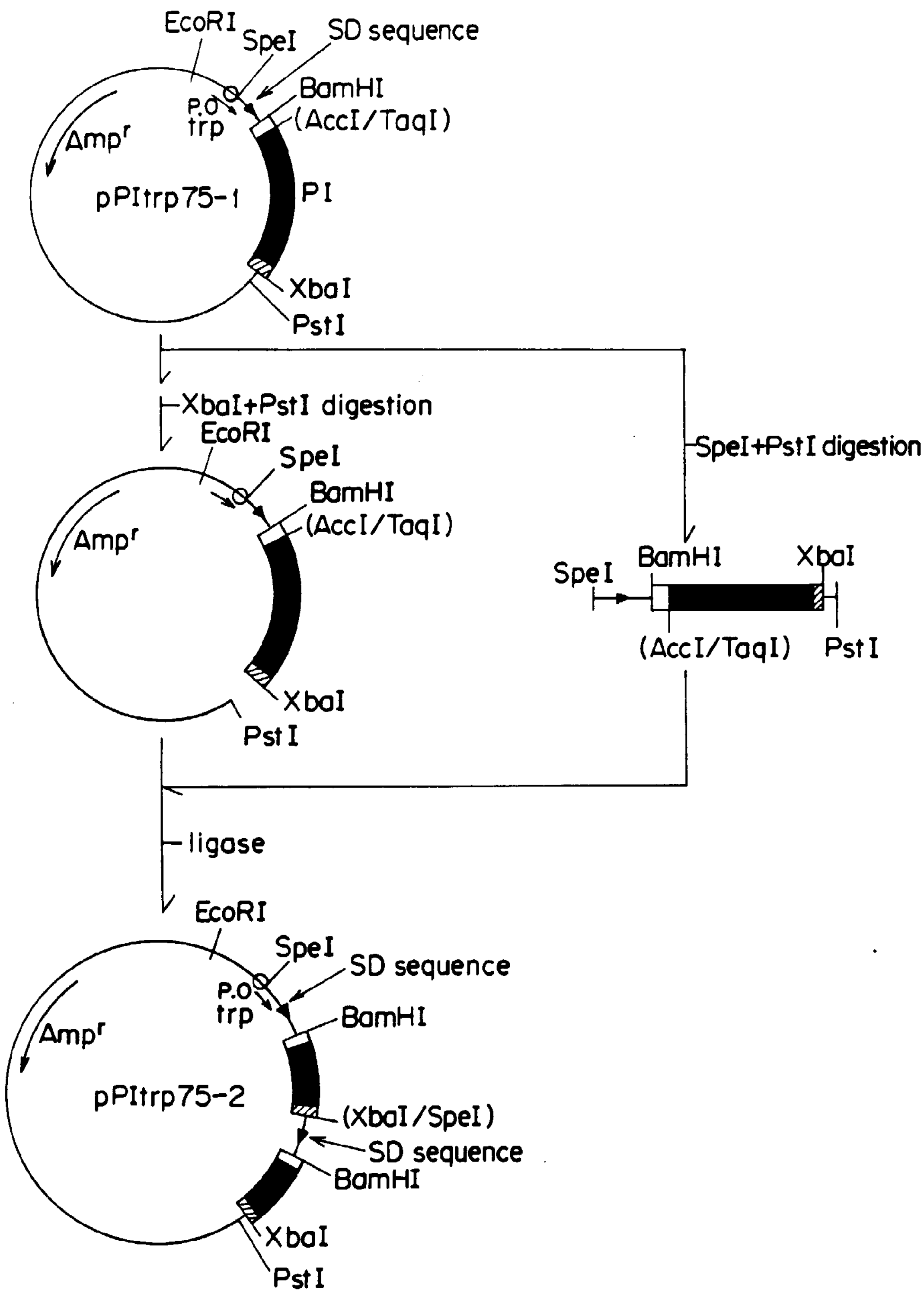
EcoRI
SpeI
SD sequence
BamHI
(AccI/TaqI)
P.O
trp
Amp^r
pPItrp75-1
PI
XbaI
PstI
XbaI+PstI digestion
EcoRI
SpeI
BamHI
(AccI/TaqI)
Amp^r
XbaI
PstI
SpeI+PstI digestion
SpeI
BamHI
XbaI
(AccI/TaqI)
PstI
ligase
EcoRI
SpeI
SD sequence
P.O
trp
BamHI
Amp^r
pPItrp75-2
(XbaI/SpeI)
SD sequence
BamHI
XbaI
PstI

# FIG. 12 (G)

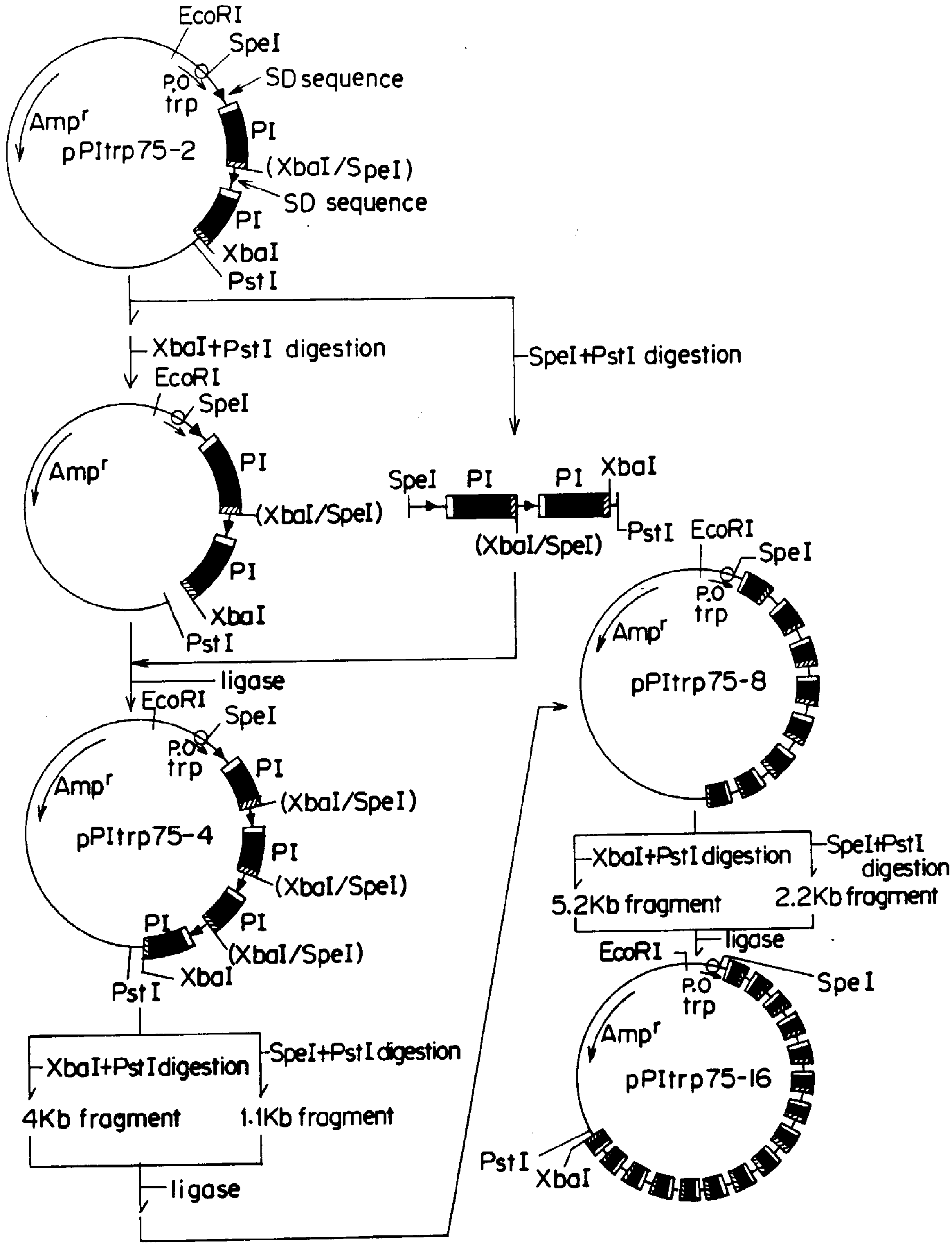
EcoRI
SpeI
SD sequence
P.O
trp
Amp^r
pPItrp75-2
PI
(XbaI/SpeI)
SD sequence
PI
XbaI
PstI
XbaI+PstI digestion
SpeI+PstI digestion
EcoRI
SpeI
Amp^r
PI
(XbaI/SpeI)
PI
XbaI
PstI
SpeI
PI
PI
XbaI
(XbaI/SpeI)
PstI
ligase
EcoRI
SpeI
P.O
trp
Amp^r
pPItrp75-4
PI
(XbaI/SpeI)
PI
(XbaI/SpeI)
PI
PI
(XbaI/SpeI)
XbaI
PstI
XbaI+PstIdigestion
SpeI+PstI digestion
4Kb fragment
1.1Kb fragment
ligase
EcoRI
SpeI
P.O
trp
Amp^r
pPItrp75-8
XbaI+PstI digestion
SpeI+PstI digestion
5.2Kb fragment
2.2Kb fragment
ligase
EcoRI
SpeI
P.O
trp
Amp^r
pPItrp75-16
PstI
XbaI

# FIG. 12 (H)

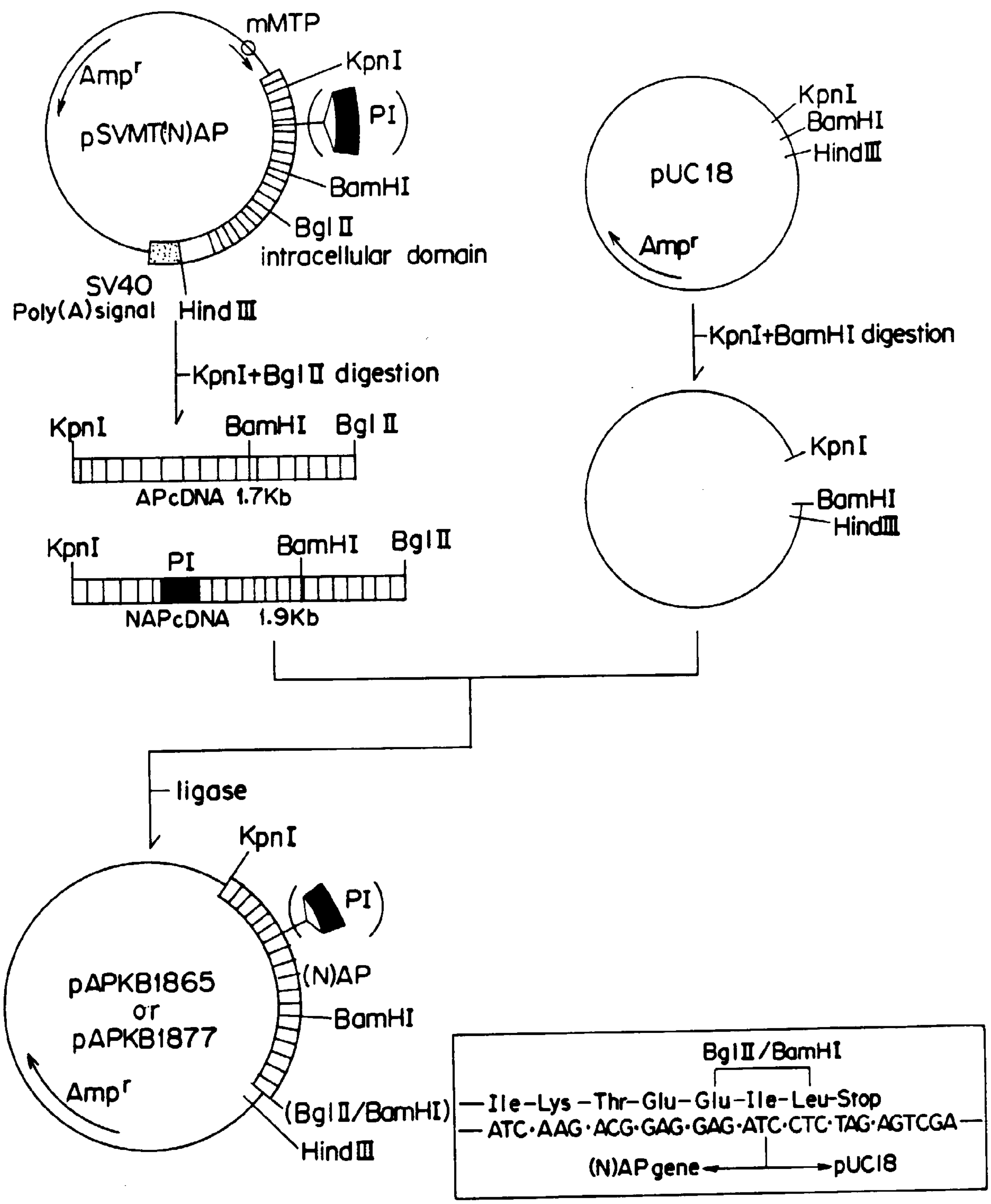
mMTP
KpnI
Amp^r
pSVMT(N)AP
PI
BamHI
Bgl II
intracellular domain
SV40
Poly(A)signal
Hind III
KpnI+Bgl II digestion
KpnI
BamHI
Bgl II
APcDNA 1.7Kb
KpnI
PI
BamHI
Bgl II
NAPcDNA 1.9Kb
KpnI
BamHI
Hind III
pUC 18
Amp^r
KpnI+BamHI digestion
KpnI
BamHI
Hind III
ligase
KpnI
PI
(N)AP
BamHI
pAPKB1865
or
pAPKB1877
Amp^r
(Bgl II/BamHI)
Hind III
Bgl II/BamHI
—Ile-Lys-Thr-Glu-Glu-Ile-Leu-Stop
—ATC·AAG·ACG·GAG·GAG·ATC·CTC·TAG·AGTCGA—
(N)AP gene
pUC18

FIG.12 (I)

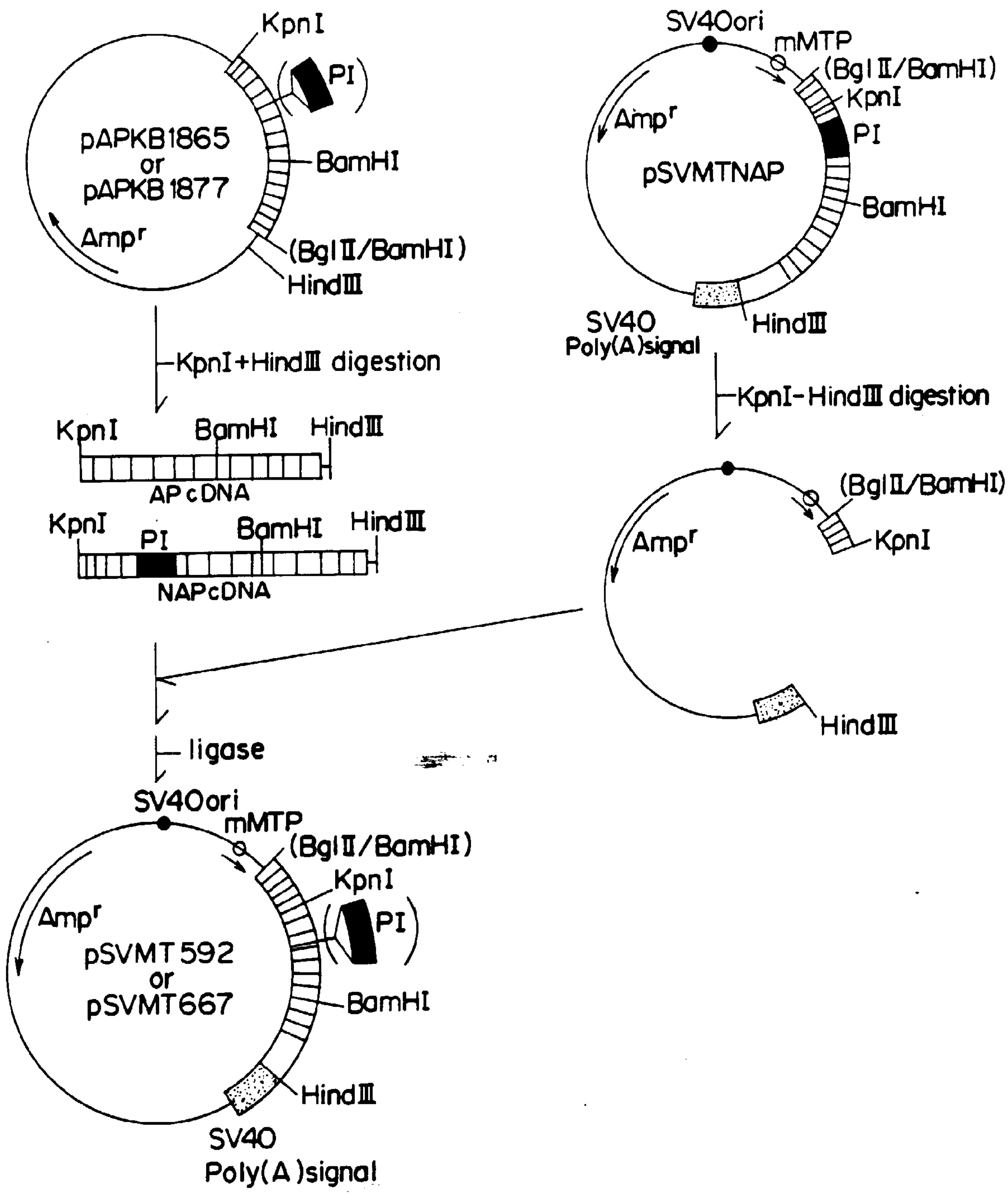
KpnI
PI
pAPKB1865
or
pAPKB1877
BamHI
Amp^r
(BglII/BamHI)
HindIII
KpnI+HindIII digestion
KpnI
BamHI
HindIII
AP cDNA
KpnI
PI
BamHI
HindIII
NAPcDNA
SV40ori
mMTP
(BglII/BamHI)
KpnI
PI
Amp^r
pSVMTNAP
BamHI
SV40
Poly(A)signal
HindIII
KpnI-HindIII digestion
(BglII/BamHI)
Amp^r
KpnI
HindIII
ligase
SV40ori
mMTP
(BglII/BamHI)
KpnI
PI
Amp^r
pSVMT592
or
pSVMT667
BamHI
HindIII
SV40
Poly(A)signal

FIG. 12 (J)

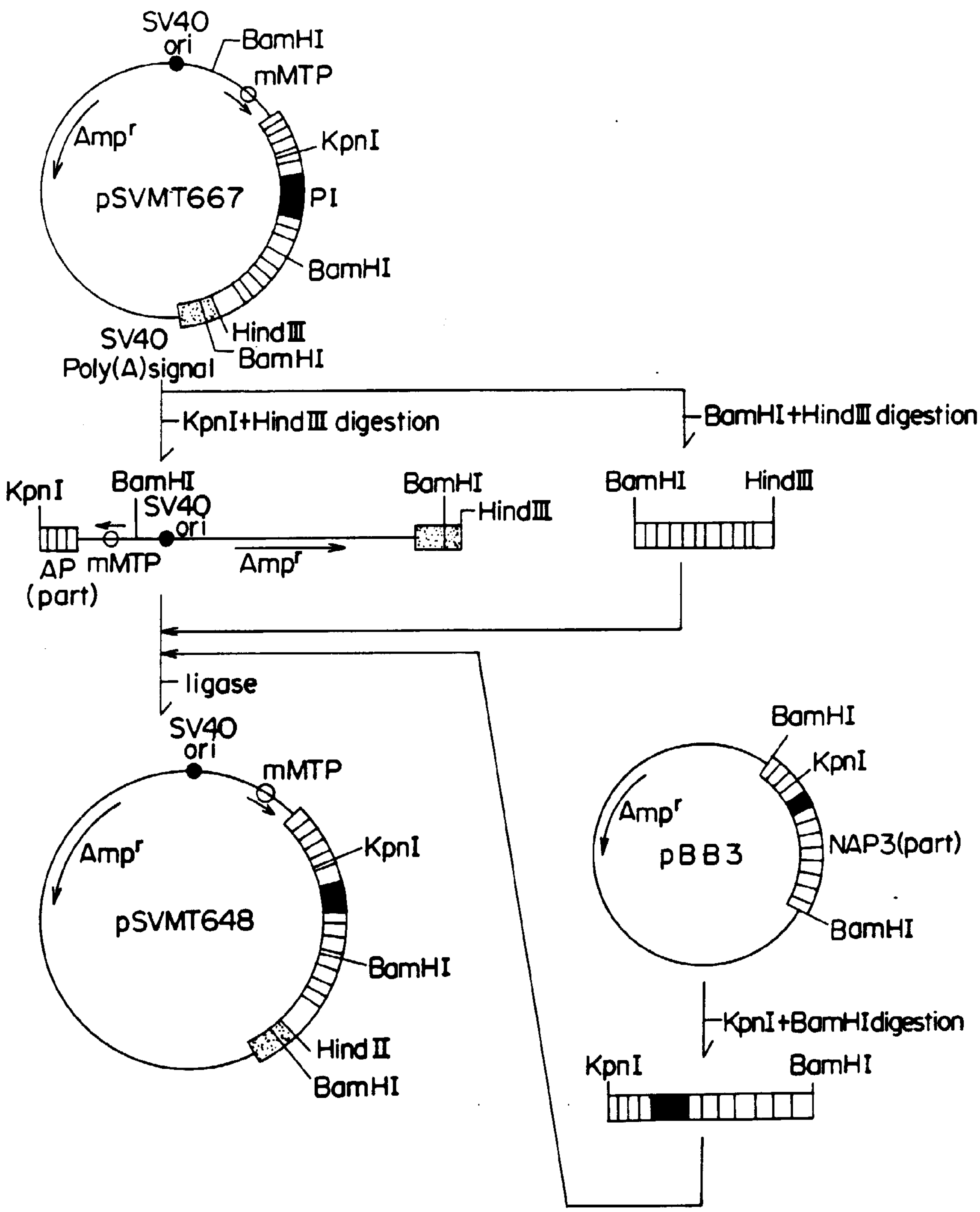
SV40
ori
BamHI
mMTP
Amp^r
KpnI
pSVMT667
PI
BamHI
HindIII
SV40
Poly(A)signal
BamHI
KpnI+HindIII digestion
BamHI+HindIII digestion
KpnI
BamHI
SV40
ori
AP
(part)
mMTP
Amp^r
BamHI
HindIII
BamHI
HindIII
ligase
SV40
ori
mMTP
Amp^r
KpnI
pSVMT648
BamHI
HindII
BamHI
BamHI
KpnI
Amp^r
pBB3
NAP3(part)
BamHI
KpnI+BamHI digestion
KpnI
BamHI

FIG. 12 (K)

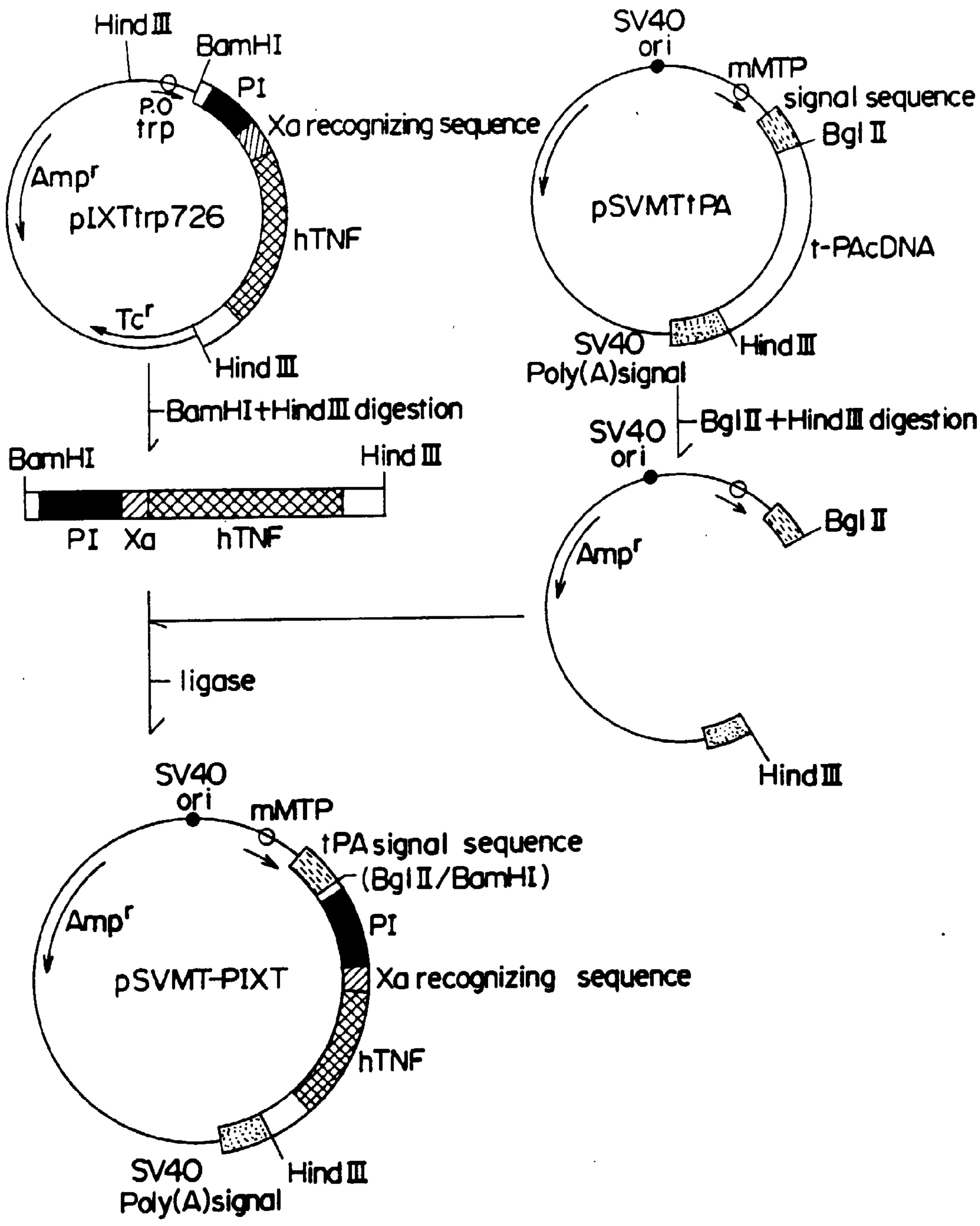

Hind III
BamHI
PI
P.O
trp
Xa recognizing sequence
Ampr
pIXTtrp726
hTNF
Tcr
Hind III
BamHI+Hind III digestion
BamHI
Hind III
PI
Xa
hTNF
SV40
ori
mMTP
signal sequence
Bgl II
pSVMTtPA
t-PAcDNA
SV40
Poly(A)signal
Hind III
Bgl II+Hind III digestion
SV40
ori
Bgl II
Ampr
Hind III
ligase
SV40
ori
mMTP
tPA signal sequence
(Bgl II/BamHI)
PI
Ampr
pSVMT-PIXT
Xa recognizing sequence
hTNF
SV40
Poly(A)signal
Hind III

FIG.12 (L)

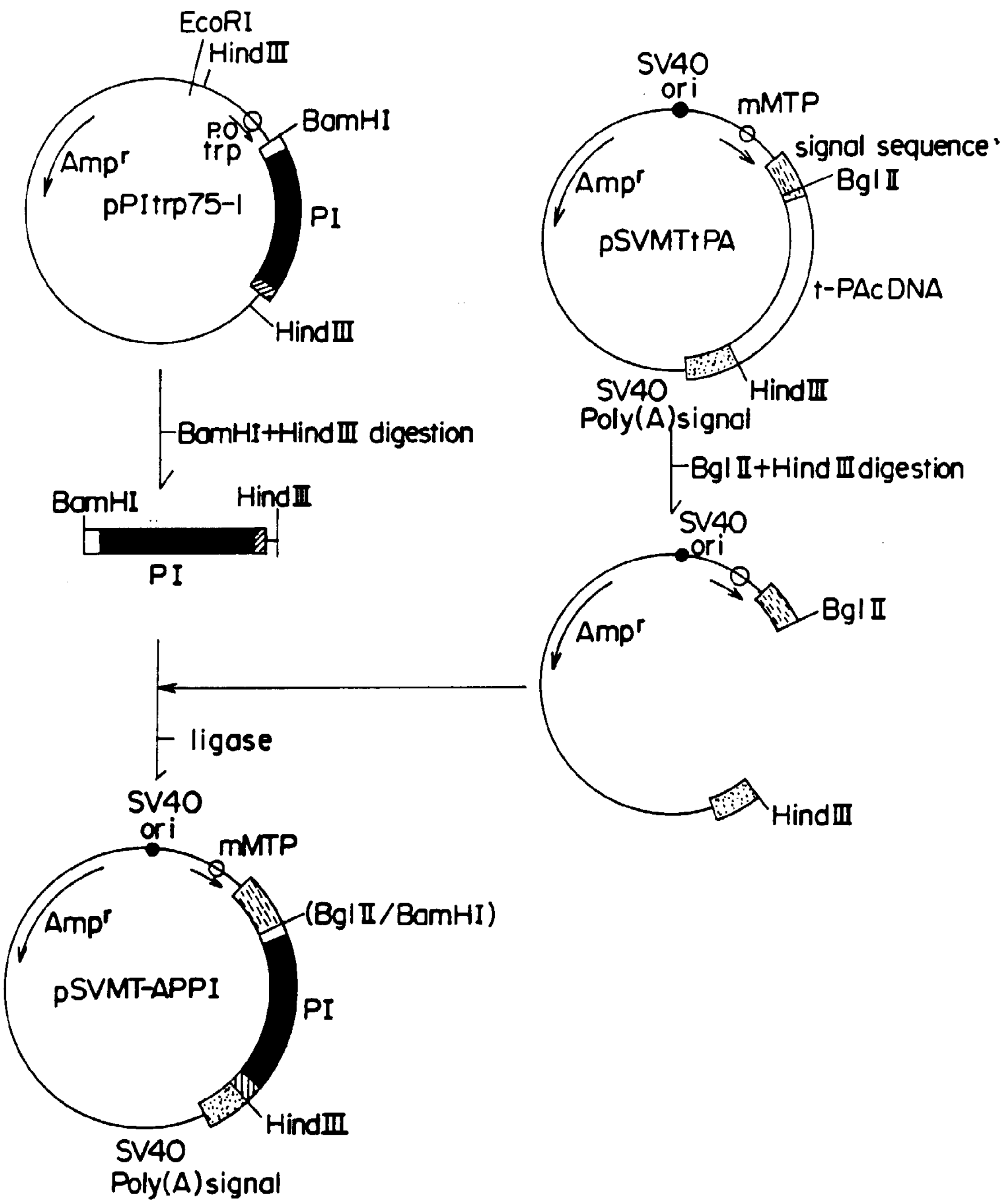
EcoRI
HindIII
P.O
trp
BamHI
Amp^r
pPItrp75-I
PI
HindIII
BamHI+HindIII digestion
BamHI
HindIII
PI
SV40
ori
mMTP
signal sequence
BglII
Amp^r
pSVMTtPA
t-PAcDNA
SV40
Poly(A)signal
HindIII
BglII+HindIIIdigestion
SV40
ori
BglII
Amp^r
HindIII
ligase
SV40
ori
mMTP
(BglII/BamHI)
Amp^r
pSVMT-APPI
PI
HindIII
SV40
Poly(A)signal

# FIG. 12 (M)

```
*   1                                                                               60*
ATG.GAT.GCA.ATG.AAG.AGA.GGG.CTC.TGC.TGT.GTG.CTG.CTG.CTG.TGT.GGA.GCA.GTC.TTC.GTT
Met-Asp-Ala-Met-Lys-Arg-Gly-Leu-Cys-Cys-Val-Leu-Leu-Leu-Cys-Gly-Ala-Val-Phe-Val

                                         t-PA signal                              
*  61                                                                            120*
TCG.CCC.AGC.CAG.GAA.ATC.CAT.GCC.CGA.TTC.AGA.AGA.GGA.GCC.AGA.TCC.ATG.CGA.GAG.GTG
Ser-Pro-Ser-Gln-Glu-Ile-His-Ala-Arg-Phe-Arg-Arg-Gly-Ala-Arg-Ser-Met-Arg-Glu-Val

*  121                                                                           180*
TGC.TCT.GAA.CAA.GCC.GAG.ACG.GGG.CCG.TGC.CGA.GCA.ATG.ATC.TCC.CGC.TGG.TAC.TTT.GAT
Cys-Ser-Glu-Gln-Ala-Glu-Thr-Gly-Pro-Cys-Arg-Ala-Met-Ile-Ser-Arg-Trp-Tyr-Phe-Asp

*  181                                                                           240*
GTG.ACT.GAA.GGG.AAG.TGT.GCC.CCA.TTC.TTT.TAC.GGC.GGA.TGT.GGC.GGC.AAC.CGG.AAC.AAC
Val-Thr-Glu-Gly-Lys-Cys-Ala-Pro-Phe-Phe-Tyr-Gly-Gly-Cys-Gly-Gly-Asn-Arg-Asn-Asn

*  241                                                                           300*
TTT.GAC.ACA.GAA.GAG.TAC.TGC.ATG.GCC.GTG.TGT.GGC.AGC.GCC.ATG.TCC.CAA.AGT.TTA.CTC
Phe-Asp-Thr-Glu-Glu-Tyr-Cys-Met-Ala-Val-Cys-Gly-Ser-Ala-Met-Ser-Gln-Ser-Leu-Leu

                 PI
*  301
AAG.ACT.ACC.CAG.GAA.CCT.CTT.GCC.CGA.AGG.CTC.TAG.
Lys-Thr-Thr-Gln-Glu-Pro-Leu-Ala-Arg-Arg-Leu-***-
```

FIG.12 (N)

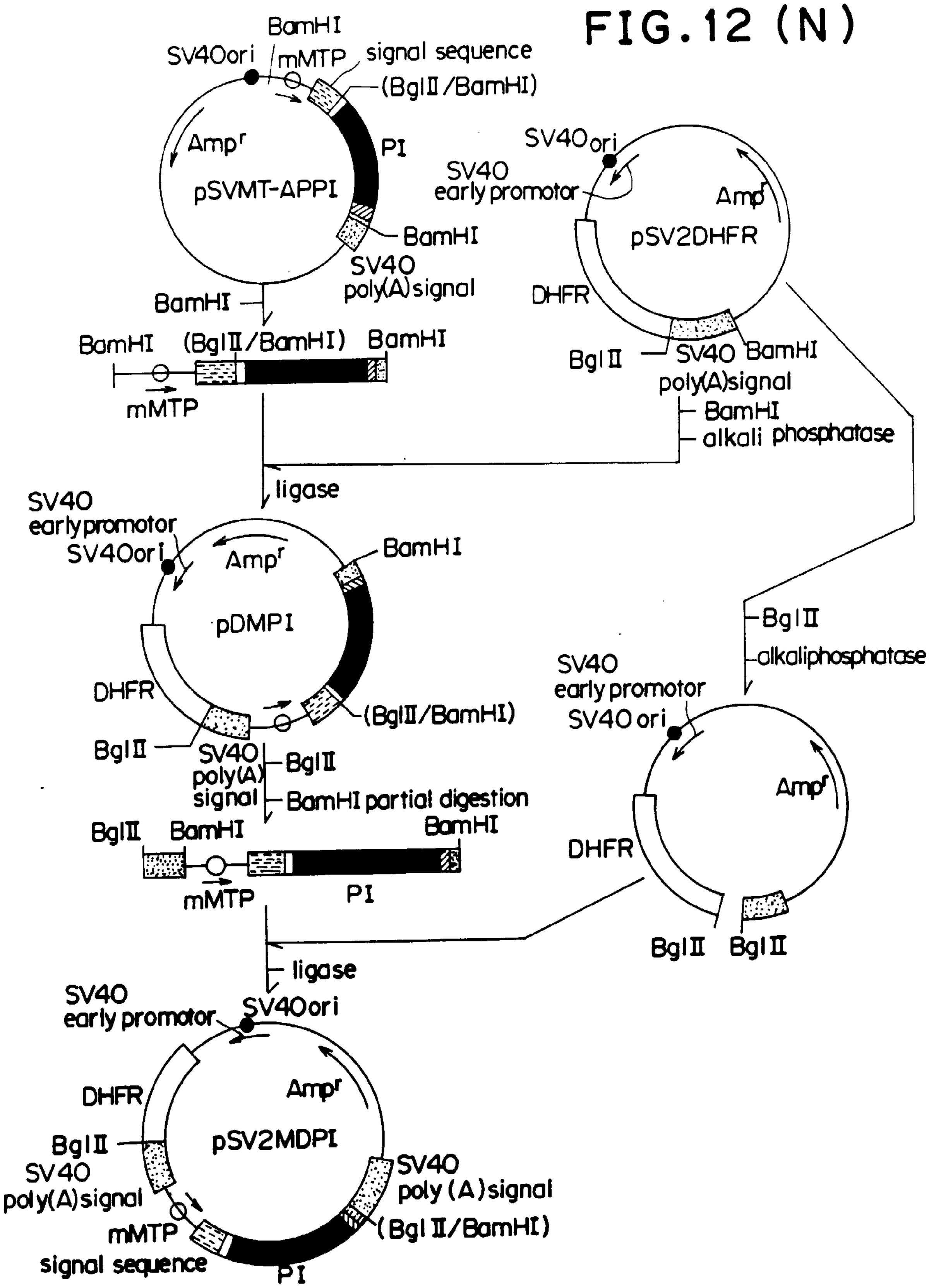
BamHI
SV40ori
mMTP
signal sequence
(BglII/BamHI)
Ampr
PI
pSVMT-APPI
BamHI
SV40
poly(A)signal
BamHI
SV40ori
SV40
early promotor
Ampr
pSV2DHFR
DHFR
BglII
SV40
poly(A)signal
BamHI
BamHI
alkali phosphatase
BamHI
(BglII/BamHI)
BamHI
mMTP
ligase
SV40
earlypromotor
SV40ori
Ampr
BamHI
pDMPI
DHFR
(BglII/BamHI)
BglII
SV40
poly(A)
signal
BglII
BamHI partial digestion
BglII
BamHI
BamHI
mMTP
PI
BglII
alkaliphosphatase
SV40
early promotor
SV40 ori
Ampr
DHFR
BglII
BglII
ligase
SV40
early promotor
SV40ori
DHFR
Ampr
pSV2MDPI
BglII
SV40
poly(A)signal
SV40
poly (A)signal
(BglII/BamHI)
mMTP
signal sequence
PI

FIG.13

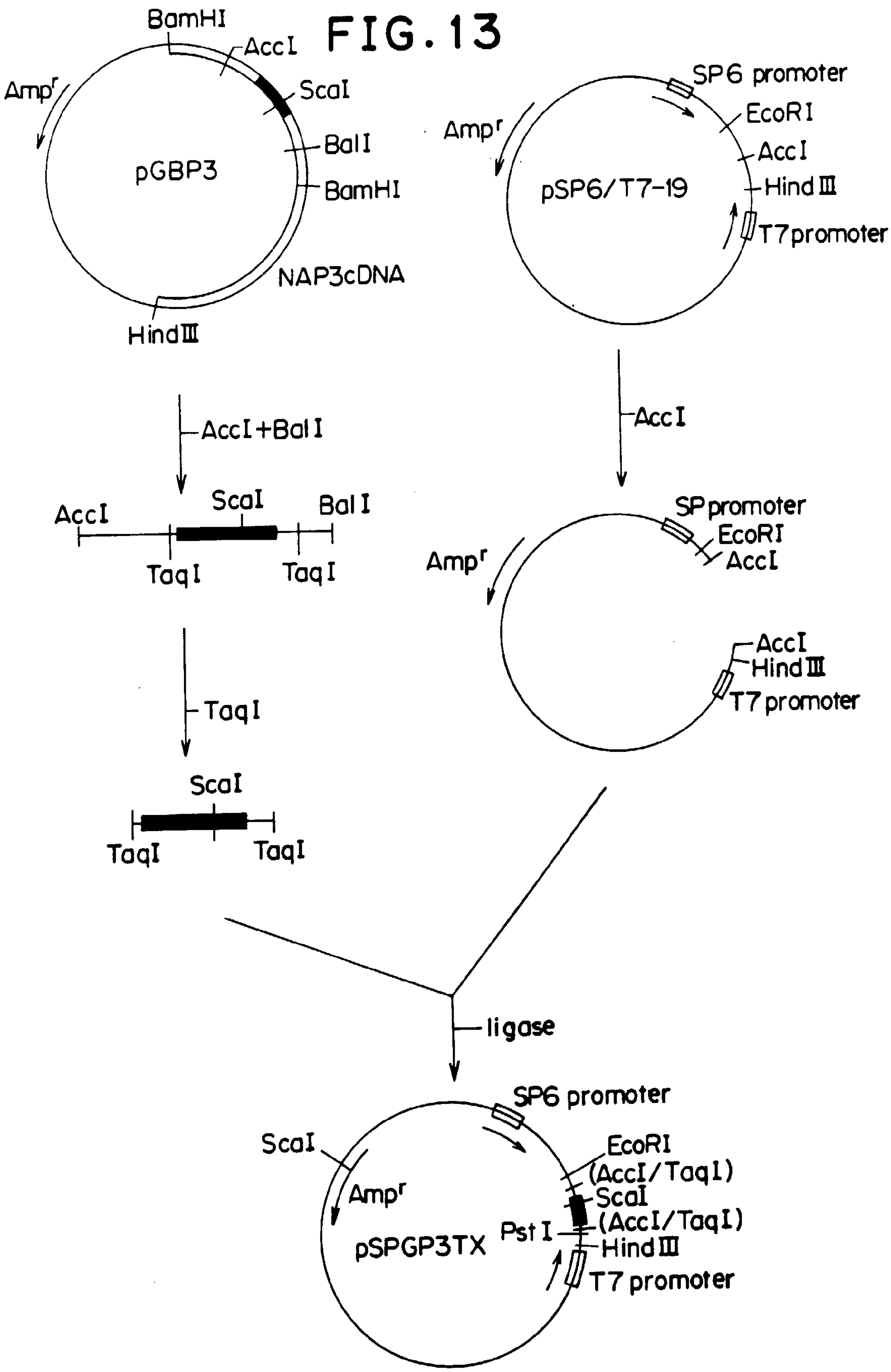
BamHI
AccI
ScaI
BalI
BamHI
Amp^r
pGBP3
NAP3cDNA
HindIII
SP6 promoter
EcoRI
AccI
HindIII
T7promoter
Amp^r
pSP6/T7-19
AccI+BalI
AccI
ScaI
BalI
TaqI
TaqI
AccI
SPpromoter
EcoRI
AccI
Amp^r
AccI
HindIII
T7promoter
TaqI
ScaI
TaqI
TaqI
ligase
SP6 promoter
ScaI
Amp^r
EcoRI
(AccI/TaqI)
ScaI
(AccI/TaqI)
PstI
HindIII
T7 promoter
pSPGP3TX

FIG. 14

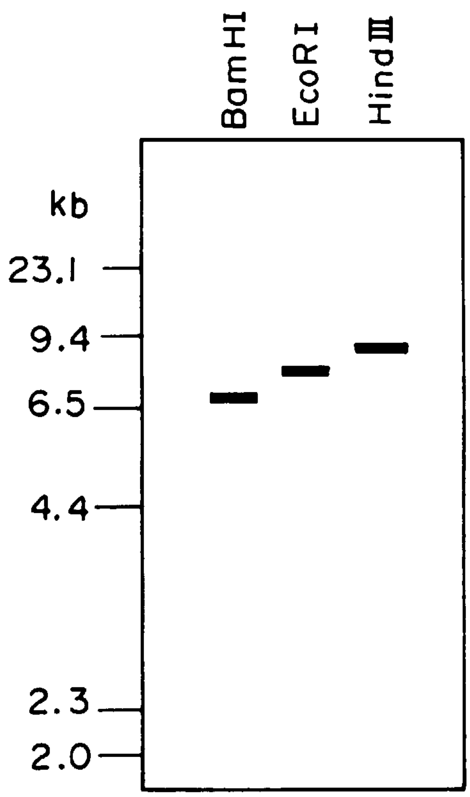

FIG. 15

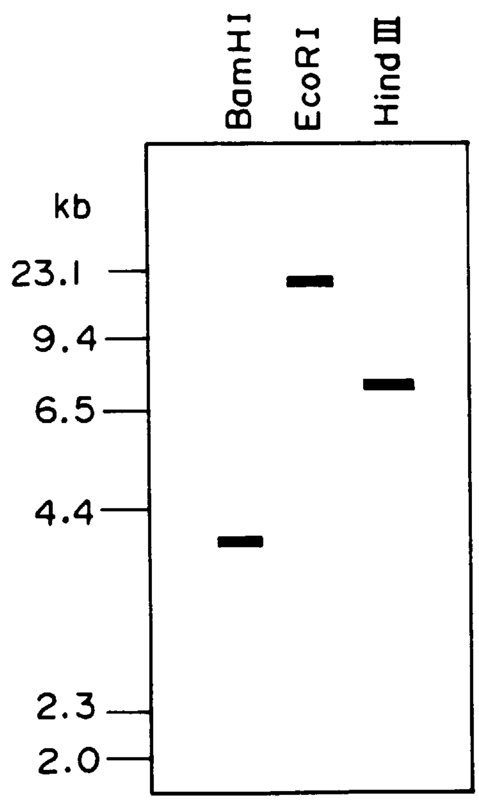

FIG.16

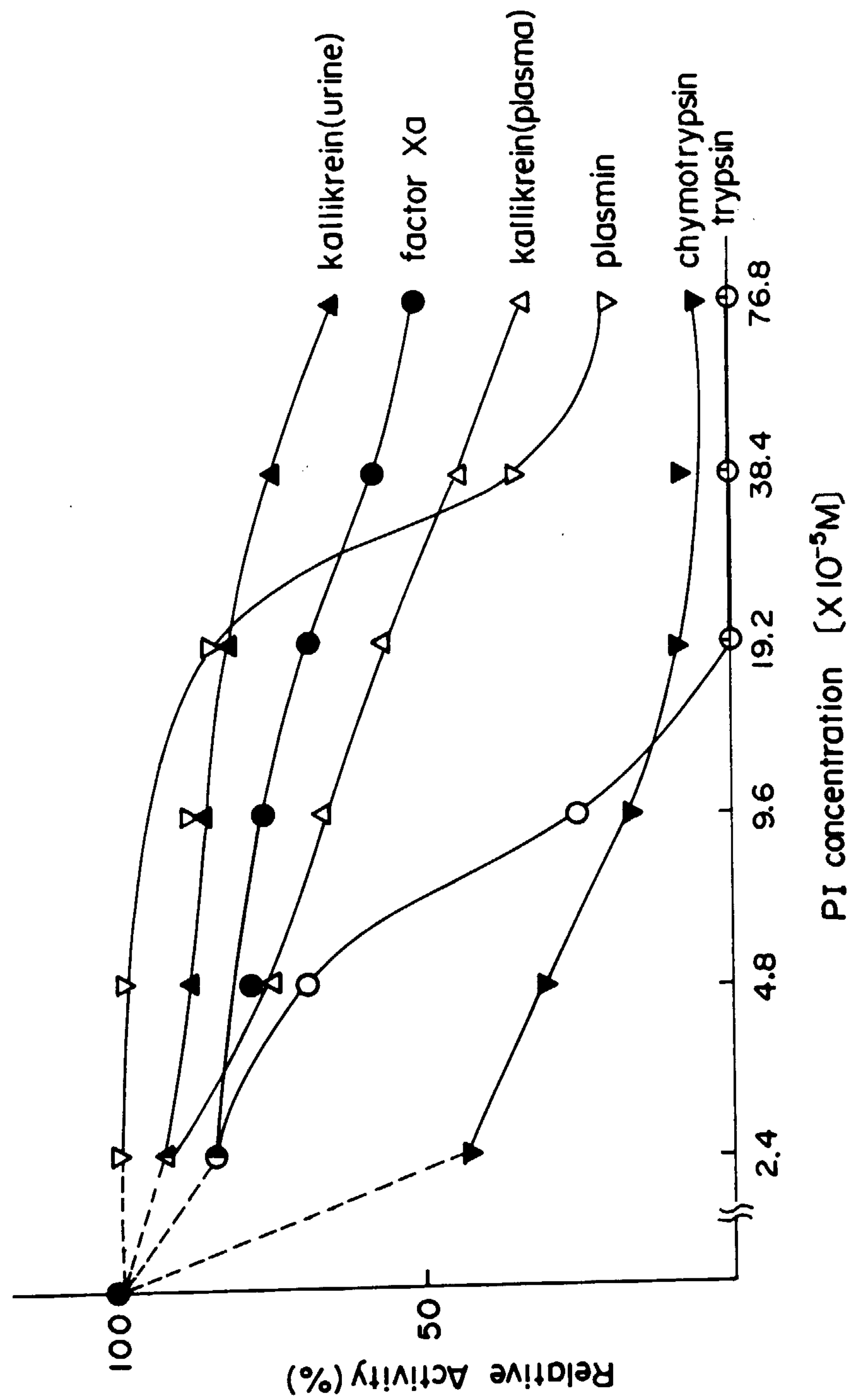
kallikrein(urine)
factor Xa
kallikrein(plasma)
plasmin
chymotrypsin
trypsin
100
50
Relative Activity (%)
2.4
4.8
9.6
19.2
38.4
76.8
PI concentration [X10$^{-5}$M]